# EUROPEAN PATENT SPECIFICATION

(11) **EP 3 672 614 B1**
(45) Date of publication and mention of the grant of the patent: **06.10.2021**
(21) Application number: 19731027.9
(22) Date of filing: 22.05.2019
(51) Int. Cl.: A61K 38/00, A61K 39/00, A61K 45/06

(54) **ENGINEERED EXTRACELLULAR VESICLES AND USES THEREOF**
MANIPULIERTE EXTRAZELLULÄRE VESIKEL UND VERWENDUNGEN DAVON
VÉSICULES EXTRACELLULAIRES MODIFIÉES ET LEURS UTILISATIONS

(30) Priority: 17.04.2019 US 201962835430 P; 16.11.2018 WO PCT/US2018/061679
(43) Date of publication of application: 01.07.2020
(73) Proprietor: Codiak BioSciences, Inc., Cambridge, MA 02140 (US)
(72) Inventor: DOOLEY, Kevin P., Cambridge, Massachusetts 02140 (US); HARRISON, Rane A., Cambridge, Massachusetts 02140 (US); XU, Ke, Cambridge, Massachusetts 02140 (US); HOUDE, Damian J., Cambridge, Massachusetts 02140 (US); HAUPT, Sonya, Cambridge, Massachusetts 02140 (US); KULMAN, John D., Cambridge, Massachusetts 02140 (US); WILLIAMS, Douglas E., Cambridge, Massachusetts 02140 (US); MCCONNELL, Russell E., Cambridge, Massachusetts 02140 (US); YOUNISS, Madeleine, Cambridge, Massachusetts 02140 (US)
(74) Representative: Carpmaels & Ransford LLP
(86) International application number: PCT/US2019/033629
(87) International publication number: WO 2020/101740

(56) References cited:
- WO-A1-2016/138525
- WO-A1-2018/039119
- WO-A1-2018/112154
- WO-A1-2019/099942
- WO-A1-2020/041720
- US-A1- 2018 015 182
- BENCE GYÖRGY ET AL: "Therapeutic Applications of Extracellular Vesicles: Clinical Promise and Open Questions", ANNUAL REVIEW OF PHARMACOLOGY AND TOXICOLOGY., vol. 55, no. 1, 3 October 2014 (2014-10-03), pages 439-464, XP055619511, US ISSN: 0362-1642, DOI: 10.1146/annurev-pharmtox-010814-124630
- PABLO GARCÍA-MANRIQUE ET AL: "Therapeutic biomaterials based on extracellular vesicles: classification of bio-engineering and mimetic preparation routes", JOURNAL OF EXTRACELLULAR VESICLES, vol. 7, no. 1, 17 January 2018 (2018-01-17), page 1422676, XP055541251, DOI: 10.1080/20013078.2017.1422676
- JING LI ET AL: "Identification and Characterization of 293T Cell-Derived Exosomes by Profiling the Protein, mRNA and MicroRNA Components", PLOS ONE, vol. 11, no. 9, 20 September 2016 (2016-09-20), page e0163043, XP055619543, DOI: 10.1371/journal.pone.0163043
- Ryo Tamura: "1. Study on the Effector Domain of MARCKS Protein as a Cell Penetrating Peptide; 2. Development of the Photoactivatable Prodrugs of Doxazolidine", , 1 January 2017 (2017-01-01), XP055739069, Retrieved from the Internet: URL:https://scholar.colorado.edu/concern/g raduate_thesis_or_dissertations/n583xv070 [retrieved on 2020-10-12]

## Description

### TECHNICAL FIELD

The present disclosure provides extracellular vesicles, e.g., exosomes, enriched in a scaffold protein associated with the extracellular vesicle's luminal surface which can be useful as an agent for the prophylaxis or treatment of cancer and other diseases.

### BACKGROUND

Exosomes are important mediators of intercellular communication. They are also important biomarkers in the diagnosis and prognosis of many diseases, such as cancer. As drug delivery vehicles, exosomes offer many advantages over traditional drug delivery methods as a new treatment modality in many therapeutic areas.

A central feature of exosomes is their ability to contain biologically active payload within their interior space, or lumen. It is well known that exosomes contain endogenous payload including mRNA, miRNA, DNA, proteins, carbohydrates, and lipids, but the ability to direct specific loading of desired therapeutic payload is currently limited. Exosomes may be loaded by overexpressing desired therapeutic payloads in a producer cell, but this loading is often of limited efficiency due to stochastic localization of the payload to cellular exosome processing centers. Alternatively, purified exosomes may be loaded *ex vivo* by, for example, electroporation. These methods may suffer from low efficiency or be limited to small payloads, such as siRNAs. Therefore, suitable methods for generating highly efficient and well-defined loaded exosomes are needed to better enable therapeutic use and other applications of exosome-based technologies.

US 2018/0015182 A1 describes engineered exosomes for the delivery of bioactive cargo using transmembrane tetraspanins. WO 2018/112154 A1 relates to methods of measuring exosomes using intrinsic fluorescence. WO 2018/039119 A1 discloses methods of suppressing delivery of exosomes to liver and spleen. György et al. (Annu Rev Pharmacol Toxicol. 2015; 55: 439-464) discusses therapeutic applications of extracellular vesicles. Garcia-Manrique et al. (J Extracell Vesicles. 2018; 7(1): 1422676) reviews therapeutic biomaterials based on extracellular vesicles. Li et al. (PLoS One. 2016; 11(9): e0163043) describes the identification and characterization of 293T Cell-derived exosomes. WO 2016/138525 A1 relates to polypeptide assemblies and methods for the production thereof. Ryo Tamura, "A thesis submitted to the Faculty of the Graduate School of the University of Colorado in partial fulfillment of the requirement for the degree of Doctor of Philosophy Department of Chemistry and Biochemistry", 2017, is a study on the effector domain of MARCKS protein as a cell penetrating peptide and the development of the photoactivatable prodrugs of doxazolidine.

### BRIEF SUMMARY

The invention provides an isolated extracellular vesicle (EV) comprising a biologically active molecule linked to a scaffold protein, wherein the scaffold protein comprises an N-terminus domain (ND) and an effector domain (ED), wherein the ND is associated with the luminal surface of the EV and the ED is associated with the luminal surface of the EV, wherein the ND comprises the amino acid sequence GGKLSK (SEQ ID NO: 203), but does not comprise a methionine (Met) at the N-terminus, wherein the ED comprises a lysine (Lys) at its N terminus that is linked directly to the lysine at the C-terminus of SEQ ID NO: 203 in the ND; and wherein the biologically active molecule comprises one or more heterologous proteins fused to the C-terminus of the scaffold protein. The invention also provides a pharmaceutical composition comprising an EV of the invention and a pharmaceutically acceptable carrier. The invention also provides an EV of the invention for use in preventing or treating a disease in a subject in need thereof. Further embodiments of the invention are defined in the appended claims.

An aspect of the present disclosure relates to novel methods of loading Extracellular Vesicles (EVs), *e.g.,* exosomes for therapeutic use. Specifically, the methods use protein markers that are newly identified from the luminal surface of exosomes. In particular, a group of proteins (*e.g*., myristoylated alanine rich Protein Kinase C substrate (MARCKS); myristoylated alanine rich Protein Kinase C substrate like 1 (MARCKSL1); and brain acid soluble protein 1 (BASP1)) were identified to be highly enriched on the luminal surface of exosomes. Furthermore, a short sequence of the amino terminus of BASP1, *e.g.,* at least seven amino acids, was shown to be sufficient to direct high efficiency loading of fluorescent protein molecules to the same extent as the full length BASP1 protein. This fragment, which is at least seven amino acids and less than ten amino acids, presents a significant advance in the field of engineered EV loading, and allows for the efficient, reproducible loading of any biologically active molecule, *e.g.,* a therapeutic protein payload, into the lumen of EVs, *e.g.,* exosomes, with no additional steps of *ex vivo* manipulation. The loading of EVs, *e.g.,* exosomes, using the fusion proteins described herein produces engineered EVs, e.g, engineered exosomes, with significantly higher payload levels compared to any other genetic engineering method described thus far.

The proteins and peptide sequences newly identified from exosomes are used in various embodiments of the present disclosure. For example, some embodiments relate to generating a fusion protein by conjugating the EV, *e.g.,* exosome protein or protein fragment *(i.e.,* a scaffold protein) and a biologically active molecule *(e.g.,* therapeutically relevant protein) and producing an EV, *e.g.,* an exosome, containing the fusion protein on the luminal surface of the EV. The native full-length protein or a biologically active fragment of the biologically active molecule *(e.g.,* a therapeutically relevant protein) can be transported to the luminal surface of EVs, *e.g.,* exosomes, by being conjugated to the exosome-enriched proteins or protein fragments.

The present disclosure further relates to generation or use of a lumen-engineered EVs, *e.g.,* lumen-engineered exosomes, designed for more efficient loading, or for loading of a biologically active molecule *(e.g.,* a therapeutically relevant protein) in the lumen of an EV, *e.g.,* an exosome. For example, the EVs' luminal surface can be modified to contain a higher concentration of the native full-length EV, *e.g.,* exosome protein and/or a fragment or a modified protein of the native EV, *e.g.,* exosome protein on the luminal surface.

Some embodiments of the present disclosure relate to a producer cell or a method of generating the producer cell for producing such a lumen-engineered EVs. An exogenous polynucleotide can be introduced transiently or stably into a producer cell to generate a lumen-engineered EV, *e.g.,* a lumen-engineered exosome, from the producer cell.

Accordingly, in an aspect, the present disclosure provides an EV, *e.g.,* an exosome, comprising a scaffold protein, wherein at least a part of the scaffold protein is expressed from an exogenous sequence, and the scaffold protein comprises MARCKS, MARCKSL1, BASP1 or a fragment, variant, derivative, or a modification thereof.

In some aspects, the scaffold protein is present in the EV, *e.g.,* an exosome, at a higher density than a different scaffold protein in a different EV, *e.g.,* an exosome, wherein the different scaffold protein comprises a conventional EV, *e.g.,* exosome protein or a variant thereof. In some embodiments, the conventional EV, *e.g.,* exosome protein is selected from the group consisting of CD9, CD63, CD81, PDGFR, GPI anchor proteins, lactadherin, LAMP2, LAMP2B, and a fragment thereof.

In some embodiments, the EV, *e.g.,* an exosome, is produced from a cell genetically modified to comprise the exogenous sequence, optionally wherein the cell is an HEK293 cell.

In some embodiments, the cell comprises a plasmid comprising the exogenous sequence.

In some embodiments, the exogenous sequence is inserted into a genomic site located 3' or 5' relative to a genomic sequence encoding MARCKS, MARCKSL1, or BASP1. In some embodiments, the exogenous sequence is inserted into a genomic sequence encoding MARCKS, MARCKSL1, or BASP1.

In some embodiments, the scaffold protein is a fusion protein comprising MARCKS, MARCKSL1, BASP1, or a fragment thereof, and a therapeutic peptide.

In some embodiments, the biologically active molecule comprises a therapeutic peptide selected from the group consisting of a natural peptide, a recombinant peptide, a synthetic peptide, or a linker to a therapeutic compound. In some embodiments, the biologically active molecule *(e.g.,* a therapeutic compound) is selected from the group consisting of nucleotides, amino acids, lipids, carbohydrates, and small molecules. In some embodiments, the biologically active molecule *(e.g.,* a therapeutic peptide) is an antibody or a fragment or a modification thereof. In some embodiments, the therapeutic peptide is an enzyme, a ligand, a receptor, a transcription factor, or a fragment or a modification thereof. In some embodiments, the therapeutic peptide is an antimicrobial peptide or a fragment or a modification thereof.

In some embodiments, the EV, *e.g.,* an exosome, further comprises a second scaffold protein, wherein the second scaffold protein comprises MARCKS, MARCKSL1, BASP1, or a fragment thereof. In some embodiments, the EV, *e.g.,* an exosome, further comprises a second scaffold protein, wherein the second scaffold protein comprises PTGFRN, BSG, IGSF2, IGSF3, IGSF8, ITGB1, ITGA4, SLC3A2, ATP transporter or a fragment thereof.

In some embodiments, the scaffold protein comprises a peptide sequence corresponding to the pattern (M)(G)(G/A/S)(K/Q)(L/F/S/Q)(S/A)(K)(K) (SEQ ID NO: 118) or the said pattern without the N-terminal (M). In some embodiments, the scaffold protein comprises a peptide of (M)(O(π)(X)(Φ/π)(π)(+)(+), or the peptide without the N-terminal (M), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). In some embodiments, the scaffold protein comprises a peptide of sequence (M)(G)(π)(ξ)(Φ/π)(S/A/G/N)(+)(+), or the peptide without the N-terminal (M), wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). See R. Aasland et al., FEBS Letters 513 (2002): 141-144 for the nomenclature of amino acids.

In some embodiments, the scaffold protein comprises a peptide of any one of SEQ ID NOS: 4-110. In some embodiments, the scaffold protein comprises a peptide of MGXKLSKKK (SEQ ID NO: 116), or the peptide without the N-terminal M, wherein X is any amino acid. In some embodiments, the scaffold protein comprises a peptide of SEQ ID NO: 110, or the corresponding peptide without the N-terminal M. In some embodiments, the scaffold protein comprises the peptide of SEQ ID NO: 13, or the corresponding peptide without the N-terminal (M).

In some embodiments, the scaffold protein further comprises a payload, *e.g.,* a peptide.

In another embodiment, the present disclosure provides a pharmaceutical composition comprising an EV of the present disclosure, *e.g.,* an exosome, and an excipient.

In some embodiments, the pharmaceutical composition is substantially free of macromolecules, wherein the macromolecules are selected from nucleic acids, exogenous proteins, lipids, carbohydrates, metabolites, and a combination thereof

In yet another embodiment, the present disclosure provides a population of cells for producing the EV, *e.g.,* an exosome, provided herein.

In some embodiments, the population of cells comprises an exogenous sequence encoding the scaffold protein comprising MARCKS, MARCKSL1, BASP1 or a fragment or a modification thereof. In some embodiments, the population of cells further comprise a second exogenous sequence encoding a second scaffold protein, wherein the second scaffold protein comprises MARCKS, MARCKSL1, BASP1 or a fragment or a modification thereof. In some embodiments, the population of cells further comprises a second exogenous sequence encoding a second scaffold protein, wherein the second scaffold protein comprises PTGFRN, BSG, IGSF2, IGSF3, IGSF8, ITGB1, ITGA4, SLC3A2, ATP transporter or a fragment thereof.

In some embodiments, the exogenous sequence is inserted into a genomic sequence encoding MARCKS, MARCKSL1, or BASP1, wherein the exogenous sequence and the genomic sequence encodes the scaffold protein. In some embodiments, the exogenous sequence is in a plasmid.

In some embodiments, the exogenous sequence encodes a biologically active molecule *(e.g.,* a therapeutic peptide). In some embodiments, the therapeutic peptide is selected from a group consisting of a natural peptide, a recombinant peptide, a synthetic peptide, or a linker to a therapeutic compound. In some embodiments, the therapeutic compound is selected from the group consisting of nucleotides, amino acids, lipids, carbohydrates, and small molecules. In some embodiments, the therapeutic peptide is an antibody or a fragment or a modification thereof. In a particular embodiment, the antibody is a nanobody. A person of ordinary skill in the art would understand that the antibody used in an EV of the present disclosure can be any antigen-binding molecule known in the arts, including, *e.g.,* alternative antibody formats, antigen-drug conjugates (ADC) or immunotoxins.

In some embodiments, the therapeutic peptide is an enzyme, a ligand, a receptor, a transcription factor, or a fragment or a modification thereof. In some embodiments, the therapeutic peptide is an antimicrobial peptide or a fragment or a modification thereof.

In some embodiments, the exogenous sequence encodes a targeting moiety. In some embodiments, the targeting moiety is specific to an organ, a tissue, or a cell.

In some embodiments, the second scaffold protein further comprises a targeting moiety. In some embodiments, the targeting moiety is specific to an organ, a tissue, or a cell.

In one embodiment, the present disclosure provides a polypeptide for modifying an EV, *e.g.,* an exosome, comprising a sequence of
(i) (M)(G)(G/A/S)(K/Q)(L/F/S/Q)(S/A)(K)(K) (SEQ ID NO: 118), or the corresponding sequence without the N-terminal (M);
(ii) (M)(G)(π)(X)(Φ/*π*)(*π*)(+)(+), or the corresponding sequence without the N-terminal (M), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu); or
(iii) (M)(G)(π)(ξ)(Φ/π)(S/A/G/N)(+)(+), or the corresponding sequence without the N-terminal (M),wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu).

In some embodiments, the polypeptide of the scaffold comprises a sequence of any of SEQ ID NO: 4-110, or any of the corresponding sequences without the N-terminal M. In some embodiments, the polypeptide comprises a sequence of SEQ ID NO: 13, or the corresponding sequence without the N-terminal M. In some embodiments, the polypeptide comprises a sequence of SEQ ID NO: 110, or the corresponding sequence without the N-terminal M. In some embodiments, the polypeptide comprises a sequence of MGXKLSKKK (SEQ ID NO: 116), wherein X is any amino acid, or the corresponding sequence without the N-terminal M.

In one aspect, the present disclosure provides a polynucleotide construct comprising a coding sequence encoding the polypeptide provided herein. In some embodiments, the coding sequence is codon optimized.

In another aspect, the present disclosure provides a method of making an engineered EV, *e.g.,* an exosome, comprising the steps of:
a. introducing into a cell a nucleic acid construct encoding a fusion polypeptide comprising
   (i) a first sequence encoding MARCKS, MARCKSL1, BASP1 or a fragment or a modification thereof, and
   (ii) a second sequence encoding a payload, *e.g.,* a biologically active molecule such as a therapeutic peptide;
b. maintaining the cell under conditions allowing the cell to express the fusion polypeptide; and,
c. obtaining the engineered exosome comprising the fusion polypeptide from said cell.

In some embodiments, the first sequence comprises a sequence of
(i) (M)(G)(G/A/S)(K/Q)(L/F/S/Q)(S/A)(K)(K) (SEQ ID NO: 118), or the corresponding sequence without the N-terminal (M);
(ii) (M)(G)(π)(X)(Φ/*π*)(*π*)(+)(+), or the corresponding sequence without the N-terminal (M). wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu); or
(iii) (M)(G)(π)(ξ)(Φ/π)(S/A/G/N)(+)(+), or the corresponding sequence without the N-terminal (M), wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu).

In some embodiments, the polynucleotide comprises a sequence of any of SEQ ID NO: 4-110, or any of the corresponding sequences without the N-terminal M. In some embodiments, the polynucleotide comprises a sequence of SEQ ID NO: 13, or the corresponding sequence without the N-terminal M. In some embodiments, the polynucleotide comprises a sequence of SEQ ID NO: 110, or the corresponding sequence without the N-terminal M. In some embodiments, the polynucleotide comprises a sequence of MGXKLSKKK (SEQ ID NO: 116), or the corresponding sequence without the N-terminal M, wherein X is any amino acid.

In some embodiments, the fusion polypeptide is present on the luminal surface of the engineered EV, *e.g.,* an engineered exosome, at a higher density than a different scaffold protein in a different EV, *e.g.,* an exosome, wherein the different scaffold protein comprises a conventional EV, *e.g.,* exosome protein or a variant thereof. In some embodiments, the fusion polypeptide is present at more than 2 fold higher density than the different scaffold protein in the different EV, *e.g.,* an exosome.

In some embodiments, the fusion polypeptide is present at more than 4 fold, 16 fold, 100 fold, or 10,000 fold higher density than the different scaffold protein in the different EV, *e.g.,* an exosome.

The present disclosure is directed to an isolated extracellular vesicle (EV) comprising a biologically active molecule linked to a scaffold protein, wherein the scaffold protein comprises an N-terminus domain (ND) and an effector domain (ED), wherein the ND and the ED are associated with the luminal surface of the EV by an ionic interaction, wherein the ED comprises at least two contiguous lysines (Lys) in sequence. In some embodiments, the ND is associated with the luminal surface of the EV via myristoylation. In other embodiments, the ND has Gly at the N-terminus.

In some embodiments, the ED comprises at least three Lys, at least four Lys, at least five Lys, at least six Lys, or at least seven Lys. In other embodiments, the ED is linked to the ND by a peptide bond. In some embodiments, the ED comprises (Lys)n, wherein n is an integer between 1 and 10. In other embodiments, the ED comprises KK, KKK, KKKK (SEQ ID NO: 151), KKKKK (SEQ ID NO: 152), or any combination thereof.

In other embodiments, the ND comprises the amino acid sequence as set forth in G:X2:X3:X4:X5:X6, wherein G is a glycine represented as Gly; wherein ":" represents a peptide bond, wherein each of the X2 to the X6 is independently an amino acid; and wherein the X6 comprises a basic amino acid. In some embodiments, the X6 is selected from the group consisting of Lys, Arg, and His.

In other embodiments, the disclosure is an isolated extracellular vesicle (EV) comprising a biologically active molecule linked to a scaffold protein, wherein the scaffold protein comprises an N-terminus domain (ND) and an effector domain (ED), wherein the ND comprises the amino acid sequence as set forth in G:X2:X3 :X4:X5 :X6, wherein G is a glycine, represented by Gly; wherein ":" represents a peptide bond, wherein each of the X2 to the X6 is independently an amino acid; wherein the X6 comprises a basic amino acid, and wherein the ED is linked to X6 by a peptide bond and comprises at least one lysine at the N-terminus of the ED. In some embodiments, the ED does not comprise a transmembrane domain or a cytoplasmic domain of a virus. In some embodiments, the X2 is selected from the group consisting of Pro, Gly, Ala, and Ser. In other embodiments, the X4 is selected from the group consisting of Pro, Gly, Ala, Ser, Val, Ile, Leu, Phe, Trp, Tyr, Gln, and Met. In some embodiments, the X5 is selected from the group consisting of Pro, Gly, Ala, and Ser.

In some embodiments, the ND of the scaffold protein comprises the amino acid sequence of G:X2:X3:X4:X5:X6, wherein
G represents Gly;
":" represents a peptide bond;
the X2 is an amino acid selected from the group consisting of Pro, Gly, Ala, and Ser;
the X3 is an amino acid;
the X4 is an amino acid selected from the group consisting of Pro, Gly, Ala, Ser, Val, Ile, Leu, Phe, Trp, Tyr, Gln, and Met;
the X5 is an amino acid selected from the group consisting of Pro, Gly, Ala, and Ser; and
the X6 is an amino acid selected from the group consisting of Lys, Arg, and His.

In some embodiments, the X3 is selected from the group consisting of Asn, Gln, Ser, Thr, Asp, Glu, Lys, His, and Arg.

In some embodiments, the ND and the ED are joined by a linker. In some embodiments, the linker comprises one or more amino acids.

In some embodiments, the disclosure is directed to an isolated extracellular vesicle (EV) comprising a biologically active molecule linked to a scaffold protein, wherein the scaffold protein comprises ND-ED, wherein:
ND comprises G:X2:X3:X4:X5:X6; wherein:
G represents Gly;
":" represents a peptide bond;
the X2 is an amino acid selected from the group consisting of Pro, Gly, Ala, and Ser;
the X3 is an amino acid;
the X4 is an amino acid selected from the group consisting of Pro, Gly, Ala, Ser, Val, Ile, Leu, Phe, Trp, Tyr, Glu, and Met;
the X5 is an amino acid selected from the group consisting of Pro, Gly, Ala, and Ser;
the X6 is an amino acid selected from the group consisting of Lys, Arg, and His;
"-" is an optional linker comprising one or more amino acids; and
ED is an effector domain comprising (i) at least two contiguous lysines (Lys), which is linked to the X6 by a peptide bond or one or more amino acids or (ii) at least one lysine, which is directly linked to the X6 by a peptide bond.

In other embodiments, the X2 is selected from the group consisting of Gly and Ala. In some embodiments, the X3 is Lys. In some embodiments, the X4 is Leu or Glu. In some embodiments, the X5 is selected from the group consisting of Ser and Ala. In some embodiments, the X6 is Lys. In other embodiments, the X2 is Gly, Ala, or Ser; the X3 is Lys or Glu, the X4 is Leu, Phe, Ser, and Glu, the X5 is Ser or Ala; and the X6 is Lys.

In some embodiments, the ND and the ED are linked by a linker comprising one or more amino acids. In some embodiments, the ED comprises Lys (K), KK, KKK, KKKK (SEQ ID NO: 151), KKKKK (SEQ ID NO: 152), or any combination thereof.

In some embodiments, the scaffold protein comprises an amino acid sequence selected from the group consisting of (i) GGKLSKK (SEQ ID NO: 157), (ii) GAKLSKK (SEQ ID NO: 158), (iii) GGKQSKK (SEQ ID NO: 159), (iv) GGKLAKK (SEQ ID NO: 160), or (v) any combination thereof. In some embodiments, the scaffold protein comprises an amino acid sequence selected from the group consisting of (i) GGKLSKKK (SEQ ID NO: 161), (ii) GGKLSKKS (SEQ ID NO: 162), (iii) GAKLSKKK (SEQ ID NO: 163), (iv) GAKLSKKS (SEQ ID NO: 164), (v) GGKQSKKK (SEQ ID NO: 165), (vi) GGKQSKKS (SEQ ID NO: 166), (vii) GGKLAKKK (SEQ ID NO: 167), (viii) GGKLAKKS (SEQ ID NO: 168), and (ix) any combination thereof. In some embodiments, the scaffold protein is at least about 8, at least about 9, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, at least about 100, at least about 105, at least about 110, at least about 120, at least about 130, at least about 140, at least about 150, at least about 160, at least about 170, at least about 180, at least about 190, or at least about 200 amino acids in length.

In some embodiments, the Scaffold protein comprises (i) GGKLSKKKKGYNVN (SEQ ID NO: 169), (ii) GAKLSKKKKGYNVN (SEQ ID NO: 170), (iii) GGKQSKKKKGYNVN (SEQ ID NO: 171), (iv) GGKLAKKKKGYNVN (SEQ ID NO: 172), (v) GGKLSKKKKGYSGG (SEQ ID NO: 173), (vi) GGKLSKKKKGSGGS (SEQ ID NO: 174), (vii) GGKLSKKKKSGGSG (SEQ ID NO: 175), (viii) GGKLSKKKSGGSGG (SEQ ID NO: 176), (ix) GGKLSKKSGGSGGS (SEQ ID NO: 177), (x) GGKLSKSGGSGGSV (SEQ ID NO: 178), or (xi) GAKKSKKRFSFKKS (SEQ ID NO: 179).

In some embodiments, the scaffold protein does not comprise Met at the N-terminus. In some embodiments, the scaffold protein comprises a myristoylated amino acid residue at the N-terminus of the scaffold protein. In some embodiments, the amino acid residue at the N-terminus of the scaffold protein is Gly. In some embodiments, the amino acid residue at the N-terminus of the scaffold protein is synthetic. In some embodiments, the amino acid residue at the N-terminus of the scaffold protein is a glycine analog.

In some embodiments, the scaffold protein comprises an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to SEQ ID NO: 1 (MARKS), SEQ ID NO: 2 (MARCKSL1), or SEQ ID NO: 3 (BASP1).

In some embodiments, the biologically active molecule is on the luminal surface or lumen of the EV. In some embodiments, the scaffold protein further comprises a transmembrane domain. In some embodiments, the transmembrane domain is between the ED domain of the scaffold protein and the biologically active molecule. In some embodiments, the scaffold protein further comprises an extravesicular domain. In some embodiments, the biologically active molecule is linked to the extravesicular domain.

In some embodiments, the scaffold protein is linked to the biologically active molecule by a linker. In some embodiments, the ND domain is linked to the ED domain by a linker. In some embodiments, the linker comprises one or more amino acids. In some embodiments, the linker comprises a cleavable linker. In some embodiments, the linker comprises a flexible linker.

In other embodiments, the biologically active molecule comprises a protein, a polypeptide, a peptide, a polynucleotide (DNA and/or RNA), a chemical compound, a virus, an ionophore, a carrier for an ionophore, a moiety that forms a channel or a pore, or any combination thereof. In some embodiments, the protein comprises a recombinant peptide, a natural peptide, a synthetic peptide, an antibody, a fusion protein, or any combination thereof. In some embodiments, the protein comprises an enzyme, a cytokine, a ligand, a receptor, a transcription factor, or a combination thereof. In some embodiments, the virus comprises an adeno-associated virus, a parvovirus, a retrovirus, an adenovirus, or any combination thereof

In other embodiments, the EV further comprises a second scaffold protein. In some embodiments, the second scaffold protein comprises a PTGFRN polypeptide, a BSG polypeptide, an IGSF2 polypeptide, an IGSF3 polypeptide, an IGSF8 polypeptide, an ITGB1 polypeptide, an ITGA4 polypeptide, a SLC3A2 polypeptide, an ATP transporter polypeptide, an aminopeptidase N (ANPEP) polypeptide, an ectonucleotide pyrophosphatase/phosphodiesterase family member 1 (ENPP1) polypeptide, a neprilysin (MME) polypeptide, a neuropilin-1 (NRP1) polypeptide, or a fragment thereof. In some embodiments, the biologically active molecule is an inhibitor for a negative checkpoint regulator or an inhibitor for a binding partner of a negative checkpoint regulator. In some embodiments, the negative checkpoint regulator is selected from the group consisting of: cytotoxic T-lymphocyte-associated protein 4 (CTLA-4), programmed cell death protein 1 (PD-1), lymphocyte-activated gene 3 (LAG-3), T-cell immunoglobulin mucin-containing protein 3 (TIM-3), B and T lymphocyte attenuator (BTLA), T cell immunoreceptor with Ig and ITIM domains (TIGIT), V-domain Ig suppressor of T cell activation (VISTA), adenosine A2a receptor (A2aR), killer cell immunoglobulin like receptor (KIR), indoleamine 2,3-dioxygenase (IDO), CD20, CD39, and CD73. In some embodiments, the biologically active molecule is an immunogenic protein.

In other embodiments, the biologically active molecule is a toxin, toxoid, or a non-toxic mutant of a toxin. In some embodiments, the toxin is a diphtheria toxin. In some embodiments, the toxoid is a tetanus toxoid. In some embodiments, the biologically active molecule is a non-toxic mutant of diphtheria toxin.

In some embodiments, the biologically active molecule is an activator for a positive co-stimulatory molecule or an activator for a binding partner of a positive co-stimulatory molecule. In some embodiments, the positive co-stimulatory molecule is a TNF receptor superfamily member. In some embodiments, the TNF receptor superfamily member is selected from the group consisting of: CD120a, CD120b, CD18, OX40, CD40, Fas receptor, M68, CD27, CD30, 4-1BB, TRAILR1, TRAILR2, TRAILR3, TRAILR4, RANK, OCIF, TWEAK receptor, TACI, BAFF receptor, ATAR, CD271, CD269, AITR, TROY, CD358, TRAMP, and XEDAR. In some embodiments, the activator for a positive co-stimulatory molecule is a TNF superfamily member. In some embodiments, the TNF superfamily member is selected from the group consisting of: TNFα, TNF-C, OX40L, CD40L, FasL, LIGHT, TL1A, CD27L, Siva, CD153, 4-1BB ligand, TRAIL, RANKL, TWEAK, APRIL, BAFF, CAMLG, NGF, BDNF, NT-3, NT-4, GITR ligand, and EDA-2. In some embodiments, the positive co-stimulatory molecule is a CD28-superfamily co-stimulatory molecule. In some embodiments, the CD28-superfamily co-stimulatory molecule is ICOS or CD28. In some embodiments, the activator for a positive co-stimulatory molecule is ICOSL, CD80, or CD86.

In other embodiments, the cytokine is selected from the group consisting of: IL-2, IL-7, IL-10, IL-12, and IL-15. In some embodiments, the protein comprises a T-cell receptor (TCR), a T-cell co-receptor, a major histocompatibility complex (MHC), a human leukocyte antigen (HLA), or a derivative thereof. In some embodiments, the protein comprises a tumor antigen. In some embodiments, the tumor antigen is selected from the group consisting of: alpha-fetoprotein (AFP), carcinoembryonic antigen (CEA), epithelial tumor antigen (ETA), mucin 1 (MUC1), Tn-MUC1, mucin 16 (MUC16), tyrosinase, melanoma-associated antigen (MAGE), tumor protein p53 (p53), CD4, CD8, CD45, CD80, CD86, programmed death ligand 1 (PD-L1), programmed death ligand 2 (PD-L2), NY-ESO-1, PSMA, TAG-72, HER2, GD2, cMET, EGFR, Mesothelin, VEGFR, alpha-folate receptor, CE7R, IL-3, Cancer-testis antigen, MART-1 gp100, and TNF-related apoptosis-inducing ligand.

In other embodiments, the EV is an exosome.

In some embodiments, the disclosure is directed to a pharmaceutical composition comprising the EV of the present disclosure and a pharmaceutically acceptable carrier. In some embodiments, the disclosure is directed to a cell that produces the EV of the present disclosure. In some embodiments, the disclosure is directed to a kit comprising the EV of the present disclosure and instructions for use.

In other embodiments, the disclosure is directed to a method of making EVs comprising culturing the cell of the present disclosure under a suitable condition and obtaining the EVs. In some embodiments, the disclosure is directed to a method of anchoring a biologically active molecule to an extracellular vesicle comprising linking the biologically active molecule to the scaffold protein of disclosed herein.

In other embodiments, the disclosure is directed to a method of preventing or treating a disease in a subject in need thereof, comprising administering the EV of the present disclosure, wherein the disease is associated with the antigen. In some embodiments, the EV is administered parenterally, orally, intravenously, intramuscularly, intra-tumorally, intranasally, subcutaneously, or intraperitoneally.

### EMBODIMENTS

E1. An EV, *e.g.,* an exosome, comprising a scaffold protein, wherein at least a part of the scaffold protein is expressed from an exogenous sequence, and the scaffold protein comprises MARCKS, MARCKSL1, BASP1 or a fragment or a modification thereof.
E2. The EV, *e.g.,* exosome, of embodiment E1, wherein the scaffold protein is present in the EV, *e.g.,* exosome, at a higher density than a different scaffold protein in a different EV, *e.g.,* exosome, wherein the different scaffold protein comprises a conventional EV, *e.g.,* exosome, protein or a variant thereof.
E3. The EV, *e.g.,* exosome, of embodiment E2, wherein the conventional EV, *e.g.,* exosome, protein is selected from the group consisting of CD9, CD63, CD81, PDGFR, GPI anchor proteins, lactadherin, LAMP2, LAMP2B, and a fragment thereof.
E4. The EV, *e.g.,* exosome, of any of embodiments E1 to E3, wherein the EV, *e.g.,* exosome, is produced from a cell genetically modified to comprise the exogenous sequence, optionally wherein the cell is an HEK293 cell.
E5. The EV, *e.g.,* exosome, of embodiments E1 to E4, wherein the cell comprises a plasmid comprising the exogenous sequence.
E6. The EV, *e.g.,* exosome, of embodiments E1 to E5, wherein the cell comprises the exogenous sequence inserted into a genome of the cell.
E7. The EV, *e.g.,* exosome, of embodiments E1 to E6, wherein the exogenous sequence is inserted into a genomic site located 3' or 5' relative to a genomic sequence encoding MARCKS, MARCKSL1, or BASP1.
E8. The EV, *e.g.,* exosome, of embodiments E1 to E7, wherein the exogenous sequence is inserted into a genomic sequence encoding MARCKS, MARCKSL1, or BASP1.
E9. The EV, *e.g.,* exosome, of any of embodiments E1 to E8, wherein the scaffold protein is a fusion protein comprising MARCKS, MARCKSL1, BASP1, or a fragment thereof, and a therapeutic peptide.
E10. The EV, *e.g.,* exosome, of embodiment E9, wherein the therapeutic peptide is selected from the group consisting of a natural peptide, a recombinant peptide, a synthetic peptide, or a linker to a therapeutic compound.
E11. The EV, *e.g.,* exosome, of embodiment E9, wherein the therapeutic compound is selected from the group consisting of nucleotides, amino acids, lipids, carbohydrates, and small molecules.
E12. The EV, *e.g.,* exosome, of embodiment E9, wherein the therapeutic peptide is an antibody or a fragment or a modification thereof.
E13. The EV, *e.g.,* exosome, of embodiment E9, wherein the therapeutic peptide is an enzyme, a ligand, a receptor, a transcription factor, or a fragment or a modification thereof.
E14. The EV, *e.g.,* exosome, of embodiment E9, wherein the therapeutic peptide is an antimicrobial peptide or a fragment or a modification thereof.
E15. The EV, *e.g.,* exosome, of any of embodiments E1 to E14, further comprising a second scaffold protein, wherein the second scaffold protein comprises MARCKS, MARCKSL1, BASP1, or a fragment thereof.
E16. The EV, *e.g.,* exosome, of any of embodiments E1 to E14, further comprising a second scaffold protein, wherein the second scaffold protein comprises PTGFRN, BSG, IGSF2, IGSF3, IGSF8, ITGB1, ITGA4, SLC3A2, ATP transporter or a fragment thereof.
E17. The EV, *e.g.,* exosome, of any of embodiments E1 to E16, wherein the scaffold protein comprises a peptide of sequence (M)(G)(G/A/S)(K/Q)(L/F/S/Q)(S/A)(K)(K) (SEQ ID NO: 118), or the corresponding sequence without the N-terminal (M).
E18. The EV, *e.g.,* exosome, of any of embodiments E1 to E17, wherein the scaffold protein comprises a peptide of sequence (M)(G)(π)(X)(Φ/*π*)(*π*)(+)(+), or the corresponding sequence without the N-terminal (M), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu).
E19. The EV, *e.g.,* exosome, of any of embodiments E1 to E18, wherein the scaffold protein comprises a peptide of sequence (M)(G)(π)(ξ)(Φ/π)(S/A/G/N)(+)(+), or the corresponding sequence without the N-terminal (M), wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu).
E20. The EV, *e.g.,* exosome, of any of embodiments E18 or E19, wherein the scaffold protein comprises a peptide of any one of SEQ ID NO: 4-110, or any of the corresponding sequences without the N-terminal M.
E21. The EV, *e.g.,* exosome, of any of embodiments E18 or E19, wherein the scaffold protein comprises a peptide of MGXKLSKKK (SEQ ID NO: 116), or the corresponding sequence without the N-terminal M, wherein X is any amino acid.
E22. The EV, *e.g.,* exosome, of embodiment E20, wherein the scaffold protein comprises a peptide of SEQ ID NO: 110, or the corresponding sequence without the N-terminal M.
E23. The EV, *e.g.,* exosome, of embodiment E20, wherein the scaffold protein comprises the peptide of SEQ ID NO: 13, or the corresponding sequence without the N-terminal M.
E24. The EV, *e.g.,* exosome, of any of embodiments E1 to E24, wherein the scaffold protein further comprises a payload, *e.g.,* a biologically active molecule such as a peptide.
E25. A pharmaceutical composition comprising the EV, *e.g.,* exosome, of any of embodiments E1 to E24 and an excipient.
E26. The pharmaceutical composition of embodiment E25, substantially free of macromolecules, wherein the macromolecules are selected from nucleic acids, exogenous proteins, lipids, carbohydrates, metabolites, and a combination thereof
E27. A population of cells for producing the EV, *e.g.,* exosome, of any of embodiments E1 to E24.
E28. The population of cells of embodiment E27, comprising an exogenous sequence encoding the scaffold protein comprising MARCKS, MARCKSL1, BASP1 or a fragment or a modification thereof.
E29. The population of cells of embodiment E28, further comprising a second exogenous sequence encoding a second scaffold protein, wherein the second scaffold protein comprises MARCKS, MARCKSL1, BASP1 or a fragment or a modification thereof.
E30. The population of cells of embodiment E28, further comprising a second exogenous sequence encoding a second scaffold protein, wherein the second scaffold protein comprises PTGFRN, BSG, IGSF2, IGSF3, IGSF8, ITGB1, ITGA4, SLC3A2, ATP transporter or a fragment thereof.
E31. The population of cells of any of embodiments E27 to E30, wherein the exogenous sequence is inserted into a genomic sequence encoding MARCKS, MARCKSL1, or BASP1, wherein the exogenous sequence and the genomic sequence encodes the scaffold protein.
E32. The population of cells of any of embodiments E27 to E30, wherein the exogenous sequence is in a plasmid.
E33. The population of cells of any of embodiments E27 to E32, wherein the exogenous sequence encodes a biologically active molecule, *e.g.,* a therapeutic peptide.
E34. The population of cells of embodiment E33, wherein the therapeutic peptide is selected from a group consisting of a natural peptide, a recombinant peptide, a synthetic peptide, or a linker to a therapeutic compound.
E35. The population of cells of embodiment E33, wherein the therapeutic compound is selected from the group consisting of nucleotides, amino acids, lipids, carbohydrates, and small molecules.
E36. The population of cells of embodiment E33, wherein the therapeutic peptide is an antibody or a fragment or a modification thereof.
E37. The population of cells of embodiment E33, wherein the therapeutic peptide is an enzyme, a ligand, a receptor, a transcription factor, or a fragment or a modification thereof.
E38. The population of cells of embodiment E33, wherein the therapeutic peptide is an antimicrobial peptide or a fragment or a modification thereof.
E39. The population of cells of embodiment E28, wherein the exogenous sequence encodes a targeting moiety.
E40. The population of cells of embodiment E39, wherein the targeting moiety is specific to an organ, a tissue, or a cell.
E41. The population of cells of embodiments E29 or E30, wherein the second scaffold protein further comprises a targeting moiety.
E42. The population of cells of embodiment E41, wherein the targeting moiety is specific to an organ, a tissue, or a cell.
E43. A polypeptide for modifying an EV, *e.g.,* exosome, comprising a sequence of
   (i) (M)(G)(G/A/S)(K/Q)(L/F/S/Q)(S/A)(K)(K) (SEQ ID NO: 118), or the corresponding sequence without the N-terminal (M);
   (ii) (M)(G)(π)(X)(Φ/*π*)(*π*)(+)(+), or the corresponding sequence without the N-terminal (M), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu); or
   (iii) (M)(G)(π)(ξ)(Φ/π)(S/A/G/N)(+)(+), or the corresponding sequence without the N-terminal (M), wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu).
E44. The polypeptide of embodiment E43, comprising a sequence of any of SEQ ID NO: 4-110, or any of the corresponding sequences without the N-terminal M.
E45. The polypeptide of embodiment E43, comprising a sequence of SEQ ID NO: 13, or the corresponding sequence without the N-terminal M.
E46. The polypeptide of embodiment E43, comprising a sequence of SEQ ID NO: 110, or the corresponding sequence without the N-terminal M.
E47. The polypeptide of embodiment E43, comprising a sequence of MGXKLSKKK (SEQ ID NO: 116), or the corresponding sequence without the N-terminal M, wherein X is any amino acid.
E48. The polypeptide of any of embodiments E43 to E47, wherein the polypeptide is fused to a payload, *e.g.,* a biologically active molecule such as a peptide.
E49. The polypeptide of embodiment E48, wherein the polypeptide is fused to the N-terminus of the peptide.
E50. A polynucleotide construct comprising a coding sequence encoding the polypeptide of any of embodiments E43 to E49.
E51. The polynucleotide construct of embodiment E50, wherein the coding sequence is codon optimized.
E52. A method of making an engineered EV, *e.g.,* exosome, comprising the steps of:
   a. introducing into a cell a nucleic acid construct encoding a fusion polypeptide comprising (i) a first sequence encoding MARCKS, MARCKSL1, BASP1 or a fragment or a modification thereof, and (ii) a second sequence encoding a payload, *e.g.,* a biologically active molecule such as a peptide;
   b. maintaining the cell under conditions allowing the cell to express the fusion polypeptide; and
   c. obtaining the engineered EV, *e.g.,* exosome, comprising the fusion polypeptide from said cell.
E53. The method of embodiment E52, wherein the first sequence comprises a sequence of
   (i) (M)(G)(G/A/S)(K/Q)(L/F/S/Q)(S/A)(K)(K) (SEQ ID NO: 118), or the corresponding sequence without the N-terminal (M);
   (ii) (M)(G)(π)(X)(Φ/*π*)(*π*)(+)(+), or the corresponding sequence without the N-terminal (M), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu); or
   (iii) (M)(G)(π)(ξ)(Φ/π)(S/A/G/N)(+)(+), or the corresponding sequence without the N-terminal (M), wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu).
E54. The method of any of embodiments E52 or E53, wherein the first sequence comprises any of SEQ ID NO: 4-110, or any of the corresponding sequences without the N-terminal M.
E55. The method of embodiment E54, wherein the first sequence comprises SEQ ID NO: 13, or the corresponding sequence without the N-terminal M.
E56. The method of embodiment E55, wherein the first sequence comprises SEQ ID NO: 110, or the corresponding sequence without the N-terminal M.
E57. The method of embodiment E53, wherein the first sequence comprises MGXKLSKKK (SEQ ID NO: 116), or the corresponding sequence without the N-terminal M, wherein X is any amino acid.
E58. The method of any of embodiments E52 to E57, wherein the fusion polypeptide is present on the luminal surface of the engineered EV, *e.g.,* exosome, at a higher density than a different scaffold protein in a different EV, *e.g.,* exosome, wherein the different scaffold protein comprises a conventional EV, *e.g.,* exosome protein or a variant thereof.
E59. The method of embodiment E58, wherein the fusion polypeptide is present at more than 2 fold higher density than the different scaffold protein in the different EV, *e.g.,* exosome.
E60. The method of embodiment E59, wherein the fusion polypeptide is present at more than 4 fold, 16 fold, 100 fold, or 10,000 fold higher density than the different scaffold protein in the different EV, *e.g.,* exosome.

### BRIEF DESCRIPTION OF THE DRAWINGS

The figures depict various aspects of the present disclosure for purposes of illustration only. One skilled in the art will readily recognize from the following discussion that alternative aspects of the structures and methods illustrated herein may be employed without departing from the principles of the disclosure described herein.

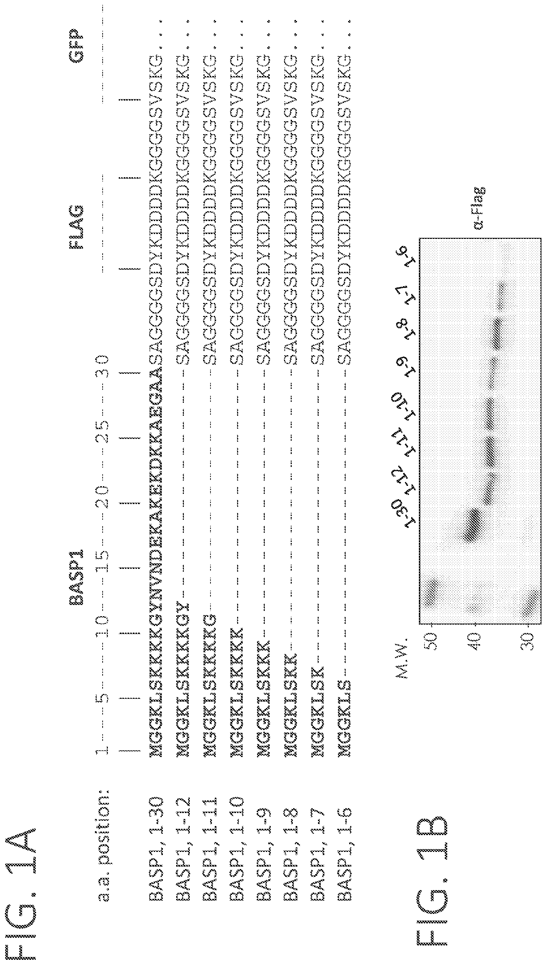

**FIG. 1A** shows sequences of fusion proteins comprising a BASP1 fragment fused to a FLAG® tag and GFP (SEQ ID NOs: 135-142, respectively, in order of appearance). **FIG. 1B** shows the anti-FLAG® Western blot results for exosomes purified from cells stably expressing one of the fusion proteins in FIG. 1A. For both FIGs. 1A and 1B, the amino acid positions, *e.g.,* BASP1, 1-30, refer to a BASP-1 fragment that is initially encoded by the gene construct. The first Met is cleaved off during processing.

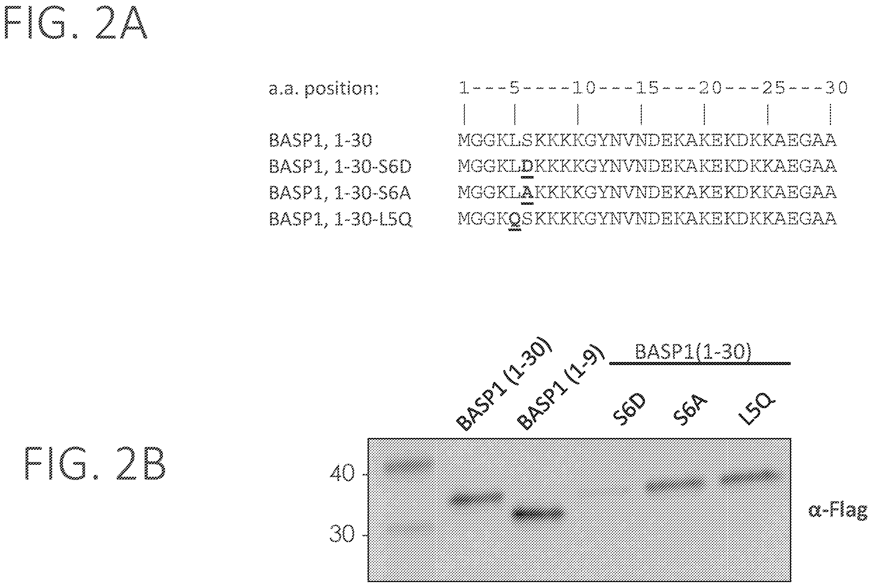

**FIG. 2A** shows sequences from a BASP1 fragment (1-30) (SEQ ID NO: 4) and its modifications (1-30-S6D, 1-30-S6A, and 1-30-L5Q) fused to a FLAG® tag and GFP (SEQ ID NOs: 143-145, respectively, in order of appearance).
**FIG. 2B** shows the anti-FLAG® Western blot results for exosomes purified from cells stably expressing one of the fusion proteins in FIG. 2A. For both FIGs. 2A and 2B, the amino acid positions, *e.g.,* BASP1, 1-30, refer to a BASP-1 fragment that is initially encoded by the gene construct. The first Met is cleaved off during processing.

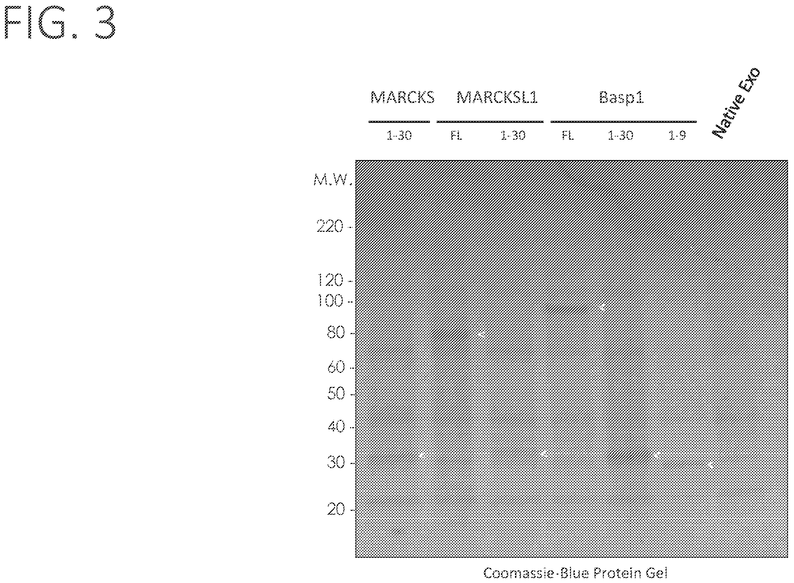

**FIG. 3** shows an image of a COOMMASSIE® stained protein gel with exosome samples purified from cells stably expressing full-length MARCKSL1, BASP1, or amino acids 1-30 of MARCKS, MARCKSL1, or BASP1, all fused to FLAG®-GFP. White arrows on the image indicate bands corresponding to the fusion proteins. For FIG. 3, the amino acid positions, *e.g.,* BASP1, 1-30, refer to a BASP-1 fragment that is initially encoded by the gene construct. The first Met is cleaved off during processing.

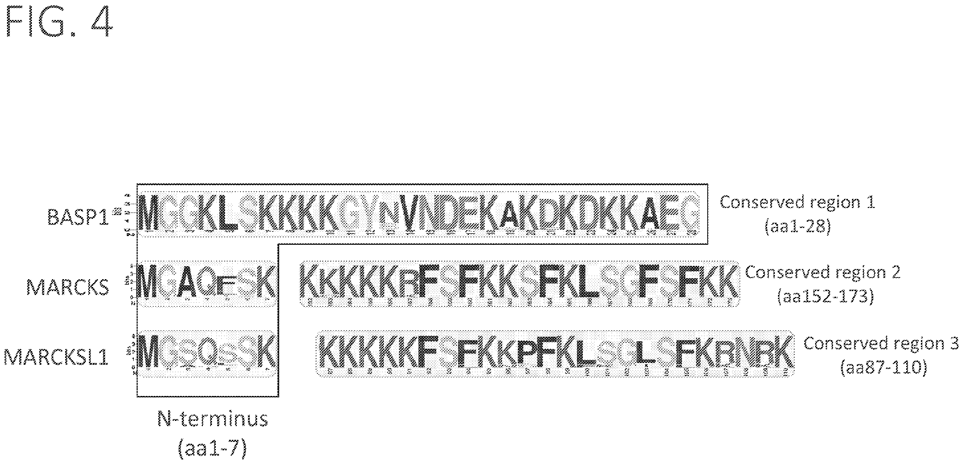

**FIG. 4** shows a protein sequence alignment between the first 28 amino acids of BASP1 (conserved region 1), amino acids 1-7 and 152-173 of MARCKS (conserved region 2), and amino acids 1-7 and 87-110 of MARCKSL1 (conserved region 3).

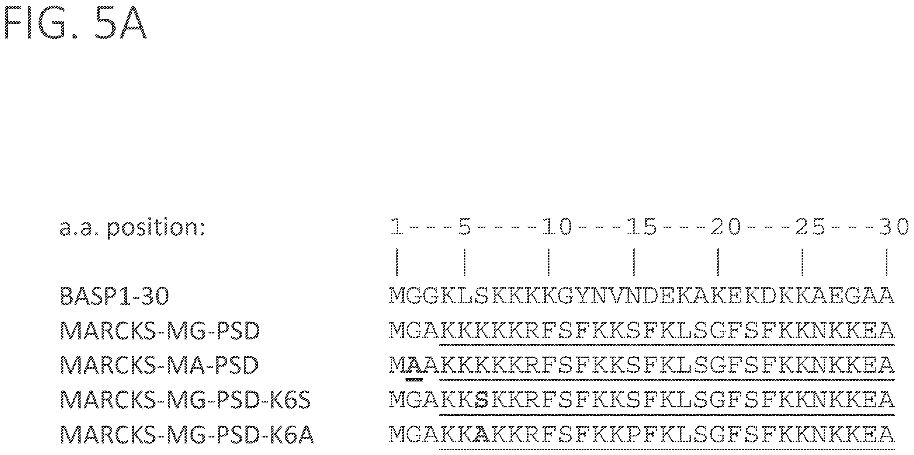

**FIG. 5A** shows sequences of amino acids 1-30 of BASP1 ("BASP1-30") (SEQ ID NO: 4) and fusion proteins comprising amino acids 1-3 of MARCKS or its modification fused to the PSD domain of MARCKS or its modification ("MARCKS-MG-PSD", "MARCKS-MA-PSD", "MARCKS-MG-PSD-K6S" and "MARCKS-MG-PSD-K6A") (SEQ ID NOS: 146-149, respectively, in order of appearance). Point mutations introduced into the MARCKS sequences are bolded. The amino acid positions, *e.g.,* aa 1-30 of BASP1 or aa 1-3 of MARCKS, refer to a fragment that is initially encoded by the gene construct. The first Met is cleaved off during processing.

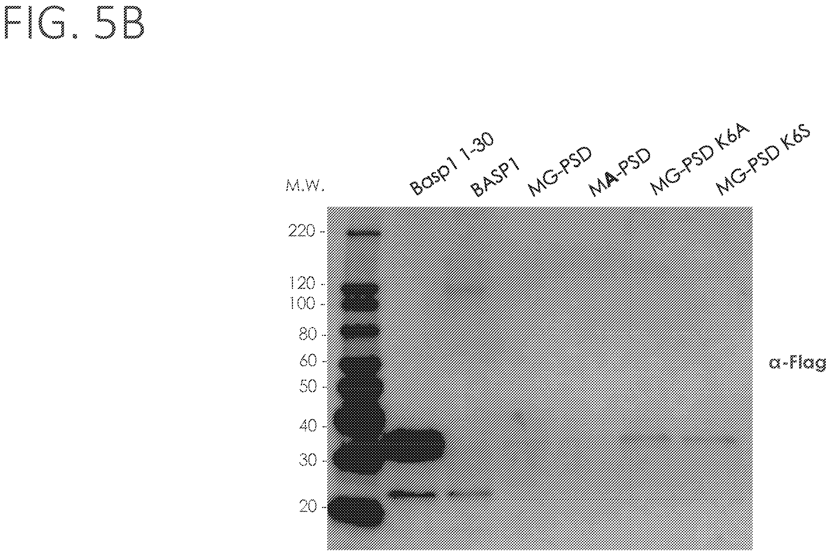

**FIG. 5B** shows anti-FLAG® Western blotting results of purified exosomes from cells stably expressing the fusion proteins comprising the amino acid sequences of FIG. 5A and FLAG®.

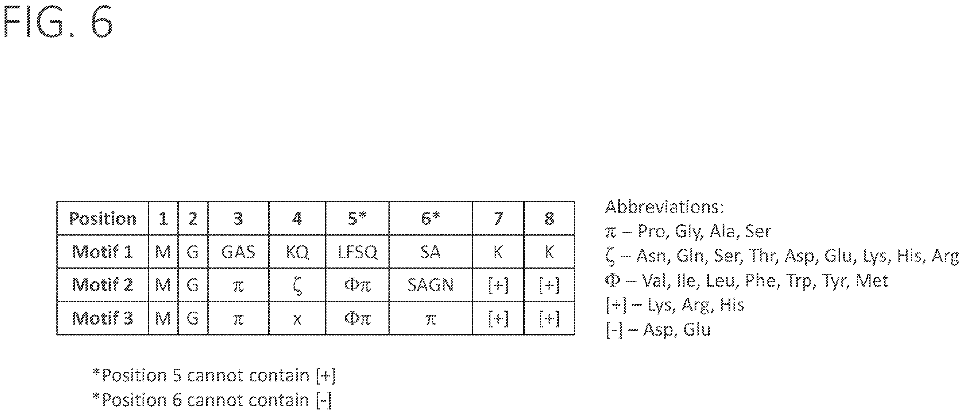

**FIG. 6** shows three different consensus sequences (all three sequences are represented by SEQ ID NO: 118) derived from functional studies of MARCKS, MARCKSL1, and BASP1, and the amino acid requirements of each of the sequences for loading a payload into the lumen of exosomes via attachment to the luminal surface.

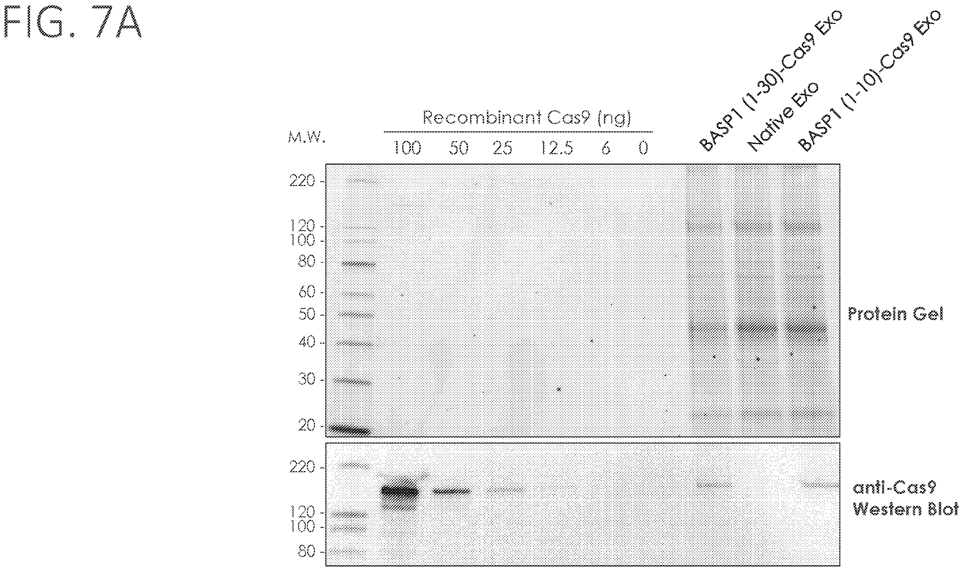

**FIG. 7A** shows total protein (top) and an anti-Cas9 Western blot (bottom) of native exosomes or exosomes purified from cells stably expressing Cas9 fused to amino acids 1-10 or 1-30 of BASP1, as well as decreasing amounts of recombinant Cas9. The amino acid positions, *e.g.,* aa 1-10 of BASP or aa 1-30 of BASP1, refer to a fragment that is initially encoded by the gene construct. The first Met is cleaved off during processing.

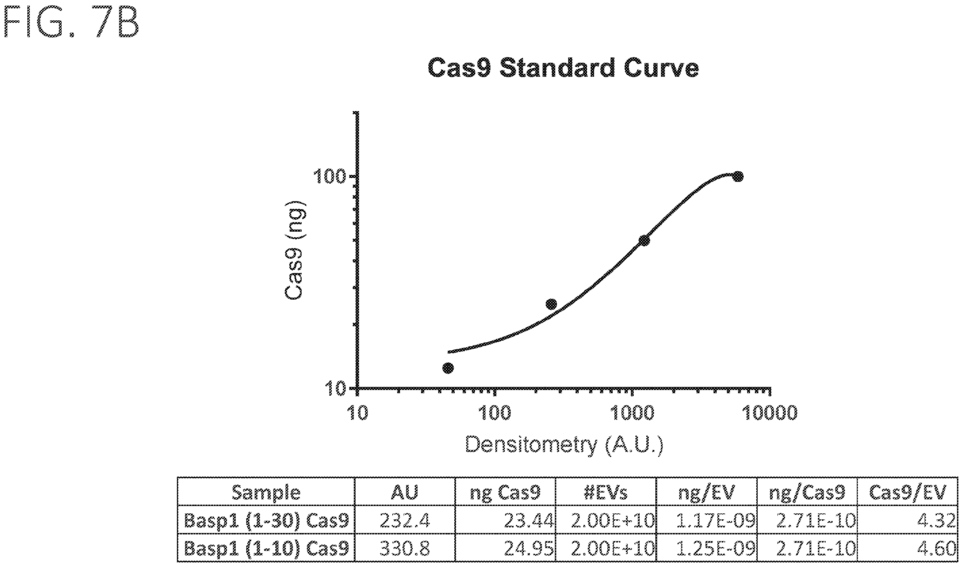

**FIG. 7B** The top panel shows a standard curve derived from Cas9 densitometry of the Western blotting results of FIG. 7A. The bottom panel further provides amounts of Cas9 loaded per each purified exosome as fusion proteins conjugated to 1-30 amino acids or 1-10 amino acids fragments of BASP1 (2-30 amino acids or 2-10 amino acids fragments of BASP1 after processing *(i.e.,* the first Met being cleaved off)), estimated based on the standard curve.

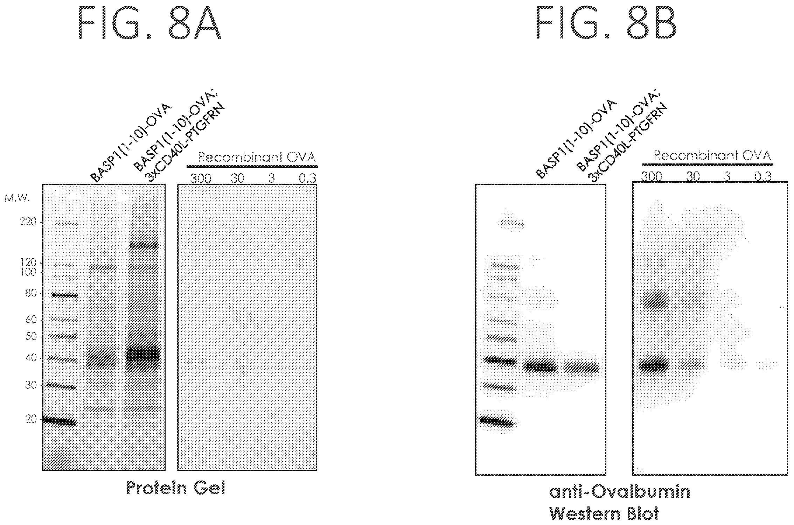

**FIG. 8A** shows protein gel images of exosomes purified from cells stably transfected with a construct expressing BASP1 N-terminal (amino acids 1-10) fusion to ovalbumin ("BASP1 (1-10)-OVA") or cells stably transfected with two constructs, one expressing BASP1 N-terminal (amino acids 1-10) fusion to ovalbumin, and the other expressing CD40L fused to a transmembrane protein PTGFRN ("BASP1 (1-10)-OVA; 3XCD40L-PTGFRN"). FIG. 8A further shows an image of the protein gel loading decreasing amounts of recombinant OVA. The amino acid numbering, *e.g.,* amino acids 1-10, refers to a fragment that is initially encoded by the gene construct. The first Met is cleaved off during processing.
**FIG. 8B** shows anti-Ovalbumin Western blot results of the samples from FIG. 8A.

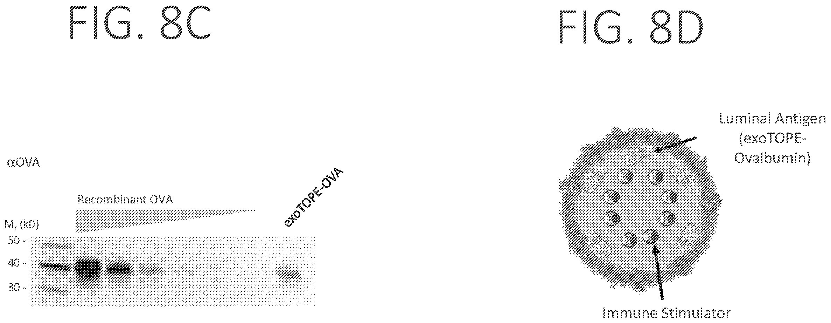

**FIG. 8C** shows Westernblot results comparing recombinant ova with ova fused to exoTOPE. **FIG. 8D** shows a diagram of exosome comprising (i) exoTOPE linked to Ovalbumin in the luminal side of the exosome and (ii) immune stimulator in the lumen of the exosome.

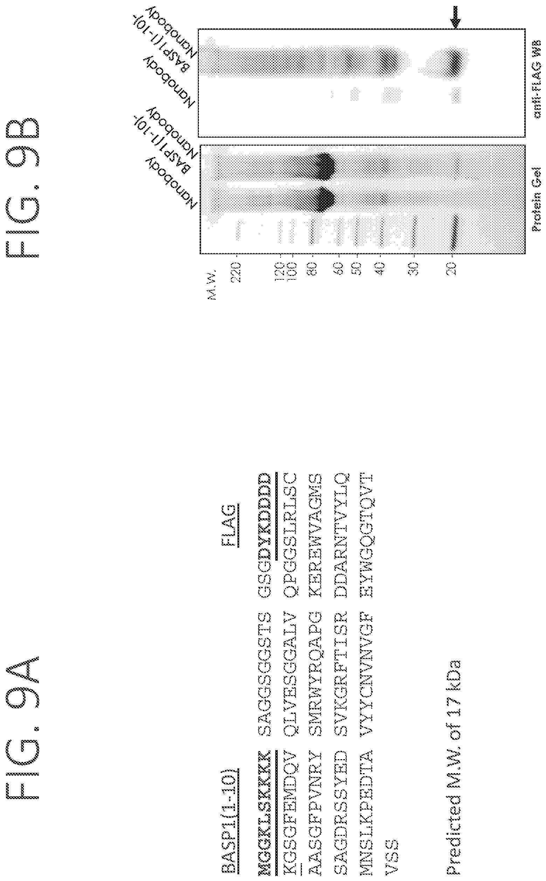

**FIG. 9A** shows the sequence of a camelid nanobody directed against GFP fused to amino acids 1-10 of BASP1 and a FLAG® tag (SEQ ID NO: 150). The amino acid numbering, *e.g.,* amino acids 1-10 of BASP1, refers to a fragment that is initially encoded by the gene construct. The first Met is cleaved off during processing.

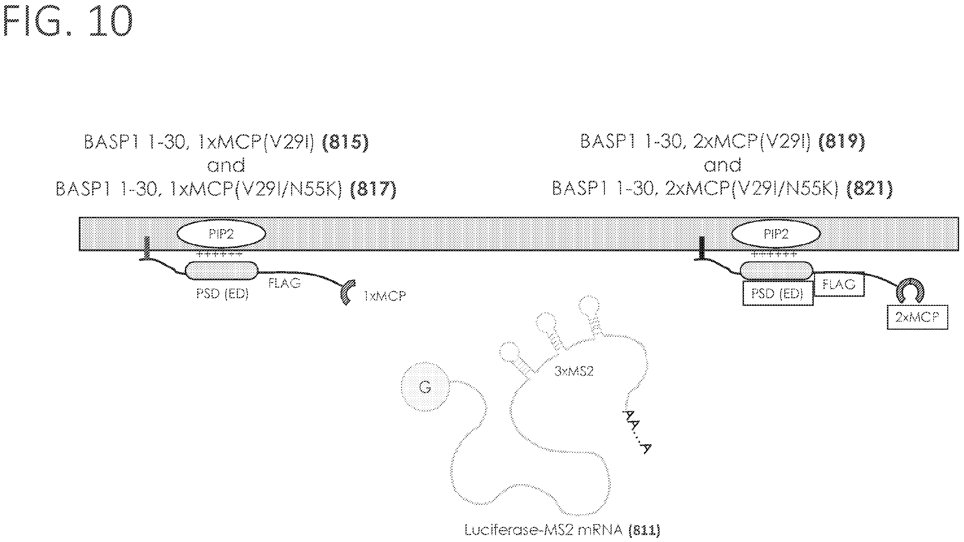

**FIG. 9B** shows a protein gel and an anti-FLAG® Western blotting results of purified exosomes from cells stably expressing the fusion protein of FIG. 10A ("BASP1(1-10)-Nanobody") or the protein lacking the BASP1 sequence ("Nanobody"). The amino acid numbering, *e.g.,* BASP1 (1-10)-Nanobody, refers to a fragment that is initially encoded by the gene construct. The first Met is cleaved off during processing.
**FIG. 10** shows a schematic of an exosome mRNA loading system comprising (i) BASP1 (1-30) fused to FLAG® and monomeric or dimeric MCP variants (1XMCP(V29I) ("815"; SEQ ID NO: 111), 1XMCP (V29I/N55K) ("817"; SEQ ID NO: 112), 2XMCP(V29I) ("819"; SEQ ID NO: 113) or 2XMCP(V29I/N55K)) ("821"; SEQ ID NO: 114) and (ii) a luciferase mRNA containing 3x MS2 hairpin loops ("Luciferase-MS2 mRNA" or "811"; SEQ ID NO: 115). The amino acid numbering, *e.g.,* BASP1 (1-30), refers to a fragment that is initially encoded by the gene construct. The first Met is cleaved off during processing.

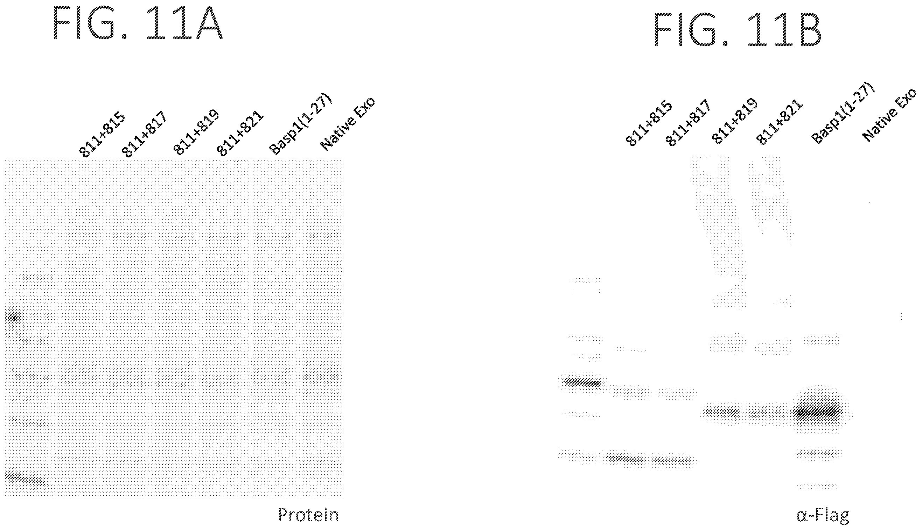

**FIG. 11A** shows a protein gel of the exosomes containing the mRNA loading constructs described in FIG. 10, a luciferase mRNA (811) in combination with various BASP1 fusion proteins (815, 817, or 819).
**FIG. 11B** shows an anti-FLAG® Western blot of the samples in FIG. 11A.

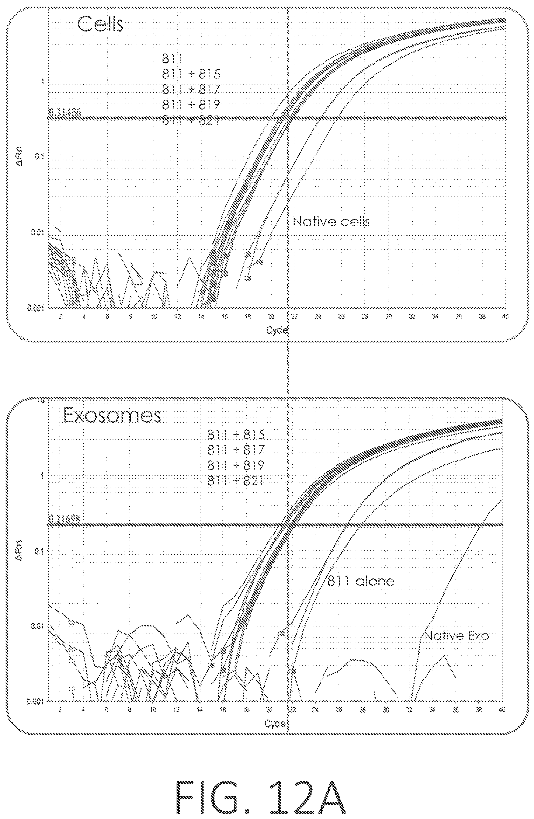

**FIG. 12A** shows RT-qPCR results for the amount of Luciferase mRNA in cells (top) or exosomes (bottom) containing the mRNA loading constructs shown in FIG. 10.

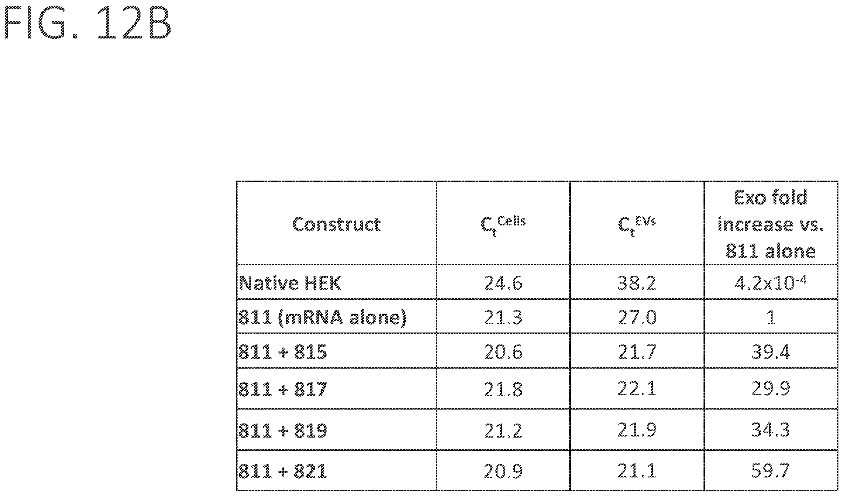

**FIG. 12B** shows a table quantitating the amount of Luciferase mRNA in purified exosomes from the samples in FIG. 12A, including fold-enrichment from stochastic loading of Luciferase mRNA.

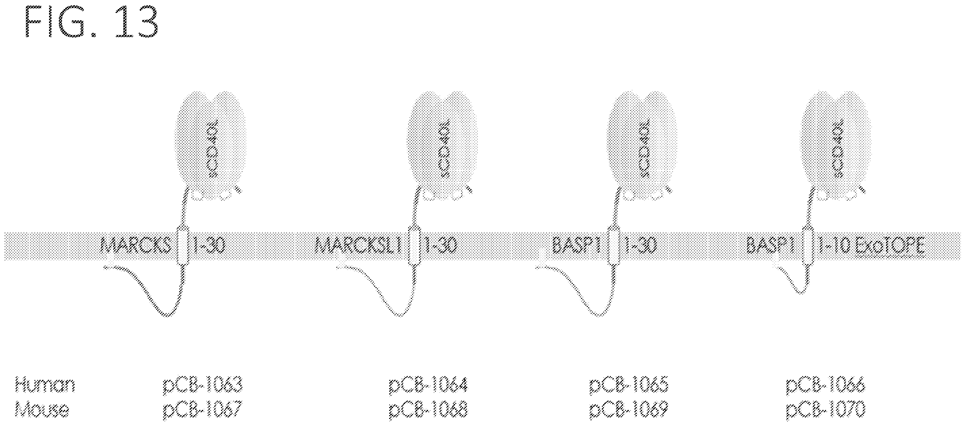

**FIG.** 13 shows schematic diagrams of CD40L trimers fused to N-terminal fragments of MARCKS, MARCKSL1, and BASP1 to allow for external surface display of transmembrane proteins anchored to the luminal surface of the exosome. The amino acid numbering, *e.g.,* MARCKS 1-30, MARCKSL1 1-30, BASP1 1-30, BASP1 1-10 ExoTOPE, refers to a fragment that is initially encoded by the gene construct. The first Met is cleaved off during processing.

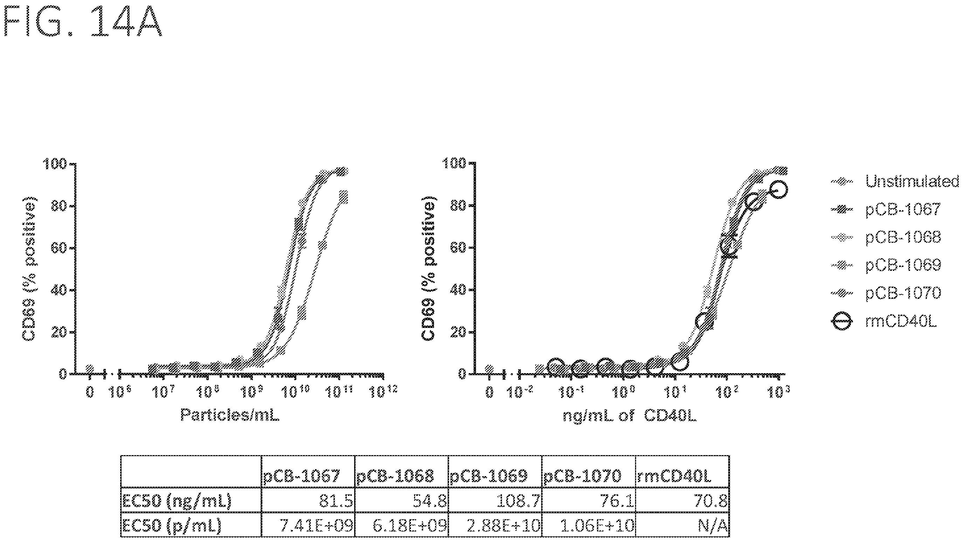

**FIG. 14A** shows the results of mouse B-cell activation in cultures incubated with CD40L surface expression exosomes fused to N-terminal fragments of MARCKS, MARCKSL1, and BASP1.

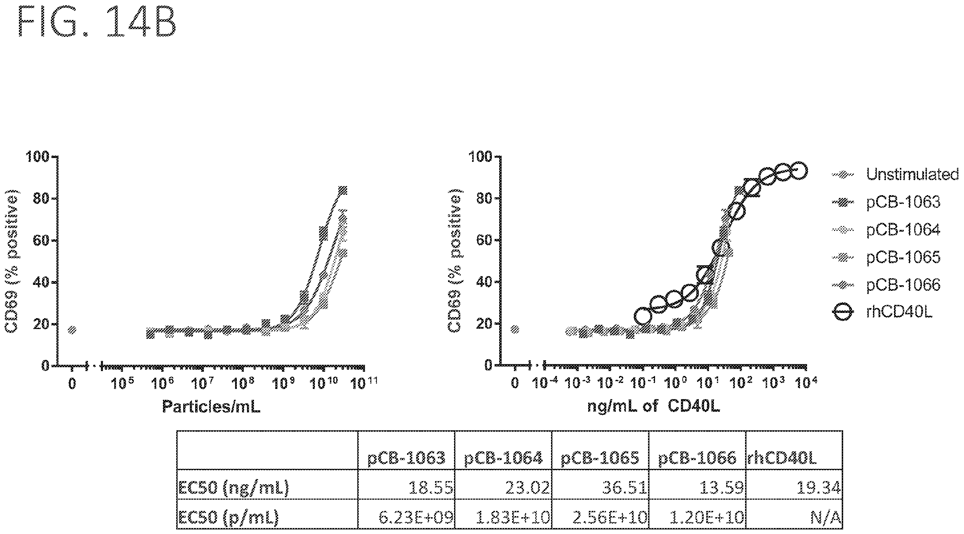

**FIG. 14B** shows the results of human B-cell activation in cultures incubated with CD40L surface expression exosomes fused to N-terminal fragments of MARCKS, MARCKSL1, and BASP1.

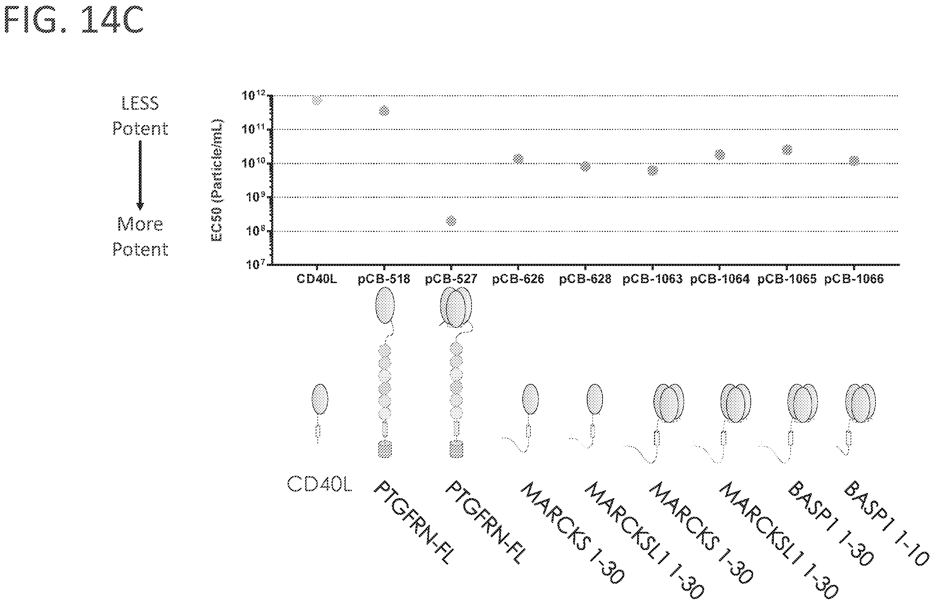

**FIG. 14C** shows a chart of relative potency for different CD40L surface display exosomes when fused to N-terminal sequences of MARCKS, MARCKSL1, BASP1, or full-length PTGFRN. The amino acid numbering, *e.g.,* BASP1 1-30, refers to a fragment that is initially encoded by the gene construct. The first Met is cleaved off during processing.

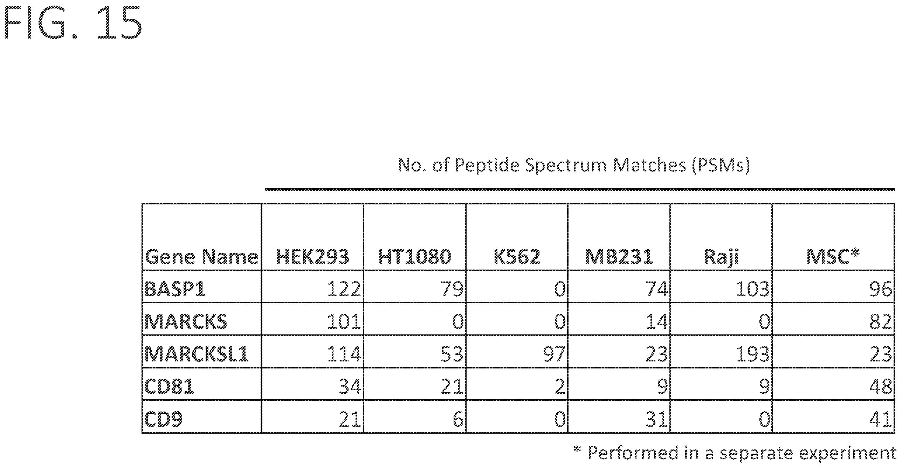

**FIG. 15** shows the number of peptide spectrum matches (PSMs) of luminal proteins (MARCKS, MARCKSL1, and BASP 1) and conventional EV, *e.g.,* exosome proteins (CD81 and CD9) in exosomes purified from various cell lines of different origins (HEK293SF, kidney; HT1080, connective tissue; K562, bone marrow; MDA-MB-231, breast; Raji, lymphoblast; mesenchymal stem cell (MSC), bone marrow).

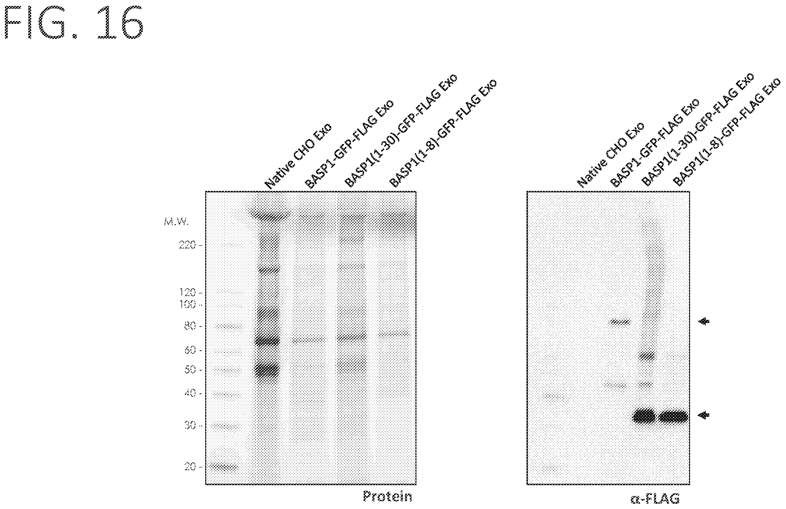

**FIG. 16** shows a protein gel (left) and an anti-FLAG® Western blot (right) of Chinese hamster ovary (CHO) cell-derived exosomes alone, or from cells overexpressing BASP1 or BASP1 N-terminal fragments (1-30 or 1-8) fused to FLAG®-GFP. The amino acid numbering, *e.g.,* BASP1 (1-30), refers to a fragment that is initially encoded by the gene construct. The first Met is cleaved off during processing.

### DETAILED DESCRIPTION

The present disclosure is directed to extracellular vesicles (EVs), *e.g.,* exosomes, comprising at least one biologically active molecule linked to the EV, *e.g.,* exosome, via a scaffold protein, wherein the scaffold protein comprises an N-terminus domain (ND) and an effector domain (ED), wherein the ND and/or the ED are associated with the luminal surface of the EV, *e.g.,* an exosome, wherein the ED comprises (i) a lysine repeat in the ED or (ii) a lysine repeat when combined with the ND, *e.g.,* K at the C-terminus in the ND and K at the N-terminus in the ED, wherein the ND and ED are linked directly, *i.e.,* by a peptide bond. The present disclosure also provides the minimum number of amino acids that are capable of anchoring a payload, *e.g.,* a biologically active molecule, to the luminal surface of the EV, *e.g.,* exosome, e.g., seven to 15, seven to 14, seven to 13, seven to 12, seven to 11, seven to 10, seven to 9, or seven to 8 amino acid fragments. The ND can be associated with the luminal surface of the EV, *e.g.,* an exosome, via myristoylation, whereas the ED can be associated with the luminal surface of the EV, *e.g.,* an exosome, by an ionic interaction, *e.g.,* via attractive electrostatic interaction. Non-limiting examples of the various aspects are shown in the present disclosure.

Before the present disclosure is described in greater detail, it is to be understood that this disclosure is not limited to the particular compositions or process steps described, as such can, of course, vary. As will be apparent to those of skill in the art upon reading this disclosure, each of the individual aspects described and illustrated herein has discrete components and features which can be readily separated from or combined with the features of any of the other several aspects. Any recited method can be carried out in the order of events recited or in any other order which is logically possible.

The headings provided herein are not limitations of the various aspects of the disclosure, which can be defined by reference to the specification as a whole. It is also to be understood that the terminology used herein is for the purpose of describing particular aspects only, and is not intended to be limiting.

Accordingly, the terms defined immediately below are more fully defined by reference to the specification in its entirety.

### I. Definitions

Unless defined otherwise, all technical and scientific terms used herein have the meaning commonly understood by a person skilled in the art to which this disclosure belongs. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure. As used herein, the following terms have the meanings ascribed to them below.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein. It is further noted that the claims can be drafted to exclude any optional element. As such, this statement is intended to serve as antecedent basis for use of such exclusive terminology as "solely," "only" and the like in connection with the recitation of claim elements, or use of a negative limitation.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Units, prefixes, and symbols are denoted in their Systeme International de Unites (SI) accepted form.

Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the disclosure.

Thus, ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

Unless otherwise indicated, reference to a compound that has one or more stereocenters intends each stereoisomer, and all combinations of stereoisomers, thereof.

Where a value is explicitly recited, it is to be understood that values which are about the same quantity or amount as the recited value are also within the scope of the disclosure. Where a combination is disclosed, each subcombination of the elements of that combination is also specifically disclosed and is within the scope of the disclosure. Conversely, where different elements or groups of elements are individually disclosed, combinations thereof are also disclosed. Where any element of a disclosure is disclosed as having a plurality of alternatives, examples of that disclosure in which each alternative is excluded singly or in any combination with the other alternatives are also hereby disclosed; more than one element of a disclosure can have such exclusions, and all combinations of elements having such exclusions are hereby disclosed.

Nucleotides are referred to by their commonly accepted single-letter codes. Unless otherwise indicated, nucleotide sequences are written left to right in 5' to 3' orientation. Nucleotides are referred to herein by their commonly known one-letter symbols recommended by the IUPAC-IUB Biochemical Nomenclature Commission. Accordingly, A represents adenine, C represents cytosine, G represents guanine, T represents thymine, U represents uracil.

Amino acid sequences are written left to right in amino to carboxy orientation. Amino acids are referred to herein by either their commonly known three letter symbols or by the one-letter symbols recommended by the IUPAC-IUB Biochemical Nomenclature Commission.

The term **"about"** is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, *e.g.,* 10 percent, up or down (higher or lower).

As used herein, the term **"approximately,"** as applied to one or more values of interest, refers to a value that is similar to a stated reference value. In certain aspects, the term "approximately" refers to a range of values that fall within 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The terms **"administration," "administering,"** and grammatical variants thereof refer to introducing a composition, such as an EV *(e.g.,* exosome) of the present disclosure, into a subject via a pharmaceutically acceptable route. The introduction of a composition, such as an EV *(e.g.,* exosome) of the present disclosure, into a subject is by any suitable route, including intratumorally, orally, pulmonarily, intranasally, parenterally (intravenously, intra-arterially, intramuscularly, intraperitoneally, or subcutaneously), rectally, intralymphatically, intrathecally, periocularly or topically. Administration includes self-administration and the administration by another. A suitable route of administration allows the composition or the agent to perform its intended function. For example, if a suitable route is intravenous, the composition is administered by introducing the composition or agent into a vein of the subject.

As used herein, the term **"agonist"** refers to a molecule that binds to a receptor and activates the receptor to produce a biological response. Receptors can be activated by either an endogenous or an exogenous agonist. Non-limiting examples of endogenous agonist include hormones, neurotransmitters, and cyclic dinucleotides. Non-limiting examples of exogenous agonist include drugs, small molecules, and cyclic dinucleotides. The agonist can be a full, partial, or inverse agonist.

As used herein, the term **"antagonist"** refers to a molecule that blocks or dampens an agonist mediated response rather than provoking a biological response itself upon bind to a receptor. Many antagonists achieve their potency by competing with endogenous ligands or substrates at structurally defined binding sites on the receptors. Non-limiting examples of antagonists include alpha blockers, beta-blocker, and calcium channel blockers. The antagonist can be a competitive, non-competitive, or uncompetitive antagonist.

As used herein, the term **"extracellular vesicle"** or **"EV"** refers to a cell-derived vesicle comprising a membrane that encloses an internal space (lumen). Extracellular vesicles comprise all membrane-bound vesicles that have a smaller diameter than the cell from which they are derived. Generally extracellular vesicles range in diameter from 20 nm to 1000 nm, and can comprise various payloads either within the internal space *(i.e.,* the lumen), displayed on the external surface or luminal surface of the extracellular vesicle, and/or spanning the membrane. The payload can comprise, *e.g.,* nucleic acids, proteins, carbohydrates, lipids, small molecules, and/or combinations thereof. By way of example and without limitation, extracellular vesicles include apoptotic bodies, fragments of cells, vesicles derived from cells by direct or indirect manipulation *(e.g.,* by serial extrusion or treatment with alkaline solutions), vesiculated organelles, and vesicles produced by living cells *(e.g.,* by direct plasma membrane budding or fusion of the late endosome with the plasma membrane). Extracellular vesicles can be derived from a living or dead organism, explanted tissues or organs, and/or cultured cells. In some aspects, the EV comprises a scaffold protein disclosed herein.

As used herein the term **"exosome"** refers to a cell-derived small (between 20-300 nm in diameter, more preferably 40-200 nm in diameter) extracellular vesicle (EV) comprising a membrane that encloses an internal space (lumen), and which, in some aspects, is generated from a cell *(e.g.,* a producer cell), for example, by direct plasma membrane budding or by fusion of the late endosome with the plasma membrane. The exosome is a species of extracellular vesicle (EV). In some aspects, the exosome comprises lipid or fatty acid and polypeptide and optionally comprises a payload *(e.g.,* a biologically active molecule such as a therapeutic agent), a receiver *(e.g.,* a targeting moiety), a polynucleotide *(e.g.,* a nucleic acid, RNA, or DNA), a sugar *(e.g.,* a simple sugar, polysaccharide, or glycan), or other molecules. The exosome can be derived from a producer cell, and isolated from the producer cell based on its size, density, biochemical parameters, or a combination thereof. In some aspects, the exosome comprises a scaffold protein of the present disclosure. In some aspects, the exosomes of the present disclosure are produced by cells that express one or more transgene products.

As used herein, the term **"exosome lumen protein"** refers to a scaffold protein, *i.e.,* a protein attached or associated to the luminal surface of EV, *e.g.,* exosome, such as MARCKS, MARKSL1, BASP1, or any functional fragment thereof, any variant thereof, any derivative thereof, or any combination thereof, and which is suitable for use as a scaffold to target a payload, *e.g.,* a biologically active molecule *(e.g.,* therapeutics proteins) to the luminal surface of EVs, *e.g.,* exosomes.

As used herein, the term **"nanovesicle"** refers to a cell-derived small (between 20-250 nm in diameter, *e.g.,* 30-150 nm in diameter) vesicle comprising a membrane that encloses an internal space, and which is generated from a cell *(e.g.,* producer cell) by direct or indirect manipulation such that the nanovesicle would not be produced by the producer cell without such manipulation. Appropriate manipulations of the producer cell to produce the nanovesicles include but are not limited to serial extrusion, treatment with alkaline solutions, sonication, or combinations thereof. The production of nanovesicles can, in some instances, result in the destruction of the producer cell. In some aspects, populations of nanovesicles described herein are substantially free of vesicles that are derived from producer cells by way of direct budding from the plasma membrane or fusion of the late endosome with the plasma membrane. In some aspects, the nanovesicle comprises lipid or fatty acid and polypeptide, and optionally comprises a payload *(e.g.,* a therapeutic agent), a receiver *(e.g.,* a targeting moiety), a polynucleotide *(e.g.,* a nucleic acid, RNA, or DNA), a sugar *(e.g.,* a simple sugar, polysaccharide, or glycan) or other molecules. The nanovesicle, once it is derived from a producer cell according to the manipulation, may be isolated from the producer cell based on its size, density, biochemical parameters, or a combination thereof. In some aspects, the nanovesicle can comprise a scaffold protein disclosed herein.

As used herein the term **"lumen-engineered EV"** refers to an EV, *e.g.,* an exosome with the luminal surface of the membrane or the lumen of the EV, *e.g.,* exosome, modified in its composition so that the luminal surface or the lumen of the engineered EV, *e.g.,* exosome, is different from that of the EV, *e.g.,* exosome, prior to the modification or of the naturally occurring EV, *e.g.,* exosome.

The engineering can be directly in the lumen *(i.e.,* the void within the EV) or in the membrane of the EV *(e.g.,* exosome) in particular the luminal surface of the EV, so that the lumen and/or the luminal surface of the EV, *e.g.,* exosome, is changed. For example, the membrane is modified in its composition of a protein, a lipid, a small molecule, a carbohydrate, *etc.* so that the luminal surface of the EV, *e.g.,* exosome, is modified. Similarly, the contents of the lumen can be modified. The composition can be changed by a chemical, a physical, or a biological method or by being produced from a cell previously modified by a chemical, a physical, or a biological method. Specifically, the composition can be changed by genetic engineering or by being produced from a cell previously modified by genetic engineering. In some aspects, a lumen-engineered EV, *e.g.,* lumen-engineered exosome, comprises an exogenous protein *(i.e.,* a protein that the EV, *e.g.,* exosome, does not naturally express) or a fragment or variant thereof that can be exposed in the luminal surface or lumen of the EV, *e.g.,* exosome, or can be an anchoring point (attachment) for a moiety exposed on the inner layer of the EV, *e.g.,* exosome. In other aspects, a lumen-engineered EV, *e.g.,* a lumen-engineered exosome, comprises a higher expression of a natural EV, *e.g.,* exosome, protein *(e.g.,* a scaffold protein) or a fragment or variant thereof that can be exposed to the lumen of the EV, *e.g.,* exosome, or can be an anchoring point (attachment) for a moiety exposed on the luminal surface of the EV, *e.g.,* exosome.

As used herein the term **"surface-engineered EV"** refers to an EV with the external surface of the EV modified in its composition so that the external surface of the engineered EV is different from that of the EV prior to the modification or of the naturally occurring EV.

As used herein the term **"surface-engineered exosome"** refers to an exosome with the external surface of the exosome modified in its composition so that the external surface of the engineered exosome is different from that of the exosome prior to the modification or of the naturally occurring exosome.

The term **"modified,"** when used in the context of EVs, *e.g.,* exosomes, described herein, refers to an alteration or engineering of an EV, *e.g.,* exosome and/or its producer cell, such that the modified EV, *e.g.,* exosome, is different from a naturally-occurring EV, *e.g.,* exosome. In some aspects, a modified EV, *e.g.,* exosome, described herein comprises a membrane that differs in composition of a protein, a lipid, a small molecular, a carbohydrate, *etc.* compared to the membrane of a naturally-occurring EV, *e.g.,* exosome. *E.g.,* the membrane comprises higher density or number of natural EV, *e.g.,* exosome, proteins and/or membrane comprises proteins that are not naturally found in EV, *e.g.,* exosomes. In certain aspects, such modifications to the membrane change the exterior surface of the EV, *e.g.,* exosome *(e.g.,* surface-engineered EVs and exosomes described herein). In certain aspects, such modifications to the membrane change the luminal surface of the EV, *.e.g.,* exosome *(e.g.,* lumen-engineered EV and exosomes described herein).

For example, the lumen is modified in its composition of a protein, a lipid, a small molecule, a carbohydrate, *etc.* The composition can be changed by a chemical, a physical, or a biological method or by being produced from a cell previously modified by a chemical, a physical, or a biological method. Specifically, the composition can be changed by a genetic engineering or by being produced from a cell previously modified by genetic engineering.

As used herein the term **"modification"** of a protein, *e.g.,* as in "modified protein" or "protein modification" or grammatical variants thereof, refers to a protein having at least 15% identity to the non-mutant amino acid sequence of the protein. A modification of a protein includes a fragment or a variant of the protein. A modification of a protein can further include chemical, or physical modification to a fragment or a variant of the protein. In some aspects, a modified protein retains at least one physiological function of the non-modified protein.

As used herein the term "a **fragment"** of a protein *(e.g.,* a biologically active molecule such as a therapeutic protein, or a scaffold protein disclosed herein) refers to a protein that is shorter than the naturally-occurring sequence, *e.g.,* N- and/or C-terminally deleted in comparison to the naturally occurring protein.

As used herein, the term **"functional fragment"** refers to a protein fragment that retains protein function. Accordingly, in some aspects, a functional fragment of a scaffold protein disclosed herein such as MARCKS, MARCKSL1, or BASP1 retains the ability to anchor a biologically active molecule on the luminal surface of the EV, *e.g.,* exosome.

Whether a fragment is a functional fragment in that sense can be assessed by any art known methods to determine the protein content of EVs, *e.g.,* exosomes including Western Blots, FACS analysis and fusions of the fragments with autofluorescent proteins like, *e.g.,* GFP. In a particular aspect, the fragment of MARCKS, MARCKSL1, or BASP1 retains, *e.g.,* at least about 50%, at least about 55%, at least about 60%, at least about 65%, at least about 70%, at least about 75%, at least about 80%, at least about 85%, at least about 90%, at least about 95%, at least about 99%, or at least about 100% of the ability of the naturally occurring MARCKS, MARCKSL1, or BASP1 to anchor a payload, *e.g.,* a biologically active molecule, on the luminal or on the external surface of the EV, *e.g.,* exosome. In a particular aspect, the ability of a variant of MARCKS, MARCKSL1, BASP1 or of a fragment of MARCKS, MARCKSL1, or BASP1 is at least about 70%, at least about 75%, at least about 80%, at least about 85%, at least about 90%, at least about 95%, at least about 99%, or at least about 100% of the ability of MARCKS, MARCKSL1, and BASP1, respectively, to anchor a payload, *e.g.,* biologically active molecule, on the luminal or on the external surface of the EV, *e.g.,* exosome. This ability can be assessed, e.g. by fluorescently labeled variants, in the assays described in the experimental section.

The term **"derivative"** as used herein refers to an EV, *e.g.,* exosome, component *(e.g.,* a protein or a lipid), a scaffold protein of the present disclosure, or to a payload, *e.g.,* a biologically active molecule *(e.g.,* a polypeptide, polynucleotide, lipid, carbohydrate, antibody or fragment thereof) that has been chemically or enzymatically modified.

As used herein the term **"variant"** of a protein refers to a protein that shares a certain structural *(e.g.,* amino acid sequence identity) and functional identities with another protein upon comparison *(e.g.,* by sequence alignment) by a method known in the art. For example, a variant of a protein can include a substitution, insertion, deletion, frameshift or rearrangement in another protein. In some aspects, a variant of a protein retains at least one physiological function of the non-variant protein.

In a particular aspect, the variant is a variant protein having at least 70% identity to the full length, mature MARCKS, MARCKSL1, BASP1 or a fragment of MARCKS, MARCKSL1, or BASP1.

In some aspects, variants or variants of fragments of MARCKS share at least about 70%, at least about 75%, at least about 80%, at least about 85%, at least about 90%, at least about 95%, or at least about 99% sequence identity with MARCKS according to SEQ ID NO: 1 or with a functional fragment thereof, as determined, *e.g.,* by pairwise alignment using the Needleman-Wunsch algorithm.

In some aspects, variants or variants of fragments of MARCKSL1 share at least 70%, at least about 75%, at least about 80%, at least about 85%, at least about 90%, at least about 95%, or at least about 99% sequence identity with MARCKSL1 according to SEQ ID NO: 2 or with a functional fragment thereof, as determined, *e.g.,* by pairwise alignment using the Needleman-Wunsch algorithm.

In some aspects, variants or variants of fragments of BASP1 share at least about 70%, at least about 75%, at least about 80%, at least about 85%, at least about 90%, at least about 95%, or at least about 99% sequence identity with BASP1 according to SEQ ID NO: 3 or with a functional fragment thereof, as determined, *e.g.,* by pairwise alignment using the Needleman-Wunsch algorithm..

In some aspects, a variant of a scaffold protein *(e.g.,* MARCKS, MARCKSL1, or BASP1), a functional fragment of a scaffold protein, or a variant of a functional fragment of a scaffold protein, is a derivative.

In each of above cases, the variant or variant of a fragment of a scaffold protein *(e.g.,* a variant of MARCKS, MARCKSL1, BASP1 or a fragment of MARCKS, MARCKSL1, or BASP1) retains the ability to anchor payload, *e.g.,* a biologically active molecule, on the luminal or on the external surface of the EV, *e.g.,* exosome.

Recitation of any protein provided herein encompasses a functional variant of the protein. The term **"functional variant"** of a protein refers to a variant of the protein that retains the ability to anchor a biologically active molecule on the luminal or on the external surface of the EV, *e.g.,* exosome. In a particular aspect the ability of the functional variant of MARCKS, MARCKSL1, BASP1 or of a fragment of MARCKS, MARCKSL1, or BASP1 is at least about 70%, at least about 80%, at least about 85%, at least about 90%, at least about 95% or at least about 99% of the ability of MARCKS, MARCKSL1, and BASP1, respectively, to anchor a payload, *e.g.,* a biologically active molecule, on the luminal or on the external surface of the EV, *e.g.,* exosome.

Naturally occurring variants are called **"allelic variants,"** and refer to one of several alternate forms of a gene occupying a given locus on a chromosome of an organism (Genes II, Lewin, B., ed., John Wiley & Sons, New York (1985)). These allelic variants can vary at either the polynucleotide and/or polypeptide level and are included in the present disclosure. Alternatively, non-naturally occurring variants can be produced by mutagenesis techniques or by direct synthesis.

Using known methods of protein engineering and recombinant DNA technology, variants can be generated to improve or alter the characteristics of the polypeptides. For instance, one or more amino acids can be deleted from the N-terminus or C-terminus of the secreted protein without substantial loss of biological function. Ron et al., J. Biol. Chem. 268: 2984-2988 (1993), reported variant KGF proteins having heparin binding activity even after deleting 3, 8, or 27 amino-terminal amino acid residues. Similarly, interferon gamma exhibited up to ten times higher activity after deleting 8-10 amino acid residues from the carboxy terminus of this protein. (Dobeli et al., J. Biotechnology 7:199-216 (1988))

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See Abstract.) In fact, only 23 unique amino acid sequences, out of more than 3,500 nucleotide sequences examined, produced a protein that significantly differed in activity from wild-type.

As stated above, variants or derivatives include, *e.g.,* modified polypeptides. In some aspects, variants or derivatives of, *e.g.,* polypeptides, polynucleotides, lipids, glycoproteins, are the result of chemical modification and/or endogenous modification. In some aspects, variants or derivatives are the result *of in vivo* modification. In some aspects, variants or derivatives are the result of *in vitro* modification. In yet other aspects, variant or derivatives are the result of intracellular modification in producer cells.

Modifications present in variants and derivatives include, *e.g.,* acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation (Mei et al., Blood 116:270-79 (2010)), proteolytic processing, phosphorylation, prenylation, racemization, selenoylation, sulfation, transfer-RNA mediated addition of amino acids to proteins such as arginylation, and ubiquitination.

The term **"amino acid substitution"** refers to replacing an amino acid residue present in a parent or reference sequence *(e.g.,* a wild type sequence) with another amino acid residue. An amino acid can be substituted in a parent or reference sequence *(e.g.,* a wild type polypeptide sequence), for example, via chemical peptide synthesis or through recombinant methods known in the art. Accordingly, a reference to a "substitution at position X" refers to the substitution of an amino acid present at position X with an alternative amino acid residue. In some aspects, substitution patterns can be described according to the schema AnY, wherein A is the single letter code corresponding to the amino acid naturally or originally present at position n, and Y is the substituting amino acid residue. In other aspects, substitution patterns can be described according to the schema An(YZ), wherein A is the single letter code corresponding to the amino acid residue substituting the amino acid naturally or originally present at position n, and Y and Z are alternative substituting amino acid residues that can replace A.

As used herein, the term **"antibody"** encompasses an immunoglobulin whether natural or partly or wholly synthetically produced, and fragments thereof. The term also covers any protein having a binding domain that is homologous to an immunoglobulin binding domain. "Antibody" further includes a polypeptide comprising a framework region from an immunoglobulin gene or fragments thereof that specifically binds and recognizes an antigen. Use of the term antibody is meant to include whole antibodies, polyclonal, monoclonal and recombinant antibodies, fragments thereof, and further includes single-chain antibodies, humanized antibodies, murine antibodies, chimeric, mouse-human, mouse-primate, primate-human monoclonal antibodies, anti-idiotype antibodies, antibody fragments, such as, e.g., scFv, (scFv)₂, Fab, Fab', and F(ab')₂, F(ab1)₂, Fv, dAb, and Fd fragments, diabodies, and antibody-related polypeptides. Antibody includes bispecific antibodies and multispecific antibodies so long as they exhibit the desired biological activity or function. In some aspects of the present disclosure, the payload is a biologically active molecule comprising an antibody or an antigen binding fragment thereof. In some aspects, the antibody (*e.g.,* a biologically active molecule of the present disclosure) is a nanobody.

The terms **"antibody-drug conjugate"** and **"ADC"** are used interchangeably and refer to an antibody linked, e.g., covalently, to a therapeutic agent (sometimes referred to herein as agent, drug, or active pharmaceutical ingredient) or agents. In some aspects of the present disclosure, the payload is a biologically active molecule comprising an antibody-drug conjugate.

A **"conservative amino acid substitution"** is one in which the amino acid residue is replaced with an amino acid residue having a similar side chain. Families of amino acid residues having similar side chains have been defined in the art, including basic side chains *(e.g.,* lysine, arginine, histidine), acidic side chains *(e.g.,* aspartic acid, glutamic acid), uncharged polar side chains *(e.g.,* glycine, asparagine, glutamine, serine, threonine, tyrosine, cysteine), nonpolar side chains *(e.g.,* alanine, valine, leucine, isoleucine, proline, phenylalanine, methionine, tryptophan), beta-branched side chains *(e.g.,* threonine, valine, isoleucine) and aromatic side chains *(e.g.,* tyrosine, phenylalanine, tryptophan, histidine). Thus, if an amino acid in a polypeptide is replaced with another amino acid from the same side chain family, the substitution is considered to be conservative. In another aspect, a string of amino acids can be conservatively replaced with a structurally similar string that differs in order and/or composition of side chain family members.

As used herein, the term **"conserved"** refers to nucleotides or amino acid residues of a polynucleotide sequence or polypeptide sequence, respectively, that are those that occur unaltered in the same position of two or more sequences being compared. Nucleotides or amino acids that are relatively conserved are those that are conserved amongst more related sequences than nucleotides or amino acids appearing elsewhere in the sequences.

In some aspects, two or more sequences are said to be **"completely conserved"** or **"identical"** if they are 100% identical to one another. In some aspects, two or more sequences are said to be **"highly conserved"** if they are at least 70% identical, at least 80% identical, at least 90% identical, or at least 95% identical to one another.

In some aspects, two or more sequences are said to be **"highly conserved"** if they are about 70% identical, about 80% identical, about 90% identical, about 95%, about 98%, or about 99% identical to one another. In some aspects, two or more sequences are said to be **"conserved"** if they are at least 30% identical, at least 40% identical, at least 50% identical, at least 60% identical, at least 70% identical, at least 80% identical, at least 90% identical, or at least 95% identical to one another. In some aspects, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to one another. Conservation of sequence may apply to the entire length of a polynucleotide or polypeptide or may apply to a portion, region or feature thereof.

As used herein, the term **"homology"** refers to the overall relatedness between polymeric molecules, *e.g.* between nucleic acid molecules *(e.g.* DNA molecules and/or RNA molecules) and/or between polypeptide molecules. Generally, the term "homology" implies an evolutionary relationship between two molecules. Thus, two molecules that are homologous will have a common evolutionary ancestor. In the context of the present disclosure, the term homology encompasses both to identity and similarity.

In some aspects, polymeric molecules are considered to be **"homologous"** to one another if at least 25%, 30%, 35%, 40%, 45%, 50%, 55%, 60%, 65%, 70%, 75%, 80%, 85%, 90%, 95%, or 99% of the monomers in the molecule are identical (exactly the same monomer) or are similar (conservative substitutions). The term "homologous" necessarily refers to a comparison between at least two sequences (polynucleotide or polypeptide sequences).

In the context of the present disclosure, substitutions (even when they are referred to as amino acid substitution) are conducted at the nucleic acid level, *i.e.,* substituting an amino acid residue with an alternative amino acid residue is conducted by substituting the codon encoding the first amino acid with a codon encoding the second amino acid.

As used herein, the term **"identity"** refers to the overall monomer conservation between polymeric molecules, *e.g.,* between polypeptide molecules or polynucleotide molecules *(e.g.* DNA molecules and/or RNA molecules). The term "identical" without any additional qualifiers, *e.g.,* protein A is identical to protein B, implies the sequences are 100% identical (100% sequence identity). Describing two sequences as, *e.g.,* "70% identical," is equivalent to describing them as having, *e.g.,* "70% sequence identity."

Calculation of the percent identity of two polypeptide sequences, for example, can be performed by aligning the two sequences for optimal comparison purposes *(e.g.,* gaps can be introduced in one or both of a first and a second polypeptide sequences for optimal alignment and non-identical sequences can be disregarded for comparison purposes). In certain aspects, the length of a sequence aligned for comparison purposes is at least 30%, at least 40%, at least 50%, at least 60%, at least 70%, at least 80%, at least 90%, at least 95%, or 100% of the length of the reference sequence. The amino acids at corresponding amino acid positions are then compared.

When a position in the first sequence is occupied by the same amino acid as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm.

Methods of alignment of sequences for comparison are well-known in the art. Various programs and alignment algorithms are described in: Smith and Waterman, Adv. Appl. Math. 2: 482 (1981); Needleman and Wunsch, J. Mol. Bio. 48: 443 (1970); Pearson and Lipman, Methods in Mol. Biol. 24: 307-31 (1988); Higgins and Sharp, Gene 73: 15 237-44 (1988); Higgins and Sharp, CABIOS 5: 151-3 (1989) Corpet et al., Nuc. Acids Res. 16: 10881-90 (1988); Huang et al., Comp. Appl. BioSci. 8: 155-65 (1992); and Pearson et al., Meth. Mol. Biol. 24: 307-31 (1994). The NCBI Basic Local Alignment Search Tool (BLAST) [Altschul 20 et al., J. Mol. Biol. 215: 403-10 (1990) J is available from several sources, including the National Center for Biological Information (NBCl, Bethesda, Md.) and on the Internet, for use in connection with the sequence analysis programs blastp, blastn, blastx, tblastn and tblastx. BLAST and a description of how to determine sequence identify using the program can be accessed at the official website of NCBI (National Center for Biotechnology Information) under NIH (National Institute of Health).

Other suitable programs are, *e.g.,* Needle, Stretcher, Water, or Matcher, part of the EMBOSS suite of bioinformatics programs and also available from the European Bioinformatics Institute (EBI) at www.ebi.ac.uk/Tools/psa. Sequence alignments can be conducted using methods known in the art such as MAFFT, Clustal (ClustalW, Clustal X or Clustal Omega), MUSCLE, etc.

Different regions within a single polynucleotide or polypeptide target sequence that aligns with a polynucleotide or polypeptide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

In certain aspects, the percentage identity (%ID) or of a first amino acid sequence (or nucleic acid sequence) to a second amino acid sequence (or nucleic acid sequence) is calculated as %ID = 100 x (Y/Z), where Y is the number of amino acid residues (or nucleobases) scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be higher than the percent identity of the second sequence to the first sequence.

One skilled in the art will appreciate that the generation of a sequence alignment for the calculation of a percent sequence identity is not limited to binary sequence-sequence comparisons exclusively driven by primary sequence data. It will also be appreciated that sequence alignments can be generated by integrating sequence data with data from heterogeneous sources such as structural data (e.g., crystallographic protein structures), functional data (e.g., location of mutations), or phylogenetic data. A suitable program that integrates heterogeneous data to generate a multiple sequence alignment is T-Coffee, available at www.tcoffee.org, and alternatively available, e.g., from the EBI. It will also be appreciated that the final alignment used to calculate percent sequence identity can be curated either automatically or manually.

As used herein, the term **"similarity"** refers to the overall relatedness between polymeric molecules, *e.g.* between polynucleotide molecules *(e.g.* DNA molecules and/or RNA molecules) and/or between polypeptide molecules. Calculation of percent similarity of polymeric molecules to one another can be performed in the same manner as a calculation of percent identity, except that calculation of percent similarity takes into account conservative substitutions as is understood in the art. It is understood that percentage of similarity is contingent on the comparison scale used, *i.e.,* whether the amino acids are compared, *e.g.,* according to their evolutionary proximity, charge, volume, flexibility, polarity, hydrophobicity, aromaticity, isoelectric point, antigenicity, or combinations thereof.

As used herein the term **"producer cell"** refers to a cell used for generating an EV, *e.g.,* an exosome. A producer cell can be a cell cultured *in vitro,* or a cell *in vivo.* A producer cell includes, but not limited to, a cell known to be effective in generating EVs, *e.g.,* exosomes, e.g., HEK293 cells, Chinese hamster ovary (CHO) cells, and mesenchymal stem cells (MSCs), BJ human foreskin fibroblast cells, fHDF fibroblast cells, AGE.HN® neuronal precursor cells, CAP® amniocyte cells, adipose mesenchymal stem cells, RPTEC/TERT1 cells. In certain aspects, a producer cell is not an antigen-presenting cell. In some aspects, a producer cell is not a dendritic cell, a B cell, a mast cell, a macrophage, a neutrophil, Kupffer-Browicz cell, cell derived from any of these cells, or any combination thereof.

As used herein, the terms **"isolate," "isolated,"** and **"isolating"** or **"purify," "purified,"** and **"purifying"** as well as **"extracted"** and **"extracting"** and grammatical variants thereof are used interchangeably and refer to the state of a preparation *(e.g.,* a plurality of known or unknown amount and/or concentration) of desired EVs, *e.g.,* exosomes, that have undergone one or more processes of purification, *e.g.,* a selection or an enrichment of the desired EV, *e.g.,* exosome, preparation. In some embodiments, isolating or purifying as used herein is the process of removing, partially removing *(e.g.,* a fraction) of the EVs, *e.g.,* exosomes, from a sample containing producer cells. In some embodiment, an isolated EV, *e.g.,* exosome, composition has no detectable undesired activity or, alternatively, the level or amount of the undesired activity is at or below an acceptable level or amount.

In other embodiments, an isolated EV, *e.g.,* exosome, composition has an amount and/or concentration of desired EVs, *e.g.,* exosomes, at or above an acceptable amount and/or concentration. In other aspects, the isolated EV, *e.g.,* exosome, composition is enriched as compared to the starting material *(e.g.,* producer cell preparations) from which the composition is obtained. This enrichment can be by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, at least about 99%, at least about 99.9%, at least about 99.99%, at least about 99.999%, at least about 99.9999%, or greater than about 99.9999% as compared to the starting material.

In some aspects, isolated EV, *e.g.,* exosome, preparations are substantially free of residual biological products *(e.g.,* contaminants). In some aspects, the isolated EV, *e.g.,* exosome, preparations are about 100% free, at least about 99% free, at least about 98% free, at least about 97% free, at least about 96% free, at least about 95% free, at least about 94% free, at least about 93% free, at least about 92% free, at least about 91% free, or at least about 90% free of any contaminating biological matter. Residual biological products can include abiotic materials (including chemicals) or unwanted nucleic acids, proteins, lipids, or metabolites. In certain aspects, the isolated EV, *e.g.,* exosome, preparations are about 100% free, at least about 99% free, at least about 98% free, at least about 97% free, at least about 96% free, at least about 95% free, at least about 94% free, at least about 93% free, at least about 92% free, at least about 91% free, or at least about 90% free of any macromolecules, e.g., of any nucleic acids, proteins, lipids, and/or carbohydrates. Substantially free of residual biological products can also mean that the EV, *e.g.,* exosome, composition contains no detectable producer cells and that only EVs, *e.g.,* exosomes, are detectable.

The term **"excipient"** or **"carrier"** refers to an inert substance added to a pharmaceutical composition to further facilitate administration of a compound. The terms **"pharmaceutically-acceptable carrier," "pharmaceutically-acceptable excipient,"** and grammatical variants thereof encompass any of the agents approved by a regulatory agency of the U.S. Federal government or listed in the U.S. Pharmacopeia for use in animals, including humans, as well as any carrier or diluent that does not cause significant irritation to a subject and does not abrogate the biological activity and properties of the administered compound. Included are excipients and carriers that are useful in preparing a pharmaceutical composition and are generally safe, non-toxic, and desirable.

As used herein, the term **"payload"** refers to any molecule that can be attached to a scaffold of the present disclosure and subsequently anchored to the membrane of an EV, *e.g.,* an exosome, of the present disclosure. In some embodiments, the payload is attached to the luminal surface of the EV, *e.g.,* exosome, membrane. The term payload encompasses biologically active molecules, *e.g.,* molecules that can have a therapeutic and/or prophylactic effect, as well as diagnostic molecules. Accordingly, the term payload also encompasses detectable moieties such as radionuclides, fluorescent molecules, contrast agents, tags, or molecular entities that can be recognized by a detectable moiety, *e.g.,* ligands or tags.

As used herein, the term payload is equivalent to and interchangeable with the term "cargo." Accordingly "cargo protein" or "cargo peptide" refer to specific types of payload molecules (protein and peptides, *respectively)* attached to a scaffold of the present disclosure.

The term **"biologically active molecule"** as use herein refers to any molecule that can be attached to an EV, *e.g.,* exosome, via a scaffold of the present disclosure wherein the molecule can have a therapeutic or prophylactic effect in a subject in need thereof, or affect the homeostasis of a cell or tissue in a subject. Non-limiting examples of biologically active molecules that can be introduced into an EV, *e.g.,* exosome, and/or a producer cell include, *e.g.,* therapeutic agents such as nucleotides *(e.g.,* nucleotides comprising a detectable moiety or a toxin or that disrupt transcription), nucleic acids *(e.g.,* DNA or mRNA molecules that encode a polypeptide such as an enzyme, or RNA molecules that have regulatory function such as miRNA, dsDNA, IncRNA, and siRNA), amino acids *(e.g.,* amino acids comprising a detectable moiety or a toxin or that disrupt translation), polypeptides *(e.g.,* enzymes or antibodies), lipids, carbohydrates, viruses and viral particles *(e.g.,* adeno-associated viruses and viral particles, retroviruses, adenoviruses, etc.) and small molecules *(e.g.,* small molecule drugs and toxins), including small molecule STING agonists including cyclic dinucleotides such as ML-RR S2 and 3'-3' cAIMPdFSH). In certain aspects, a payload comprises an antigen. In certain aspects, the biologically active molecule, *e.g.,* the therapeutic agent, *e.g.,* therapeutic agent to human, retains a biological function, *e.g.,* a therapeutic function, when fused to a scaffold protein disclosed herein. In some aspects, the biologically active molecule can have at least 5 amino acids, at least 6 amino acids, at least 7 amino acids, at least 8 amino acids, at least 9 amino acids, at least 10 amino acids, at least 15 amino acids, at least 20 amino acids, at least 25 amino acids, at least 30 amino acids, at least 35 amino acids, at least 40 amino acids, at least 45 amino acids, at least 50 amino acids, at least 55 amino acids, at least 60 amino acids, at least 65 amino acids, at least 70 amino acids, at least 75 amino acids, at least 100 amino acids, at least 150 amino acids, at least 200 amino acids, at least 250 amino acids, at least 300 amino acids, at least 350 amino acids, at least 400 amino acids, or at least 500 amino acids. In some aspects, the biologically active molecule can have at least 5 amino acids. In some aspects, the biologically active molecule can have at least 10 amino acids. In some aspects, the biologically active molecule can have at least 50 amino acids. In some aspects, the biologically active molecule can have at least 100 amino acids. In some aspects, the biologically active molecule can have at least 5 amino acids, *e.g.,* 5-500, 5-450, 5-400, 5-350, 5-300, 5-250, 5-200, 5-150, 5-100, 5-90, 5-80, 5-70, 5-60, 5-50, 5-40, 5-30, 5-20, 5-15, or 5-10 amino acids. In some aspects, the biologically active molecule can have at least 10 amino acids, *e.g.,* 10-500, 10-450, 10-400, 10-350, 10-300, 10-250, 10-200, 10-150, 10-100, 10-90, 10-80, 10-70, 10-60, 10-50, 10-40, 10-30, 10-20, or 10-15 amino acids. In some aspects, the biologically active molecule can have at least 50 amino acids, *e.g.,* 50-500, 50-450, 50-400, 50-350, 50-300, 50-250, 50-200, 50-150, 50-100, 50-90, 50-80, 50-50, or 50-60 amino acids. In some aspects, the biologically active molecule can have at least 100 amino acids, *e.g.,* 100-500, 100-450, 100-400, 100-350, 100-300, 100-250, 100-200, or 100-150 amino acids. In other aspects, the biologically active molecule has at least two, at least three, at least four, at least five, at least six, at least seven, at least eight, at least nine, at least ten, at least 11, at least 12, at least 15, at least 20, at least 25, at least 30, at least 35, at least 40, at least 45, at least 50, at least 60, at least 70, at least 80, at least 90, at least 100, at least 110, at least 120, at least 130, at least 140, at least 150, at least 160, at least 170, at least 180, at least 190, at least 200, at least 300, at least 400, or at least 500 nucleotides. In other aspects, the biologically active molecule has at least two nucleotides, e.g., 2-1000, 2-900, 2-500, 2-300, or 2-50. In other aspects, the biologically active molecule has at least 10 nucleotides, e.g., 10-1000, 10-900, 10-500, 10-300, or 10-50. In other aspects, the biologically active molecule has at least 15 nucleotides, e.g., 15-1000, 15-900, 15-500, 15-300, or 15-50. In other aspects, the biologically active molecule has at least 20 nucleotides, e.g., 20-1000, 20-900, 20-500, 20-300, or 20-50. In other aspects, the biologically active molecule has at least 50 nucleotides, e.g., 50-1000, 50-900, 50-500, 50-300, or 50-100. In other aspects, the biologically active molecule has at least 100 nucleotides, e.g., 100-10000, 100-9000, 100-5005, 100-3000, or 100-500. In other aspects, the biologically active molecule has at least 200 nucleotides, e.g., 200-10000, 200-9000, 200-5000, 200-3000, or 200-500. In other aspects, the biologically active molecule has at least 500 nucleotides, e.g., 500-10000, 500-9000, 500-5000, or 500-3000. As used herein, the term **"antigen"** refers to any agent that when introduced into a subject elicits an immune response (cellular or humoral) to itself. In some aspects, the payload molecules are covalently linked to the EV, e.g., exosome. In other aspects, a payload comprises an adjuvant.

A **"recombinant"** polypeptide or protein refers to a polypeptide or protein produced via recombinant DNA technology. Recombinantly produced polypeptides and proteins expressed in engineered host cells are considered isolated for the purpose of the disclosure, as are native or recombinant polypeptides which have been separated, fractionated, or partially or substantially purified by any suitable technique. The polypeptides disclosed herein can be recombinantly produced using methods known in the art. Alternatively, the proteins and peptides disclosed herein can be chemically synthesized. In some aspects of the present disclosure, scaffold proteins present in EVs, *e.g.,* exosomes, are recombinantly produced by overexpressing the scaffold proteins in the producer cells, so that levels of scaffold proteins in the resulting EVs, *e.g.,* exosomes *are* significantly increased with respect to the levels of scaffold proteins present in EVs, *e.g.,* exosomes, of producer cells not overexpressing such scaffold proteins.

As used herein the terms **"anchor"** or **"anchoring"** a biologically active molecule on the luminal or external surface of an EV *(e.g.,* exosome) of the present disclosure via a scaffold protein refers to attaching covalently the biologically active molecule to the portion of the scaffold molecule located on the luminal or external surface of the EV *(e.g.,* exosome), respectively.

The terms **"associated," "association,"** and grammatical variants thereof are used interchangeably and refer to a first moiety, *e.g.,* a first amino acid sequence or nucleotide sequence, covalently or non-covalently joined to a second moiety, *e.g.,* a second amino acid sequence or nucleotide sequence, respectively. The first moiety can be directly joined or juxtaposed to the second moiety or alternatively an intervening moiety can covalently join the first moiety to the second moiety. In some embodiments, the term "associated" includes myristoylation, ionic interaction, or any combination thereof.

The term **"linked"** or **"fused"** as used herein refers to a fusion of a first moiety to a second moiety at the C-terminus or the N-terminus by a peptide bond or by a linker of one or more amino acids. The term "linked" or "fused" also includes insertion of the whole first moiety (or the second moiety) into, *e.g.,* between, any two points, *e.g.,* amino acids, in the second moiety (or the first moiety, respectively). In one aspect, the first moiety is linked to a second moiety by a peptide bond or a linker. The first moiety can be linked to a second moiety by a phosphodiester bond or a linker. The linker can be a peptide or a polypeptide (for polypeptide chains) or a nucleotide or a nucleotide chain (for nucleotide chains) or any chemical moiety (for polypeptide or polynucleotide chains or any chemical molecules). The term "linked" can also be indicated by a hyphen (-). In some aspects, a scaffold protein on an EV, *e.g.,* exosome, can be linked or fused to a payload, *e.g.,* a biologically active molecule.

As used herein, **"a mammalian subject"** includes all mammals, including without limitation, humans, domestic animals *(e.g.,* dogs, cats and the like), farm animals *(e.g.,* cows, sheep, pigs, horses and the like) and laboratory animals (*e.g.,* monkey, rats, mice, rabbits, guinea pigs and the like).

The terms **"individual," "subject," "host," and "patient,"** and variants thereof, are used interchangeably herein and refer to any mammalian subject for whom diagnosis, treatment, or therapy is desired, particularly humans. The methods described herein are applicable to both human therapy and veterinary applications. In some aspects, the subject is a mammal, and in other aspects the subject is a human.

As used herein, the term **"substantially free"** means that the sample comprising EVs, *e.g.,* exosomes comprise less than 10% of macromolecules, *e.g.,* contaminants, by mass/volume (m/v) percentage concentration. Some fractions may contain less than about 0.001%, less than about 0.01%, less than about 0.05%, less than about 0.1%, less than about 0.2%, less than about 0.3 %, less than about 0.4%, less than about 0.5%, less than about 0.6%, less than about 0.7%, less than about 0.8%, less than about 0.9%, less than about 1%, less than about 2%, less than about 3%, less than about 4%, less than about 5%, less than about 6%, less than about 7%, less than about 8%, less than about 9%, or less than about 10% (m/v) of macromolecules.

As used herein, the term **"macromolecule"** means nucleic acids, exogenous proteins, lipids, carbohydrates, metabolites (e.g., polymeric metabolites), or a combination thereof.

As used herein, the term **"conventional EV protein"** means a protein previously known to be enriched in EVs.

As used herein, the term **"conventional EV,** *e.g.,* **exosome protein"** means a protein previously known to be enriched in exosomes, including but not limited to CD9, CD63, CD81, PDGFR, GPI anchor proteins, lactadherin LAMP2, and LAMP2B, a fragment thereof, or a peptide that binds thereto. For the avoidance of doubt, PTGFRN, BSG, IGSF2, IGSF3, IGSF8, ITGB1, ITGA4, SLC3A2, ATP transporter or a fragment or a variant thereof are not conventional EV, **e.g.,** exosome proteins.

As used herein, the term **"pharmaceutical composition"** refers to one or more of the compounds described herein, such as, *e.g.,* an EV, such as exosome of the present disclosure, mixed or intermingled with, or suspended in one or more other chemical components, such as pharmaceutically-acceptable carriers and excipients. One purpose of a pharmaceutical composition is to facilitate administration of preparations of EVs, *e.g.,* exosomes, to a subject.

The term **"polynucleotide"** as used herein refers to polymers of nucleotides of any length, including ribonucleotides, deoxyribonucleotides, analogs thereof, or mixtures thereof. This term refers to the primary structure of the molecule. Thus, the term includes triple-, double- and single-stranded deoxyribonucleic acid ("DNA"), as well as triple-, double- and single-stranded ribonucleic acid ("RNA"). It also includes modified, for example by alkylation, and/or by capping, and unmodified forms of the polynucleotide. More particularly, the term "polynucleotide" includes polydeoxyribonucleotides (containing 2-deoxy-D-ribose), polyribonucleotides (containing D-ribose), including tRNA, rRNA, hRNA, siRNA and mRNA, whether spliced or unspliced, any other type of polynucleotide which is an N- or C-glycoside of a purine or pyrimidine base, and other polymers containing normucleotidic backbones, for example, polyamide **(e.g.,** peptide nucleic acids "PNAs") and polymorpholino polymers, and other synthetic sequence-specific nucleic acid polymers providing that the polymers contain nucleobases in a configuration which allows for base pairing and base stacking, such as is found in DNA and RNA. In particular aspects, the polynucleotide comprises an mRNA. In other aspect, the mRNA is a synthetic mRNA. In some aspects, the synthetic mRNA comprises at least one unnatural nucleobase. In some aspects, all nucleobases of a certain class have been replaced with unnatural nucleobases (*e.g.,* all uridines in a polynucleotide disclosed herein can be replaced with an unnatural nucleobase, *e.g.,* 5-methoxyuridine). In some aspects of the present disclosure, the biologically active molecule is a polynucleotide.

The terms **"polypeptide," "peptide,"** and **"protein"** are used interchangeably herein to refer to polymers of amino acids of any length. The polymer can comprise modified amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids such as homocysteine, ornithine, p-acetylphenylalanine, D-amino acids, and creatine), as well as other modifications known in the art. In some aspects of the present disclosure, the payload, *e.g.,* a biologically active molecule, attached to the EV, *e.g.,* exosome, is a polypeptide, *e.g.,* an antibody or a derivative thereof such as an ADC, a PROTAC, a toxin, a fusion protein, or an enzyme.

The term **"polypeptide,"** as used herein, refers to proteins, polypeptides, and peptides of any size, structure, or function. Polypeptides include gene products, naturally occurring polypeptides, synthetic polypeptides, homologs, orthologs, paralogs, fragments and other equivalents, variants, and analogs of the foregoing. A polypeptide can be a single polypeptide or can be a multi-molecular complex such as a dimer, trimer or tetramer. They can also comprise single chain or multichain polypeptides. Most commonly disulfide linkages are found in multichain polypeptides. The term polypeptide can also apply to amino acid polymers in which one or more amino acid residues are an artificial chemical analogue of a corresponding naturally occurring amino acid. In some aspects, a "peptide" can be less than or equal to 50 amino acids long, *e.g.,* about 5, 10, 15, 20, 25, 30, 35, 40, 45, or 50 amino acids long. In some aspects, a "peptide" can be at least about 2 to about 50, at least about 3 to about 50, at least about 4 to about 50, at least about 5 to about 50, at least about 10 to about 50, at least about 15 to about 50, at least about 20 to about 50, at least about 25 to about 50, at least about 30 to about 50, at least about 35 to about 50, at least about 40 to about 50, or at least about 45 to about 50 amino acids long.

As used herein, the term **"scaffold protein of the present disclosure,"** or grammatical variants, refers to
(i) a protein (naturally expressed, chemically or enzymatically synthesized, or produced recombinantly) that locates to the luminal surface of EVs, *e.g.,* exosomes, such as MARCKS, MARKSL1, or BASP1;
(ii) any functional fragment of (i);
(iii) any functional variant of (i)-(ii);
(iv) any derivative of (i)-(iii);
(v) any peptide corresponding to a domain or combination thereof derived from a protein in (i) that can bind to the luminal surface of EVs, *e.g.,* exosomes, or a molecule comprising such peptide;
(vi) any peptide derived from a motif derived from a protein in (i) that can bind to the luminal surface of EVs, e.g., exosomes, or a molecule comprising such peptide;
(vii) a molecule of (i) to (vi) comprising at least one non-natural amino acid;
(viii) or any combination thereof,
which is suitable for use as a scaffold to target (attach) payloads, e.g., biologically active molecules *(e.g.,* therapeutics proteins) to the luminal surface of EVs, *e.g.,* exosomes.

As used herein, the term **"EV, e.g., exosome, of the present disclosure,"** or grammatical variants, refers to an EV, *e.g.,* an exosome, that comprises at least one scaffold protein of the present disclosure.

As used herein, the term **"producer cell of the present disclosure,"** or grammatical variants, refers to a cell that can produce an EV, e.g., exosome, of the present disclosure.

### II. Extracellular Vesicle Proteins, e.g., Exosome Proteins

Some aspects of the present disclosure relate to identification, use and modification **of** EV, e.g., exosome proteins (scaffold proteins), which are highly enriched in EV, e.g., exosome, luminal surfaces. Such EV proteins or exosome proteins (scaffold proteins) can be identified by analyzing highly purified EV, e.g., exosomes, with mass spectrometry or other methods known in the art.

The scaffold proteins of the present disclosure include various luminal proteins or membrane proteins, such as transmembrane proteins *(i.e.,* proteins spanning the EV membrane through one or more transmembrane helices), integral proteins, and peripheral proteins *(i.e.,* proteins on the luminal surface interacting with the surface via electrostatic interactions and/or anchoring moieties), enriched on the EV, e.g., exosome, membranes. Specifically, the scaffold proteins of the present disclosure include, but not limited to,
(1) myristoylated alanine rich Protein Kinase C substrate (MARCKS);
(2) myristoylated alanine rich Protein Kinase C substrate like 1 (MARCKSL1); and
(3) brain acid soluble protein 1 (BASP1).

One or more EV, *e.g.,* exosome, proteins identified herein (scaffold proteins) can be selectively used depending on a producer cell, production condition, purification methods, or intended application of the exosomes.

EV, *e.g.,* exosome, proteins enriched in the lumen *(e.g.,* on the luminal surface of the EV membrane) of certain EVs, *e.g.,* exosomes, with a specific size range, a targeting moiety, a charge density, a payload, *etc.* can be identified and used in some aspects of the present disclosure.

In some aspects, more than one EV, *e.g.,* exosome, protein disclosed herein *(e.g.,* scaffold proteins such as MARCKS, MARKSL1, BASP1, any functional fragment, variant, or derivative thereof, or any combination thereof) can be used concurrently or subsequently for generation and isolation of therapeutic EV, *e.g.,* exosomes, of the present disclosure.

### III. Lumen-engineered EVs, e.g., Exosomes

Extracellular vesicles (EVs), *e.g.,* exosomes, typically have 20 nm to 1000 nm in diameter. Exosomes, which are small extracellular vesicles, have typically 100-200 nm in diameter. EVs, *e.g.,* exosomes, are composed of a limiting lipid bilayer and a diverse set of proteins and nucleic acids (Maas, S.L.N., et al., Trends. Cell Biol. 27(3):172-188 (2017)). EVs, *e.g.,* exosomes, exhibit preferential uptake in discrete cell types and tissues, and their tropism can be directed by adding proteins to their surface that interact with receptors on the surface of target cells (Alvarez-Erviti, L., et al., Nat. Biotechnol. 29(4):341-345 (2011)).

Unlike antibodies, EVs, *e.g.,* exosomes, can accommodate large numbers of molecules attached to their surface, on the order of thousands to tens of thousands of molecules per EV, *e.g.,* exosome. Conjugates or complexes comprising EVs, *e.g.,* exosomes, and payloads, *e.g.,* biologically active molecules *(e.g.,* therapeutic molecules), thus represent a platform to deliver a high concentration of therapeutic or diagnostic compound to discrete cell types, while at the same time limiting overall systemic exposure to the compound, which in turn reduces off-target toxicity. Furthermore, EVs, *e.g.,* exosomes, offer the possibility of accommodating multiple payloads, *e.g.,* biologically active molecules, in different compartments. For example, the EV, *e.g.,* exosome, can comprise, *e.g.,* targeting moieties attached to the EV's external surface which would direct the EV to a certain target cell *(e.g.,* a cancer cell) or target tissue *(e.g.,* liver or brain), one or more therapeutics moieties *(e.g.,* drugs), and/or one or more detectable moieties *(e.g.,* contrast reagents or radionuclides).

The EV, *e.g.,* exosome, can also comprise different payloads, *e.g.,* therapeutics moieties *(e.g.,* drugs) and/or detectable moieties *(e.g.,* contrast reagents or radionuclides) attached to the EV's luminal surface. In addition, the EV, *e.g.,* exosome, can also comprise one or more payloads, *e.g.,* biologically active molecules *(e.g.,* therapeutics agents, diagnostic reagents, adjuvants, etc.), in the lumen of the EV, *e.g.,* an exosome.

Thus, EVs, e.g., exosomes, provide a delivery format that combine multiple payloads, e.g., biologically active molecules, with the same or different roles in a single delivery vehicle, and at a very high density.

EVs, e.g.*,* exosomes, described herein are extracellular vesicles with a diameter between about 20-300 nm. In certain embodiments, an EV, e.g., exosome, of the present disclosure has a diameter between about 20-290 nm, 20-280 nm, 20-270 nm, 20-260 nm, 20-250 nm, 20-240 nm, 20-230 nm, 20-220 nm, 20-210 nm, 20-200 nm, 20-190 nm, 20-180 nm, 20-170 nm, 20-160 nm, 20-150 nm, 20-140 nm, 20-130 nm, 20-120 nm, 20-110 nm, 20-100 nm, 20-90 nm, 20-80 nm, 20-70 nm, 20-60 nm, 20-50 nm, 20-40 nm, 20-30 nm, 30-300 nm, 30-290 nm, 30-280 nm, 30-270 nm, 30-260 nm, 30-250 nm, 30-240 nm, 30-230 nm, 30-220 nm, 30-210 nm, 30-200 nm, 30-190 nm, 30-180 nm, 30-170 nm, 30-160 nm, 30-150 nm, 30-140 nm, 30-130 nm, 30-120 nm, 30-110 nm, 30-100 nm, 30-90 nm, 30-80 nm, 30-70 nm, 30-60 nm, 30-50 nm, 30-40 nm, 40-300 nm, 40-290 nm, 40-280 nm, 40-270 nm, 40-260 nm, 40-250 nm, 40-240 nm, 40-230 nm, 40-220 nm, 40-210 nm, 40-200 nm, 40-190 nm, 40-180 nm, 40-170 nm, 40-160 nm, 40-150 nm, 40-140 nm, 40-130 nm, 40-120 nm, 40-110 nm, 40-100 nm, 40-90 nm, 40-80 nm, 40-70 nm, 40-60 nm, 40-50 nm, 50-300 nm, 50-290 nm, 50-280 nm, 50-270 nm, 50-260 nm, 50-250 nm, 50-240 nm, 50-230 nm, 50-220 nm, 50-210 nm, 50-200 nm, 50-190 nm, 50-180 nm, 50-170 nm, 50-160 nm, 50-150 nm, 50-140 nm, 50-130 nm, 50-120 nm, 50-110 nm, 50-100 nm, 50-90 nm, 50-80 nm, 50-70 nm, 50-60 nm, 60-300 nm, 60-290 nm, 60-280 nm, 60-270 nm, 60-260 nm, 60-250 nm, 60-240 nm, 60-230 nm, 60-220 nm, 60-210 nm, 60-200 nm, 60-190 nm, 60-180 nm, 60-170 nm, 60-160 nm, 60-150 nm, 60-140 nm, 60-130 nm, 60-120 nm, 60-110 nm, 60-100 nm, 60-90 nm, 60-80 nm, 60-70 nm, 70-300 nm, 70-290 nm, 70-280 nm, 70-270 nm, 70-260 nm, 70-250 nm, 70-240 nm, 70-230 nm, 70-220 nm, 70-210 nm, 70-200 nm, 70-190 nm, 70-180 nm, 70-170 nm, 70-160 nm, 70-150 nm, 70-140 nm, 70-130 nm, 70-120 nm, 70-110 nm, 70-100 nm, 70-90 nm, 70-80 nm, 80-300 nm, 80-290 nm, 80-280 nm, 80-270 nm, 80-260 nm, 80-250 nm, 80-240 nm, 80-230 nm, 80-220 nm, 80-210 nm, 80-200 nm, 80-190 nm, 80-180 nm, 80-170 nm, 80-160 nm, 80-150 nm, 80-140 nm, 80-130 nm, 80-120 nm, 80-110 nm, 80-100 nm, 80-90 nm, 90-300 nm, 90-290 nm, 90-280 nm, 90-270 nm, 90-260 nm, 90-250 nm, 90-240 nm, 90-230 nm, 90-220 nm, 90-210 nm, 90-200 nm, 90-190 nm, 90-180 nm, 90-170 nm, 90-160 nm, 90-150 nm, 90-140 nm, 90-130 nm, 90-120 nm, 90-110 nm, 90-100 nm, 100-300 nm, 110-290 nm, 120-280 nm, 130-270 nm, 140-260 nm, 150-250 nm, 160-240 nm, 170-230 nm, 180-220 nm, or 190-210 nm. The size of the EV, e.g., exosome, described herein can be measured according to methods described, *infra.*

In some aspects, an EV, e.g., exosome, of the present disclosure comprises a bi-lipid membrane ("EV, e.g., exosome, membrane"), comprising an interior surface and an exterior surface. In certain embodiments, the interior surface faces the inner core *(i.e.,* lumen) of the EV, *e.g.,* exosome. In certain aspects, the exterior surface can be in contact with the endosome, the multivesicular bodies, or the membrane/cytoplasm of a producer cell or a target cell.

In some aspects, the EV, *e.g.,* exosome, membrane comprises lipids and fatty acids. In some aspects, the EV, *e.g.,* exosome, membrane comprises phospholipids, glycolipids, fatty acids, sphingolipids, phosphoglycerides, sterols, cholesterols, and phosphatidylserines.

In some aspects, the EV, *e.g.,* exosome, membrane comprises an inner leaflet and an outer leaflet. The composition of the inner and outer leaflet can be determined by transbilayer distribution assays known in the art, *see, e.g.,* Kuypers et al., Biohim Biophys Acta 1985 819:170. In some aspects, the composition of the outer leaflet is between approximately 70-90% choline phospholipids, between approximately 0-15% acidic phospholipids, and between approximately 5-30% phosphatidylethanolamine. In some aspects, the composition of the inner leaflet is between approximately 15-40% choline phospholipids, between approximately 10-50% acidic phospholipids, and between approximately 30-60% phosphatidylethanolamine.

In one aspect, the present disclosure relates to generation and use of lumen-engineered EVs, e.g., exosomes. Lumen-engineered exosomes have an internal space (the EV's luminal surface) modified in its compositions, e.g., modified with respect to the compositions of exosomes found in nature. For example, the composition of the luminal surface can be modified by changing the protein, lipid or glycan content of the components of the luminal side of the EV, *e.g.,* exosome, membrane. In some aspects, the luminal surface of the EVs, *e.g.,* exosomes, can comprise one or more recombinantly expressed proteins, e.g., scaffold proteins of the present disclosure, e.g., exosome lumen protein, that are not natural to the EVs, e.g., exosomes. Accordingly, the present disclosure provides compositions that allow the anchoring of a payload, *e.g.,* a biologically active molecule, to an EV, *e.g.,* an exosome, comprising linking the payload, *e.g.,* a biologically active molecule, to a scaffold protein, e.g., exosome lumen protein, of the present disclosure. The present disclosure also provides a method of anchoring a payload, *e.g.,* a biologically active molecule, to an EV, *e.g.,* an exosome, comprising linking the biologically active molecule to a scaffold protein, e.g., exosome lumen protein, of the present disclosure.

In some aspects, the scaffold proteins, e.g., exosome lumen protein, of the present disclosure are attached to the luminal surface of the EV, e.g., exosome, via lipidation. In some aspects, the scaffold protein of the present disclosure is fatty acylated. In some aspects, the scaffold protein is myristoylated.

Myristoylation is a lipidation modification where a myristoyl group, derived from myristic acid, is covalently attached by an amide bond to the alpha-amino group of an N-terminal glycine. Myristic acid is a 14-carbon saturated fatty acid (14:4) with the systematic name of n-tetradecanoic acid. This modification can be added either co-translationally or posttranslationally. During co-translational addition of the myristoyl group, the N-terminal glycine is modified following cleavage of the N-terminal methionine residue in the newly forming, growing polypeptide. This occurs in approximately 80% of myristoylated proteins. Post-translational myristoylation typically occurs following a caspase cleavage event resulting in the exposure of an internal glycine residue, which would then be available for myristic acid addition. In addition to co-translational or post-translational myristoylation (conduct either *in vivo* or *in vitro*, *e.g*., enzymatically), the myristoylation of the scaffold proteins, e.g., exosome lumen proteins, of the present disclosure can also occur via chemical synthesis, e.g., by appending, as a synthetic step, the myristic acid to a scaffold protein during chemical synthesis.

In some embodiments, the lipidic anchoring to biological membranes is not palmitoylation *(i.e.,* attachment of palmitic acid, generally to cysteine and less frequently to serine or threonine). In other embodiments, the lipidic anchoring to biological membranes is prenylation (attachment of prenyl groups) or glycosylphosphatidylinositol-linking (GPI-linking). Prenylated proteins are proteins with covalently attached hydrophobic isoprene polymers (*i.e*., branched five-carbon hydrocarbon) at cysteine residues of the protein. GPI-linked proteins are attached to a GPI complex molecular group via an amide linkage to the protein's C-terminal carboxyl group. The GPI attachment occurs through the action of GPI-transamidase complex. The fatty acid chains of the phosphatidylinositol are inserted into the membrane and thus are what anchor the protein to the membrane.

In some embodiments, the lumen-engineered EVs, e.g., exosomes, are generated by chemical and/or physical methods, such as PEG-induced fusion and/or ultrasonic fusion.

In other embodiments, the lumen-engineered EVs, e.g., exosomes, are generated by genetic engineering. Exosomes produced from a genetically-modified producer cell or a progeny of the genetically-modified cell can contain modified lumen compositions. In some embodiments, lumen-engineered EVs, *e.g.,* exosomes, have the EV, *e.g.,* exosome, protein (*e*.*g*., a scaffold protein, e.g., exosome lumen protein, such as MARCKS, MARKSL1, BASP1, or a combination thereof) at a higher or lower density or include a modification or a fragment of the EV, *e.g.,* exosome, protein *(e.g.,* any functional fragment, variant, or derivative thereof, or any combination thereof).

For example, lumen-engineered EVs, e.g., exosomes, can be produced from a cell transformed with an exogenous sequence encoding the EV, e.g., exosome, protein or a modification or a fragment of the EV, *e.g.,* exosome, protein (*e.g*., a scaffold protein, e.g., exosome lumen protein, such as MARCKS, MARKSL1, BASP1, any functional fragment, variant, or derivative thereof, or any combination thereof). EVs, *e.g*., exosomes, including proteins expressed from the exogenous sequence can include modified luminal surface protein (scaffold protein) compositions.

Various modifications or fragments of the EV protein, *e.g*., exosome protein *(e.g.,* a scaffold protein, e.g., exosome lumen protein, such as MARCKS, MARKSL1, BASP1, any functional fragment, variant, or derivative thereof, or any combination thereof), can be used for the embodiments of the present disclosure. For example, proteins modified to be more effectively targeted to EV, *e.g*., exosome, luminal surfaces can be used. Proteins modified to comprise a minimal fragment required for specific and effective targeting to EV, *e.g*., exosome, luminal surfaces can be also used.

In some aspects, the scaffold protein, e.g., exosome lumen protein, of the present disclosure comprises the MARCKS protein, or a fragment, variant, or derivative thereof. The MARCKS protein (Uniprot accession no. P29966) is also known as protein kinase C substrate, 80 kDa protein, light chain. The full-length human MARCKS protein is 332 amino acids in length and comprises a calmodulin-binding domain at amino acid residues 152-176. In some embodiments, the scaffold protein of the present disclosure comprises a mature MARCKS protein *(i.e.,* without N-terminal methionine). In some aspects, the scaffold protein of the present disclosure is derived from a mature MARCKS protein, *i.e.,* it is a fragment, variant, or derivate of a mature MARCKS protein and therefore it lacks the N-terminal methionine present in the non-mature protein.

In some aspects, the scaffold protein, e.g., exosome lumen protein, of the present disclosure comprises the MARCKSL1 protein (Uniprot accession no. P49006), also known as MARCKS-like protein 1, and macrophage myristoylated alanine-rich C kinase substrate. The full-length human MARCKSL1 protein is 195 amino acids in length. The MARCKSL1 protein has an effector domain involved in lipid-binding and calmodulin-binding at amino acid residues 87-110. In some embodiments, the scaffold protein of the present disclosure comprises a mature MARCKSL1 protein *(i.e.,* without N-terminal methionine). In some aspects, the scaffold protein of the present disclosure is derived from a mature MARCKSL1 protein, *i.e.,* it is a fragment, variant, or derivate of a mature MARCKSL1 protein and therefore it lacks the N-terminal methionine present in the non-mature protein.

In some aspects, the scaffold of the present disclosure comprises the BASP1 protein (Uniprot accession number P80723), also known as 22 kDa neuronal tissue-enriched acidic protein or neuronal axonal membrane protein NAP-22. The full-length human BASP1 protein sequence (isomer 1) is 227 amino acids in length. An isomer produced by an alternative splicing is missing amino acids 88 to 141 from isomer 1. In some embodiments, the scaffold protein, e.g., exosome lumen protein, of the present disclosure comprises a mature BASP1 protein *(i.e.,* without N-terminal methionine). In some aspects, the scaffold protein of the present disclosure is derived from a mature BASP1 protein, *i.e.,* it is a fragment, variant, or derivate of a mature BASP1 protein and therefore it lacks the N-terminal methionine present in the non-mature protein.

In some aspects, the scaffold protein, e.g., exosome lumen protein, of the present disclosure comprises an "N-terminus domain" (ND) and an "effector domain," wherein the ND and/or the ED are associated with the luminal surface of the EV, *e.g.,* an exosome. As used herein the term "associated with" refers to the interaction between a scaffold protein of the present disclosure with the luminal surface of the EV, *e.g.,* an exosome, that does not involve covalent linking to a membrane component. For example, the scaffolds of the present disclosure can be associated with the luminal surface of the EV, *e.g.,* via a lipid anchor *(e.g.,* myristic acid), and/or a polybasic domain that interacts electrostatically with the negatively charged head of membrane phospholipids. In other aspects, the scaffold protein comprises an N-terminus domain (ND) and an effector domain (ED), wherein the ND is associated with the luminal surface of the EV and the ED are associated with the luminal surface of the EV by an ionic interaction, wherein the ED comprises at least two, at least three, at least four, at least five, at least six, or at least seven contiguous lysines (Lys) in sequence.

In other aspects, the ED further comprises one or more low complexity regions, *e.g.,* a PEST motif. A PEST sequence is a peptide sequence that is rich in proline (P), glutamic acid (E), serine (S), and threonine (T). In some aspects, the ED further comprises negatively charged residues (for example, Glu) and many Ser and Thr that undergo transient phosphorylation (thus, both adding negative charges to the areas out of ED).

In some aspects, the ND is associated with the luminal surface of the EV, *e.g.,* an exosome, via lipidation, e.g., via myristoylation. In some embodiments, the ND has Gly at the N-terminus. In some embodiments, the N-terminal Gly is myristoylated. In some aspects, the ND does not comprise Met at the N-terminus. In other embodiments, the ND comprises Gly, which is myristoylated, and does not comprise Met at the N-terminus.

In some aspects, the ED is associated with the luminal surface of the EV, *e.g.,* an exosome, by an ionic interaction. In some embodiments, the ED is associated with the luminal surface of the EV, *e.g.,* an exosome, by an electrostatic interaction, in particular, an attractive electrostatic interaction.

In some aspects, the ED comprises (i) a basic amino acid *(e.g.,* lysine), or (ii) two or more basic amino acids (*e.g*., lysine) next to each other in a polypeptide sequence. In some aspects, the basic amino acid is lysine (Lys; K), arginine (Arg, R), or Histidine (His, H). In some embodiments, the basic amino acid is (Lys)n, wherein n is an integer between 1 and 10.

In other aspects, the ED comprises at least a lysine and the ND comprises a lysine at the C-terminus if the N-terminus of the ED is directly linked to lysine at the C-terminus of the ND, *i.e.,* the lysine is in the N-terminus of the ED and is fused to the lysine in the C-terminus of the ND. In other embodiments, the ED comprises at least two lysines, at least three lysines, at least four lysines, at least five lysines, at least six lysines, or at least seven lysines when the N-terminus of the ED is linked to the C-terminus of the ND by a linker, *e.g.,* one or more amino acids.

In some aspects, the ED comprises K, KK, KKK, KKKK (SEQ ID NO: 151), KKKKK (SEQ ID NO: 152), or any combination thereof. The present disclosure also provides that in some aspects, the lysine repeats can be replaced with arginines. In other aspects, the arginine repeats in the ED or in the scaffold proteins provide lower loading efficacy compared to the lysine efficacy.

In some aspects, the scaffold protein, e.g., exosome lumen protein, useful for the present disclosure requires at least two lysines or at least three lysines, repeated in sequence in the ED or in the ED together with the ND, *i.e.,* lysine at the C-terminus of the ND and K in the N-terminus of the ED. In some embodiments, the ED comprises K, KK, KKK, KKKK (SEQ ID NO: 151), KKKKK (SEQ ID NO: 152), or any combination thereof. In some aspects, the ND comprises the amino acid sequence as set forth in G:X2:X3:X4:X5:X6, wherein G represents Gly; wherein ":" represents a peptide bond, wherein each of the X2 to the X6 independently represents an amino acid; and wherein the X6 represents a basic amino acid. In some aspects, the X6 amino acid is selected is selected from the group consisting of Lys, Arg, and His. In some aspects, the X5 amino acid is selected from the group consisting of Pro, Gly, Ala, and Ser. In some embodiments, the X2 amino acid is selected from the group consisting of Pro, Gly, Ala, and Ser. In some aspects, the X4 is selected from the group consisting of Pro, Gly, Ala, Ser, Val, Ile, Leu, Phe, Trp, Tyr, Gln, and Met. In some aspects, the scaffold protein does not comprise Met at the N-terminus, *e.g*., myristoylated.

In some aspects, the scaffold protein, e.g., exosome lumen protein, comprises an ND and an ED, wherein ND comprises the amino acid sequence as set forth in G:X2:X3:X4:X5:X6, wherein G represents Gly; wherein ":" represents a peptide bond, wherein each of the X2 to the X6 independently represents an amino acid; wherein the X6 represents a basic amino acid, the X5 amino acid is selected from the group consisting of Pro, Gly, Ala, and Ser, the X4 is selected from the group consisting of Pro, Gly, Ala, Ser, Val, Ile, Leu, Phe, Trp, Tyr, Gln, and Met, the X3 represents an amino acid, and the X2 amino acid is selected from the group consisting of Pro, Gly, Ala, and Ser, wherein the ED comprises at least one amino acid, e.g., at least one Lys, and wherein the scaffold protein is not (i) the amino acid sequence comprising SEQ ID NO: 4 to 114, 116 to 118, 122-150, or 180-190 or (ii) the amino acid sequence encoded by SEQ ID NO: 115 or 118 to 121.

In some aspects, the scaffold protein, e.g., exosome lumen protein, comprises an N-terminus domain (ND) and an effector domain (ED), wherein the ND comprises the amino acid sequence as set forth in G:X2:X3:X4:X5:X6, wherein G is a glycine, represented as Gly; wherein ":" represents a peptide bond, wherein each of the X2 to the X6 is independently an amino acid; wherein the X6 comprises a basic amino acid, and wherein the ED is linked to X6 by a peptide bond and comprises at least one lysine at the N-terminus of the ED. In some aspects, the scaffold protein does not comprise Met at the N-terminus, *e.g*., myristoylated. In other aspects, the scaffold protein does not comprise or does not consist of (i) the amino acid sequence comprising SEQ ID NO: 4 to 114, 116 to 118, 122 to 150, or 180 to 190 or (ii) the amino acid sequence encoded by SEQ ID NO: 115 or 118 to 121.

In some embodiments, the ND of the scaffold protein, e.g., exosome lumen protein, comprises the amino acid sequence of G:X2:X3:X4:X5:X6, wherein
a. G represents Gly;
b. ":" represents a peptide bond;
c. the X2 represents an amino acid selected from the group consisting of Pro, Gly, Ala, and Ser;
d. the X3 represents any amino acid;
e. the X4 represents an amino acid selected from the group consisting of Pro, Gly, Ala, Ser, Val, Ile, Leu, Phe, Trp, Tyr, Gln, and Met;
f. the X5 represents an amino acid selected from the group consisting of Pro, Gly, Ala, and Ser; and
g. the X6 represents an amino acid selected from the group consisting of Lys, Arg, and His.

In some aspects, the scaffold protein, e.g., exosome lumen protein, does not comprise Met at the N-terminus, *e.g*., myristoylated. In other aspects, the scaffold protein does not comprise or consist of (i) the amino acid sequence comprising SEQ ID NO: 4 to 114, 116 to 118, 122 to 150 or 180 to 190 or (ii) the amino acid sequence encoded by SEQ ID NO: 115 or 118 to 121. In some aspects, the X3 amino acid is selected from the group consisting of Asn, Gln, Ser, Thr, Asp, Glu, Lys, His, and Arg.

In some aspects, the ND and ED are joined by a linker. In some embodiments, the linker comprises one or more amino acids. In some aspects, the term "linker" refers to a peptide or polypeptide sequence (*e.g*., a synthetic peptide or polypeptide sequence) or to a non-polypeptide, *e.g*., an alkyl chain. In some aspects, two or more linkers can be linked in tandem. Generally, linkers provide flexibility or prevent/ameliorate steric hindrances. Linkers are not typically cleaved; however, in certain aspects, such cleavage can be desirable. Accordingly, in some embodiments a linker can comprise one or more protease-cleavable sites, which can be located within the sequence of the linker or flanking the linker at either end of the linker sequence. When the ND and ED are joined by a linker, the ED comprise at least two lysines, at least three lysines, at least four lysines, at least five lysines, at least six lysines, or at least seven lysines.

In some aspects, the linker is a peptide linker. In some aspects, the peptide linker can comprise at least about two, at least about three, at least about four, at least about five, at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 amino acids.

In some aspects, the linker is a glycine/serine linker. In some embodiments, the peptide linker is glycine/serine linker according to the formula [(Gly)n-Ser]m where n is any integer from 1 to 100 and m is any integer from 1 to 100. In other aspects, the glycine/serine linker is according to the formula [(Gly)x-Sery]z wherein x in an integer from 1 to 4, y is 0 or 1, and z is an integers from 1 to 50. In some aspects, the peptide linker comprises the sequence Gn, where n can be an integer from 1 to 100. In some aspects, the peptide linker can comprise the sequence (GlyAla)n, wherein n is an integer between 1 and 100. In other aspects, the peptide linker can comprise the sequence (GlyGlySer)n, wherein n is an integer between 1 and 100.

In some aspects, the peptide linker is synthetic, *i.e.,* non-naturally occurring. In one embodiment, a peptide linker includes peptides (or polypeptides) (*e.g*., natural or non-naturally occurring peptides) which comprise an amino acid sequence that links or genetically fuses a first linear sequence of amino acids to a second linear sequence of amino acids to which it is not naturally linked or genetically fused in nature. For example, in one aspect the peptide linker can comprise non-naturally occurring polypeptides which are modified forms of naturally occurring polypeptides (*e.g*., comprising a mutation such as an addition, substitution or deletion).

In other aspects, the peptide linker can comprise non-naturally occurring amino acids. In yet other aspects, the peptide linker can comprise naturally occurring amino acids occurring in a linear sequence that does not occur in nature. In still other aspects, the peptide linker can comprise a naturally occurring polypeptide sequence.

The present disclosure also provides an isolated extracellular vesicle (EV), *e.g.,* an exosome, comprising a biologically active molecule linked to a scaffold protein, e.g., exosome lumen protein, wherein the scaffold protein comprises ND-ED, wherein:
a. ND comprises G:X2:X3:X4:X5:X6; wherein:
   i. G represents Gly;
   ii. ":" represents a peptide bond;
   iii. X2 represents an amino acid selected from the group consisting of Pro, Gly, Ala, and Ser;
   iv. X3 represents any amino acid;
   v. X4 represents an amino acid selected from the group consisting of Pro, Gly, Ala, Ser, Val, Ile, Leu, Phe, Trp, Tyr, Glu, and Met;
   vi. X5 represents an amino acid selected from the group consisting of Pro, Gly, Ala, and Ser;
   vii. X6 represents an amino acid selected from the group consisting of Lys, Arg, and His;
b. "_" represents an optional linker; and
c. ED is an effector domain comprising (i) at least two contiguous lysines (Lys), the N terminal lysine linked to the X6 by a peptide bond or one or more amino acids or (ii) at least one lysine, which is directly linked to the X6 by a peptide bond. In some aspects, the scaffold protein does not comprise Met at the N-terminus, *e.g*., myristoylated. In other aspects, the scaffold protein does not comprise or does not consist of (i) the amino acid sequence comprising SEQ ID NO: 4 to 114, 116 to 118, 122-150 or 180 to 190 or (ii) the amino acid sequence encoded by SEQ ID NO: 115 or 118 to 121.

In some aspects, the X2 amino acid is selected from the group consisting of Gly and Ala. In some aspects, the X3 amino acid is Lys. In some aspects, the X4 amino acid is Leu or Glu. In some aspects, the X5 amino acid is selected from the group consisting of Ser and Ala. In some aspects, the X6 amino acid is Lys. In some aspects, the X2 amino acid is Gly, Ala, or Ser; the X3 amino acid is Lys or Glu; the X4 amino acid is Leu, Phe, Ser, or Glu; the X5 amino acid is Ser or Ala; and X6 amino acid is Lys. In some aspects, the - linker comprises a peptide bond or one or more amino acids.

In some aspects, the ED in the scaffold protein, e.g., exosome lumen protein, comprises Lys (K), KK, KKK, KKKK (SEQ ID NO: 151), KKKKK (SEQ ID NO: 152), Arg (R), RR, RRR, RRRR (SEQ ID NO: 153); RRRRR (SEQ ID NO: 154), KR, RK, KKR, KRK, RKK, KRR, RRK, (K/R)(K/R)(K/R)(K/R) (SEQ ID NO: 155), (K/R)(K/R)(K/R)(K/R)(K/R) (SEQ ID NO: 156), or any combination thereof.

In some aspects, the scaffold protein, e.g., exosome lumen protein, comprises an amino acid sequence as set forth in (i) GGKLSKK (SEQ ID NO: 157), (ii) GAKLSKK (SEQ ID NO: 158), (iii) GGKQSKK (SEQ ID NO: 159), (iv) GGKLAKK (SEQ ID NO: 160), or (v) any combination thereof. In some aspects, the scaffold protein does not comprise the amino acid sequence having Met at the N-terminus of the sequence in (i) to (v). In other aspects, the scaffold protein does not comprise or consist of (i) the amino acid sequence comprising SEQ ID NO: 4 to 114, 116 to 118, or 122-150 or (ii) the amino acid sequence encoded by SEQ ID NO: 115 or 118 to 121.

In some aspects, the ND in the scaffold protein, e.g., exosome lumen protein, comprises an amino acid sequence of (i) GGKLSK (SEQ ID NO: 203), (ii) GAKLSK (SEQ ID NO: 204), (iii) GGKQSK (SEQ ID NO: 205), (iv) GGKLAK (SEQ ID NO: 206), or (v) any combination thereof and the ED in the scaffold protein comprises (a) K, (b) KK, (c) KKK, (d) KKKG (SEQ ID NO: 207), (e) KKKGY (SEQ ID NO: 208), (f) KKKGYN (SEQ ID NO: 209), (g) KKKGYNV (SEQ ID NO: 210), (h) KKKGYNVN (SEQ ID NO: 211), (i) KKKGYS (SEQ ID NO: 212), (k) KKKGYG (SEQ ID NO: 213), (1) KKKGYGG (SEQ ID NO: 214), (m) KKKGS (SEQ ID NO: 215), (n) KKKGSG (SEQ ID NO: 216), (o) KKKGSG (SEQ ID NO: 217), (p) KKKGSGS (SEQ ID NO: 218), (q) KKKS (SEQ ID NO: 219), (r) KKKSG (SEQ ID NO: 220), (s) KKKSGG (SEQ ID NO: 221), (t) KKKSGGS (SEQ ID NO: 222), (u) KKKSGGSG (SEQ ID NO: 223), (v) KKSGGSGG (SEQ ID NO: 224), (w) KKKSGGSGGS (SEQ ID NO: 225), (x) KRFSFKKS (SEQ ID NO: 226), or any combination thereof. In some aspects, the scaffold protein does not comprise the amino acid sequence having Met at the N-terminus of the sequence in (i) to (v). In some aspects, the scaffold protein does not comprise or consist of (i) the amino acid sequence comprising SEQ ID NO: 4 to 114, 116 to 118, 122 to 150 or 180 to 190 or (ii) the amino acid sequence encoded by SEQ ID NO: 115 or 118 to 121.

In some aspects, the polypeptide sequence of a scaffold protein, e.g., exosome lumen protein, of the present disclosure consists of an amino acid sequence selected from the group consisting of (i) GGKLSKK (SEQ ID NO: 157), (ii) GAKLSKK (SEQ ID NO: 158), (iii) GGKQSKK (SEQ ID NO: 159), (iv) GGKLAKK (SEQ ID NO: 160), or (v) any combination thereof.

In some aspects, the scaffold protein, e.g., exosome lumen protein, comprises an amino acid sequence of (i) GGKLSKKK (SEQ ID NO: 161), (ii) GGKLSKKS (SEQ ID NO: 162), (iii) GAKLSKKK (SEQ ID NO: 163), (iv) GAKLSKKS (SEQ ID NO: 164), (v) GGKQSKKK (SEQ ID NO: 165), (vi) GGKQSKKS (SEQ ID NO: 166), (vii) GGKLAKKK (SEQ ID NO: 167), (viii) GGKLAKKS (SEQ ID NO: 168), or (ix) any combination thereof. In some aspects, the scaffold protein does not comprise the amino acid sequence having Met at the N-terminus of the sequence in (i) to (ix). In some aspects, the scaffold protein does not comprise or consist of (i) the amino acid sequence comprising SEQ ID NO: 4 to 114, 116 to 118, 122 to 150 or 180 to 190 or (ii) the amino acid sequence encoded by SEQ ID NO: 115 or 118 to 121.

In some aspects, the polypeptide sequence of a scaffold protein, e.g., exosome lumen protein, of the present disclosure consists of an amino acid sequence selected from the group consisting of (i) GGKLSKKK (SEQ ID NO: 161), (ii) GGKLSKKS (SEQ ID NO: 162), (iii) GAKLSKKK (SEQ ID NO: 163), (iv) GAKLSKKS (SEQ ID NO: 164), (v) GGKQSKKK (SEQ ID NO: 165), (vi) GGKQSKKS (SEQ ID NO: 166), (vii) GGKLAKKK (SEQ ID NO: 167), (viii) GGKLAKKS (SEQ ID NO: 168), and (ix) any combination thereof.

In some aspects, the scaffold protein, e.g., exosome lumen protein, is at least about 8, at least about 9, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 26, at least about 27, at least about 28, at least about 29, at least about 30, at least 31, at least about 32, at least about 33, at least about 34, at least about 35, at least about 36, at least about 37, at least about 38, at least about 39, at least about 39, at least about 40, at least about 41, at least about 42, at least about 43, at least about 44, at least about 50, at least about 46, at least about 47, at least about 48, at least about 49, at least about 50, at least about 55, at least about 60, at least about 65, at least about 70, at least about 75, at least about 80, at least 85, at least about 90, at least about 95, at least about 100, at least about 105, at least about 110, at least about 115, at least about 120, at least about 125, at least about 130, at least about 135, at least about 140, at least about 145, at least about 150, at least about 155, at least about 160, at least about 165, at least about 170, at least about 175, at least about 180, at least about 185, at least about 190, at least about 195, at least about 200, at least about 205, at least about 210, at least about 215, at least about 220, at least about 225, at least about 230, at least about 235, at least about 240, at least about 245, at least about 250, at least about 255, at least about 260, at least about 265, at least about 270, at least about 275, at least about 280, at least about 285, at least about 290, at least about 295, at least about 300, at least about 305, at least about 310, at least about 315, at least about 320, at least about 325, at least about 330, at least about 335, at least about 340, at least about 345, or at least about 350 amino acids in length.

In some aspects, the scaffold protein, e.g., exosome lumen protein, is between about 5 and about 10, between about 10 and about 20, between about 20 and about 30, between about 30 and about 40, between about 40 and about 50, between about 50 and about 60, between about 60 and about 70, between about 70 and about 80, between about 80 and about 90, between about 90 and about 100, between about 100 and about 110, between about 110 and about 120, between about 120 and about 130, between about 130 and about 140, between about 140 and about 150, between about 150 and about 160, between about 160 and about 170, between about 170 and about 180, between about 180 and about 190, between about 190 and about 200, between about 200 and about 210, between about 210 and about 220, between about 220 and about 230, between about 230 and about 240, between about 240 and about 250, between about 250 and about 260, between about 260 and about 270, between about 270 and about 280, between about 280 and about 290, between about 290 and about 300, between about 300 and about 310, between about 310 and about 320, between about 320 and about 330, between about 330 and about 340, or between about 340 and about 250 amino acids in length.

In some aspects, the scaffold protein, e.g., exosome lumen protein, comprises (i) GGKLSKKKKGYNVN (SEQ ID NO: 169), (ii) GAKLSKKKKGYNVN (SEQ ID NO: 170), (iii) GGKQSKKKKGYNVN (SEQ ID NO: 171), (iv) GGKLAKKKKGYNVN (SEQ ID NO: 172), (v) GGKLSKKKKGYSGG (SEQ ID NO: 173), (vi) GGKLSKKKKGSGGS (SEQ ID NO: 174), (vii) GGKLSKKKKSGGSG (SEQ ID NO: 175), (viii) GGKLSKKKSGGSGG (SEQ ID NO: 176), (ix) GGKLSKKSGGSGGS (SEQ ID NO: 177), (x) GGKLSKSGGSGGSV (SEQ ID NO: 178), or (xi) GAKKSKKRFSFKKS (SEQ ID NO: 179). In some aspects, the scaffold protein does not comprise the amino acid sequence having Met at the N-terminus of the sequence in (i) to (xi). In some aspects, the scaffold protein does not comprise or consist of (i) the amino acid sequence comprising SEQ ID NO: 4 to 114, 116 to 118, 122 to 150 or 180 to 190 or (ii) the amino acid sequence encoded by SEQ ID NO: 115 or 118 to 121.

In some aspects, the polypeptide sequence of a scaffold protein, e.g., exosome lumen protein, of the present disclosure consists of (i) GGKLSKKKKGYNVN (SEQ ID NO: 169), (ii) GAKLSKKKKGYNVN (SEQ ID NO: 170), (iii) GGKQSKKKKGYNVN (SEQ ID NO: 171), (iv) GGKLAKKKKGYNVN (SEQ ID NO: 172), (v) GGKLSKKKKGYSGG (SEQ ID NO: 173), (vi) GGKLSKKKKGSGGS (SEQ ID NO: 174), (vii) GGKLSKKKKSGGSG (SEQ ID NO: 175), (viii) GGKLSKKKSGGSGG (SEQ ID NO: 176), (ix) GGKLSKKSGGSGGS (SEQ ID NO: 177), (x) GGKLSKSGGSGGSV (SEQ ID NO: 178), or (xi) GAKKSKKRFSFKKS (SEQ ID NO: 179). In some aspects, the scaffold protein does not comprise the amino acid sequence having Met at the N-terminus of the sequence in (i) to (xi). In some aspects, the scaffold protein does not comprise or consist of (i) the amino acid sequence comprising SEQ ID NO: 4 to 114, 116 to 118, 122 to 150 or 180 to 190 or (ii) the amino acid sequence encoded by SEQ ID NO: 115 or 118 to 121.

In other aspects, the scaffold protein, e.g., exosome lumen protein, comprises any one of the sequences disclosed herein, but does not comprise the corresponding sequence having Met at the N-terminus, e.g., SEQ ID NO: 4 to 109. In some aspects, the scaffold protein comprises any one of the sequences in TABLE 1, but does not comprise or consist of (i) the amino acid sequence comprising SEQ ID NO: 4 to 114, 116 to 118, 122 to 150 or 180 to 190 or (ii) the amino acid sequence encoded by SEQ ID NO: 115 or 118 to 121.

Non-limiting examples of the scaffold proteins, e.g., exosome lumen proteins, useful for the present disclosure is listed below.

**TABLE 1.**

| SEQ ID NO: | Scaffold Protein: GX2X3X4X5X6-ED |
|---|---|
| 227 | GGKLSKKKKGYNVNDEKAKEKDKKAEGAA |
| 228 | GGKLSKKKKGYNVNDEKAKEKDKKAEGA |
| 229 | GGKLSKKKKGYNVNDEKAKEKDKKAEG |
| 230 | GGKLSKKKKGYNVNDEKAKEKDKKAE |
| 231 | GGKLSKKKKGYNVNDEKAKEKDKKA |
| 232 | GGKLSKKKKGYNVNDEKAKEKDKK |
| 233 | GGKLSKKKKGYNVNDEKAKEKDK |
| 234 | GGKLSKKKKGYNVNDEKAKEKD |
| 235 | GGKLSKKKKGYNVNDEKAKEK |
| 236 | GGKLSKKKKGYNVNDEKAKE |
| 237 | GGKLSKKKKGYNVNDEKAK |
| 238 | GGKLSKKKKGYNVNDEKA |
| 239 | GGKLSKKKKGYNVNDEK |
| 240 | GGKLSKKKKGYNVNDE |
| 241 | GGKLSKKKKGYNVND |
| 169 | GGKLSKKKKGYNVN |
| 242 | GGKLSKKKKGYNV |
| 243 | GGKLSKKKKGYN |
| 244 | GGKLSKKKKGY |
| 245 | GGKLSKKKKG |
| 246 | GGKLSKKKK |
| 161 | GGKLSKKK |
| 157 | GGKLSKK |
| 247 | GAKKSKKRFSFKKSFKLSGFSFKKNKKEA |
| 248 | GAKKSKKRFSFKKSFKLSGFSFKKNKKE |
| 249 | GAKKSKKRFSFKKSFKLSGFSFKKNKK |
| 250 | GAKKSKKRFSFKKSFKLSGFSFKKNK |
| 251 | GAKKSKKRFSFKKSFKLSGFSFKKN |
| 252 | GAKKSKKRFSFKKSFKLSGFSFKK |
| 253 | GAKKSKKRFSFKKSFKLSGFSFK |
| 254 | GAKKSKKRFSFKKSFKLSGFSF |
| 255 | GAKKSKKRFSFKKSFKLSGFS |
| 256 | GAKKSKKRFSFKKSFKLSGF |
| 257 | GAKKSKKRFSFKKSFKLSG |
| 258 | GAKKSKKRFSFKKSFKLS |
| 259 | GAKKSKKRFSFKKSFKL |
| 260 | GAKKSKKRFSFKKSFK |
| 261 | GAKKSKKRFSFKKSF |
| 179 | GAKKSKKRFSFKKS |
| 262 | GAKKSKKRFSFKK |
| 263 | GAKKSKKRFSFK |
| 264 | GAKKSKKRFSF |
| 265 | GAKKSKKRFS |
| 266 | GAKKSKKRF |
| 267 | GAKKSKKR |
| 268 | GAKKSKK |
| 269 | GAKKAKKRFSFKKSFKLSGFSFKKNKKEA |
| 270 | GAKKAKKRFSFKKSFKLSGFSFKKNKKE |
| 271 | GAKKAKKRFSFKKSFKLSGFSFKKNKK |
| 272 | GAKKAKKRFSFKKSFKLSGFSFKKNK |
| 273 | GAKKAKKRFSFKKSFKLSGFSFKKN |
| 274 | GAKKAKKRFSFKKSFKLSGFSFKK |
| 275 | GAKKAKKRFSFKKSFKLSGFSFK |
| 276 | GAKKAKKRFSFKKSFKLSGFSF |
| 277 | GAKKAKKRFSFKKSFKLSGFS |
| 278 | GAKKAKKRFSFKKSFKLSGF |
| 279 | GAKKAKKRFSFKKSFKLSG |
| 280 | GAKKAKKRFSFKKSFKLS |
| 281 | GAKKAKKRFSFKKSFKL |
| 282 | GAKKAKKRFSFKKSFK |
| 283 | GAKKAKKRFSFKKSF |
| 284 | GAKKAKKRFSFKKS |
| 285 | GAKKAKKRFSFKK |
| 286 | GAKKAKKRFSFK |
| 287 | GAKKAKKRFSF |
| 288 | GAKKAKKRFS |
| 289 | GAKKAKKRF |
| 290 | GAKKAKKR |
| 291 | GAKKAKK |
| 292 | GAQESKKKKKKRFSFKKSFKLSGFSFKK |
| 293 | GAQESKKKKKKRFSFKKSFKLSGFSFK |
| 294 | GAQESKKKKKKRFSFKKSFKLSGFSF |
| 295 | GAQESKKKKKKRFSFKKSFKLSGFS |
| 396 | GAQESKKKKKKRFSFKKSFKLSGF |
| 297 | GAQESKKKKKKRFSFKKSFKLSG |
| 298 | GAQESKKKKKKRFSFKKSFKLS |
| 299 | GAQESKKKKKKRFSFKKSFKL |
| 300 | GAQESKKKKKKRFSFKKSFK |
| 301 | GAQESKKKKKKRFSFKKSF |
| 302 | GAQESKKKKKKRFSFKKS |
| 303 | GAQESKKKKKKRFSFKK |
| 304 | GAQESKKKKKKRFSFK |
| 305 | GAQESKKKKKKRFSF |
| 306 | GAQESKKKKKKRFS |
| 307 | GAQESKKKKKKRF |
| 308 | GAQESKKKKKKR |
| 309 | GAQESKKKKKK |
| 310 | GAQESKKKKK |
| 311 | GAQESKKKK |
| 312 | GAQESKKK |
| 313 | GAQESKK |
| 314 | GSQSSKKKKKKFSFKKPFKLSGLSFKRNRK |
| 315 | GSQSSKKKKKKFSFKKPFKLSGLSFKRNR |
| 316 | GSQSSKKKKKKFSFKKPFKLSGLSFKRN |
| 317 | GSQSSKKKKKKtSFKKPFKLSGLSFKR |
| 318 | GSQSSKKKKKKtSFKKPFKLSGLSFK |
| 319 | GSQSSKKKKKKFSFKKPFKLSGLSF |
| 320 | GSQSSKKKKKKFSFKKPFKLSGLS |
| 321 | GSQSSKKKKKKSFKKPFKLSGL |
| 322 | GSQSSKKKKKKFSFKKPFKLSG |
| 323 | GSQSSKKKKKKFSFKKPFKLS |
| 324 | GSQSSKKKKKKFSFKKPFKL |
| 325 | GSQSSKKKKKKFSFKKPFK |
| 326 | GSQSSKKKKKKFSFKKPF |
| 327 | GSQSSKKKKKKFSFKKP |
| 328 | GSQSSKKKKKKFSFKK |
| 329 | GSQSSKKKKKKFSFK |
| 330 | GSQSSKKKKKKFSF |
| 331 | GSQSSKKKKKKFS |
| 332 | GSQSSKKKKKKF |
| 333 | GSQSSKKKKKK |
| 334 | GSQSSKKKKK |
| 335 | GSQSSKKKK |
| 336 | GSQSSKKK |
| 337 | GSQSSKK |

In some aspects, the scaffold protein, e.g., exosome lumen protein, of the present disclosure consists or consists essentially of any of the sequences in the present disclosure. In some aspects, the scaffold of the present disclosure consists of a sequence disclosed in TABLE 1. In some aspects, the scaffold of the present disclosure consists of a sequence disclosed herein, *e.g.,* a sequence disclosed in TABLE 1, covalently attached to a membrane anchor *(e.g.,* a myristic acid). In some aspects, membrane anchoring can be conducted via lipidation. In some embodiments, the lipidation can be, *e.g.,* fatty acylation. In some aspects, the fatty aceylation can be, e.g., myristoylation. In some aspects, the membrane anchor is covalently attached to the N-terminal amino acid of a sequence disclosed herein, *e.g.,* a sequence disclosed in TABLE 1. In some aspects, the membrane anchor is covalently attached to N-terminal glycine of a sequence disclosed herein, *e.g.,* a sequence disclosed in TABLE 1. Accordingly, in some aspects, the scaffold of the present disclosure consists of a sequence disclosed, *e.g.,* a sequence disclosed in TABLE 1, that has been N-myristoylated.

In some aspects, the scaffold protein, e.g., exosome lumen protein, of the present disclosure does not contain an N-terminal Met. In some aspects, the scaffold protein comprises a lipidated amino acid, *e.g.,* a myristoylated amino acid, at the N-terminus of the scaffold protein, which functions as a lipid anchor. In some aspects, the amino acid residue at the N-terminus of the scaffold protein in Gly. The presence of an N-terminal Gly is a requirement for N-myristoylation. In some aspects, the amino acid residue at the N-terminus of the scaffold protein is synthetic. In some aspects, the amino acid residue at the N-terminus of the scaffold protein is a glycine analog, e.g., allylglycine, butylglycine, or propargylglycine.

In some aspects, a lipid anchor can be attached by chemical synthesis or enzymatically to any N-terminal amino acid of a scaffold protein of the present disclosure.

In other aspects, the lipid anchor can be any lipid anchor known in the art, *e.g.,* palmitic acid or glycosylphosphatidylinositols. Under unusual circumstances, *e.g*., by using a culture medium where myristic acid is limiting, some other fatty acids including shorter-chain and unsaturated, can be attached to the N-terminal glycine. For example, in BK channels, myristate has been reported to be attached posttranslationally to internal serine/threonine or tyrosine residues via a hydroxy ester linkage. Membrane anchors known in the art are presented in the following table.

**TABLE 2**

| *Modification* | *Modifying Group* |
|---|---|
| S-Palmitoylation | 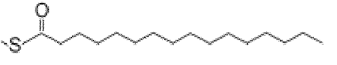 |
| N-Palmitoylation | 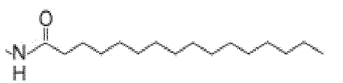 |
| N-Myristoylation | 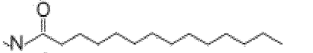 |
| Farnesylation | |
| Geranylgeranylation | 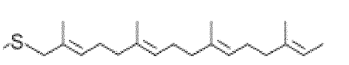 |
| Cholesterol | 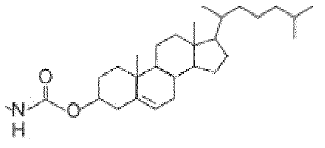 |

In some embodiments, the scaffold protein, e.g., exosome lumen protein, comprises an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to the mature form of SEQ ID NO: 1 (MARCKS), SEQ ID NO: 2 (MARCKSL1), or SEQ ID NO: 3 (BASP1), *i.e.,* without the N-terminal methionine amino acid present in SEQ ID NO: 1, SEQ ID NO: 2, or SEQ ID NO: 3.

In some embodiments, the scaffold protein, e.g., exosome lumen protein, comprises an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to a functional fragment of the mature form of SEQ ID NO: 1 (MARCKS), SEQ ID NO: 2 (MARCKSL1), or SEQ ID NO: 3 (BASP1), *i.e.,* without the N-terminal methionine amino acid present in SEQ ID NO: 1, SEQ ID NO: 2, or SEQ ID NO: 3.

In some aspects, a biologically active molecule attached to the scaffold protein of the present disclosure is on the luminal side of the EV *(e.g.,* an exosome).

In some aspects, the scaffold protein, e.g., exosome lumen protein, further comprises a transmembrane domain. In some aspects, the transmembrane domain is interposed between the ED domain of the scaffold protein, which is located on the luminal side of the EV *(e.g.,* an exosome) and the payload, *e.g.,* a biologically active molecule. Thus, the payload, *e.g.,* a biologically active molecule, is anchored to the external surface of the EV *(e.g.,* an exosome).

In some aspects, the scaffold protein, e.g., exosome lumen protein, further comprises an extravesicular domain. In some embodiments, the extravesicular domain is interposed between the transmembrane domain and the biologically active molecule.

In some aspects, the scaffold protein is linked to the biologically active molecule via at least one linker, *e.g.,* a peptide linker. In some aspects, the ND is linked to the ED by a linker directly interposed between both domains. In some aspects, the linker comprises, e.g., a peptide bond or one of more amino acids. In some aspects, the linker comprises a cleavable linker. In some aspects, the linker comprises a flexible linker. In some aspects, the linker comprises a self-immolative linker.

Fusions between a scaffold protein of the present disclosure and biologically active molecule *(e.g.,* a molecule having a therapeutic activity) can be also used. For example, the fusion protein can comprise a scaffold protein such as MARCKS, MARCKSL1, BASP1, or a modification thereof, in particular a fragment or variant thereof, and a payload, *e.g.,* a biologically active molecule *(e.g.,* a therapeutic peptide). In some aspects, the fusion protein comprises a scaffold protein comprising a fragment of the amino terminus of BASP1. In some aspects, the biologically active molecule comprises a protein, a polypeptide, a peptide, a polynucleotide (DNA and/or RNA), a chemical compound, a virus, an ionophore, a carrier for an ionophore, a moiety that forms a channel or a pore, or any combination thereof.

The biologically active molecule *(e.g.,* a therapeutic peptide) can be selected from a group consisting of a natural peptide, a recombinant peptide, a synthetic peptide, fusion protein, or a linker to a therapeutic compound. The biologically active molecule *(e.g.,* a therapeutic compounds) can also be a nucleotide, amino acid, lipid, carbohydrate, or a small molecule. The biologically active molecule *(e.g.,* a therapeutic peptide) can be an antibody, an enzyme, a ligand, an antigen *(e.g.,* a tumor antigen or an antigen from an infectious agent such as a bacteria, virus, fungus, or protozoa), a receptor, an antimicrobial peptide, a transcription factor, or a fragment or a modification thereof.

The fusion protein comprising the scaffold of the present disclosure fused or conjugated to a payload, *e.g.,* a biologically active molecule, can be attached to the luminal surface of the EV, *e.g.,* an exosomes, and provide, *e.g.,* a therapeutic activity to the EV, *e.g.,* exosome.

In some embodiments, the biologically active molecule *(e.g.,* a therapeutic peptide) is a component of a genome editing complex. In some embodiments, said genome editing complex is a transcription activator-like effector nuclease (TAL-effector nuclease or TALEN); a zinc finger nuclease (ZFN); a recombinase; a CRISPR/Cas9 complex, a CRISPR/Cpf1 complex, a CRISPR/C2c1, C2c2, or C2c3 complex, a CRISPR/CasY or CasX complex, or any other appropriate CRISPR complex known in the art; or any other appropriate genome editing complex known in the art or any combination thereof.

In some embodiments, the biologically active molecule *(e.g.,* a therapeutic peptide) is a transmembrane peptide. The transmembrane peptides described herein may be expressed as fusion proteins to any of the sequences described herein or any fragments or variants thereof. In some embodiments, the transmembrane protein has a first end fused to the luminal sequence in the lumen of the EV, *e.g*., exosome, and a second end expressed on the surface of the EV, *e.g*., exosome.

In some embodiments, an EV, *e.g.,* of the present disclosure can comprise a second scaffold protein. In some embodiments, the second scaffold protein comprises a transmembrane protein comprising a PTGFRN polypeptide, a BSG polypeptide, an IGSF2 polypeptide, an IGSF3 polypeptide, an IGSF8 polypeptide, an ITGB1 polypeptide, an ITGA4 polypeptide, a SLC3A2 polypeptide, an ATP transporter polypeptide, an aminopeptidase N (ANPEP) polypeptide, an ectonucleotide pyrophosphatase/phosphodiesterase family 1 (ENPP1) polypeptide, a neprilysin (MME) polypeptide, a neuropilin-1 (NRP1) polypeptide, or a fragment, variant, or derivative thereof. Non limiting examples of the second scaffold protein can be found at US Patent No. 10,195,290 and PCT Publication No. WO 2019/040920.

In some embodiments, the biologically active molecule *(e.g.,* a therapeutic peptide) is a nucleic acid binding protein. In some embodiments, the nucleic acid binding protein is Dicer, an Argonaute protein, TRBP, MS2 bacteriophage coat protein. In some embodiments, the nucleic acid binding protein additionally comprises one or more RNA or DNA molecules. In some embodiments, the one or more RNA is a miRNA, siRNA, guide RNA, lincRNA, mRNA, antisense RNA, dsRNA, or combinations thereof.

In some embodiments, the biologically active molecule *(e.g.,* a therapeutic peptide) is a part of a protein-protein interaction system. In some embodiments, the protein-protein interaction system comprises an FRB-FKBP interaction system, e.g., the FRB-FKBP interaction system as described in Banaszynski et al., J Am Chem Soc. 2005 Apr 6; 127(13):4715-21.

The fusion proteins can be targeted to the luminal surface of EVs, *e.g*., exosomes and provide a therapeutic activity to the EV, *e.g*., exosome.

In some embodiments, fusion proteins having a targeting moiety are used. For example, fusion proteins can comprise (i) a scaffold protein such as MARCKS, MARCKSL1, BASP1, or a fragment, variant, or a modification thereof, and (ii) a targeting moiety. The targeting moiety can be used for targeting the EV, *e.g*., exosome, to a specific organ, tissue, or cell for a treatment using the EV, *e.g*., exosome. In some embodiments, the targeting moiety is an antibody or antigen-binding fragment thereof.

Antibodies and antigen-binding fragments thereof include whole antibodies, polyclonal, monoclonal and recombinant antibodies, fragments thereof, and further includes single-chain antibodies, humanized antibodies, murine antibodies, chimeric, mouse-human, mouse-primate, primate-human monoclonal antibodies, anti-idiotype antibodies, antibody fragments, such as, *e.g*., scFv, (scFv)₂, Fab, Fab', and F(ab')₂, F(ab1)₂, Fv, dAb, and Fd fragments, diabodies, minibodies, camelid antibodies, and antibody-related polypeptides.

Antibodies and antigen-binding fragments thereof also includes bispecific antibodies and multispecific antibodies so long as they exhibit the desired biological activity or function. The modular architecture of antibodies has been exploited to create more than 60 different bispecific antibody formats. See Spiess et al. (2015) Molecular Immunology 67:95-106. Accordingly, in some embodiments, the bispecific antibody format is selected from crossMab, DAF (Dual Action Fab) (two-in-one), DAF (four-in-one), DutaMab, DT-IgG, Knobs-in-holes common LC, Knobs-in-holes assembly, Charge pair, Fab-arm exchange, SEEDbody, Triomab, LUZ-Y (bispecific antibody with a leucize zipper inducing heterodimerization of two HCs), Fcab, Kλ-body, Orthogonal Fab, DVD-IgG (dual variable domain IgG), IgG(H)-scFv, scFv-(H)IgG, IgG(L)-scFv, scFv-(L)IgG, IgG(L,H)-Fv, IgG(H)-V, V(H)-IgG, IgG(L)-V, V(L)-IgG, KIH IgG-scFab, 2scFv-IgG, IgG-2scFv, scFv4-Ig, Zybody, DVI-IgG (four-in-one), Nanobody, Nanobody-HSA, BiTE (bispecific T cell engager), Diabody, DART (dual-affinity-retargeting), TandAb (tandem antibody), scDiabody, scDiabody-CH3, Triple Body, Miniantibody, Minibody, TriBi minibody, scFv-CH3 KIH, Fab-scFv, scFv-CH-CL-scFv, F(ab')2, F(ab')2-ScFv2, scFv-KIH, Fab-scFv-Fc, Tetravalent HC Ab, scDiabody-Fc, Diabody-Fc, Tandem scFv-Fc, Intrabody, Dock and Locck, ImmTAC, HSAbody, scDiabody-HSA, Tandem scFv-Toxin, IgG-IgG, Cov-X-Body, and scFv1-PEG-scFV2. Bispecific antibodies can be monospecific antibodies engineered for bispecificity by appending either the amino or carboxy termini of either the light or heavy chains with additional antigen-binding units. Alternatives for these additional antigen-binding units include single domain antibodies (unpaired VL or VH), paired antibody variable domains (*e.g*., Fv or scFv) or engineered protein scaffolds. Numerous bispecific fragment forms lacking some or all of the bispecific antibody constant domains are known in the art.

In some embodiments, the biologically active molecule *(e.g.,* a therapeutic peptide) is a fusion protein comprising a scaffold protein of the present disclosure and a viral protein. In some embodiments, the viral protein comprises a viral capsid, an envelope protein, or a combination thereof. In some embodiments, the fusion proteins allow for the assembly of intact viruses that are retained on the luminal surface of an EV, *e.g.,* an exosome.

In some embodiments, the biologically active molecule is an inhibitor a negative checkpoint regulator or an inhibitor for a binding partner of a negative checkpoint regulator. In some embodiments, the negative checkpoint regulator is selected from the group consisting of: cytotoxic T-lymphocyte-associated protein 4 (CTLA-4), programmed cell death protein 1 (PD-1), lymphocyte-activated gene 3 (LAG-3), T-cell immunoglobulin mucin-containing protein 3 (TIM-3), B and T lymphocyte attenuator (BTLA), T cell immunoreceptor with Ig and ITIM domains (TIGIT), V-domain Ig suppressor of T cell activation (VISTA), adenosine A2a receptor (A2aR), killer cell immunoglobulin like receptor (KIR), indoleamine 2,3-dioxygenase (IDO), CD20, CD39, and CD73.

In some embodiments, the biologically active molecule is an immunogenic protein. In some embodiments, the biologically active molecule is a toxin, toxoid, or a non-toxic mutant of a toxin. In some embodiments, the toxin is diphtheria toxin. In some embodiments, the toxoid is a tetanus toxoid. In some embodiments, the diphtheria toxin is a non-toxic mutant of diphtheria toxin.

In some embodiments, the biologically active molecule is an activator for a positive co-stimulatory molecule or an activator for a binding partner of a positive co-stimulatory molecule. In some embodiments, the positive co-stimulatory molecule is a TNF receptor superfamily member selected from the group consisting of: CD120a, CD120b, CD18, OX40, CD40, Fas receptor, M68, CD27, CD30, 4-1BB, TRAILR1, TRAILR2, TRAILR3, TRAILR4, RANK, OCIF, TWEAK receptor, TACI, BAFF receptor, ATAR, CD271, CD269, AITR, TROY, CD358, TRAMP, and XEDAR. In some embodiments the activator for a positive co-stimulatory molecule is a TNF superfamily member selected from the group consisting of: TNFα, TNF-C, OX40L, CD40L, FasL, LIGHT, TL1A, CD27L, Siva, CD153, 4-1BB ligand, TRAIL, RANKL, TWEAK, APRIL, BAFF, CAMLG, NGF, BDNF, NT-3, NT-4, GITR ligand, and EDA-2. In some embodiments, the positive co-stimulatory molecule is a CD28-superfamily co-stimulatory molecule. In some embodiments, the CD28-superfamily co-stimulatory molecule is ICOS or CD28. In some embodiments, the activator for a positive co-stimulatory molecule is ICOSL, CD80, or CD86.

In some embodiments, the biologically active molecule is a cytokine selected from the group consisting of: IL-2, IL-7, IL-10, IL-12, and IL-15. In some embodiments, the biologically active molecule is a protein comprising a T-cell receptor (TCR), a T-cell co-receptor, a major histocompatibility complex (MHC), a human leukocyte antigen (HLA), or a derivative thereof. In some embodiments, the biologically active molecule is a protein comprising a tumor antigen. In some embodiments, the tumor antigen is selected from the group consisting of: alpha-fetoprotein (AFP), carcinoembryonic antigen (CEA), epithelial tumor antigen (ETA), mucin 1 (MUC1), Tn-MUC1, mucin 16 (MUC16), tyrosinase, melanoma-associated antigen (MAGE), tumor protein p53 (p53), CD4, CD8, CD45, CD80, CD86, programmed death ligand 1 (PD-L1), programmed death ligand 2 (PD-L2), NY-ESO-1, PSMA, TAG-72, HER2, GD2, cMET, EGFR, Mesothelin, VEGFR, alpha-folate receptor, CE7R, IL-3, Cancer-testis antigen, MART-1 gp100, and TNF-related apoptosis-inducing ligand.

In some aspects, fusion proteins that comprise a biologically active molecule and a scaffold protein of the present disclosure (*e.g*., MARCKS, MARCKSL1, BASP1, any of SEQ ID NO: 4-109, the corresponding sequence without the N-terminal M, or any scaffold protein sequences disclosed herein (e.g., Table 1), or a modification thereof, in particular a fragment or variant thereof), result in enrichment of the biologically active molecule in EVs, *e.g*., exosomes, compared to expression of the biologically active molecule lacking the scaffold protein. In some aspects, fusion proteins that comprises a biologically active molecule and a scaffold protein of the present disclosure (*e.g*., MARCKS, MARCKSL1, BASP1 without the N-terminal M, the amino acid sequence of SEQ ID NO: 4 to 114, 116 to 118, 122 to 150 or 180 to 190 without the N-terminal M, the amino acid sequence encoded by SEQ ID NO: 115, or 118 to 121, or any scaffold protein sequences disclosed herein (e.g., Table 1) without the N-terminal M) result in enrichment of the biologically active molecule in EVs, *e.g*., exosomes, compared to expression of the biologically active molecule lacking the scaffold protein.

In some embodiments, the scaffold protein useful for the present disclosure comprises any of SEQ ID NO: 4-109, the corresponding sequence without the N-terminal M, or any sequences disclosed in TABLE 1. In some aspects, the scaffold protein useful for the present disclosure comprises any of SEQ ID NO: 4-109, wherein the scaffold protein does not comprise an N-terminal Met. In some aspects, the scaffold protein comprises a sequence disclosed in Table 1, wherein the scaffold protein does not comprise an N-terminal Met. In some aspects, the scaffold protein useful for the present disclosure comprises MARCKS, MARCKSL1, BASP1 without the N-terminal M, the amino acid sequence of SEQ ID NO: 4 to 114, 116 to 118, 122 to 150 or 180 to 190 without the N-terminal M, the amino acid sequence encoded by SEQ ID NO: 115, or 118 to 121, or any scaffold protein sequences disclosed herein (e.g., Table 1) without the N-terminal M. In some aspects, the scaffold protein useful for the present disclosure comprises MARCKS, MARCKSL1, or BASP1, wherein the scaffold protein does not comprise an N-terminal Met. In some aspects, the scaffold protein comprises the amino acid sequence of SEQ ID NO: 4 to 114, 116 to 118, 122 to 150 or 180 to 190, wherein the scaffold protein does not comprise an N-terminal Met. In some aspects, the scaffold protein comprises the amino acid sequence encoded by SEQ ID NO: 115, or 118 to 121, wherein the scaffold protein does not comprise an N-terminal Met. In other embodiments, the scaffold protein useful for the present disclosure comprises any sequence disclosed herein, but is not any one of SEQ ID NO: 4-109, the corresponding sequence without the N-terminal M, or any sequences disclosed in TABLE 1 without the N-terminal M. In other embodiments, the scaffold protein useful for the present disclosure comprises any sequence disclosed herein, but does not comprise any one of SEQ ID NO: 4-109.

In some embodiments, the fusion proteins comprise a scaffold protein comprising a peptide with the sequence MGXKLSKKK, where X is alanine or any other amino acid (SEQ ID NO: 117); or a peptide with sequence of (M)(G)(π)(ξ)(Φ/π)(S/A/G/N)(+)(+), wherein the N-terminal M is cleaved, such that the resulting fusion protein comprises GXKLSKKK (SEQ ID NO: 425) or (G)(π)(ξ)(Φ/π)(S/A/G/N)(+)(+) joined to the biologically active molecule, wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). In some embodiments, the scaffold protein useful for the present disclosure is not MGXKLSKKK, where X is alanine or any other amino acid (SEQ ID NO: 117) or is not a peptide with sequence of (M)(G)(π)(ξ)(Φ/π)(S/A/G/N)(+)(+). In some embodiments, the scaffold protein useful for the present disclosure does not comprise MGXKLSKKK, where X is alanine or any other amino acid (SEQ ID NO: 117) or does not comprise a peptide with sequence of (M)(G)(π)(ξ)(Φ/π)(S/A/G/N)(+)(+).

In some embodiments, the fusion protein comprises a scaffold protein comprising a peptide with sequence of (M)(G)(π)(X)(Φ/π)(π)(+)(+) or (G)(π)(X)(Φ/π)(π)(+)(+), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). In some embodiments, the fusion protein comprises a scaffold protein comprising a peptide with sequence of (G)(π)(X)((Φ/π)(π)(+)(+), wherein the scaffold protein does not comprise an N-terminal M *(e.g.,* an N-terminal methionine).

In some aspects, the fusion protein comprises a scaffold protein comprising a peptide with sequence of (G)(π)(X)((Φ/π)(π)(+)(+), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu), wherein the sequence is not (M)(G)(π)(X)(Φ/π)(π)(+)(+). In some aspects, the scaffold protein comprises the peptide with sequence of (G)(π)(X)(Φ/π)(π)(+)(+), wherein the scaffold protein does not comprise an N-terminal Met.

In some embodiments, the conventional EV, *e.g*., exosome, protein is selected from the list consisting of CD9, CD63, CD81, PDGFR, GPI anchor proteins, LAMP2, LAMP2B, and a fragment, variant, or derivative thereof.

In some embodiments, the enrichment of the fusion protein comprising MARCKS, MARCKSL1, BASP1, or any of SEQ ID NO: 4-109 in exosomes is >2-fold, >4-fold, >8-fold, >16-fold, >25-fold, >50-fold, >100-fold, >200-fold, >500-fold, >750-fold, >1,000-fold, >2,000-fold, >5,000-fold, >7,500-fold, >10,000-fold higher than the fusion protein lacking MARCKS, MARCKSL1, BASP1, any of SEQ ID NO: 4-109 or compared to fusion proteins that comprise conventional EV, e.g., exosome, proteins. In some aspects, the enrichment of the fusion protein comprising MARCKS, MARCKSL1, BASP1 without the N-terminal M, any of SEQ ID NO: 4-109 without the N-terminal M or any sequences disclosed herein, e.g., TABLE 1, without the N-terminal M in exosomes is >2-fold, >4-fold, >8-fold, >16-fold, >25-fold, >50-fold, >100-fold, >200-fold, >500-fold, >750-fold, >1,000-fold, >2,000-fold, >5,000-fold, >7,500-fold, >10,000-fold higher than the fusion protein lacking MARCKS, MARCKSL1, BASP1 without the N-terminal M, any of SEQ ID NO: 4-109 without the N-terminal M or any sequences disclosed herein, *e.g*., TABLE 1, without the N-terminal M or compared to fusion proteins that comprise conventional EV, *e.g*., exosome, proteins.

In some embodiments, the enrichment of the fusion protein comprising MARCKS, MARCKSL1, BASP1, any of SEQ ID NO: 4-109, the corresponding sequence without the N-terminal M, or any scaffold protein sequences disclosed herein (e.g., Table 1) without the N-terminal M in exosomes is at least about 2-fold, at least about 4-fold, at least about 6-fold, at least about 8-fold, at least about 10-fold, at least about 12-fold, at least about 14-fold, at least about 16-fold, at least about 18-fold, at least about 20-fold, at least about 25-fold, at least about 30-fold, at least about 35-fold, at least about 40-fold, at least about 45-fold, at least about 50-fold, at least about 60-fold, at least about 70-fold, at least about 80-fold, at least about 90-fold, at least about100-fold, at least about 200-fold, at least about 300-fold, at least about 400-fold, at least about 500-fold, at least about 750-fold, at least about 1,000-fold, at least about 1,500-fold, at least about 2,000-fold, at least about 2,500-fold, at least about 3,000-fold, at least about 3,500-fold, at least about 4,000-fold, at least about 4,500-fold, at least about 5,000-fold, at least about 5,500-fold, at least about 6,000-fold, at least about 6,500-fold, at least about 7,000-fold, at least about 7,500-fold, at least about 8,000-fold, at least about 8,500-fold, at least about 9,000-fold, at least about 9,500-fold or at least about 10,000-fold higher than the fusion protein lacking MARCKS, MARCKSL1, BASP1, any of SEQ ID NO: 4-109, the corresponding sequence without the N-terminal M, or any scaffold protein sequences disclosed herein (e.g., Table 1) without the N-terminal M, or compared to fusion proteins that comprise conventional EV, *e.g*., exosome, proteins.

In some embodiments, the protein sequence of any of SEQ ID NO: 1-109, the corresponding sequence without the N-terminal M, or any scaffold protein sequences disclosed herein (e.g., Table 1) without the N-terminal M is sufficient to load the EVs, *e.g*., exosomes, with the fusion protein.

In some embodiments, the lumen-engineered EV, *e.g*., exosome, comprising a fusion protein containing an exogenous sequence (*e.g*., a biologically active molecule) and an EV, *e.g*., exosome, lumen protein (scaffold protein) newly-identified herein has a higher density of the fusion protein than similarly engineered EVs, *e.g*., exosomes, comprising an exogenous sequence conjugated to a conventional EV, *e.g.,* exosome, protein known in the art *(e.g.,* CD9, CD63, CD81, PDGFR, GPI anchor proteins, lactadherin, LAMP2, and LAMP2B, a fragment thereof, or a peptide that binds thereto).

In some embodiments, the fusion protein containing an EV, *e.g*., exosome, lumen protein (scaffold protein) newly-identified herein is present at 2-, 4-, 8-, 16-, 32-, 64-, 100-, 200-, 400-, 800-, 1,000-fold or a higher density in the EV, *e.g*., exosome, luminal surface than fusion proteins in other EV, *e.g*., exosome, luminal surfaces similarly modified using a conventional EV, *e.g*., exosome, protein.

In some embodiments, the fusion protein containing an EV, *e.g*., exosome, lumen protein (scaffold protein) newly-identified herein is present at 2 to 4-fold, 4 to 8-fold, 8 to 16-fold, 16 to 32-fold, 32 to 64-fold, 64 to 100-fold, 100 to 200-fold, 200 to 400-fold, 400 to 800-fold, 800 to 1,000-fold or to a higher density in the EV, *e.g*., exosome, luminal surface than fusion proteins in other EV, *e.g*., exosome, luminal surfaces similarly modified using a conventional EV, *e.g*., exosome, protein.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD9.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD63.

In some embodiments, a fusion protein comprising a scaffold protein, e.g., exosome lumen protein, of the present disclosure (e.g., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD81.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using PDGFR.

In some embodiments, a fusion protein comprising a scaffold protein, e.g., exosome lumen protein, of the present disclosure (*e.g.*, MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using GPI anchor proteins.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using lactadherin.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using LAMP2.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using LAMP2B.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using an conventional protein.

In some embodiments, a fusion protein comprising a scaffold protein, e.g., exosome lumen protein, of the present disclosure (*e.g*., MARCKS, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes similarly modified using a variant of a conventional EV, *e.g*., exosome, protein.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD9.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD63.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD81.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using PDGFR.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher, density than at EVs, *e.g*., exosomes, similarly modified using GPI anchor proteins.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using lactadherin.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using LAMP2.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using LAMP2B.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using an conventional protein.

In some embodiments, a fusion protein comprising a scaffold protein, e.g., exosome lumen protein, of the present disclosure (*e.g*., MARCKSL1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes similarly modified using a variant of a conventional EV, e.g., exosome, protein.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD9.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD63.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using CD81.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using PDGFR.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using GPI anchor proteins.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using lactadherin.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using LAMP2.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using LAMP2B.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using an conventional protein.

In some embodiments, a fusion protein comprising a scaffold protein, e.g., exosome lumen protein, of the present disclosure (*e.g*., BASP1, a variant, a fragment, a variant of a fragment, or a modification thereof) is present at 2-, 4-, 8-, 16-, 32-, 64-, 100-, 200-, 400-, 800-, 1,000-fold or a higher density than at EVs, *e.g*., exosomes, similarly modified using a variant of a conventional EV, *e.g*., exosome protein.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using CD9. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using CD9.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using CD63. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using CD63.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using CD81. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using CD81.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using PDGFR. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using PDGFR.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using GPI anchor proteins. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using GPI anchor proteins.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using lactadherin. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using lactadherin.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using LAMP2. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using LAMP2.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using LAMP2B. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using LAMP2Bt.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using a conventional protein. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g*., exosomes, similarly modified using a conventional protien.

In some embodiments, a fusion protein comprising any of SEQ ID NO: 1-109, the corresponding sequence without the N-terminal M or any scaffold protein sequences disclosed herein (e.g., Table 1) without the N-terminal M is present at 2-, 4-, 8-, 16-, 32-, 64-, 100-, 200-, 400-, 800-, 1,000-fold or a higher density than at EVs, *e.g*., exosomes, similarly modified using a variant of a conventional EV protein, *e.g.*, exosome protein. In some aspects, the EV, *e.g*., exosome, comprises a fusion protein comprising any of SEQ ID NO: 1-109, wherein the fusion protein does not comprise an N-terminal Met, and wherein the fusion protein is present at 2-, 4-, 8-, 16-, 32-, 64-, 100-, 200-, 400-, 800-, 1,000-fold or a higher density than at EVs, *e.g.,* exosomes, similarly modified using a conventional EV protein, *e.g*., exosome protein

In some embodiments, the lumen-engineered EVs, *e.g*., exosomes, described herein demonstrate superior characteristics compared to lumen-engineered EVs, *e.g*., exosomes known in the art. For example, lumen-engineered EVs, *e.g*., exosomes, produced by using the newly-identified EV, *e.g*., exosome, proteins provided herein contain modified proteins more highly enriched on their luminal surface compared to EVs, *e.g.,* exosomes in prior art, *e.g.,* those produced using conventional EV, *e.g*., exosome, proteins.

Moreover, the lumen-engineered EVs, *e.g*., exosomes, of the present disclosure can have greater, more specific, or more controlled biological activity compared to lumen-engineered EVs, *e.g.,* exosomes, known in the art. For example, a lumen-engineered EV, *e.g.,* exosome, comprising a payload, *e.g.,* a therapeutic or biologically relevant exogenous sequence fused to an EV, *e.g.,* exosome, protein or a fragment thereof described herein (*e*.*g*., a scaffold of the present disclosure such as BASP1 or a fragment, variant, or derivative thereof) can have more of the desired engineered characteristics than fusion to scaffolds known in the art.

Scaffold proteins known in the art include tetraspanin molecules (*e.g*., CD63, CD81, CD9 and others), lysosome-associated membrane protein 2 (LAMP2 and LAMP2B), platelet-derived growth factor receptor (PDGFR), GPI anchor proteins, lactadherin and fragments thereof, and peptides that have affinity to any of these proteins or fragments thereof. For the avoidance of doubt, PTGFRN, BSG, IGSF2, IGSF3, IGSF8, ITGB1, ITGA4, SLC3A2, ATP transporter or a fragment or a variant thereof are not conventional EV, *e.g*., exosome, proteins. Previously, overexpression of exogenous proteins relied on stochastic or random disposition of the exogenous proteins into the exosome for producing lumen-engineered exosomes. This resulted in low-level, unpredictable density of the exogenous proteins in exosomes. Thus, the EV, e.g., exosome proteins and fragments thereof described herein provide important advancements in novel EV, *e.g*., exosome compositions and methods of making the same.

Fusion proteins provided herein can comprise a scaffold protein, e.g., exosome lumen protein, of the present disclosure such as MARCKS, MARCKSL1, BASP1, or a fragment, a variant, or a derivative thereof, and an additional peptide. The additional peptide can be attached to either the N-terminus or the C-terminus of the EV, *e.g*., exosome, protein (scaffold protein) or a fragment, a variant, or a derivative thereof.

In some embodiments, fusion proteins provided herein comprise a scaffold protein of the present disclosure such as MARCKS, MARCKSL1, BASP1, or a fragment, a variant, or a derivative thereof, and two additional peptides. Both of the two additional peptides can be attached to either the N-terminus or the C-terminus of the EV, *e.g*., exosome, protein (scaffold protein) or a fragment, a variant, or derivative thereof. In some embodiments, one of the two additional peptides is attached to the N-terminus and the other of the two additional peptides is attached to the C-terminus of the EV, *e.g*., exosome, protein (scaffold protein) or a fragment, a variant, or a derivative thereof.

In some embodiments, the compositions and methods of generating lumen-engineered extracellular vesicles described herein comprise nanovesicles.

### IV. Producer Cell For Production Of Lumen-engineered EVs, e.g., Exosomes

EV, *e.g*., exosomes, of the present disclosure can be produced from a cell grown *in vitro* or a body fluid of a subject. When EVs, *e.g.,* exosomes, are produced from *in vitro* cell culture, various producer cells, *e.g*., HEK293 cells, can be used for the present disclosure. Additional cell types that can be used for the production of the lumen-engineered EVs, *e.g.,* exosomes, described herein include, without limitation, mesenchymal stem cells, T-cells, B-cells, dendritic cells, macrophages, and cancer cell lines.

Accordingly, the present disclosure provides cells that produce the EVs, *e.g.,* exosomes, of the present disclosure. In some embodiments, the comprises one or more vectors, wherein the vectors comprise a nucleic acid sequence encoding the scaffold protein and the payload, *e.g.,* a biologically active molecule. In some embodiments, a nucleic acid sequence encodes the scaffold protein and a second nucleic acid sequence encodes the payload, *e.g.,* a biologically active molecule. In some embodiments, the nucleic acid sequence encoding the scaffold protein and the nucleic acid sequence encoding the payload, *e.g.,* a biologically active molecule, are in a single open reading frame; thus, the expression product would be a fusion protein comprising the scaffold protein fused to the payload, *e.g.,* a biologically active molecule. In some embodiments, the nucleic acid sequence is operably linked to a promoter.

The producer cell can be genetically modified to comprise one or more exogenous sequences to produce lumen-engineered EVs, *e.g*., exosomes. The genetically-modified producer cell can contain the exogenous sequence by transient or stable transformation. The exogenous sequence can be transformed as a plasmid. The exogenous sequences can be stably integrated into a genomic sequence of the producer cell, at a targeted site or in a random site. In some embodiments, a stable cell line is generated for production of lumen-engineered EVs, *e.g*., exosomes.

The exogenous sequences can be inserted into a genomic sequence of the producer cell, located within, upstream (5'-end) or downstream (3'-end) of an endogenous sequence encoding the EV, *e.g*., exosome, protein. Various methods known in the art can be used for the introduction of the exogenous sequences into the producer cell. For example, cells modified using various gene editing methods (*e.g*., methods using a homologous recombination, transposon-mediated system, loxP-Cre system, CRISPR/Cas9 or TALEN) are within the scope of the present disclosure.

The exogenous sequences can comprise a sequence encoding a scaffold protein of the present disclosure (*e.g*., an EV, *e.g.,* exosome, protein or a modification or a fragment of the EV, *e.g*., exosome, protein). An extra copy of the sequence encoding the scaffold protein (*e.g.,* an EV, *e.g.,* exosome, protein) can be introduced to produce a lumen-engineered EV, *e.g.,* exosome, having a higher density of the EV, *e.g*., exosome, protein. An exogenous sequence encoding a modification or a fragment of the EV, *e.g*., exosome, protein can be introduced to produce a lumen-engineered EV, *e.g*., exosome, containing the modification or the fragment of the EV, *e.g*., exosome, protein. An exogenous sequence encoding an affinity tag can be introduced to produce a lumen-engineered EV, *e.g*., exosome, containing a fusion protein comprising the affinity tag attached to the EV, *e.g*., exosome, protein.

In some embodiments, a lumen-engineered EV, *e.g*., exosome, has a higher density of the EV, *e.g.,* exosome, protein (scaffold protein) than native EVs, *e.g.,* exosomes, isolated from the same or similar producer cell types. In some embodiments, said EV, *e.g.,* exosome, protein (scaffold protein) is present at 2-, 4-, 8-, 16-, 32-, 64-, 100-, 200-, 400-, 800-, 1,000-fold or to a higher density on said lumen-engineered EV, *e.g*., exosome, than the native EV< *e.g.,* exosome. In some embodiments, the EV, *e.g.,* exosome, protein (scaffold protein) is present at 2 to 4-fold, 4 to 8-fold, 8 to 16-fold, 16 to 32-fold, 32 to 64-fold, 64 to 100-fold, 100 to 200-fold, 200 to 400-fold, 400 to 800-fold, 800 to 1,000-fold or to a higher density on the lumen-engineered EV, *e.g.,* exosome, than the native EV, *e.g.,* exosome. In some embodiments, a fusion protein comprising the EV, *e.g*., exosome, protein (scaffold protein) is present at 2 to 4-fold, 4 to 8-fold, 8 to 16-fold, 16 to 32-fold, 32 to 64-fold, 64 to 100-fold, 100 to 200-fold, 200 to 400-fold, 400 to 800-fold, 800 to 1,000-fold or to a higher density on said lumen-engineered EV, *e.g.,* exosome, than the unmodified EV, *e.g.,* exosome, protein (scaffold protein) on the native EV, *e.g.,* exosome. In some embodiments, a fragment or a variant of the EV, *e.g.,* exosome, protein (scaffold protein) is present at 2 to 4-fold, 4 to 8-fold, 8 to 16-fold, 16 to 32-fold, 32 to 64-fold, 64 to 100-fold, 100 to 200-fold, 200 to 400-fold, 400 to 800-fold, 800 to 1,000-fold or to a higher density on the lumen-engineered EV, *e.g*., exosome, than the unmodified EV, *e.g.,* exosome, protein (scaffold protein) on the native EV, *e.g.,* exosome.

In particular embodiments, MARCKS, a fragment or a variant of MARCKS, or a modification thereof is present at 2 to 4-fold, 4 to 8-fold, 8 to 16-fold, 16 to 32-fold, 32 to 64-fold, 64 to 100-fold, 100 to 200-fold, 200 to 400-fold, 400 to 800-fold, 800 to 1,000-fold or to a higher density on said lumen-engineered EV, *e.g*., exosome, than the unmodified MARCKS on said native EV, *e.g*., exosome. In some embodiments, MARCKSL1, a fragment or a variant of MARCKSL1, or a modification thereof is present at 2 to 4-fold, 4 to 8-fold, 8 to 16-fold, 16 to 32-fold, 32 to 64-fold, 64 to 100-fold, 100 to 200-fold, 200 to 400-fold, 400 to 800-fold, 800 to 1,000-fold or to a higher density on said lumen-engineered EV, *e.g*., exosome, than the unmodified MARCKSL1 on said native EV, *e.g*., exosome. In some embodiments, BASP1, a fragment or a variant of BASP1, or a modification thereof is present at 2 to 4-fold, 4 to 8-fold, 8 to 16-fold, 16 to 32-fold, 32 to 64-fold, 64 to 100-fold, 100 to 200-fold, 200 to 400-fold, 400 to 800-fold, 800 to 1,000-fold or to a higher density on said lumen-engineered EV, *e.g*., exosome, than the unmodified BASP1 on the native EV, *e.g*., exosome.

In some embodiments, the producer cell is further modified to comprise an additional exogenous sequence. For example, an additional exogenous sequence can be included to modulate endogenous gene expression, or produce an exosome including a certain polypeptide as a payload. In some embodiments, the producer cell is modified to comprise two exogenous sequences, one encoding the EV, *e.g*., exosome, protein (scaffold protein) or a modification or a fragment of the EV, *e.g*., exosome, protein (scaffold protein), and the other encoding a payload.

More specifically, lumen-engineered EVs, *e.g*., exosomes, can be produced from a cell transformed with a sequence encoding one or more exosome lumen proteins (scaffold proteins) including, but not limited to, (1) myristoylated alanine rich Protein Kinase C substrate (MARCKS); (2) myristoylated alanine rich Protein Kinase C substrate like 1 (MARCKSL1); and (3) brain acid soluble protein 1 (BASP1). Any of the one or more EV, e.g., exosome, lumen proteins (scaffold proteins) described herein can be expressed from a plasmid, an exogenous sequence inserted into the genome or other exogenous nucleic acid such as a synthetic messenger RNA (mRNA).

In some embodiments, the one or more EV, *e.g*., exosome, lumen protein (scaffold protein) is expressed in a cell transformed with an exogenous sequence encoding its full length, endogenous form. In some embodiments, such an exogenous sequence encodes MARCKS protein of SEQ ID NO: 1 or the corresponding sequence without the N-terminal M. In certain aspects, such an exogenous sequence encodes MARCKS protein of SEQ ID NO: 1. In some embodiments, such an exogenous sequence encodes MARCKS protein of SEQ ID NO: 1, wherein the MARKS protein encoded by the exogenous sequence does not comprise an N-terminal Met. In some embodiments, such an exogenous sequence encodes MARCKSL1 protein of SEQ ID NO: 2 or the corresponding sequence without the N-terminal M. In certain aspects, such an exogenous sequence encodes MARCKSL1 protein of SEQ ID NO: 2. In some embodiments, such an exogenous sequence encodes MARCKSL1 protein of SEQ ID NO: 2, wherein the MARCKSL1 protein encoded by the exogenous sequence does not comprise an N-terminal Met. In some embodiments, such an exogenous sequence encodes BASP1 protein of SEQ ID NO: 3 or the corresponding sequence without the N-terminal M. In certain aspects, such an exogenous sequence encodes BASP1 protein of SEQ ID NO: 3. In some embodiments, such an exogenous sequence encodes BASP1 protein of SEQ ID NO: 3, wherein the BASP1 protein encoded by the exogenous sequence does not comprise an N-terminal Met.

Lumen-engineered EVs, *e.g*., exosomes, can be produced from a cell transformed with a polynucleotide sequence encoding a fragment of one or more EV, *e.g*., exosome, lumen proteins (scaffold proteins) including, but not limited to, (1) myristoylated alanine rich Protein Kinase C substrate (MARCKS); (2) myristoylated alanine rich Protein Kinase C substrate like 1 (MARCKSL1); and (3) brain acid soluble protein 1 (BASP1).

In some embodiments, the polynucleotide sequence encodes a fragment of the EV, *e.g.,* exosome, lumen protein *(e.g.,* a scaffold protein such as MARCKS, MARCKSL1, or BASP1) lacking at least 5, 10, 50, 100, 200, or 300 amino acids from the N-terminus of the native protein. In some embodiments, the polynucleotide sequence encodes a fragment of the EV, *e.g.,* exosome, lumen protein *(e.g.,* a scaffold protein such as MARCKS, MARCKSL1, or BASP1) lacking at least 5, 10, 50, 100, 200, or 300 amino acids from the C-terminus of the native protein. In some embodiments, the polynucleotide sequence encodes a fragment of the EV, *e.g.,* exosome, lumen protein *(e.g.,* a scaffold protein such as MARCKS, MARCKSL1, or BASP1) lacking at least 5, 10, 50, 100, 200, or 300 amino acids from both the N-terminus and C-terminus of the native protein. In some embodiments, the polynucleotide sequence encodes a fragment of the EV, *e.g.,* exosome, lumen protein *(e.g.,* a scaffold protein such as MARCKS, MARCKSL1, or BASP1) lacking one or more functional or structural domains of the native protein.

In some embodiments, the fusion protein comprises a peptide of SEQ ID NO: 4-109, the corresponding sequence without the N-terminal M, or any scaffold protein sequences disclosed herein (e.g., Table 1) without the N-terminal M. In some embodiments, the fusion protein comprises a peptide of SEQ ID NO: 4-109 or any scaffold protein sequence disclosed herein (e.g., Table 1), wherein the fusion protein does not comprise an N-terminal Met. In some embodiments, the fusion protein comprises the peptide of SEQ ID NO: 13. In some embodiments, the fusion protein comprises the peptide of SEQ ID NO: 13, wherein the fusion protein does not comprise an N-terminal Met. In some embodiments, the fusion protein comprises a scaffold protein, e.g., exosome lumen protein, comprising a peptide with the sequence MGXKLSKKK (SEQ ID NO: 117) or GXKLSKKK (SEQ ID NO:425), where X is alanine or any other amino acid. In some embodiments, the fusion protein comprises a scaffold protein, e.g., exosome lumen protein, comprising a peptide with the sequence GXKLSKKK (SEQ ID NO:425), where X is alanine or any other amino acid, and wherein the scaffold protein, e.g., exosome lumen protein, does not comprise an N-terminal Met. In some embodiments, the fusion protein comprises a scaffold protein, e.g., exosome lumen protein, comprising a peptide with the sequence GXKLSKKK (SEQ ID NO:425), where X is alanine or any other amino acid, and wherein the fusion protein does not comprise an N-terminal Met. In some embodiments, the fusion protein comprises a scaffold protein comprising the amino acid sequence of GXKLSKKK (SEQ ID NO:425), wherein X is alanine or any other amino acid, wherein the scaffold protein does not have Met at the N-terminus of the amino acid sequence, e.g., comprise MGXKLSKKK (SEQ ID NO: 117). In other embodiments, the fusion protein comprises a scaffold protein, e.g., exosome lumen protein, comprising an amino acid sequence of GXKLSKKK (SEQ ID NO: 425), wherein the fusion protein does not comprise MGXKLSKKK. In some embodiments, the fusion protein comprises a scaffold protein comprising a peptide with sequence of (M)(G)(π)(ξ)(Φ/π)(S/A/G/N)(+)(+) or (G)(π)(ξ)(Φ/π)(S/A/G/N)(+)(+), wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). In some embodiments, the fusion protein comprises a scaffold protein, e.g., exosome lumen protein, an amino acid sequence of (G)(π)(ξ)(Φ/π)(S/A/G/N)(+)(+), wherein the fusion protein does not comprise (M)(G)(π)(ξ)(Φ/π)(S/A/G/N)(+)(+). In some embodiments, the fusion protein comprises a scaffold protein, e.g., exosome lumen protein, comprising a peptide with sequence of (M)(O(π)(X)(Φ/π)(π)(+)(+) or (G)(π)(X)(Φ/π)(π)(+)(+), wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). In other embodiments, the fusion protein comprises a scaffold protein, e.g., exosome lumen protein, comprising an amino acid sequence of (G)(π)(X)((Φ/π)(π)(+)(+), wherein the fusion protein does not comprise (M)(G)(π)(X)(Φ/π)(π)(+)(+).

In some embodiments, lumen-engineered EVs, e.g., exosomes, can be produced from a cell transformed with a sequence encoding an EV, *e.g.,* exosome, protein *(e.g.,* a scaffold protein e.g., exosome lumen protein, such as MARCKS, MARCKSL1, or BASP1) or a fragment or a modification thereof fused to one or more heterologous proteins. In some embodiments, the one or more heterologous proteins are fused to the N-terminus of the EV, e.g., exosome, protein *(e.g.,* a scaffold protein such as MARCKS, MARCKSL1, or BASP1) or a modification thereof, in particular a fragment or variant thereof. In some embodiments, the one or more heterologous proteins are fused to the C-terminus of the EV, *e.g.,* exosome, protein *(e.g.,* a scaffold protein such as MARCKS, MARCKSL1, or BASP1) or a modification thereof, in particular a fragment or variant thereof. In some embodiments, the one or more heterologous proteins are fused to the N-terminus and the C-terminus of the EV, *e.g.,* exosome, protein *(e.g.,* a scaffold protein such as MARCKS, MARCKSL1, or BASP1) or a modification thereof, in particular a fragment or variant thereof. In some embodiments, the one or more heterologous proteins are mammalian proteins. In some embodiments, the one or more heterologous proteins are human proteins.

In some embodiments lumen-engineered EVs, *e.g*., exosomes, are produced from a cell transformed with a sequence encoding a polypeptide of a sequence identical or similar to a full-length or a fragment of a native EV, *e.g*., exosome, lumen protein (scaffold protein) including, but not limited to,
(1) myristoylated alanine rich Protein Kinase C substrate (MARCKS);
(2) myristoylated alanine rich Protein Kinase C substrate like 1 (MARCKSL1); and
(3) brain acid soluble protein 1 (BASP1).

In some embodiments, the polypeptide is at least about 50% identical to a full-length or a fragment of a native EV, *e.g.,* exosome, lumen protein (scaffold protein), *e.g.,* 50% identical to SEQ ID NO: 1-3 or any of the corresponding sequences without the N-terminal M. In some embodiments, the polypeptide is at least about 55% identical to a full-length or a fragment of a native EV, *e.g.,* exosome, lumen protein (scaffold protein), *e.g.,* at least about 55% identical to SEQ ID NO: 1-3 or any of the corresponding sequences without the N-terminal M. In some embodiments, the polypeptide is at least about 60% identical to a full-length or a fragment of a native EV, *e.g.,* exosome, lumen protein (scaffold protein), *e.g.,* at least about 60% identical to SEQ ID NO: 1-3 or any of the corresponding sequences without the N-terminal M. In some embodiments, the polypeptide is at least about 65% identical to a full-length or a fragment of a native EV, *e.g.,* exosome, lumen protein (scaffold protein), *e.g.,* at least about 65% identical to SEQ ID NO: 1-3 or any of the corresponding sequences without the N-terminal M. In some embodiments, the polypeptide is at least about 70% identical to a full-length or a fragment of a native EV, *e.g.,* exosome, lumen protein (scaffold protein), *e.g.,* at least about 70% identical to SEQ ID NO: 1-3 or any of the corresponding sequences without the N-terminal M. In some embodiments, the polypeptide is at least about 75% identical to a full-length or a fragment of a native EV, *e.g.,* exosome, lumen protein (scaffold protein), *e.g.,* at least about 75% identical to SEQ ID NO: 1-3 or any of the corresponding sequences without the N-terminal M. In some embodiments, said polypeptide is at least about 80% identical to a full-length or a fragment of a native EV, *e.g.,* exosome, lumen protein (scaffold protein), *e.g.,* at least about 80% identical to SEQ ID NO: 1-3 or any of the corresponding sequences without the N-terminal M. In some embodiments, the polypeptide is at least about 85% identical to a full-length or a fragment of a native EV, *e.g.,* exosome, lumen protein (scaffold protein), *e.g.,* at least about 85% identical to SEQ ID NO: 1-3 or any of the corresponding sequences without the N-terminal M. In some embodiments, said polypeptide is at least about 90% identical to a full-length or a fragment of a native EV, *e.g.,* exosome, lumen protein (scaffold protein), *e.g.,* at least about 90% identical to SEQ ID NO: 1-3 or any of the corresponding sequences without the N-terminal M. In some embodiments, the polypeptide is at least about 95% identical to a full-length or a fragment of a native EV, *e.g.,* exosome, lumen protein (scaffold protein), *e.g.,* at least about 95% identical to SEQ ID NO: 1-3 or any of the corresponding sequences without the N-terminal M. In some embodiments, the polypeptide is at least about 99% identical to a full-length or a fragment of a native EV, *e.g.,* exosome, lumen protein (scaffold protein), *e.g.,* at least about 99% identical to SEQ ID NO: 1-3 or any of the corresponding sequences without the N-terminal M. In some embodiments, the polypeptide is at least about 99.9% identical to a full-length or a fragment of a native EV, *e.g.,* exosome, lumen protein (scaffold protein), *e.g.,* at least about 99.9% identical to SEQ ID NO: 1-3 or any of the corresponding sequences without the N-terminal M.

In some embodiments, lumen-engineered EVs, *e.g*., exosomes, produced from the cell comprise a polypeptide of a sequence identical or similar to a fragment of brain acid soluble protein 1 (BASP1). In some embodiments, the polypeptide is at least about 50% identical to a full-length or a fragment of BASP1, *e.g.,* at least about 50% identical to SEQ ID NO: 4-109, the corresponding sequence without the N-terminal M, or any BASP1 sequences described herein, e.g., TABLE 1, without the N terminal M. In some embodiments, the polypeptide is at least about 55% identical to a full-length or a fragment of BASP1, *e.g.,* at least about 50% identical to SEQ ID NO: 4-109, the corresponding sequence without the N-terminal M, or any BASP1 sequences described herein, e.g., TABLE 1, without the N terminal M. In some embodiments, said polypeptide is at least about 60% identical to a full-length or a fragment of BASP1, *e.g.,* at least about 60% identical to SEQ ID NO: 4-109, the corresponding sequence without the N-terminal M, or any BASP1 sequences described herein, e.g., TABLE 1, without the N terminal M. In some embodiments, the polypeptide is at least about 65% identical to a full-length or a fragment of BASP1, *e.g.,* at least about 65% identical to SEQ ID NO: 4-109, the corresponding sequence without the N-terminal M, or any BASP1 sequences described herein, e.g., TABLE 1, without the N terminal M. In some embodiments, the polypeptide is at least about 70% identical to a full-length or a fragment of BASP1, *e.g.,* at least about 70% identical to SEQ ID NO: 4-109, the corresponding sequence without the N-terminal M, or any BASP1 sequences described herein, e.g., TABLE 1, without the N terminal M. In some embodiments, the polypeptide is at least about 75% identical to a full-length or a fragment of BASP1, *e.g.,* at least about 75% identical to SEQ ID NO: 4-109, the corresponding sequence without the N-terminal M, or any BASP1 sequences described herein, e.g., TABLE 1, without the N terminal M. In some embodiments, the polypeptide is at least about 80% identical to a full-length or a fragment of BASP1, *e.g.,* at least about 80% identical to SEQ ID NO4-109, the corresponding sequence without the N-terminal M, or any BASP1 sequences described herein, e.g., TABLE 1, without the N terminal M. In some embodiments, the polypeptide is at least about 85% identical to a full-length or a fragment of BASP1, *e.g.,* at least about 85% identical to SEQ ID NO: 4-109, the corresponding sequence without the N-terminal M, or any BASP1 sequences described herein, e.g., TABLE 1, without the N terminal M. In some embodiments, the polypeptide is at least about 90% identical to a full-length or a fragment of BASP1, *e.g.,* at least about 90% identical to SEQ ID NO: 4-109, the corresponding sequence without the N-terminal M, or any BASP1 sequences described herein, e.g., TABLE 1, without the N terminal M. In some embodiments, the polypeptide is at least about 95% identical to a full-length or a fragment of BASP1, *e.g.,* at least about 95% identical to SEQ ID NO: 4-109, the corresponding sequence without the N-terminal M, or any BASP1 sequences described herein, e.g., TABLE 1, without the N terminal M. In some embodiments, the polypeptide is at least about 99% identical to a full-length or a fragment of BASP1, *e.g.,* at least about 99% identical to SEQ ID NO: 4-109, the corresponding sequence without the N-terminal M, or any BASP1 sequences described herein, e.g., TABLE 1, without the N terminal M. In some embodiments, the polypeptide is at least about 99.9% identical to a full-length or a fragment of BASP1, *e.g.,* at least about 99.9% identical to SEQ ID NO: 4-109, the corresponding sequence without the N-terminal M, or any BASP1 sequences described herein, e.g., TABLE 1, without the N terminal M. In some embodiments, the polypeptide is about 100% identical to a fragment of BASP1, e.g., about 100% identical to 4-109, the corresponding sequence without the N-terminal M, or any BASP1 sequences described herein, e.g., TABLE 1, without the N terminal M.

In some embodiments, lumen-engineered EVs, *e.g*., exosomes, produced from the cell comprise a polypeptide of a sequence identical or similar to a fragment of brain acid soluble protein 1 (BASP1), wherein the polypeptide does not comprise an N-terminal Met. In some embodiments, the polypeptide is at least about 50% identical to a full-length or a fragment of BASP1, *e.g.,* at least about 50% identical to SEQ ID NO: 4-109 or any BASP1 sequences described herein, e.g., TABLE 1, wherein the polypeptide does not comprise an N-terminal Met. In some embodiments, the polypeptide is at least about 55% identical to a full-length or a fragment of BASP1, *e.g.,* at least about 50% identical to SEQ ID NO: 4-109 or any BASP1 sequences described herein, e.g., TABLE 1, wherein the polypeptide does not comprise an N-terminal Met. In some embodiments, said polypeptide is at least about 60% identical to a full-length or a fragment of BASP1, *e.g.,* at least about 60% identical to SEQ ID NO: 4-109 or any BASP1 sequences described herein, e.g., TABLE 1, wherein the polypeptide does not comprise an N-terminal Met. In some embodiments, the polypeptide is at least about 65% identical to a full-length or a fragment of BASP1, *e.g.,* at least about 65% identical to SEQ ID NO: 4-109 or any BASP1 sequences described herein, e.g., TABLE 1, wherein the polypeptide does not comprise an N-terminal Met. In some embodiments, the polypeptide is at least about 70% identical to a full-length or a fragment of BASP1, *e.g.,* at least about 70% identical to SEQ ID NO: 4-109 or any BASP1 sequences described herein, e.g., TABLE 1, wherein the polypeptide does not comprise an N-terminal Met. In some embodiments, the polypeptide is at least about 75% identical to a full-length or a fragment of BASP1, *e.g.,* at least about 75% identical to SEQ ID NO: 4-109 or any BASP1 sequences described herein, e.g., TABLE 1, wherein the polypeptide does not comprise an N-terminal Met. In some embodiments, the polypeptide is at least about 80% identical to a full-length or a fragment of BASP1, *e.g.,* at least about 80% identical to SEQ ID NO4-109 or any BASP1 sequences described herein, e.g., TABLE 1, wherein the polypeptide does not comprise an N-terminal Met. In some embodiments, the polypeptide is at least about 85% identical to a full-length or a fragment of BASP1, *e.g.,* at least about 85% identical to SEQ ID NO: 4-109 or any BASP1 sequences described herein, e.g., TABLE 1, wherein the polypeptide does not comprise an N-terminal Met. In some embodiments, the polypeptide is at least about 90% identical to a full-length or a fragment of BASP1, *e.g.,* at least about 90% identical to SEQ ID NO: 4-109 or any BASP1 sequences described herein, e.g., TABLE 1, wherein the polypeptide does not comprise an N-terminal Met. In some embodiments, the polypeptide is at least about 95% identical to a full-length or a fragment of BASP1, *e.g.,* at least about 95% identical to SEQ ID NO: 4-109 or any BASP1 sequences described herein, e.g., TABLE 1, wherein the polypeptide does not comprise an N-terminal Met. In some embodiments, the polypeptide is at least about 99% identical to a full-length or a fragment of BASP1, *e.g.,* at least about 99% identical to SEQ ID NO: 4-109 or any BASP1 sequences described herein, e.g., TABLE 1, wherein the polypeptide does not comprise an N-terminal Met. In some embodiments, the polypeptide is at least about 99.9% identical to a full-length or a fragment of BASP1, *e.g.,* at least about 99.9% identical to SEQ ID NO: 4-109 or any BASP1 sequences described herein, e.g., TABLE 1, wherein the polypeptide does not comprise an N-terminal Met. In some embodiments, the polypeptide is about 100% identical to a fragment of BASP1, *e.g*., about 100% identical to 4-109 or any BASP1 sequences described herein, e.g., TABLE 1, wherein the polypeptide does not comprise an N-terminal Met.

### VI. Methods of making

EVs (*e.g*., exosomes) of the present disclosure can be produced by chemical synthesis, recombinant DNA technology, biochemical or enzymatic fragmentation of larger molecules, combinations of the foregoing or by any other method. In one embodiment, the present disclosure provides a method of conjugating a biologically active molecule to an EV *(e.g.,* exosome). The method comprises linking a biologically active molecule to an EV *(e.g.,* exosome) as described above.

In some embodiments of the present disclosure, the EVs, e.g., exosomes of the present disclosure can be manufactured using producer cells as described above. Accordingly, the present disclosure provides a method of making EVs, *e.g*., exosomes, comprising culturing a producer cell disclosed herein under suitable conditions and obtaining the EVs, *e.g*., exosomes of the present disclosure.

In other embodiments, the scaffold proteins, e.g., exosome lumen proteins, of the present disclosure can be produced recombinantly and subsequently incorporated into an EV, *e.g*., exosome, of the present disclosure. In other embodiments, only the polypeptide portion of the scaffold protein is produced recombinantly. In some embodiments, the polypeptide portion of the scaffold protein produced recombinantly is subsequently modified, *e.g*., chemically or enzymatically, to incorporate a membrane anchor (*e.g*., an N-terminal myristic acid). In some embodiments, the scaffold protein produced semi-recombinantly (*i*.*e*., combining recombinant product of the polypeptide portion followed by chemical or enzymatic modification) is subsequently incorporated into an EV, *e.g*., exosome, of the present disclosure.

In other embodiments, the scaffold proteins, *e.g*., exosome lumen proteins, of the present disclosure can be produced chemically, *e.g*., using solid phase peptide synthesis, and subsequently incorporated into an EV, *e.g*., exosome, of the present disclosure. In other embodiments, only the polypeptide portion of the scaffold protein is produced synthetically. In some embodiments, the polypeptide portion of the scaffold protein produced synthetically is subsequently modified, *e.g*., chemically or enzymatically, to incorporate a membrane anchor (*e*.*g*., an N-terminal myristic acid). In some embodiments, the scaffold protein produced semi-synthetically (*i*.*e*., combining synthetic product of the polypeptide portion followed by chemical or enzymatic modification) is subsequently incorporated into an EV, *e.g*., exosome, of the present disclosure.

In other embodiments, the scaffold proteins, e.g., exosome lumen proteins, of the present disclosure can be produced *in vitro*, *e.g*., using a cell-free expression such a reticulocyte system, and subsequently incorporated into an EV, *e.g*., exosome, of the present disclosure. In other embodiments, only the polypeptide portion of the scaffold protein is produced *in vitro, e.g.,* in a cell-free system. In some embodiments, the polypeptide portion of the scaffold protein produced *in vitro*, *e.g*., in a cell-free system is subsequently modified, *e.g.,* chemically or enzymatically, to incorporate a membrane anchor (*e.g*., an N-terminal myristic acid). In some embodiments, the scaffold protein produced semi-*in vitro* (*i.e*., combining *in vitro* product of the polypeptide portion followed by chemical or enzymatic modification) is subsequently incorporated into an EV, *e.g*., exosome, of the present disclosure.

In some embodiments, the methods described herein further comprise the step of characterizing EVs, *e.g*., exosomes, contained in each collected fraction during their production and purification. In some embodiments, contents of the EVs, *e.g*., exosomes, can be extracted for study and characterization. In some embodiments, EVs, *e.g*., exosomes, are isolated and characterized by metrics including, but not limited to, size, shape, morphology, or molecular compositions such as nucleic acids, proteins, metabolites, and lipids.

### VII. Pharmaceutical Compositions and Methods of Administration

The present disclosure also provides pharmaceutical compositions comprising EVs (*e.g*., exosomes) described herein that are suitable for administration to a subject. The pharmaceutical compositions generally comprise a plurality of EVs (*e.g*., exosomes) comprising at least one payload, *e.g.,* a biologically active molecule, covalently or non-covalently linked to the plurality of EVs (*e.g*., exosomes) and a pharmaceutically-acceptable excipient or carrier in a form suitable for administration to a subject.

Pharmaceutically acceptable excipients or carriers are determined in part by the particular composition being administered, as well as by the particular method used to administer the composition. Accordingly, there is a wide variety of suitable formulations of pharmaceutical compositions comprising a plurality of EVs *(e.g.,* exosomes). (See, *e.g.,* Remington's Pharmaceutical Sciences, Mack Publishing Co., Easton, Pa. 18th ed. (1990)). The pharmaceutical compositions are generally formulated sterile and in full compliance with all Good Manufacturing Practice (GMP) regulations of the U.S. Food and Drug Administration. In some embodiments, the pharmaceutical composition comprises one or more chemical compounds, such as for example, small molecules covalently linked to an EV (*e.g*., exosome) described herein.

In some embodiments, a pharmaceutical composition comprises one or more therapeutic or diagnostic agents and an EV (*e.g*., exosome) described herein. In certain embodiments, the EVs (*e.g*., exosomes) are co-administered with of one or more additional therapeutic or diagnostic agents, in a pharmaceutically acceptable carrier. In some embodiments, the pharmaceutical composition comprising the EV (*e.g*., exosome) is administered prior to administration of the additional therapeutic or diagnostic agents. In other embodiments, the pharmaceutical composition comprising the EV (*e.g*., exosome) is administered after the administration of the additional therapeutic or diagnostic agents. In further embodiments, the pharmaceutical composition comprising the EV (*e.g*., exosome) is administered concurrently with the additional therapeutic or diagnostic agents.

Provided herein are pharmaceutical compositions comprising an EV (*e.g*., exosome) of the present disclosure having the desired degree of purity, and a pharmaceutically acceptable carrier or excipient, in a form suitable for administration to a subject. Pharmaceutically acceptable excipients or carriers can be determined in part by the particular composition being administered, as well as by the particular method used to administer the composition. Accordingly, there is a wide variety of suitable formulations of pharmaceutical compositions comprising a plurality of extracellular vesicles. (*See*, *e.g*., Remington's Pharmaceutical Sciences, Mack Publishing Co., Easton, Pa. 21st ed. (2005)). The pharmaceutical compositions are generally formulated sterile and in full compliance with all Good Manufacturing Practice (GMP) regulations of the U.S. Food and Drug Administration.

In some embodiments, a pharmaceutical composition comprises one or more therapeutic or diagnostic agents and an EV (*e.g*., exosome) described herein. In certain embodiments, the EVs (*e.g*., exosomes) are co-administered with of one or more additional therapeutic agents or diagnostic agents, in a pharmaceutically acceptable carrier. In some embodiments, the pharmaceutical composition comprising the EV (*e.g*., exosome) is administered prior to administration of the additional therapeutic or diagnostic agents. In other embodiments, the pharmaceutical composition comprising the EV (*e.g*., exosome) is administered after the administration of the additional therapeutic or diagnostic agents. In further embodiments, the pharmaceutical composition comprising the EV (*e.g*., exosome) is administered concurrently with the additional therapeutic or diagnostic agents.

Acceptable carriers, excipients, or stabilizers are nontoxic to recipients (*e.g*., animals or humans) at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN™, PLURONICS™ or polyethylene glycol (PEG).

Examples of carriers or diluents include, but are not limited to, water, saline, Ringer's solutions, dextrose solution, and 5% human serum albumin. The use of such media and compounds for pharmaceutically active substances is well known in the art. Except insofar as any conventional media or compound is incompatible with the extracellular vesicles described herein, use thereof in the compositions is contemplated. Supplementary therapeutic agents can also be incorporated into the compositions. Typically, a pharmaceutical composition is formulated to be compatible with its intended route of administration. The EVs (*e.g*., exosomes) of the present disclosure can be administered by parenteral, topical, intravenous, oral, subcutaneous, intra-arterial, intradermal, transdermal, rectal, intracranial, intraperitoneal, intranasal, intratumoral, intramuscular route or as inhalants. In certain embodiments, the pharmaceutical composition comprising EVs (*e.g*., exosomes) is administered intravenously, *e.g.* by injection. The EVs (*e*.*g*., exosomes) can optionally be administered in combination with other therapeutic agents that are at least partly effective in treating the disease, disorder or condition for which the EVs (*e.g*., exosomes) are intended.

Solutions or suspensions can include the following components: a sterile diluent such as water, saline solution, fixed oils, polyethylene glycols, glycerine, propylene glycol or other synthetic solvents; antibacterial compounds such as benzyl alcohol or methyl parabens; antioxidants such as ascorbic acid or sodium bisulfite; chelating compounds such as ethylenediaminetetraacetic acid (EDTA); buffers such as acetates, citrates or phosphates, and compounds for the adjustment of tonicity such as sodium chloride or dextrose. The pH can be adjusted with acids or bases, such as hydrochloric acid or sodium hydroxide. The preparation can be enclosed in ampoules, disposable syringes or multiple dose vials made of glass or plastic.

Pharmaceutical compositions suitable for injectable use include sterile aqueous solutions (if water soluble) or dispersions and sterile powders. For intravenous administration, suitable carriers include physiological saline, bacteriostatic water, Cremophor EL™ (BASF, Parsippany, N.J.) or phosphate buffered saline (PBS). The composition is generally sterile and fluid to the extent that easy syringeability exists. The carrier can be a solvent or dispersion medium containing, *e.g.,* water, ethanol, polyol *(e.g.,* glycerol, propylene glycol, and liquid polyethylene glycol, and the like), and suitable mixtures thereof. The proper fluidity can be maintained, *e.g*., by the use of a coating such as lecithin, by the maintenance of the required particle size in the case of dispersion and by the use of surfactants. Prevention of the action of microorganisms can be achieved by various antibacterial and antifungal compounds, e.g., parabens, chlorobutanol, phenol, ascorbic acid, thimerosal, and the like. If desired, isotonic compounds, e.g., sugars, polyalcohols such as mannitol, sorbitol, and sodium chloride can be added to the composition. Prolonged absorption of the injectable compositions can be brought about by including in the composition a compound which delays absorption, *e.g*., aluminum monostearate and gelatin.

Sterile injectable solutions can be prepared by incorporating the EVs (*e.g.*, exosomes) of the present disclosure in an effective amount and in an appropriate solvent with one or a combination of ingredients enumerated herein, as desired. Generally, dispersions are prepared by incorporating the EVs (*e.g*., exosomes) into a sterile vehicle that contains a basic dispersion medium and any desired other ingredients. In the case of sterile powders for the preparation of sterile injectable solutions, methods of preparation are vacuum drying and freeze-drying that yields a powder of the active ingredient plus any additional desired ingredient from a previously sterile-filtered solution thereof. The EVs (*e.g*., exosomes) can be administered in the form of a depot injection or implant preparation which can be formulated in such a manner to permit a sustained or pulsatile release of the EVs (*e.g*., exosomes).

Systemic administration of compositions comprising EVs (*e.g*., exosomes) of the present disclosure can also be by transmucosal means. For transmucosal administration, penetrants appropriate to the barrier to be permeated are used in the formulation. Such penetrants are generally known in the art, and include, *e.g.*, for transmucosal administration, detergents, bile salts, and fusidic acid derivatives. Transmucosal administration can be accomplished through the use of, *e.g.*, nasal sprays.

In certain embodiments the pharmaceutical composition comprising EVs (*e.g.*, exosomes) of the present disclosure is administered intravenously into a subject that would benefit from the pharmaceutical composition. In certain other embodiments, the composition is administered to the lymphatic system, *e.g*., by intralymphatic injection or by intranodal injection (*see e.g.*, Senti et a/., PNAS 105( 46): 17908 (2008)), or by intramuscular injection, by subcutaneous administration, by intratumoral injection, by direct injection into the thymus, or into the liver.

In certain embodiments, the pharmaceutical composition comprising EVs (*e.g.*, exosomes) of the present disclosure is administered as a liquid suspension. In certain embodiments, the pharmaceutical composition is administered as a formulation that is capable of forming a depot following administration. In certain preferred embodiments, the depot slowly releases the EVs (*e.g*., exosomes) into circulation, or remains in depot form.

Typically, pharmaceutically-acceptable compositions are highly purified to be free of contaminants, are biocompatible and not toxic, and are suited to administration to a subject. If water is a constituent of the carrier, the water is highly purified and processed to be free of contaminants, *e.g*., endotoxins.

The pharmaceutically-acceptable carrier can be lactose, dextrose, sucrose, sorbitol, mannitol, starch, gum acacia, calcium phosphate, alginates, gelatin, calcium silicate, micro-crystalline cellulose, polyvinylpyrrolidone, cellulose, water, syrup, methyl cellulose, methylhydroxy benzoate, propylhydroxy benzoate, talc, magnesium stearate, and/or mineral oil, but is not limited thereto. The pharmaceutical composition can further include a lubricant, a wetting agent, a sweetener, a flavor enhancer, an emulsifying agent, a suspension agent, and/or a preservative.

The pharmaceutical compositions described herein comprise the EVs (*e.g.*, exosomes) described herein and optionally a pharmaceutically active or therapeutic agent, and/or a diagnostic agent. The therapeutic agent can be a biological agent, a small molecule agent, or a nucleic acid agent. The diagnostic agent can be, *e.g.*, a contrast reagent.

Dosage forms are provided that comprise a pharmaceutical composition comprising the EVs (*e.g*., exosomes) described herein. In some embodiments, the dosage form is formulated as a liquid suspension for intravenous injection. In some embodiments, the dosage form is formulated as a liquid suspension for intratumoral injection.

In certain embodiments, the preparation of EVs (*e.g*., exosomes) of the present disclosure is subjected to radiation, *e.g*., X rays, gamma rays, beta particles, alpha particles, neutrons, protons, elemental nuclei, UV rays in order to damage residual replication-competent nucleic acids.

In certain embodiments, the preparation of EVs (*e.g*., exosomes) of the present disclosure is subjected to gamma irradiation using an irradiation dose of more than 1, 5, 10, 15, 20, 25, 30, 35, 40, 50, 60, 70, 80, 90, 100, or more than 100 kGy.

In certain embodiments, the preparation of EVs (*e.g*., exosomes) of the present disclosure is subjected to X-ray irradiation using an irradiation dose of more than 0.1, 0.5, 1, 5, 10, 15, 20, 25, 30, 35, 40, 50, 60, 70, 80, 90, 100, 200, 300, 400, 500, 600, 700, 800, 900, 1000, 2000, 3000, 4000, 5000, 6000, 7000, 8000, 9000, 10000, or

The EVs (e.g., exosomes) of the present disclosure may be used concurrently with other drugs. To be specific, the EVs (e.g., exosomes) of the present disclosure may be used together with medicaments such as hormonal therapeutic agents, chemotherapeutic agents, immunotherapeutic agents, medicaments inhibiting the action of cell growth factors or cell growth factor receptors and the like.

### VIII. Therapeutic Uses

The present disclosure provides methods of treating a disease or condition in a subject in need thereof comprising administering a composition comprising EVs (*e.g.*, exosomes) of the present disclosure to the subject. The present disclosure also provides methods of preventing or ameliorating the symptoms of a disease or condition in a subject in need thereof comprising administering a composition comprising EVs (*e.g*., exosomes) of the present disclosure to the subject. Also provided are methods to diagnose a disease or condition in a subject in need thereof comprising administering a composition comprising EVs (*e.g.*, exosomes) of the present disclosure to the subject. Also provided are EVs (*e.g*., exosomes) of the present disclosure for use in therapy, for use as a medicament, for use in treating a disease or condition in a subject in need thereof, for use in preventing or ameliorating the symptoms of a disease or condition in a subject in need thereof, or for diagnosing a disease or condition in a subject in need thereof.

In one embodiment, the disease or disorder is a cancer, an inflammatory disease, a neurodegenerative disorder, a central nervous disease or a metabolic disease.

Present disclosure also provides methods of preventing and/or treating a disease or disorder in a subject in need thereof, comprising administering an EV (*e.g*., exosome) disclosed herein to the subject. In some embodiments, a disease or disorder that can be treated with the present methods comprises a cancer, graft-versus-host disease (GvHD), autoimmune disease, infectious diseases, or fibrotic diseases. In some embodiments, the treatment is prophylactic. In other embodiments, the EVs (*e.g*., exosomes) for the present disclosure are used to induce an immune response. In other embodiments, the EVs (*e.g*., exosomes) for the present disclosure are used to vaccinate a subject. In some embodiments, the disease or disorder is a cancer.

In some embodiments, the disease or disorder is an infectious disease. In certain embodiments, the disease or disorder is an oncogenic virus. In some embodiments, infectious diseases that can be treated with the present disclosure includes, but not limited to, Human Gamma herpes virus 4 (Epstein Barr virus), influenza A virus, influenza B virus, cytomegalovirus, staphylococcus aureus, mycobacterium tuberculosis, chlamydia trachomatis, HIV-1, HIV-2, corona viruses (*e.g.*, MERS-CoV and SARS CoV), filoviruses (*e.g.*, Marburg and Ebola), Streptococcus pyogenes, Streptococcus pneumoniae, Plasmodia species (*e.g*., vivax and falciparum), Chikunga virus, Human Papilloma virus (HPV), Hepatitis B, Hepatitis C, human herpes virus 8, herpes simplex virus 2 (HSV2), Klebsiella sp., Pseudomonas aeruginosa, Enterococcus sp., Proteus sp., Enterobacter sp., Actinobacter sp., coagulase-negative staphylococci (CoNS), Mycoplasma sp., or combinations thereof.

In some embodiments, the EVs (*e.g*., exosomes) are administered intravenously to the circulatory system of the subject. In some embodiments, the EVs (*e.g*., exosomes) are infused in suitable liquid and administered into a vein of the subject.

In some embodiments, the EVs (*e.g*., exosomes) are administered intra-arterialy to the circulatory system of the subject. In some embodiments, the EVs (*e.g*., exosomes) are infused in suitable liquid and administered into an artery of the subject.

In some embodiments, the EVs (*e.g*., exosomes) are administered to the subject by intrathecal administration. In some embodiments, the EVs (*e.g*., exosomes) are administered via an injection into the spinal canal, or into the subarachnoid space so that it reaches the cerebrospinal fluid (CSF).

In some embodiments, the EVs (*e.g*., exosomes) are administered intratumorally into one or more tumors of the subject.

In some embodiments, the EVs (*e*.*g*., exosomes) are administered to the subject by intranasal administration. In some embodiments, the EVs (*e.g*., exosomes) can be insufflated through the nose in a form of either topical administration or systemic administration. In certain embodiments, the EVs (*e.g*., exosomes) are administered as nasal spray.

In some embodiments, the EVs (*e.g*., exosomes) are administered to the subject by intraperitoneal administration. In some embodiments, the EVs (*e.g*., exosomes) are infused in suitable liquid and injected into the peritoneum of the subject. In some embodiments, the intraperitoneal administration results in distribution of the EVs (*e.g*., exosomes) to the lymphatics. In some embodiments, the intraperitoneal administration results in distribution of the EVs (*e.g*., exosomes) to the thymus, spleen, and/or bone marrow. In some embodiments, the intraperitoneal administration results in distribution of the EVs (*e.g*., exosomes) to one or more lymph nodes. In some embodiments, the intraperitoneal administration results in distribution of the EVs (*e.g*., exosomes) to one or more of the cervical lymph node, the inguinal lymph node, the mediastinal lymph node, or the sternal lymph node. In some embodiments, the intraperitoneal administration results in distribution of the EVs (*e.g*., exosomes) to the pancreas.

In some embodiments, the EVs (*e.g*., exosomes) are administered to the subject by periocular administration. In some embodiments, the EVs (*e.g*., exosomes) are injected into the periocular tissues. Periocular drug administration includes the routes of subconjunctival, anterior sub-Tenon's, posterior sub-Tenon's, and retrobulbar administration.

### IX. Kits

The present disclosure also provides kits, or products of manufacture comprising one or more EVs (*e.g*., exosomes) of the present disclosure and optionally instructions for use. In some embodiments, the kit, or product of manufacture contains a pharmaceutical composition described herein which comprises at least one EV (*e.g*., exosome) of the present disclosure, and instructions for use.

In some embodiments, the kit, or product of manufacture comprises at least one EV (*e.g*., exosome) of the present disclosure or a pharmaceutical composition comprising the EVs (*e.g*., exosomes) in one or more containers. One skilled in the art will readily recognize that the EVs (*e.g*., exosomes) of the present disclosure, pharmaceutical composition comprising the EVs (*e.g*., exosomes) of the present disclosure, or combinations thereof can be readily incorporated into one of the established kit formats which are well known in the art.

In some embodiments, the kit, or product of manufacture comprises (i) EVs (*e.g.*, exosomes), (ii) one or more payload, *e.g*., biologically active molecules, (ii) reagents to covalently attach the one or more payloads, *e.g.*, biologically active molecules, to the EVs (*e.g.*, exosomes), or (iv) any combination thereof, and instructions to conduct the reaction to covalently attach the one or more payloads, *e.g.*, biologically active molecules to the EVs (*e.g.*, exosomes).

In some embodiments, the kit comprises reagents to conjugate a payload, *e.g.*, a biologically active molecule to an EV (*e.g*., exosome), and instructions to conduct the conjugation.

The practice of the present disclosure will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Sambrook et al., ed. (1989) Molecular Cloning A Laboratory Manual (2nd ed.; Cold Spring Harbor Laboratory Press); Sambrook et al., ed. (1992) Molecular Cloning: A Laboratory Manual, (Cold Springs Harbor Laboratory, NY); D. N. Glover ed., (1985) DNA Cloning, Volumes I and II; Gait, ed. (1984) Oligonucleotide Synthesis; Mullis et al. U.S. Pat. No. 4,683,195; Hames and Higgins, eds. (1984) Nucleic Acid Hybridization; Hames and Higgins, eds. (1984) Transcription And Translation; Freshney (1987) Culture Of Animal Cells (Alan R. Liss, Inc.); Immobilized Cells And Enzymes (IRL Press) (1986); Perbal (1984) A Practical Guide To Molecular Cloning; the treatise, Methods In Enzymology (Academic Press, Inc., N.Y.); Miller and Calos eds. (1987) Gene Transfer Vectors For Mammalian Cells, (Cold Spring Harbor Laboratory); Wu et al., eds., Methods In Enzymology, Vols. 154 and 155; Mayer and Walker, eds. (1987) Immunochemical Methods In Cell And Molecular Biology (Academic Press, London); Weir and Blackwell, eds., (1986) Handbook Of Experimental Immunology, Volumes I-IV; Manipulating the Mouse Embryo, Cold Spring Harbor Laboratory Press, Cold Spring Harbor, N.Y., (1986); ); Crooke, Antisense drug Technology: Principles, Strategies and Applications, 2nd Ed. CRC Press (2007) and in Ausubel et al. (1989) Current Protocols in Molecular Biology (John Wiley and Sons, Baltimore, Md.).

### SEQUENCES

| SEQ ID NO | Description | Sequence |
|---|---|---|
| 1 | | |
| | | |
| 2 | | |
| 3 | | |
| 4 | | MGGKLSKKKKGYNVNDEKAKEKDKKAEGAA |
| 5 | | MGGKLSKKKKGYNVNDEKAKEKDKKAE |
| 6 | | MGGKLSKKKKGYNVNDEKAKEKDK |
| 7 | | MGGKLSKKKKGYNVNDEKAKE |
| 8 | | MGGKLSKKKKGYNVNDEK |
| 9 | | MGGKLSKKKKGYNVN |
| 10 | | MGGKLSKKKKGY |
| 11 | | MGGKLSKKKKG |
| 12 | | MGGKLSKKKK |
| 13 | | MGGKLSKKK |
| 14 | | MGGKLSKK |
| 15 | | MGGKLSK |
| 16 | | MGGKLAKK |
| 17 | | MGGKFSKK |
| 18 | | MGGKFAKK |
| 19 | | MGGKSSKK |
| 20 | | MGGKSAKK |
| 21 | | MGGKQSKK |
| 22 | | MGGKQAKK |
| 23 | | MGGQLSKK |
| 24 | | MGGQLAKK |
| 25 | | MGGQFSKK |
| 26 | | MGGQFAKK |
| 27 | | MGGQSSKK |
| 28 | | MGGQSAKK |
| 29 | | MGGQQSKK |
| 30 | | MGGQQAKK |
| 31 | | MGAKLSKK |
| 32 | | MGAKLAKK |
| 33 | | MGAKFSKK |
| 34 | | MGAKFAKK |
| 35 | | MGAKSSKK |
| 36 | | MGAKSAKK |
| 37 | | MGAKQSKK |
| 38 | | MGAKQAKK |
| 39 | | MGAQLSKK |
| 40 | | MGAQLAKK |
| 41 | | MGAQFSKK |
| 42 | | MGAQFAKK |
| 43 | | MGAQSSKK |
| 44 | | MGAQSAKK |
| 45 | | MGAQQSKK |
| 46 | | MGAQQAKK |
| 47 | | MGSKLSKK |
| 48 | | MGSKLAKK |
| 49 | | MGSKFSKK |
| 50 | | MGSKFAKK |
| 51 | | MGSKSSKK |
| 52 | | MGSKSAKK |
| 53 | | MGSKQSKK |
| 54 | | MGSKQAKK |
| 55 | | MGSQLSKK |
| 56 | | MGSQLAKK |
| 57 | | MGSQFSKK |
| 58 | | MGSQFAKK |
| 59 | | MGSQSSKK |
| 60 | | MGSQSAKK |
| 61 | | MGSQQSKK |
| 62 | | MGSQQAKK |
| 63 | | MGGKLAK |
| 64 | | MGGKFSK |
| 65 | | MGGKFAK |
| 66 | | MGGKSSK |
| 67 | | MGGKSAK |
| 68 | | MGGKQSK |
| 69 | | MGGKQAK |
| 70 | | MGGQLSK |
| 71 | | MGGQLAK |
| 72 | | MGGQFSK |
| 73 | | MGGQFAK |
| 74 | | MGGQSSK |
| 75 | | MGGQSAK |
| 76 | | MGGQQSK |
| 77 | | MGGQQAK |
| 78 | | MGAKLSK |
| 79 | | MGAKLAK |
| 80 | | MGAKFSK |
| 81 | | MGAKFAK |
| 82 | | MGAKSSK |
| 83 | | MGAKSAK |
| 84 | | MGAKQSK |
| 85 | | MGAKQAK |
| 86 | | MGAQLSK |
| 87 | | MGAQLAK |
| 88 | | MGAQFSK |
| 89 | | MGAQFAK |
| 90 | | MGAQSSK |
| 91 | | MGAQSAK |
| 92 | | MGAQQSK |
| 93 | | MGAQQAK |
| 94 | | MGSKLSK |
| 95 | | MGSKLAK |
| 96 | | MGSKFSK |
| 97 | | MGSKFAK |
| 98 | | MGSKSSK |
| 99 | | MGSKSAK |
| 100 | | MGSKQSK |
| 101 | | MGSKQAK |
| 102 | | MGSQLSK |
| 103 | | MGSQLAK |
| 104 | | MGSQFSK |
| 105 | | MGSQFAK |
| 106 | | MGSQSSK |
| 107 | | MGSQSAK |
| 108 | | MGSQQSK |
| 109 | | MGSQQAK |
| 110 | | MGAKLSKKK |
| 111 | | |
| 112 | | |
| 113 | | |
| 114 | | |
| 115 | | |
| | | |
| 116 | X is any amino acid | MGXKLSKKK |
| 117 | X is alanine or any other amino acid | MGXKLSKKK |
| 118 | X1=(G/A/S); X2=(K/Q); X3=(L/F/S/Q);X4=(S/A) | MGX1X2X3X4KK |
| 119 | | tggaggtgctcaaagagttg |
| 120 | | ttgggcgtgcacttgat |
| 121 | | gggcattggcttc |
| 122 | | |
| 123 | | |
| 124 | | |
| 125 | | |
| 126 | | |
| 127 | | |
| 128 | | MGGKLSKKKKGYNVNDEKAKESAGGGGSDYKDDDDKGGGGSVSKGEELFTG |
| 129 | | MGGKLSKKKKGYNVNDEKSAGGGGSDYKDDDDKGGGGSVSKGEELFTG |
| 130 | | MGGKLSKKKKGYNVNSAGGGGSDYKDDDDKGGGGSVSKGEELFTG |
| 131 | | MGGKLSKKKKGYSAGGGGSDYKDDDDKGGGGSVSKGEELFTG |
| 132 | | MGGKLSKKKSAGGGGSDYKDDDDKGGGGSVSKGEELFTG |
| 133 | | MGGKLSSAGGGGSDYKDDDDKGGGGSVSKGEELFTG |
| 134 | | MGGSAGGGGSDYKDDDDKGGGGSVSKGEELFTG |
| 135 | | |
| 136 | | MGGKLSKKKKGYSAGGGGSDYKDDDDKGGGGSVSKG |
| 137 | | MGGKLSKKKKGSAGGGGSDYKDDDDKGGGGSVSKG |
| 138 | | MGGKLSKKKKSAGGGGSDYKDDDDKGGGGSVSKG |
| 139 | | MGGKLSKKKSAGGGGSDYKDDDDKGGGGSVSKG |
| 140 | | MGGKLSKKSAGGGGSDYKDDDDKGGGGSVSKG |
| 141 | | MGGKLSKSAGGGGSDYKDDDDKGGGGSVSKG |
| 142 | | MGGKLSSAGGGGSDYKDDDDKGGGGSVSKG |
| 143 | | MGGKLDKKKKGYNVNDEKAKEKDKKAEGAA |
| 144 | | MGGKLAKKKKGYNVNDEKAKEKDKKAEGAA |
| 145 | | MGGKQSKKKKGYNVNDEKAKEKDKKAEGAA |
| 146 | | MGAKKKKKRFSFKKSFKLSGFSFKKNKKEA |
| 147 | | MAAKKKKKRFSFKKSFKLSGFSFKKNKKEA |
| 148 | | MGAKKSKKRFSFKKSFKLSGFSFKKNKKEA |
| 149 | | MGAKKAKKRFSFKKPFKLSGFSFKKNKKEA |
| 150 | | |
| 151 | | KKKK |
| 152 | | KKKKK |
| 153 | | RRRR |
| 154 | | RRRRR |
| 155 | | (K/R) (K/R) (K/R) (K/R) |
| 156 | | (K/R) (K/R) (K/R) (K/R) (K/R) |
| 157 | | GGKLSKK |
| 158 | | GAKLSKK |
| 159 | | GGKQSKK |
| 160 | | GGKLAKK |
| 161 | | GGKLSKKK |
| 162 | | GGKLSKKS |
| 163 | | GAKLSKKK |
| 164 | | GAKLSKKS |
| 165 | | GGKQSKKK |
| 166 | | GGKQSKKS |
| 167 | | GGKLAKKK |
| 168 | | GGKLAKKS |
| 169 | | GGKLSKKKKGYNVN |
| 170 | | GAKLSKKKKGYNVN |
| 171 | | GGKQSKKKKGYNVN |
| 172 | | GGKLAKKKKGYNVN |
| 173 | | GGKLSKKKKGYSGG |
| 174 | | GGKLSKKKKGSGGS |
| 175 | | GGKLSKKKKSGGSG |
| 176 | | GGKLSKKKSGGSGG |
| 177 | | GGKLSKKSGGSGGS |
| 178 | | GGKLSKSGGSGGSV |
| 179 | | GAKKSKKRFSFKKS |
| 180 | | (M) (G) (G) (K/Q) (L/F/S/Q) (S/A) (K) (K) |
| 181 | | (M) (G) (A) (K/Q) (L/F/S/Q) (S/A) (K) (K) |
| 182 | | (M) (G) (S) (K/Q) (L/F/S/Q) (S/A) (K) (K) |
| 183 | | (M) (G) (G/A/S) (K) (L/F/S/Q) (S/A) (K) (K) |
| 184 | | (M) (G) (G/A/S) (Q) (L/F/S/Q) (S/A) (K) (K) |
| 185 | | (M) (G) (G/A/S) (K/Q) (L) (S/A) (K) (K) |
| 186 | | (M) (G) (G/A/S) (K/Q) (F) (S/A) (K) (K) |
| 187 | | (M) (G) (G/A/S) (K/Q) (S) (S/A) (K) (K) |
| 188 | | (M) (G) (G/A/S) (K/Q) (Q) (S/A) (K) (K) |
| 189 | | (M) (G) (G/A/S) (K/Q) (L/F/S/Q) (S) (K) (K) |
| 190 | | (M) (G) (G/A/S) (K/Q) (L/F/S/Q) (A) (K) (K) |
| 191 | | (G) (G) (K/Q) (L/F/S/Q) (S/A) (K) (K) |
| 192 | | (G) (A) (K/Q) (L/F/S/Q) (S/A) (K) (K) |
| 193 | | (G) (S) (K/Q) (L/F/S/Q) (S/A) (K) (K) |
| 194 | | (G) (G/A/S) (K) (L/F/S/Q) (S/A) (K) (K) |
| 195 | | (G) (G/A/S) (Q) (L/F/S/Q) (S/A) (K) (K) |
| 196 | | (G) (G/A/S) (K/Q) (L) (S/A) (K) (K) |
| 197 | | (G) (G/A/S) (K/Q) (F) (S/A) (K) (K) |
| 198 | | (G) (G/A/S) (K/Q) (S) (S/A) (K) (K) |
| 199 | | (G) (G/A/S) (K/Q) (Q) (S/A) (K) (K) |
| 200 | | (G) (G/A/S) (K/Q) (L/F/S/Q) (S) (K) (K) |
| 201 | | (G) (G/A/S) (K/Q) (L/F/S/Q) (A) (K) (K) |
| 202 | | (G) (G/A/S) (K/Q) (L/F/S/Q) (S/A) (K) (K) |
| 203 | | GGKLSK |
| 204 | | GAKLSK |
| 205 | | GGKQSK |
| 206 | | GGKLAK |
| 207 | | KKKG |
| 208 | | KKKGY |
| 209 | | KKKGYN |
| 210 | | KKKGYNV |
| 211 | | KKKGYNVN |
| 212 | | KKKGYS |
| 213 | | KKKGYG |
| 214 | | KKKGYGG |
| 215 | | KKKGS |
| 216 | | KKKGGS |
| 217 | | KKKGSG |
| 218 | | KKKGSGS |
| 219 | | KKKS |
| 220 | | KKKSG |
| 221 | | KKKSGG |
| 222 | | KKKSGGS |
| 223 | | KKKSGGSG |
| 224 | | KKSGGSGG |
| 225 | | KKKSGGSGGS |
| 226 | | KRFSFKKS |
| 227 | | GGKLSKKKKGYNVNDEKAKEKDKKAEGAA |
| 228 | | GGKLSKKKKGYNVNDEKAKEKDKKAEGA |
| 229 | | GGKLSKKKKGYNVNDEKAKEKDKKAEG |
| 230 | | GGKLSKKKKGYNVNDEKAKEKDKKAE |
| 231 | | GGKLSKKKKGYNVNDEKAKEKDKKA |
| 232 | | GGKLSKKKKGYNVNDEKAKEKDKK |
| 233 | | GGKLSKKKKGYNVNDEKAKEKDK |
| 234 | | GGKLSKKKKGYNVNDEKAKEKD |
| 235 | | GGKLSKKKKGYNVNDEKAKEK |
| 236 | | GGKLSKKKKGYNVNDEKAKE |
| 237 | | GGKLSKKKKGYNVNDEKAK |
| 238 | | GGKLSKKKKGYNVNDEKA |
| 239 | | GGKLSKKKKGYNVNDEK |
| 240 | | GGKLSKKKKGYNVNDE |
| 241 | | GGKLSKKKKGYNVND |
| 242 | | GGKLSKKKKGYNV |
| 243 | | GGKLSKKKKGYN |
| 244 | | GGKLSKKKKGY |
| 245 | | GGKLSKKKKG |
| 246 | | GGKLSKKKK |
| 247 | | GAKKSKKRFSFKKSFKLSGFSFKKNKKEA |
| 248 | | GAKKSKKRFSFKKSFKLSGFSFKKNKKE |
| 249 | | GAKKSKKRFSFKKSFKLSGFSFKKNKK |
| 250 | | GAKKSKKRFSFKKSFKLSGFSFKKNK |
| 251 | | GAKKSKKRFSFKKSFKLSGFSFKKN |
| 252 | | GAKKSKKRFSFKKSFKLSGFSFKK |
| 253 | | GAKKSKKRFSFKKSFKLSGFSFK |
| 254 | | GAKKSKKRFSFKKSFKLSGFSF |
| 255 | | GAKKSKKRFSFKKSFKLSGFS |
| 256 | | GAKKSKKRFSFKKSFKLSGF |
| 257 | | GAKKSKKRFSFKKSFKLSG |
| 258 | | GAKKSKKRFSFKKSFKLS |
| 259 | | GAKKSKKRFSFKKSFKL |
| 260 | | GAKKSKKRFSFKKSFK |
| 261 | | GAKKSKKRFSFKKSF |
| 262 | | GAKKSKKRFSFKK |
| 263 | | GAKKSKKRFSFK |
| 264 | | GAKKSKKRFSF |
| 265 | | GAKKSKKRFS |
| 266 | | GAKKSKKRF |
| 267 | | GAKKSKKR |
| 268 | | GAKKSKK |
| 269 | | GAKKAKKRFSFKKSFKLSGFSFKKNKKEA |
| 270 | | GAKKAKKRFSFKKSFKLSGFSFKKNKKE |
| 271 | | GAKKAKKRFSFKKSFKLSGFSFKKNKK |
| 272 | | GAKKAKKRFSFKKSFKLSGFSFKKNK |
| 273 | | GAKKAKKRFSFKKSFKLSGFSFKKN |
| 274 | | GAKKAKKRFSFKKSFKLSGFSFKK |
| 275 | | GAKKAKKRFSFKKSFKLSGFSFK |
| 276 | | GAKKAKKRFSFKKSFKLSGFSF |
| 277 | | GAKKAKKRFSFKKSFKLSGFS |
| 278 | | GAKKAKKRFSFKKSFKLSGF |
| 279 | | GAKKAKKRFSFKKSFKLSG |
| 280 | | GAKKAKKRFSFKKSFKLS |
| 281 | | GAKKAKKRFSFKKSFKL |
| 282 | | GAKKAKKRFSFKKSFK |
| 283 | | GAKKAKKRFSFKKSF |
| 284 | | GAKKAKKRFSFKKS |
| 285 | | GAKKAKKRFSFKK |
| 286 | | GAKKAKKRFSFK |
| 287 | | GAKKAKKRFSF |
| 288 | | GAKKAKKRFS |
| 289 | | GAKKAKKRF |
| 290 | | GAKKAKKR |
| 291 | | GAKKAKK |
| 292 | | GAQESKKKKKKRFSFKKSFKLSGFSFKK |
| 293 | | GAQESKKKKKKRFSFKKSFKLSGFSFK |
| 294 | | GAQESKKKKKKRFSFKKSFKLSGFSF |
| 295 | | GAQESKKKKKKRFSFKKSFKLSGFS |
| 296 | | GAQESKKKKKKRFSFKKSFKLSGF |
| 297 | | GAQESKKKKKKRFSFKKSFKLSG |
| 298 | | GAQESKKKKKKRFSFKKSFKLS |
| 299 | | GAQESKKKKKKRFSFKKSFKL |
| 300 | | GAQESKKKKKKRFSFKKSFK |
| 301 | | GAQESKKKKKKRFSFKKSF |
| 302 | | GAQESKKKKKKRFSFKKS |
| 303 | | GAQESKKKKKKRFSFKK |
| 304 | | GAQESKKKKKKRFSFK |
| 305 | | GAQESKKKKKKRFSF |
| 306 | | GAQESKKKKKKRFS |
| 307 | | GAQESKKKKKKRF |
| 308 | | GAQESKKKKKKR |
| 309 | | GAQESKKKKKK |
| 310 | | GAQESKKKKK |
| 311 | | GAQESKKKK |
| 312 | | GAQESKKK |
| 313 | | GAQESKK |
| 314 | | GSQSSKKKKKKFSFKKPFKLSGLSFKRNRK |
| 315 | | GSQSSKKKKKKFSFKKPFKLSGLSFKRNR |
| 316 | | GSQSSKKKKKKFSFKKPFKLSGLSFKRN |
| 317 | | GSQSSKKKKKKFSFKKPFKLSGLSFKR |
| 318 | | GSQSSKKKKKKFSFKKPFKLSGLSFK |
| 319 | | GSQSSKKKKKKFSFKKPFKLSGLSF |
| 320 | | GSQSSKKKKKKFSFKKPFKLSGLS |
| 321 | | GSQSSKKKKKKFSFKKPFKLSGL |
| 322 | | GSQSSKKKKKKFSFKKPFKLSG |
| 323 | | GSQSSKKKKKKFSFKKPFKLS |
| 324 | | GSQSSKKKKKKFSFKKPFKL |
| 325 | | GSQSSKKKKKKFSFKKPFK |
| 326 | | GSQSSKKKKKKFSFKKPF |
| 327 | | GSQSSKKKKKKFSFKKP |
| 328 | | GSQSSKKKKKKFSFKK |
| 329 | | GSQSSKKKKKKFSFK |
| 330 | | GSQSSKKKKKKFSF |
| 331 | | GSQSSKKKKKKFS |
| 332 | | GSQSSKKKKKKF |
| 333 | | GSQSSKKKKKK |
| 334 | | GSQSSKKKKK |
| 335 | | GSQSSKKKK |
| 336 | | GSQSSKKK |
| 337 | | GSQSSKK |
| 338 | | GGKFSKK |
| 339 | | GGKFAKK |
| 340 | | GGKSSKK |
| 341 | | GGKSAKK |
| 342 | | GGKQAKK |
| 343 | | GGQLSKK |
| 344 | | GGQLAKK |
| 345 | | GGQFSKK |
| 346 | | GGQFAKK |
| 347 | | GGQSSKK |
| 348 | | GGQSAKK |
| 349 | | GGQQSKK |
| 350 | | GGQQAKK |
| 351 | | GAKLAKK |
| 352 | | GAKFSKK |
| 353 | | GAKFAKK |
| 354 | | GAKSSKK |
| 355 | | GAKSAKK |
| 356 | | GAKQSKK |
| 357 | | GAKQAKK |
| 358 | | GAQLSKK |
| 359 | | GAQLAKK |
| 360 | | GAQFSKK |
| 361 | | GAQFAKK |
| 362 | | GAQSSKK |
| 363 | | GAQSAKK |
| 364 | | GAQQSKK |
| 365 | | GAQQAKK |
| 366 | | GSKLSKK |
| 367 | | GSKLAKK |
| 368 | | GSKFSKK |
| 369 | | GSKFAKK |
| 370 | | GSKSSKK |
| 371 | | GSKSAKK |
| 372 | | GSKQSKK |
| 373 | | GSKQAKK |
| 374 | | GSQLSKK |
| 375 | | GSQLAKK |
| 376 | | GSQFSKK |
| 377 | | GSQFAKK |
| 378 | | GSQSAKK |
| 379 | | GSQQSKK |
| 380 | | GSQQAKK |
| 381 | | GGKFSK |
| 382 | | GGKFAK |
| 383 | | GGKSSK |
| 384 | | GGKSAK |
| 385 | | GGKQAK |
| 386 | | GGQLSK |
| 387 | | GGQLAK |
| 388 | | GGQFSK |
| 389 | | GGQFAK |
| 390 | | GGQSSK |
| 391 | | GGQSAK |
| 392 | | GGQQSK |
| 393 | | GGQQAK |
| 394 | | GAKLAK |
| 395 | | GAKFSK |
| 396 | | GAKFAK |
| 397 | | GAKSSK |
| 398 | | GAKSAK |
| 399 | | GAKQSK |
| 400 | | GAKQAK |
| 401 | | GAQLSK |
| 402 | | GAQLAK |
| 403 | | GAQFSK |
| 404 | | GAQFAK |
| 405 | | GAQSSK |
| 406 | | GAQSAK |
| 407 | | GAQQSK |
| 408 | | GAQQAK |
| 409 | | GSKLSK |
| 410 | | GSKLAK |
| 411 | | GSKFSK |
| 412 | | GSKFAK |
| 413 | | GSKSSK |
| 414 | | GSKSAK |
| 415 | | GSKQSK |
| 416 | | GSKQAK |
| 417 | | GSQLSK |
| 418 | | GSQLAK |
| 419 | | GSQFSK |
| 420 | | GSQFAK |
| 421 | | GSQSSK |
| 422 | | GSQSAK |
| 423 | | GSQQSK |
| 424 | | GSQQAK |
| 425 | | GXKLSKKK |

### EXAMPLES

The following examples are put forth so as to provide those of ordinary skill in the art with a complete disclosure and description of how to make and use the present disclosure, and are not intended to limit the scope of what the inventors regard as their disclosure nor are they intended to represent that the experiments below are all or the only experiments performed. Efforts have been made to ensure accuracy with respect to numbers used (e.g., amounts, temperature, etc.) but some experimental errors and deviations should be accounted for.

Unless indicated otherwise, parts are parts by weight, molecular weight is weight average molecular weight, temperature is in degrees Celsius, and pressure is at or near atmospheric. Standard abbreviations can be used, *e.g.,* bp, base pair(s); kb, kilobase(s); pl, picoliter(s); s or sec, second(s); min, minute(s); h or hr, hour(s); aa, amino acid(s); nt, nucleotide(s); and the like.

The practice of the present disclosure will employ, unless otherwise indicated, conventional methods of protein chemistry, biochemistry, recombinant DNA techniques and pharmacology, within the skill of the art. Such techniques are explained fully in the literature. See, *e.g.*, T.E. Creighton, Proteins: Structures and Molecular Properties (W.H. Freeman and Company, 1993); AL. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 21th Edition (Easton, Pennsylvania: Mack Publishing Company, 2005); Carey and Sundberg Advanced Organic Chemistry 3rd Ed. (Plenum Press) Vols A and B(1992).

### Example 1

### Identification of a Minimal Protein Sequence Sufficient for Loading Luminal Exosome Payloads

To identify the minimal BASP1 amino acid sequence between the twelve-amino acid truncation that facilitated loading and the six-amino acid truncation that failed to facilitate loading as shown above, individual truncation mutants of the N-terminus of BASP1 fused to a FLAG® tag and GFP were generated and stably expressed in HEK293SF cells (FIG. 1A). Exosomes were purified from the stable cell cultures as described above. BASP1 sequences of seven through twelve amino acids were capable of loading GFP in exosomes at high density, while the first six amino acids were not (FIG. 1B). These data demonstrate that at least one lysine residue after position six is required for luminal loading of exosomes with the N-terminus of BASP1, *i.e.*, for attachment to the luminal surface of the exosome.

The serine at position 6 of BASP1 is highly conserved across species and in MARCKS and MARCKSL1. To determine whether this amino acid was required for payload loading into exosomes, HEK293SF cells were stably transfected with expression plasmids encoding BASP1 1-30- FLAG®-GFP or BASP1 1-30- FLAG®-GFP including a point mutant, replacing serine six with aspartic acid (S6D; polar charged substitution) or alanine (S6A; small nonpolar substitution). Additionally, the lysine at position five was mutated to a glutamic acid (L5Q) to test the potential role of this position in modulating myristoylation, palmitoylation, and other membrane functions of several membrane-associated proteins (Gottlieb-Abraham et al., Mol. Biol. Cell. 2016 Dec 1;27(24):3926-3936) (FIG. 2A). BASP1 S6D completely abrogated loading of GFP into exosomes, while S6A did not alter loading. BASP1 L5Q did not impact luminal loading either, indicating that a negative charge at position six disrupts loading, while a polar amino acid substitution at position five is well-tolerated (FIG. 2B).

The first thirty amino acids of BASP1 contain the N-terminal leader sequence identified above, followed by a lysine-rich stretch of amino acids. To understand whether MARCKS and MARCKSL1 N-termini can load exosomes similarly to BASP1, HEK293SF cells were stably transfected with MARCKS and MARCKSL1 full-length proteins or amino acids 1-30 fused to FLAG®-GFP. Purified exosomes were analyzed by SDS PAGE and COOMASSIE® staining to determine the extent of loading. Full-length MARCKS and MARCKSL1 were able to load exosomes with GFP, but amino acids 1-30 were inferior to the full-length proteins, suggesting that there are additional structural or sequence features in distal regions of the MARCKS and MARCKSL1 proteins required for loading (FIG. 3). Sequence analysis of MARCKS and MARCKSL1 revealed regions with potential sequence homology to the N-terminus of BASP1.

Amino acids 152-173 of MARCKS and 87-110 of MARCKSL1 are lysine-rich with interspersed phenylalanine and serine residues and are predicted to be phosphorylation site domains (PSD) or effector domains (ED) (FIG. 4). HEK293SF cells were stably transfected with plasmid constructs fusing amino acids 1-3 of MARCKS to the PSD domain (MG-PSD). Individual point mutations were generated at the predicted myristoylation site (MA-PSD) and position six (K6S and K6A) to determine the role of these residues in loading exosomes (FIG. 5A). Western blotting of purified exosomes demonstrated that compared to the positive control of BASP1 1-30, neither MG-PSD nor MA-PSD could efficiently load exosomes. Interestingly, the K6A and K6S mutations led to improvements in loading, suggesting that a positive charge at position 6 prevents loading of an exosomal payload and that the PSD of MARCKS could functionally complement for the endogenous N-terminal sequence (FIG. 5B). Together, these studies allowed for the identification of several motifs sufficient to load a payload into exosomes (FIG. 6).

The narrowest motif, Motif 1, allows for a protein sequence of (M)(G)(G/A/S)(K/Q)(L/F/S/Q)(S/A)(K)(K) (SEQ ID NO: 118) or (G)(G/A/S)(K/Q)(L/F/S/Q)(S/A)(K)(K) (SEQ ID NO: 202) without the first Met, where each parenthetical letter or group of letters is an amino acid position, and wherein additionally position five cannot be a positively charged amino acid (K/R/H) and position six cannot be a negatively charged amino acid (D/E).

Sub-motifs of Motif 1 include, without being limiting, the protein sequences:
(M)(G)(G)(K/Q)(L/F/S/Q)(S/A)(K)(K) (SEQ ID NO: 180),
(G)(G)(K/Q)(L/F/S/Q)(S/A)(K)(K) (SEQ ID NO: 191)
(M)(G)(A)(K/Q)(L/F/S/Q)(S/A)(K)(K) (SEQ ID NO: 181),
(G)(A)(K/Q)(L/F/S/Q)(S/A)(K)(K) (SEQ ID NO: 192),
(M)(G)(S)(K/Q)(L/F/S/Q)(S/A)(K)(K) (SEQ ID NO: 182),
(G)(S)(K/Q)(L/F/S/Q)(S/A)(K)(K) (SEQ ID NO: 193),
(M)(G)(G/A/S)(K)(L/F/S/Q)(S/A)(K)(K) (SEQ ID NO: 183),
(G)(G/A/S)(K)(L/F/S/Q)(S/A)(K)(K) (SEQ ID NO: 194),
(M)(G)(G/A/S)(Q)(L/F/S/Q)(S/A)(K)(K) (SEQ ID NO: 184),
(G)(G/A/S)(Q)(L/F/S/Q)(S/A)(K)(K) (SEQ ID NO: 195),
(M)(G)(G/A/S)(K/Q)(L)(S/A)(K)(K) (SEQ ID NO: 185),
(G)(G/A/S)(K/Q)(L)(S/A)(K)(K) (SEQ ID NO: 196),
(M)(G)(G/A/S)(K/Q)(F)(S/A)(K)(K) (SEQ ID NO: 186),
(G)(G/A/S)(K/Q)(F)(S/A)(K)(K) (SEQ ID NO: 197),
(M)(G)(G/A/S)(K/Q)(S)(S/A)(K)(K) (SEQ ID NO: 187),
(G)(G/A/S)(K/Q)(S)(S/A)(K)(K) (SEQ ID NO: 198),
(M)(G)(G/A/S)(K/Q)(Q)(S/A)(K)(K) (SEQ ID NO: 188),
(G)(G/A/S)(K/Q)(Q)(S/A)(K)(K) (SEQ ID NO: 199),
(M)(G)(G/A/S)(K/Q)(L/F/S/Q)(S)(K)(K) (SEQ ID NO: 189),
(G)(G/A/S)(K/Q)(L/F/S/Q)(S)(K)(K) (SEQ ID NO: 200),
(M)(G)(G/A/S)(K/Q)(L/F/S/Q)(A)(K)(K) (SEQ ID NO: 190), and
(G)(G/A/S)(K/Q)(L/F/S/Q)(A)(K)(K) (SEQ ID NO: 201),
where position five cannot be a positively charged amino acid (K/R/H) and position six cannot be a negatively charged amino acid (D/E).

Motif 2, a broader motif, can be expressed as (M)(G)(π)(ξ)(Φ/π)(S/A/G/N)(+)(+) or (G)(π)(ξ)(Φ/π)(S/A/G/N)(+)(+) without the first Met, wherein each parenthetical position represents an amino acid, and wherein π is any amino acid selected from the group consisting of (Pro, Gly, Ala, Ser), ξ is any amino acid selected from the group consisting of (Asn, Gln, Ser, Thr, Asp, Glu, Lys, His, Arg), Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). See R. Aasland et al., FEBS Letters 513 (2002): 141-144 for the nomenclature of amino acids.

Motif 3, the broadest motif, can be expressed as (M)(G)(π)(X)((Φ/π)(π)(+)(+) or (G)(π)(X)(Φ/π)(π)(+)(+) without the first Met, wherein each parenthetical position represents an amino acid, and wherein π is any amino acid selected from the group consisting of (Pro, Gly, Ala, Ser), X is any amino acid, Φ is any amino acid selected from the group consisting of (Val, Ile, Leu, Phe, Trp, Tyr, Met), and (+) is any amino acid selected from the group consisting of (Lys, Arg, His); and wherein position five is not (+) and position six is neither (+) nor (Asp or Glu). In all cases of Motifs 1-3, the sequence may be truncated by one amino acid to be seven total amino acids in length (*i.e.*, consisting of amino acids 1-7 in the order presented in Motifs 1-3). Any of the sequences derived from any of Motifs 1, 2, or 3 (or these motifs lacking amino acid 7), can be used to load a payload into exosomes to the same extent as, or comparable to, full length BASP1 or natural truncation sequences of BASP1. This deep analysis of amino acid sequence-structure-function provides novel insights into the requirements for directing biologically expressed payload into exosomes by producer cells.

### Example 2

### The N-Terminus of BASP1 is Sufficient to Load Diverse Classes of Proteins

The results in Example 1 suggest that the N-terminus of BASP1 may be a useful engineering scaffold for generating luminally loaded exosomes directly from producer cells. To test this hypothesis, stable HEK293SF cells were generated to express full-length Cas9 protein with codon optimization (as described in Zetsche B, Volz SE, Zhang F. A split-Cas9 architecture for inducible genome editing and transcription modulation. Nat Biotechnol. 2015 Feb;33(2):139-42) fused to amino acids 1-30 or 1-10 of BASP1. Exosomes were purified from cell culture as described above and analyzed by SDS-PAGE and Western blotting using an anti-Cas9 antibody (Abcam; Catalog # ab191468, clone 7A9-3A3). As shown in FIG. 7A, both BASP1 1-30 and 1-10 were sufficient to load Cas9 in exosomes. Recombinant Cas9 protein was used as a positive control for Western blotting. Densitometry quantitation and comparison of various amounts of recombinant Cas9 and BASP1-Cas9 exosome lanes from the Western blotting experiments revealed that the exosomes were loaded with 4-5 Cas9 molecules per exosome (FIG. 7B). This Cas9 enzyme, which is ∼160kDa in mass, represents a significant increase in payload size compared to the GFP experiments shown above.

As an additional validation of the diversity of payload proteins that can be loaded as a fusion to the N-terminus of BASP1, ovalbumin was stably expressed in HEK293SF cells as a fusion to amino acids 1-10 of BASP1 ("BASP1(1-10)-OVA"). A separate cell line was co-transfected with the same plasmid and a second plasmid encoding trimeric CD40L fused to an exosome-specific surface glycoprotein PTGFRN ("3xCD40L-PTGFRN") using a second selectable marker. Exosomes were purified from the two transfected cell cultures and analyzed by SDS-PAGE (FIG. 8A) and anti-ovalbumin western blotting (Abcam; Catalog # ab17293, clone 6C8) (FIG. 8B). As a control, recombinant ovalbumin (InvivoGen; Catalog # vac-pova) was titrated in a separate gel. Ovalbumin was robustly loaded into exosomes when fused to amino acids 1-10 of BASP1 as a single construct or when in combination with an additional overexpression plasmid (3xCD40L-PTGFRN). This result demonstrates that exosomes can be combinatorially engineered, both with a luminal payload and with a simultaneous surface payload (e.g., PTGFRN) from a separate transcript.

Another class of proteins that may be useful in the context of therapeutic exosomes are antibodies and antibody fragments. A single chain camelid nanobody targeting GFP (as described in Caussinus E, Kanca O, Affolter M. Fluorescent fusion protein knockout mediated by anti-GFP nanobody. Nat Struct Mol Biol. 2011 Dec 11;19(1):117-21) was stably expressed in HEK293SF cells as a fusion protein to amino acids 1-10 of BASP1 and a FLAG® tag ("BASP1(1-10)-Nanobody") or a FLAG® tag alone ("Nanobody") (FIG. 9A). Purified exosomes were analyzed by SDS-PAGE and anti-FLAG® Western blotting, demonstrating that there was substantial enrichment of the nanobody with equal amounts of total loaded protein when the nanobody was fused to the N-terminus of BASP1 (FIG. 9B). These results demonstrate that protein payloads of diverse classes can be expressed and packaged into exosomes by producer cells using a very short protein sequence derived from the N-terminus of BASP1, *i.e.,* a scaffold.

### Example 3

### The N-Terminus of BASP1 Can Be Used to Load Nucleic Acids in the Lumen of Exosomes

Nucleic acids, and in particular RNAs (*e.g*., mRNAs, siRNAs, miRNAs) are an attractive class of therapeutic payload to be loaded in the lumen of therapeutic exosomes. Exosome loading of RNA may protect the RNA from degradation in the extracellular environment and the loaded exosome can be directed to certain cells and/or tissues through additional levels of exosome engineering, *e.g*., surface expression of a targeting construct. To understand whether the EV, *e.g*., exosome proteins (or protein fragments) identified above can be used to generate mRNA-loaded exosomes, combinatorial engineered exosomes were generated. As shown in FIG. 10, amino acids 1-30 of BASP1 were expressed as a fusion to FLAG® and variants of the phage protein MCP. MCP recognizes and binds to an mRNA stem loop called MS2, which can be expressed as a transcriptional fusion to mRNAs and other RNAs, thus driving physical association between the MCP fusion proteins and MS2 fusion RNAs of interest. Mutational analysis previously identified two positions in MCP that increases affinity to MS2; a valine to isoleucine substitution at position 29 (V29I; Lim & Peabody, RNA. Nucleic Acids Res. 1994 Sep 11;22(18):3748-52) and an asparagine to lysine substitution at position 55 (N55K; Lim et al., J Biol Chem. 1994 Mar 25;269(12):9006-10). BASP1 1-30 was fused to monomeric or dimeric MCP variants, where each MCP was either V29I or doubly mutated V29I/N55K. A luciferase reporter construct was expressed as a fusion to 3 MS2 stem loops from a separate plasmid. Five stable HEK293SF cell lines were generated, either Luciferase-MS2 alone (#811) or in combination with each of the BASP1-MCP variants (#815, 817, 819, or 821) (FIG. 10). As an additional control, HEK293SF cells were stably transfected with FLAG-tagged BASP1 1-27. Exosomes were isolated and treated with BENZONASE® to remove any externally-associated mRNAs, and purified according to the Methods above. Purified exosomes were analyzed by SDS-PAGE (FIG. 11A) and anti-FLAG® Western blotting (FIG. 11B), demonstrating equal amounts of total protein and comparable levels of BASP1-FLAGS) fusions in each exosome preparation. Importantly, the BASP1-MCP fusions expressed to comparable levels as a BASP1 1-27 FLAG fusion lacking an MCP protein, demonstrating that the addition of MCP monomers or dimers do not disrupt the BASP1-mediated loading of proteins in exosomes.

The cells stably expressing the BASP1-MCP and Luciferase-MS2 mRNA were isolated and total Luciferase mRNA was quantified by RT-qPCR (FWD Primer: 5'-TGGAGGTGCTCAAAGAGTTG-3' (SEQ ID NO: 119); REV Primer: 5'-TTGGGCGTGCACTTGAT-3' (SEQ ID NO: 120); PROBE: 5'-/56-FAM/CAGCTTTCC/ZEN/GGGCATTGGCTTC/3IABkFQ/-3' (SEQ ID NO: 121)). Untransfected cells expressed lower levels of Luciferase than all of the 811-expressing cells, which expressed comparable levels of Luciferase (FIG. 12A, top). The purified exosomes from each of the stable cell lines were also analyzed by RT-qPCR. Native exosomes had no detectable levels of Luciferase MS2, while cells expressing 811 alone had detectable but very low levels of Luciferase MS2. Importantly, each of the BASP1-MCP fusion proteins contained greater amounts of Luciferase-MS2 mRNA, demonstrating the importance of the binding between MCP and MS2 to facilitate loading of mRNA into exosomes (FIG. 12A, bottom). Quantitation of relative mRNA between the groups demonstrated an enrichment of ∼30 to 60-fold for all of the BASP1-MCP fusions over 811 alone (FIG. 12B). BASP1-MCP construct 821, which contained dimeric MCP V29I/N55K is predicted to have the greatest affinity for MS2 mRNAs, and indeed contained the greatest amount of Luciferase-MS2 in this experiment. These results demonstrate that BASP1 fragments are robust and versatile scaffold proteins for loading the lumen of exosomes with diverse payloads including nucleic acids.

### Example 4

### BASP1, MARCKS, and MARCKSL1 Can Be Used to Generate Surface-Decorated Exosomes

The results in the previous experiments demonstrate that full-length and N-terminal regions of MARCKS, MARCKSL1, and BASP1 can be used to generate luminally loaded exosomes. To further explore the potential of these proteins for exosome engineering, amino acids 1-30 of MARCKS, MARCKSL1 and BASP1, or amino acids 1-10 of BASP1 were fused to the endogenous transmembrane region of CD40L expressed as a homotrimer. Constructs were prepared for both human and mouse sequences of CD40L because the ligands do not cross-react with the cognate receptor on the other species (FIG. 13). Exosomes were purified from HEK293SF cells stably transfected with one of the CD40L expression constructs and incubated in either mouse or human B cells. Amounts of input CD40L on the exosomes was quantified by CD40L ELISA (for measurement of human CD40L, R&D Systems, Catalog # DCDL40, Lot # P168248; and for measurement of mouse CD40L, Abcam, Catalog # ab119517, Lot # GR3218850-2 were used), B cells were quantified using B-cell marker, CD19, and B cell activation was measured by percentage of gated cells positive for CD69. Dose titration curves of mouse (FIG. 14A) or human (FIG. 14B) exosomal CD40L in species-matched cultures showed comparable activity between constructs on a particle-to-particle basis (left graphs and table below) or as compared to each other and equal amounts of recombinant protein on a CD40L molar basis (right graphs and table below). Comparable activity was observed when the CD40L constructs were expressed as monomers as well, and were only slightly less potent than trimeric CD40L expressed on the N-terminus of PTGFRN, a high-density exosome display scaffold (see, e.g., International Patent Application No. PCT/US2018/048026) (FIG. 14C). These results demonstrate that MARCKS, MARCKSL1, and BASP1 are diverse, robust scaffolds useful for the generation of various classes of engineered exosomes for use in human and animal applications.

### Example 5

### Diverse Cell Types Express BASP1, MARCKS, and/or MARCKSL1

Cell lines from different tissues of origin (HEK293, kidney; HT1080, connective tissue; K562, bone marrow; MDA-MB-231, breast; Raji, lymphoblast) were grown to logarithmic phase and transferred to media supplemented with exosome-depleted serum for ∼6 days except for the HEK293 cells, which were grown in chemically-defined media. Bone marrow-derived mesenchymal stem cells (MSC) were grown on 3D microcarriers for five days and supplemented in serum-free media for three days. Supernatant from each cell line culture was isolated, and exosomes were purified using the OPTIPREP™ density-gradient ultracentrifugation method described above. Each of the purified exosome preparations was analyzed by LC-MS/MS as described above, and the number of peptide spectrum matches (PSMs) was quantified for BASP1, MARCKS, and MARCKSL1 and two widely studied EV, *e.g*., exosome proteins (CD81 and CD9). The tetraspanins CD81 and CD9 were detectable in most purified exosome populations, but were, in some cases, equal to or lower than the luminal EV, *e.g.*, exosome, proteins (*e.g.*, compare CD9 to BASP1 or MARCKSL1) (FIG. 15). This finding indicates that the newly-identified luminal exosome markers may be suitable fusion proteins for generating engineered exosomes from several unrelated cell lines derived from different tissues.

### Example 6

### Non-Human Cells Overexpressing BASP1 Produce Luminally Engineered Exosomes

The results in Example 5 demonstrate that numerous human-derived cells naturally express BASP1 and the other novel EV. To determine whether BASP1 can be used as a universal exosome scaffold protein, Chinese hamster ovary (CHO) cells were stably transfected with either a plasmid expressing full-length BASP1 fused to a FLAG® tag and GFP ("BASP1-GFP-FLAG"), a plasmid expressing amino acids 1-30 of BASP1 fused to a FLAG® tag and GFP ("BASP1(1-30)-GFP-FLAG") or a plasmid expressing amino acids 1-8 of BASP1 fused to a FLAG® tag and GFP ("BASP1(1-8)-GFP-FLAG"). Exosomes were purified from wild-type CHO cells and CHO cells transfected with one of the three BASP1 plasmids. As shown in FIGs. 16A-B, BASP1 and the BASP1 fragment fusion proteins were successfully overexpressed in CHO cells and loaded into exosomes as detected by stain-free PAGE (FIG. 16A) and Western blotting with an antibody against FLAG® (FIG. 16B). This result demonstrates that non-human cells, such CHO cells, can produce exosomes that overexpress human BASP1 fragments, and that this overexpression can drive a payload protein into the lumen of exosomes at high density. This result indicates that BASP1 is a universal scaffold protein for generating engineered exosomes from many different cell types and species.

The present disclosure has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

### Equivalents

The present disclosure provides, inter alia, compositions of EVs, *e.g*., exosomes containing modified exogenous proteins and peptides for use in enrichment of exogenous proteins in EVs, *e.g*., exosomes. The present disclosure also provides method of producing enriched EVs, *e.g*., exosomes. While various specific embodiments have been illustrated and described, the above specification is not restrictive. The breadth and scope of the present disclosure should not be limited by any of the above-described exemplary embodiments. Many variations will become apparent to those skilled in the art upon review of this specification.

This application claims the benefit of International Application No. PCT/US2018/061679, filed November 16, 2018, and U.S. Provisional Application No. 62/835,430, filed April 17, 2019.

## Claims

1. An isolated extracellular vesicle (EV) comprising a biologically active molecule linked to a scaffold protein,
wherein the scaffold protein comprises an N-terminus domain (ND) and an effector domain (ED),
wherein the ND is associated with the luminal surface of the EV and the ED is associated with the luminal surface of the EV,
wherein the ND comprises the amino acid sequence GGKLSK (SEQ ID NO: 203), but does not comprise a methionine (Met) at the N-terminus,
wherein the ED comprises a lysine (Lys) at its N-terminus that is linked directly to the lysine at the C-terminus of SEQ ID NO: 203 in the ND; and
wherein the biologically active molecule comprises one or more heterologous proteins fused to the C-terminus of the scaffold protein.

2. The EV of claim 1, wherein the ED comprises KK, KKK, KKKK (SEQ ID NO: 151), KKKKK (SEQ ID NO: 152), or any combination thereof.

3. The EV of claim 1 or 2, wherein the scaffold protein is linked to the biologically active molecule by a linker.

4. The EV of claim 3, wherein the linker comprises a cleavable linker.

5. The EV of any one of claims 1 to 4, wherein the scaffold protein comprises GGKLSKKK (SEQ ID NO: 161) or GGKLSKKS (SEQ ID NO: 162).

6. The EV of any one of claims 1 to 5, wherein the scaffold protein is at least about 8 amino acids in length.

7. The EV of any one of claims 1 to 6, wherein the biologically active molecule is on the luminal surface or in the lumen of the EV.

8. The EV of any one of claims 1 to 7, wherein the scaffold protein further comprises:
(i) a transmembrane domain,
(ii) an extravesicular domain, or
(iii) both (i) and (ii).

9. The EV of any one of claims 1 to 6, wherein the scaffold protein further comprises a transmembrane domain and an extravesicular domain and:
(i) the transmembrane domain is between the ED domain of the scaffold protein and the biologically active molecule, and
(ii) the biologically active molecule is linked to the extravesicular domain.

10. The EV of any one of claims 1 to 9, wherein the biologically active molecule comprises:
(a) a protein, wherein the protein optionally comprises
(i) a recombinant peptide, a natural peptide, a synthetic peptide, an antibody, a fusion protein, or any combination thereof;
(ii) an enzyme, a cytokine, a ligand, a receptor, a transcription factor, or a combination thereof;
(iii) a T-cell receptor (TCR), a T-cell co-receptor, a major histocompatibility complex (MHC), a human leukocyte antigen (HLA), or a derivative thereof;
(iv) a tumor antigen optionally selected from the group consisting of alpha-fetoprotein (AFP), carcinoembryonic antigen (CEA), epithelial tumor antigen (ETA), mucin 1 (MUC1), Tn-MUC1, mucin 16 (MUC16), tyrosinase, melanoma-associated antigen (MAGE), tumor protein p53 (p53), CD4, CD8, CD45, CD80, CD86, programmed death ligand 1 (PD-L1), programmed death ligand 2 (PD-L2), NY-ESO-1, PSMA, TAG-72, HER2, GD2, cMET, EGFR, Mesothelin, VEGFR, alpha-folate receptor, CE7R, IL-3, Cancer-testis antigen, MART-1 gp100, and TNF-related apoptosis-inducing ligand;
(b) a polypeptide;
(c) a peptide;
(d) a polynucleotide (DNA and/or RNA);
(e) a chemical compound;
(f) a virus, wherein the virus optionally comprises an adeno-associated virus, a parvovirus, a retrovirus, an adenovirus, or any combination thereof;
(g) an ionophore;
(h) a carrier for an ionophore;
(i) a moiety that forms a channel or a pore; or
(j) any combination thereof.

11. The EV of any one of claims 1 to 10, wherein the EV further comprises a second scaffold protein, wherein the second scaffold protein optionally comprises a PTGFRN polypeptide, a BSG polypeptide, an IGSF2 polypeptide, an IGSF3 polypeptide, an IGSF8 polypeptide, an ITGB1 polypeptide, an ITGA4 polypeptide, a SLC3A2 polypeptide, an ATP transporter polypeptide, an aminopeptidase N (ANPEP) polypeptide, an ectonucleotide pyrophosphatase/phosphodiesterase family member 1 (ENPP1) polypeptide, a neprilysin (MME) polypeptide, a neuropilin-1 (NRP1) polypeptide, or a fragment thereof.

12. The EV of any one of claims 1 to 11, wherein the biologically active molecule is:
(a) an inhibitor for a negative checkpoint regulator or an inhibitor for a binding partner of a negative checkpoint regulator, wherein the negative checkpoint regulator is optionally selected from the group consisting of:
(i) cytotoxic T-lymphocyte-associated protein 4 (CTLA-4),
(ii) programmed cell death protein 1 (PD-1),
(iii) lymphocyte-activated gene 3 (LAG-3),
(iv) T-cell immunoglobulin mucin-containing protein 3 (TIM-3),
(v) B and T lymphocyte attenuator (BTLA),
(vi) T cell immunoreceptor with Ig and ITIM domains (TIGIT),
(vii) V-domain Ig suppressor of T cell activation (VISTA),
(viii) adenosine A2a receptor (A2aR),
(ix) killer cell immunoglobulin like receptor (KIR),
(x) indoleamine 2,3-dioxygenase (IDO),
(xi) CD20,
(xii) CD39, and
(xiii) CD73;
(b) an immunogenic protein;
(c) a toxin, toxoid, or a non-toxic mutant of a toxin, wherein
(i) the toxin is optionally a diphtheria toxin, or
(ii) the toxoid is optionally a tetanus toxoid;
(d) an activator for a positive co-stimulatory molecule or an activator for a binding partner of a positive co-stimulatory molecule, wherein the positive co-stimulatory molecule is optionally selected from the group consisting of:
(i) a TNF receptor superfamily member optionally selected from the group consisting of: CD120a, CD120b, CD18, OX40, CD40, Fas receptor, M68, CD27, CD30, 4-1BB, TRAILR1, TRAILR2, TRAILR3, TRAILR4, RANK, OCIF, TWEAK receptor, TACI, BAFF receptor, ATAR, CD271, CD269, AITR, TROY, CD358, TRAMP, and XEDAR, optionally wherein the activator for the positive co-stimulatory molecule is a TNF superfamily member optionally selected from the group consisting of: TNFα, TNF-C, OX40L, CD40L, FasL, LIGHT, TL1A, CD27L, Siva, CD153, 4-1BB ligand, TRAIL, RANKL, TWEAK, APRIL, BAFF, CAMLG, NGF, BDNF, NT-3, NT-4, GITR ligand, and EDA-2; and
(ii) a CD28-superfamily co-stimulatory molecule optionally selected from ICOS and CD28, optionally wherein the activator for the positive co-stimulatory molecule is ICOSL, CD80 or CD86.

13. The EV of any one of claims 1 to 12, wherein the EV is an exosome.

14. A pharmaceutical composition comprising the EV of any one of claims 1 to 13 and a pharmaceutically acceptable carrier.

15. The EV of any one of claims 1 to 13 for use in preventing or treating a disease in a subject in need thereof.

## Patentansprüche

1. Isoliertes extrazelluläres Vesikel (EV), umfassend ein biologisch aktives Molekül, das mit einem Gerüstprotein verknüpft ist,
wobei das Gerüstprotein eine N-Terminus-Domäne (ND) und eine Effektor-Domäne (ED) umfasst,
wobei die ND mit der luminalen Oberfläche des EV und die ED mit der luminalen Oberfläche des EV assoziiert ist,
wobei die ND die Aminosäuresequenz GGKLSK (SEQ ID NO: 203), aber kein Methionin (Met) am N-Terminus umfasst,
wobei die ED ein Lysin (Lys) an ihrem N-Terminus umfasst, das mit dem Lysin am C-Terminus von SEQ ID NO: 203 in der ND direkt verknüpft ist; und
wobei das biologisch aktive Molekül ein oder mehrere heterologe Proteine umfasst, die an den C-Terminus des Gerüstproteins fusioniert sind.

2. EV nach Anspruch 1, wobei die ED KK, KKK, KKKK (SEQ ID NO: 151), KKKKK (SEQ ID NO: 152) oder eine beliebige Kombination davon umfasst.

3. EV nach Anspruch 1 oder 2, wobei das Gerüstprotein über einen Linker mit dem biologisch aktiven Molekül verknüpft ist.

4. EV nach Anspruch 3, wobei der Linker einen spaltbaren Linker umfasst.

5. EV nach einem der Ansprüche 1 bis 4, wobei das Gerüstprotein GGKLSKKK (SEQ ID NO: 161) oder GGKLSKKS (SEQ ID NO: 162) umfasst.

6. EV nach einem der Ansprüche 1 bis 5, wobei das Gerüstprotein eine Länge von wenigstens etwa 8 Aminosäuren aufweist.

7. EV nach einem der Ansprüche 1 bis 6, wobei sich das biologisch aktive Molekül auf der luminalen Oberfläche oder im Lumen des EV befindet.

8. EV nach einem der Ansprüche 1 bis 7, wobei das Gerüstprotein ferner Folgendes umfasst:
(i) eine Transmembrandomäne,
(ii) eine extravesikuläre Domäne oder
(iii) sowohl (i) als auch (ii).

9. EV nach einem der Ansprüche 1 bis 6, wobei das Gerüstprotein ferner eine Transmembrandomäne und eine extravesikuläre Domäne umfasst und:
(i) die Transmembrandomäne sich zwischen der ED-Domäne des Gerüstproteins und dem biologisch aktiven Molekül befindet und
(ii) das biologisch aktive Molekül mit der extravesikulären Domäne verknüpft ist.

10. EV nach einem der Ansprüche 1 bis 9, wobei das biologisch aktive Molekül Folgendes umfasst:
(a) ein Protein, wobei das Protein gegebenenfalls
(i) ein rekombinantes Peptid, ein natürliches Peptid, ein synthetisches Peptid, einen Antikörper, ein Fusionsprotein oder eine beliebige Kombination davon;
(ii) ein Enzym, ein Cytokin, einen Liganden, einen Rezeptor, einen Transkriptionsfaktor oder eine Kombination davon;
(iii) einen T-Zell-Rezeptor (TCR), einen T-Zell-Corezeptor, einen MHC (Major Histocompatibility Complex), ein menschliches Leukozytenantigen (HLA) oder ein Derivat davon;
(iv) ein Tumorantigen, gegebenenfalls ausgewählt aus der Gruppe bestehend aus Alpha-Fetoprotein (AFP), karzinoembryonalem Antigen (CEA), Epithel-Tumorantigen (ETA), Mucin 1 (MUC1), Tn-MUC1, Mucin 16 (MUC16), Tyrosinase, melanom-assoziiertem Antigen (MAGE), Tumorprotein p53 (p53), CD4, CD8, CD45, CD80, CD86, PD-L1 (Programmed Death Ligand 1), PD-L2 (Programmed Death Ligand 2), NY-ESO-1, PSMA, TAG-72, HER2, GD2, cMET, EGFR, Mesothelin, VEGFR, Alpha-Folatrezeptor, CE7R, IL-3, Hodenkrebsantigen, MART-1 gp100 und TNF-verwandtem Apoptose induzierendem Liganden,
umfasst;
(b) ein Polypeptid;
(c) ein Peptid;
(d) ein Polynukleotid (DNA und/oder RNA);
(e) eine chemische Verbindung;
(f) ein Virus, wobei das Virus gegebenenfalls ein adeno-assoziiertes Virus, ein Parvovirus, ein Retrovirus, ein Adenovirus oder eine beliebige Kombination davon umfasst;
(g) einen Ionophor;
(h) einen Träger für einen Ionophor;
(i) eine Gruppierung, die einen Kanal oder eine Pore bildet; oder
(j) eine beliebige Kombination davon.

11. EV nach einem der Ansprüche 1 bis 10, wobei das EV ferner ein zweites Gerüstprotein umfasst, wobei das zweite Gerüstprotein gegebenenfalls ein PTGFRN-Polypeptid, ein BSG-Polypeptid, ein IGSF2-Polypeptid, ein IGSF3-Polypeptid, ein IGSF8-Polypeptid, ein ITGB1-Polypeptid, ein ITGA4-Polypeptid, ein SLC3A2-Polypeptid, ein ATP-Transporter-Polypeptid, ein ANPEP(Aminopeptidase N)-Polypeptid, ein ENPP1(Ectonucleotide Pyrophosphatase/Phosphodiesterase Family Member 1)-Polypeptid, ein MME(Neprilysin)-Polypeptid, ein NRP1(Neuropilin-1)-Polypeptid oder ein Fragment davon umfasst.

12. EV nach einem der Ansprüche 1 bis 11, wobei es sich bei dem biologisch aktiven Molekül um Folgendes handelt:
(a) einen Inhibitor für einen negativen Checkpoint-Regulator oder einen Inhibitor für einen Bindungspartner eines negativen Checkpoint-Regulators, wobei der negative Checkpoint-Regulator gegebenenfalls ausgewählt ist aus der Gruppe bestehend aus:
(i) CTLA-4 (Cytotoxic T-Lymphocyte-Associated Protein 4),
(ii) PD-1 (Programmed Cell Death Protein 1),
(iii) LAG-3 (Lymphocyte-Activated Gene 3),
(iv) TIM-3 (T-Cell Immunoglobulin Mucin-containing Protein 3 (TIM-3),
(v) BTLA (B and T Lymphocyte Attenuator),
(vi) TIGIT (T Cell Immunoreceptor with Ig and ITIM Domains),
(vii) VISTA (V-Domain Ig Suppressor of T Cell Activation),
(viii) A2aR (Adenosine A2a Receptor),
(ix) KIR (Killer Cell Immunoglobulin-like Receptor (KIR),
(x) IDO (Indoleamine 2,3-Dioxygenase),
(xi) CD20,
(xii) CD39 und
(xiii) CD73;
(b) ein immunogenes Protein;
(c) ein Toxin, Toxoid oder eine nicht toxische Mutante eines Toxins, wobei
(i) das Toxin gegebenenfalls ein Diphtherietoxin ist oder
(ii) das Toxoid gegebenenfalls ein Tetanustoxoid ist;
(d) einen Aktivator für ein positives Costimulatormolekül oder einen Aktivator für einen Bindungspartner eines positiven Costimulatormoleküls, wobei das positive Costimulatormolekül gegebenenfalls ausgewählt ist aus der Gruppe bestehend aus:
(i) einem Mitglied der TNF-Rezeptor-Superfamilie, das gegebenenfalls ausgewählt ist aus der Gruppe bestehend aus: CD120a, CD120b, CD18, OX40, CD40, Fas-Rezeptor, M68, CD27, CD30, 4-1BB, TRAILR1, TRAILR2, TRAILR3, TRAILR4, RANK, OCIF, TWEAK-Rezeptor, TACI, BAFF-Rezeptor, ATAR, CD271, CD269, AITR, TROY, CD358, TRAMP und XEDAR, gegebenenfalls wobei es sich bei dem Aktivator für das positive Costimulatormolekül um ein Mitglied der TNF-Superfamilie handelt, das gegebenenfalls ausgewählt ist aus der Gruppe bestehend aus: TNFa, TNF-C, OX40L, CD40L, FasL, LIGHT, TL1A, CD27L, Siva, CD153, 4-1BB-Ligand, TRAIL, RANKL, TWEAK, APRIL, BAFF, CAMLG, NGF, BDNF, NT-3, NT-4, GITR-Ligand und EDA-2; und
(ii) einem CD28-Superfamilie-Costimulatormolekül, das gegebenenfalls ausgewählt ist aus ICOS und CD28, gegebenenfalls wobei es sich bei dem Aktivator für das positive Costimulatormolekül um ICOSL, CD80 oder CD86 handelt.

13. EV nach einem der Ansprüche 1 bis 12, wobei es sich bei dem EV um ein Exosom handelt.

14. Pharmazeutische Zusammensetzung, umfassend das EV nach einem der Ansprüche 1 bis 13 und einen pharmazeutisch unbedenklichen Träger.

15. EV nach einem der Ansprüche 1 bis 13 zur Verwendung beim Vorbeugen oder Behandeln einer Krankheit bei einem dies benötigenden Individuum.

## Revendications

1. Vésicule extracellulaire (VE) isolée comprenant une molécule biologiquement active liée à une protéine échafaudage,
dans laquelle la protéine échafaudage comprend un domaine N-terminal (DN) et un domaine effecteur (DE),
dans laquelle le DN est associé à la surface luminale de la VE et le DE est associé à la surface luminale de la VE,
dans laquelle le DN comprend la séquence d'acides aminés GGKLSK (SEQ ID n° : 203) mais ne comprend pas une méthionine (Met) à l'extrémité N-terminale,
dans laquelle le DE comprend une lysine (Lys) à son extrémité N-terminale qui est directement liée à la lysine à l'extrémité C-terminale de la SEQ ID n° : 203 dans le DN ; et
dans laquelle la molécule biologiquement active comprend une ou plusieurs protéine(s) hétérologue(s) fusionnée(s) avec l'extrémité C-terminale de la protéine échafaudage.

2. VE de la revendication 1, dans laquelle le DE comprend KK, KKK, KKKK (SEQ ID n° : 151), KKKKK (SEQ ID n° : 152) ou une quelconque combinaison de ceux-ci.

3. VE des revendications 1 ou 2, dans laquelle la protéine échafaudage est liée à la molécule biologiquement active par un lieur.

4. VE de la revendication 3, dans laquelle le lieur comprend un lieur pouvant être coupé.

5. VE de l'une quelconque des revendications 1 à 4, dans laquelle la protéine échafaudage comprend GGKLSKKK (SEQ ID n° : 161) ou GGKLSKKS (SEQ ID n° : 162).

6. VE de l'une quelconque des revendications 1 à 5, dans laquelle la protéine échafaudage a une longueur d'au moins 8 acides aminés environ.

7. VE de l'une quelconque des revendications 1 à 6, dans laquelle la molécule biologiquement active est sur la surface luminale ou dans le lumen de la VE.

8. VE de l'une quelconque des revendications 1 à 7, dans laquelle la protéine échafaudage comprend en outre :
(i) un domaine transmembranaire,
(ii) un domaine extra-vésiculaire ou
(iii)à la fois (i) et (ii).

9. VE de l'une quelconque des revendications 1 à 6, dans laquelle la protéine échafaudage comprend en outre un domaine transmembranaire et un domaine extra-vésiculaire et :
(i) le domaine transmembranaire se trouve entre le domaine DE de la protéine échafaudage et la molécule biologiquement active et
(ii) la molécule biologiquement active est liée au domaine extra-vésiculaire.

10. VE de l'une quelconque des revendications 1 à 9, dans laquelle la molécule biologiquement active comprend :
(a) une protéine, la protéine comprenant en option
(i) un peptide recombinant, un peptide naturel, un peptide de synthèse, un anticorps, une protéine de fusion ou une quelconque combinaison de ceux-ci ;
(ii) une enzyme, une cytokine, un ligand, un récepteur, un facteur de transcription ou une combinaison de ceux-ci ;
(iii) un récepteur de lymphocyte T (TCR), un corécepteur de lymphocyte T, un complexe majeur d'histocompatibilité (CMH), un antigène leucocytaire humain (ALH) ou un dérivé de ceux-ci ;
(iv) un antigène tumoral, en option choisi dans le groupe constitué par l'alpha-fœto-protéine (AFP), un antigène carcino-embryonnaire (CEA), un antigène de tumeur épithéliale (ETA), la mucine 1 (MUC1), la Tn-MUC1, la mucine 16 (MUC16), une tyrosinase, un antigène associé à un mélanome (MAGE), la protéine tumorale p53 (p53), le CD4, le CD8, le CD45, le CD80, le CD86, le ligand 1 de mort programmée (PD-L1), le ligand 2 de mort programmée (PD-L2), le NY-ESO-1, le PSMA, le TAG-72, l'HER2, le GD2, la cMET, l'EGFR, la mésothéline, le VEGFR, un récepteur de l'alpha-folate, le CE7R, l'IL-3, un antigène de testicule cancéreux, le MART-1 la gp100 et un ligand induisant l'apoptose associée au TNF ;
(b) un polypeptide ;
(c) un peptide ;
(d) un polynucléotide (ADN et/ou ARN) ;
(e) un composé chimique ;
(f) un virus, dans laquelle le virus comprend, en option, un virus adéno-associé, un parvovirus, un rétrovirus, un adénovirus ou une quelconque combinaison de ceux-ci ;
(g) un ionophore ;
(h) un véhicule pour un ionophore ;
(i) une fraction qui forme un canal ou un pore ;
ou
(j) une quelconque combinaison de ceux-ci.

11. VE de l'une quelconque des revendications 1 à 10, la VE comprenant en outre une deuxième protéine échafaudage, dans laquelle la deuxième protéine échafaudage comprend, en option, un polypeptide de PTGFRN, un polypeptide de BSG, un polypeptide d'IGSF2, un polypeptide d'IGSF3, un polypeptide d'IGSF8, un polypeptide d'ITGB1, un polypeptide d'ITGA4, un polypeptide de SLC3A2, un polypeptide transporteur d'ATP, un polypeptide d'aminopeptidase N (ANPEP), un polypeptide d'un membre de la famille des ectonucléotide pyrophosphatase/phosphodiestérase 1 (ENPP1), un polypeptide de néprilysine (MME), un polypeptide de neuropiline-1 (NRP1) ou un fragment de ceux-ci.

12. VE de l'une quelconque des revendications 1 à 11, dans laquelle la molécule biologiquement active est :
(a) un inhibiteur pour un régulateur d'un point de contrôle négatif ou un inhibiteur pour un partenaire de liaison d'un régulateur d'un point de contrôle négatif, dans laquelle le régulateur d'un point de contrôle négatif est choisi, en option, dans le groupe constitué par :
(i) la protéine 4 associée aux lymphocytes T cytotoxiques (CTLA-4),
(ii) la protéine 1 de mort cellulaire programmée (PD-1),
(iii) le gène d'activation lymphocytaire 3 (LAG-3),
(iv) la protéine 3 contenant un domaine de type mucine et un de type immunoglobuline de lymphocytes T (TIM-3),
(v) un atténuateur des lymphocytes B et T (BTLA),
(vi) un immuno-récepteur de lymphocytes T avec des domaines Ig et ITIM (TIGIT),
(vii) un suppresseur de type Ig à domaine V de l'activation des lymphocytes T (VISTA),
(viii)un récepteur A2a de l'adénosine (A2aR),
(ix) un récepteur de type immunoglobuline de cellules tueuses (KIR),
(x) l'indole-amine 2,3-dioxygénase (IDO),
(xi) le CD20,
(xii) le CD39 et
(xiii)le CD73 ;
(b) une protéine immunogène ;
(c) une toxine, une anatoxine ou un mutant non toxique d'une toxine, dans laquelle
(i) la toxine est, en option, la toxine diphtérique ou
(ii) l'anatoxine est l'anatoxine tétanique ;
(d) un activateur pour une molécule de co-stimulation positive ou un activateur pour un partenaire de liaison d'une molécule de co-stimulation positive, dans laquelle la molécule de co-stimulation positive est choisie, en option, dans le groupe constitué par :
(i) un membre de la superfamille des récepteurs de TNF, en option choisi dans le groupe constitué par les : CD120a, CD120b, CD18, OX40, CD40, récepteur de Fas, M68, CD27, CD30, 4-1BB, TRAILR1, TRAILR2, TRAILR3, TRAILR4, RANK, OCIF, récepteur de TWEAK, TACI, récepteur de BAFF, ATAR, CD271, CD269, AITR, TROY, CD358, TRAMP et XEDAR, en option dans laquelle l'activateur pour la molécule de co-stimulation positive est un membre de la superfamille des TNF choisi, en option, dans le groupe constitué par les : TNFα, TNF-C, OX40L, CD40L, FasL, LIGHT, TL1A, CD27L, Siva, CD153, ligand 4-1BB, TRAIL, RANKL, TWEAK, APRIL, BAFF, CAMLG, NGF, BDNF, NT-3, NT-4, ligand GITR et EDA-2 ; et
(ii) une molécule de co-stimulation de la superfamille des CD28 choisie, en option, parmi l'ICOS et le CD28, en option dans laquelle l'activateur pour une molécule de co-stimulation positive est l'ICOSL, le CD80 ou le CD86.

13. VE de l'une quelconque des revendications 1 à 12, la VE étant un exosome.

14. Composition pharmaceutique comprenant la VE de l'une quelconque des revendications 1 à 13 et un véhicule acceptable d'un point de vue pharmaceutique.

15. VE de l'une quelconque des revendications 1 à 13 destinée à être utilisée dans la prévention ou le traitement d'une maladie chez un sujet la nécessitant.


### SEQUENCE LISTING

<110> CODIAK BIOSCIENCES
<120> ENGINEERED EXTRACELLULAR VESICLES AND USES THEREOF
<130> 4000.041PC01/C-K/BMD
<140> PCT/US2018/061679
   <141> 2018-11-16
<150> US 62/835,430
   <151> 2019-04-17
<160> 425
<170> PatentIn version 3.5
<210> 1
   <211> 332
   <212> PRT
   <213> Artificial Sequence
<220>
   <223> MARCKS protein
<400> 1
   Met Gly Ala Gln Phe Ser Lys Thr Ala Ala Lys Gly Glu Ala Ala Ala
   1               5                   10                  15
   Glu Arg Pro Gly Glu Ala Ala Val Ala Ser Ser Pro Ser Lys Ala Asn
               20                  25                  30
   Gly Gln Glu Asn Gly His Val Lys Val Asn Gly Asp Ala Ser Pro Ala
           35                  40                  45
   Ala Ala Glu Ser Gly Ala Lys Glu Glu Leu Gln Ala Asn Gly Ser Ala
       50                  55                  60
   Pro Ala Ala Asp Lys Glu Glu Pro Ala Ala Ala Gly Ser Gly Ala Ala
   65                  70                  75                  80
   Ser Pro Ser Ala Ala Glu Lys Gly Glu Pro Ala Ala Ala Ala Ala Pro
                   85                  90                  95
   Glu Ala Gly Ala Ser Pro Val Glu Lys Glu Ala Pro Ala Glu Gly Glu
               100                 105                 110
   Ala Ala Glu Pro Gly Ser Pro Thr Ala Ala Glu Gly Glu Ala Ala Ser
           115                 120                 125
   Ala Ala Ser Ser Thr Ser Ser Pro Lys Ala Glu Asp Gly Ala Thr Pro
       130                 135                 140
   Ser Pro Ser Asn Glu Thr Pro Lys Lys Lys Lys Lys Arg Phe Ser Phe
   145                 150                 155                 160
   Lys Lys Ser Phe Lys Leu Ser Gly Phe Ser Phe Lys Lys Asn Lys Lys
                   165                 170                 175
   Glu Ala Gly Glu Gly Gly Glu Ala Glu Ala Pro Ala Ala Glu Gly Gly
               180                 185                 190
   Lys Asp Glu Ala Ala Gly Gly Ala Ala Ala Ala Ala Ala Glu Ala Gly
           195                 200                 205
   Ala Ala Ser Gly Glu Gln Ala Ala Ala Pro Gly Glu Glu Ala Ala Ala
       210                 215                 220
   Gly Glu Glu Gly Ala Ala Gly Gly Asp Pro Gln Glu Ala Lys Pro Gln
   225                 230                 235                 240
   Glu Ala Ala Val Ala Pro Glu Lys Pro Pro Ala Ser Asp Glu Thr Lys
                   245                 250                 255
   Ala Ala Glu Glu Pro Ser Lys Val Glu Glu Lys Lys Ala Glu Glu Ala
               260                 265                 270
   Gly Ala Ser Ala Ala Ala Cys Glu Ala Pro Ser Ala Ala Gly Pro Gly
           275                 280                 285
   Ala Pro Pro Glu Gln Glu Ala Ala Pro Ala Glu Glu Pro Ala Ala Ala
       290                 295                 300
   Ala Ala Ser Ser Ala Cys Ala Ala Pro Ser Gln Glu Ala Gln Pro Glu
   305                 310                 315                 320
   Cys Ser Pro Glu Ala Pro Pro Ala Glu Ala Ala Glu
                   325                 330
<210> 2
   <211> 195
   <212> PRT
   <213> Artificial Sequence
<220>
   <223> MARCKSL1 protein
<400> 2
   Met Gly Ser Gln Ser Ser Lys Ala Pro Arg Gly Asp Val Thr Ala Glu
   1               5                   10                  15
   Glu Ala Ala Gly Ala Ser Pro Ala Lys Ala Asn Gly Gln Glu Asn Gly
               20                  25                  30
   His Val Lys Ser Asn Gly Asp Leu Ser Pro Lys Gly Glu Gly Glu Ser
           35                  40                  45
   Pro Pro Val Asn Gly Thr Asp Glu Ala Ala Gly Ala Thr Gly Asp Ala
       50                  55                  60
   Ile Glu Pro Ala Pro Pro Ser Gln Gly Ala Glu Ala Lys Gly Glu Val
   65                  70                  75                  80
   Pro Pro Lys Glu Thr Pro Lys Lys Lys Lys Lys Phe Ser Phe Lys Lys
                   85                  90                  95
   Pro Phe Lys Leu Ser Gly Leu Ser Phe Lys Arg Asn Arg Lys Glu Gly
               100                 105                 110
   Gly Gly Asp Ser Ser Ala Ser Ser Pro Thr Glu Glu Glu Gln Glu Gln
           115                 120                 125
   Gly Glu Ile Gly Ala Cys Ser Asp Glu Gly Thr Ala Gln Glu Gly Lys
       130                 135                 140
   Ala Ala Ala Thr Pro Glu Ser Gln Glu Pro Gln Ala Lys Gly Ala Glu
   145                 150                 155                 160
   Ala Ser Ala Ala Ser Glu Glu Glu Ala Gly Pro Gln Ala Thr Glu Pro
                   165                 170                 175
   Ser Thr Pro Ser Gly Pro Glu Ser Gly Pro Thr Pro Ala Ser Ala Glu
               180                 185                 190
   Gln Asn Glu
           195
<210> 3
   <211> 227
   <212> PRT
   <213> Artificial Sequence
<220>
   <223> BASP1 protein
<400> 3
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Thr Glu
               20                  25                  30
   Glu Glu Gly Thr Pro Lys Glu Ser Glu Pro Gln Ala Ala Ala Glu Pro
           35                  40                  45
   Ala Glu Ala Lys Glu Gly Lys Glu Lys Pro Asp Gln Asp Ala Glu Gly
       50                  55                  60
   Lys Ala Glu Glu Lys Glu Gly Glu Lys Asp Ala Ala Ala Ala Lys Glu
   65                  70                  75                  80
   Glu Ala Pro Lys Ala Glu Pro Glu Lys Thr Glu Gly Ala Ala Glu Ala
                   85                  90                  95
   Lys Ala Glu Pro Pro Lys Ala Pro Glu Gln Glu Gln Ala Ala Pro Gly
               100                 105                 110
   Pro Ala Ala Gly Gly Glu Ala Pro Lys Ala Ala Glu Ala Ala Ala Ala
           115                 120                 125
   Pro Ala Glu Ser Ala Ala Pro Ala Ala Gly Glu Glu Pro Ser Lys Glu
       130                 135                 140
   Glu Gly Glu Pro Lys Lys Thr Glu Ala Pro Ala Ala Pro Ala Ala Gln
   145                 150                 155                 160
   Glu Thr Lys Ser Asp Gly Ala Pro Ala Ser Asp Ser Lys Pro Gly Ser
                   165                 170                 175
   Ser Glu Ala Ala Pro Ser Ser Lys Glu Thr Pro Ala Ala Thr Glu Ala
               180                 185                 190
   Pro Ser Ser Thr Pro Lys Ala Gln Gly Pro Ala Ala Ser Ala Glu Glu
           195                 200                 205
   Pro Lys Pro Val Glu Ala Pro Ala Ala Asn Ser Asp Gln Thr Val Thr
       210                 215                 220
   Val Lys Glu
   225
<210> 4
   <211> 30
   <212> PRT
   <213> Artificial Sequence
<220>
   <223> BASP1 fragment
<400> 4
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala
               20                  25                  30
<210> 5
   <211> 27
   <212> PRT
   <213> Artificial Sequence
<220>
   <223> fusion protein
<400> 5
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu
               20                  25
<210> 6
   <211> 24
   <212> PRT
   <213> Artificial Sequence
<220>
   <223> fusion protein
<400> 6
   Met Gly Gly Lys Leu Ser Lys Lys Lys Lys Gly Tyr Asn Val Asn Asp
   1               5                   10                  15
   Glu Lys Ala Lys Glu Lys Asp Lys
               20
<210> 7
   <211> 21
   <212> PRT
   <213> Artificial Sequence
<220>
   <223> fusion protein
<400> 7
   Met Gly Gly Lys Leu Ser Lys Lys Lys Lys Gly Tyr Asn Val Asn Asp
   1               5                   10                  15
   Glu Lys Ala Lys Glu
               20
<210> 8
   <211> 18
   <212> PRT
   <213> Artificial Sequence
<220>
   <223> fusion protein
<400> 8
   Met Gly Gly Lys Leu Ser Lys Lys Lys Lys Gly Tyr Asn Val Asn Asp
   1               5                   10                  15
   Glu Lys
<210> 9
   <211> 15
   <212> PRT
   <213> Artificial Sequence
<220>
   <223> fusion protein
<400> 9
   Met Gly Gly Lys Leu Ser Lys Lys Lys Lys Gly Tyr Asn Val Asn
   1               5                   10                  15
<210> 10
   <211> 12
   <212> PRT
   <213> Artificial Sequence
<220>
   <223> fusion protein
<400> 10
   Met Gly Gly Lys Leu Ser Lys Lys Lys Lys Gly Tyr
   1               5                   10
<210> 11
   <211> 11
   <212> PRT
   <213> Artificial Sequence
<220>
   <223> fusion protein
<400> 11
   Met Gly Gly Lys Leu Ser Lys Lys Lys Lys Gly
   1 5 10
<210> 12
   <211> 10
   <212> PRT
   <213> Artificial Sequence
<220>
   <223> fusion protein
<400> 12
   Met Gly Gly Lys Leu Ser Lys Lys Lys Lys
   1 5 10
<210> 13
   <211> 9
   <212> PRT
   <213> Artificial Sequence
<220>
   <223> fusion protein
<400> 13
   Met Gly Gly Lys Leu Ser Lys Lys Lys
   1 5
<210> 14
   <211> 8
   <212> PRT
   <213> Artificial Sequence
<220>
   <223> fusion protein
<400> 14
   Met Gly Gly Lys Leu Ser Lys Lys
   1 5
<210> 15
   <211> 7
   <212> PRT
   <213> Artificial Sequence
<220>
   <223> fusion protein
<400> 15
   Met Gly Gly Lys Leu Ser Lys
   1 5
<210> 16
   <211> 8
   <212> PRT
   <213> Artificial Sequence
<220>
   <223> fusion protein
<400> 16
   Met Gly Gly Lys Leu Ala Lys Lys
   1               5
<210> 17
   <211> 8
   <212> PRT
   <213> Artificial Sequence
<220>
   <223> fusion protein
<400> 17
   Met Gly Gly Lys Phe Ser Lys Lys
   1               5
<210> 18
   <211> 8
   <212> PRT
   <213> Artificial Sequence
<220>
   <223> fusion protein
<400> 18
   Met Gly Gly Lys Phe Ala Lys Lys
   1               5
<210> 19
   <211> 8
   <212> PRT
   <213> Artificial Sequence
<220>
   <223> fusion protein
<400> 19
   Met Gly Gly Lys Ser Ser Lys Lys
   1               5
<210> 20
   <211> 8
   <212> PRT
   <213> Artificial Sequence
<220>
   <223> fusion protein
<400> 20
   Met Gly Gly Lys Ser Ala Lys Lys
   1 5
<210> 21
   <211> 8
   <212> PRT
   <213> Artificial Sequence
<220>
   <223> fusion protein
<400> 21
   Met Gly Gly Lys Gln Ser Lys Lys
   1 5
<210> 22
   <211> 8
   <212> PRT
   <213> Artificial Sequence
<220>
   <223> fusion protein
<400> 22
   Met Gly Gly Lys Gln Ala Lys Lys
   1 5
<210> 23
   <211> 8
   <212> PRT
   <213> Artificial Sequence
<220>
   <223> fusion protein
<400> 23
   Met Gly Gly Gln Leu Ser Lys Lys
   1 5
<210> 24
   <211> 8
   <212> PRT
   <213> Artificial Sequence
<220>
   <223> fusion protein
<400> 24
   Met Gly Gly Gln Leu Ala Lys Lys
   1 5
<210> 25
   <211> 8
   <212> PRT
   <213> Artificial Sequence
<220>
   <223> fusion protein
<400> 25
   Met Gly Gly Gln Phe Ser Lys Lys
   1               5
<210> 26
   <211> 8
   <212> PRT
   <213> Artificial Sequence
<220>
   <223> fusion protein
<400> 26
   Met Gly Gly Gln Phe Ala Lys Lys
   1               5
<210> 27
   <211> 8
   <212> PRT
   <213> Artificial Sequence
<220>
   <223> fusion protein
<400> 27
   Met Gly Gly Gln Ser Ser Lys Lys
   1               5
<210> 28
   <211> 8
   <212> PRT
   <213> Artificial Sequence
<220>
   <223> fusion protein
<400> 28
   Met Gly Gly Gln Ser Ala Lys Lys
   1               5
<210> 29
   <211> 8
   <212> PRT
   <213> Artificial Sequence
<220>
   <223> fusion protein
<400> 29
   Met Gly Gly Gln Gln Ser Lys Lys
   1               5
<210> 30
   <211> 8
   <212> PRT
   <213> Artificial Sequence
<220>
   <223> fusion protein
<400> 30
   Met Gly Gly Gln Gln Ala Lys Lys
   1               5
<210> 31
   <211> 8
   <212> PRT
   <213> Artificial Sequence
<220>
   <223> fusion protein
<400> 31
   Met Gly Ala Lys Leu Ser Lys Lys
   1               5
<210> 32
   <211> 8
   <212> PRT
   <213> Artificial Sequence
<220>
   <223> fusion protein
<400> 32
   Met Gly Ala Lys Leu Ala Lys Lys
   1               5
<210> 33
   <211> 8
   <212> PRT
   <213> Artificial Sequence
<220>
   <223> fusion protein
<400> 33
   Met Gly Ala Lys Phe Ser Lys Lys
   1               5
<210> 34
   <211> 8
   <212> PRT
   <213> Artificial Sequence
<220>
   <223> fusion protein
<400> 34
   Met Gly Ala Lys Phe Ala Lys Lys
   1               5
<210> 35
   <211> 8
   <212> PRT
   <213> Artificial Sequence
<220>
   <223> fusion protein
<400> 35
   Met Gly Ala Lys Ser Ser Lys Lys
   1               5
<210> 36
   <211> 8
   <212> PRT
   <213> Artificial Sequence
<220>
   <223> fusion protein
<400> 36
   Met Gly Ala Lys Ser Ala Lys Lys
   1               5
<210> 37
   <211> 8
   <212> PRT
   <213> Artificial Sequence
<220>
   <223> fusion protein
<400> 37
   Met Gly Ala Lys Gln Ser Lys Lys
   1               5
<210> 38
   <211> 8
   <212> PRT
   <213> Artificial Sequence
<220>
   <223> fusion protein
<400> 38
   Met Gly Ala Lys Gln Ala Lys Lys
   1 5
<210> 39
   <211> 8
   <212> PRT
   <213> Artificial Sequence
<220>
   <223> fusion protein
<400> 39
   Met Gly Ala Gln Leu Ser Lys Lys
   1 5
<210> 40
   <211> 8
   <212> PRT
   <213> Artificial Sequence
<220>
   <223> fusion protein
<400> 40
   Met Gly Ala Gln Leu Ala Lys Lys
   1 5
<210> 41
   <211> 8
   <212> PRT
   <213> Artificial Sequence
<220>
   <223> fusion protein
<400> 41
   Met Gly Ala Gln Phe Ser Lys Lys
   1 5
<210> 42
   <211> 8
   <212> PRT
   <213> Artificial Sequence
<220>
   <223> fusion protein
<400> 42
   Met Gly Ala Gln Phe Ala Lys Lys
   1 5
<210> 43
   <211> 8
   <212> PRT
   <213> Artificial Sequence
<220>
   <223> fusion protein
<400> 43
   Met Gly Ala Gln Ser Ser Lys Lys
   1 5
<210> 44
   <211> 8
   <212> PRT
   <213> Artificial Sequence
<220>
   <223> fusion protein
<400> 44
   Met Gly Ala Gln Ser Ala Lys Lys
   1 5
<210> 45
   <211> 8
   <212> PRT
   <213> Artificial Sequence
<220>
   <223> fusion protein
<400> 45
   Met Gly Ala Gln Gln Ser Lys Lys
   1 5
<210> 46
   <211> 8
   <212> PRT
   <213> Artificial Sequence
<220>
   <223> fusion protein
<400> 46
   Met Gly Ala Gln Gln Ala Lys Lys
   1 5
<210> 47
   <211> 8
   <212> PRT
   <213> Artificial Sequence
<220>
   <223> fusion protein
<400> 47
   Met Gly Ser Lys Leu Ser Lys Lys
   1 5
<210> 48
   <211> 8
   <212> PRT
   <213> Artificial Sequence
<220>
   <223> fusion protein
<400> 48
   Met Gly Ser Lys Leu Ala Lys Lys
   1 5
<210> 49
   <211> 8
   <212> PRT
   <213> Artificial Sequence
<220>
   <223> fusion protein
<400> 49
   Met Gly Ser Lys Phe Ser Lys Lys
   1 5
<210> 50
   <211> 8
   <212> PRT
   <213> Artificial Sequence
<220>
   <223> fusion protein
<400> 50
   Met Gly Ser Lys Phe Ala Lys Lys
   1 5
<210> 51
   <211> 8
   <212> PRT
   <213> Artificial Sequence
<220>
   <223> fusion protein
<400> 51
   Met Gly Ser Lys Ser Ser Lys Lys
   1 5
<210> 52
   <211> 8
   <212> PRT
   <213> Artificial Sequence
<220>
   <223> fusion protein
<400> 52
   Met Gly Ser Lys Ser Ala Lys Lys
   1               5
<210> 53
   <211> 8
   <212> PRT
   <213> Artificial Sequence
<220>
   <223> fusion protein
<400> 53
   Met Gly Ser Lys Gln Ser Lys Lys
   1               5
<210> 54
   <211> 8
   <212> PRT
   <213> Artificial Sequence
<220>
   <223> fusion protein
<400> 54
   Met Gly Ser Lys Gln Ala Lys Lys
   1               5
<210> 55
   <211> 8
   <212> PRT
   <213> Artificial Sequence
<220>
   <223> fusion protein
<400> 55
   Met Gly Ser Gln Leu Ser Lys Lys
   1               5
<210> 56
   <211> 8
   <212> PRT
   <213> Artificial Sequence
<220>
   <223> fusion protein
<400> 56
   Met Gly Ser Gln Leu Ala Lys Lys
   1 5
<210> 57
   <211> 8
   <212> PRT
   <213> Artificial Sequence
<220>
   <223> fusion protein
<400> 57
   Met Gly Ser Gln Phe Ser Lys Lys
   1 5
<210> 58
   <211> 8
   <212> PRT
   <213> Artificial Sequence
<220>
   <223> fusion protein
<400> 58
   Met Gly Ser Gln Phe Ala Lys Lys
   1 5
<210> 59
   <211> 8
   <212> PRT
   <213> Artificial Sequence
<220>
   <223> fusion protein
<400> 59
   Met Gly Ser Gln Ser Ser Lys Lys
   1 5
<210> 60
   <211> 8
   <212> PRT
   <213> Artificial Sequence
<220>
   <223> fusion protein
<400> 60
   Met Gly Ser Gln Ser Ala Lys Lys
   1 5
<210> 61
   <211> 8
   <212> PRT
   <213> Artificial Sequence
<220>
   <223> fusion protein
<400> 61
   Met Gly Ser Gln Gln Ser Lys Lys
   1               5
<210> 62
   <211> 8
   <212> PRT
   <213> Artificial Sequence
<220>
   <223> fusion protein
<400> 62
   Met Gly Ser Gln Gln Ala Lys Lys
   1               5
<210> 63
   <211> 7
   <212> PRT
   <213> Artificial Sequence
<220>
   <223> fusion protein
<400> 63
   Met Gly Gly Lys Leu Ala Lys
   1               5
<210> 64
   <211> 7
   <212> PRT
   <213> Artificial Sequence
<220>
   <223> fusion protein
<400> 64
   Met Gly Gly Lys Phe Ser Lys
   1               5
<210> 65
   <211> 7
   <212> PRT
   <213> Artificial Sequence
<220>
   <223> fusion protein
<400> 65
   Met Gly Gly Lys Phe Ala Lys
   1               5
<210> 66
   <211> 7
   <212> PRT
   <213> Artificial Sequence
<220>
   <223> fusion protein
<400> 66
   Met Gly Gly Lys Ser Ser Lys
   1               5
<210> 67
   <211> 7
   <212> PRT
   <213> Artificial Sequence
<220>
   <223> fusion protein
<400> 67
   Met Gly Gly Lys Ser Ala Lys
   1               5
<210> 68
   <211> 7
   <212> PRT
   <213> Artificial Sequence
<220>
   <223> fusion protein
<400> 68
   Met Gly Gly Lys Gln Ser Lys
   1               5
<210> 69
   <211> 7
   <212> PRT
   <213> Artificial Sequence
<220>
   <223> fusion protein
<400> 69
   Met Gly Gly Lys Gln Ala Lys
   1               5
<210> 70
   <211> 7
   <212> PRT
   <213> Artificial Sequence
<220>
   <223> fusion protein
<400> 70
   Met Gly Gly Gln Leu Ser Lys
   1 5
<210> 71
   <211> 7
   <212> PRT
   <213> Artificial Sequence
<220>
   <223> fusion protein
<400> 71
   Met Gly Gly Gln Leu Ala Lys
   1 5
<210> 72
   <211> 7
   <212> PRT
   <213> Artificial Sequence
<220>
   <223> fusion protein
<400> 72
   Met Gly Gly Gln Phe Ser Lys
   1 5
<210> 73
   <211> 7
   <212> PRT
   <213> Artificial Sequence
<220>
   <223> fusion protein
<400> 73
   Met Gly Gly Gln Phe Ala Lys
   1 5
<210> 74
   <211> 7
   <212> PRT
   <213> Artificial Sequence
<220>
   <223> fusion protein
<400> 74
   Met Gly Gly Gln Ser Ser Lys
   1 5
<210> 75
   <211> 7
   <212> PRT
   <213> Artificial Sequence
<220>
   <223> fusion protein
<400> 75
   Met Gly Gly Gln Ser Ala Lys
   1 5
<210> 76
   <211> 7
   <212> PRT
   <213> Artificial Sequence
<220>
   <223> fusion protein
<400> 76
   Met Gly Gly Gln Gln Ser Lys
   1 5
<210> 77
   <211> 7
   <212> PRT
   <213> Artificial Sequence
<220>
   <223> fusion protein
<400> 77
   Met Gly Gly Gln Gln Ala Lys
   1 5
<210> 78
   <211> 7
   <212> PRT
   <213> Artificial Sequence
<220>
   <223> fusion protein
<400> 78
   Met Gly Ala Lys Leu Ser Lys
   1 5
<210> 79
   <211> 7
   <212> PRT
   <213> Artificial Sequence
<220>
   <223> fusion protein
<400> 79
   Met Gly Ala Lys Leu Ala Lys
   1               5
<210> 80
   <211> 7
   <212> PRT
   <213> Artificial Sequence
<220>
   <223> fusion protein
<400> 80
   Met Gly Ala Lys Phe Ser Lys
   1               5
<210> 81
   <211> 7
   <212> PRT
   <213> Artificial Sequence
<220>
   <223> fusion protein
<400> 81
   Met Gly Ala Lys Phe Ala Lys
   1               5
<210> 82
   <211> 7
   <212> PRT
   <213> Artificial Sequence
<220>
   <223> fusion protein
<400> 82
   Met Gly Ala Lys Ser Ser Lys
   1               5
<210> 83
   <211> 7
   <212> PRT
   <213> Artificial Sequence
<220>
   <223> fusion protein
<400> 83
   Met Gly Ala Lys Ser Ala Lys
   1 5
<210> 84
   <211> 7
   <212> PRT
   <213> Artificial Sequence
<220>
   <223> fusion protein
<400> 84
   Met Gly Ala Lys Gln Ser Lys
   1 5
<210> 85
   <211> 7
   <212> PRT
   <213> Artificial Sequence
<220>
   <223> fusion protein
<400> 85
   Met Gly Ala Lys Gln Ala Lys
   1 5
<210> 86
   <211> 7
   <212> PRT
   <213> Artificial Sequence
<220>
   <223> fusion protein
<400> 86
   Met Gly Ala Gln Leu Ser Lys
   1 5
<210> 87
   <211> 7
   <212> PRT
   <213> Artificial Sequence
<220>
   <223> fusion protein
<400> 87
   Met Gly Ala Gln Leu Ala Lys
   1 5
<210> 88
   <211> 7
   <212> PRT
   <213> Artificial Sequence
<220>
   <223> fusion protein
<400> 88
   Met Gly Ala Gln Phe Ser Lys
   1               5
<210> 89
   <211> 7
   <212> PRT
   <213> Artificial Sequence
<220>
   <223> fusion protein
<400> 89
   Met Gly Ala Gln Phe Ala Lys
   1               5
<210> 90
   <211> 7
   <212> PRT
   <213> Artificial Sequence
<220>
   <223> fusion protein
<400> 90
   Met Gly Ala Gln Ser Ser Lys
   1               5
<210> 91
   <211> 7
   <212> PRT
   <213> Artificial Sequence
<220>
   <223> fusion protein
<400> 91
   Met Gly Ala Gln Ser Ala Lys
   1               5
<210> 92
   <211> 7
   <212> PRT
   <213> Artificial Sequence
<220>
   <223> fusion protein
<400> 92
   Met Gly Ala Gln Gln Ser Lys
   1 5
<210> 93
   <211> 7
   <212> PRT
   <213> Artificial Sequence
<220>
   <223> fusion protein
<400> 93
   Met Gly Ala Gln Gln Ala Lys
   1 5
<210> 94
   <211> 7
   <212> PRT
   <213> Artificial Sequence
<220>
   <223> fusion protein
<400> 94
   Met Gly Ser Lys Leu Ser Lys
   1 5
<210> 95
   <211> 7
   <212> PRT
   <213> Artificial Sequence
<220>
   <223> fusion protein
<400> 95
   Met Gly Ser Lys Leu Ala Lys
   1 5
<210> 96
   <211> 7
   <212> PRT
   <213> Artificial Sequence
<220>
   <223> fusion protein
<400> 96
   Met Gly Ser Lys Phe Ser Lys
   1 5
<210> 97
   <211> 7
   <212> PRT
   <213> Artificial Sequence
<220>
   <223> fusion protein
<400> 97
   Met Gly Ser Lys Phe Ala Lys
   1               5
<210> 98
   <211> 7
   <212> PRT
   <213> Artificial Sequence
<220>
   <223> fusion protein
<400> 98
   Met Gly Ser Lys Ser Ser Lys
   1               5
<210> 99
   <211> 7
   <212> PRT
   <213> Artificial Sequence
<220>
   <223> fusion protein
<400> 99
   Met Gly Ser Lys Ser Ala Lys
   1               5
<210> 100
   <211> 7
   <212> PRT
   <213> Artificial Sequence
<220>
   <223> fusion protein
<400> 100
   Met Gly Ser Lys Gln Ser Lys
   1               5
<210> 101
   <211> 7
   <212> PRT
   <213> Artificial Sequence
<220>
   <223> fusion protein
<400> 101
   Met Gly Ser Lys Gln Ala Lys
   1 5
<210> 102
   <211> 7
   <212> PRT
   <213> Artificial Sequence
<220>
   <223> fusion protein
<400> 102
   Met Gly Ser Gln Leu Ser Lys
   1 5
<210> 103
   <211> 7
   <212> PRT
   <213> Artificial Sequence
<220>
   <223> fusion protein
<400> 103
   Met Gly Ser Gln Leu Ala Lys
   1 5
<210> 104
   <211> 7
   <212> PRT
   <213> Artificial Sequence
<220>
   <223> fusion protein
<400> 104
   Met Gly Ser Gln Phe Ser Lys
   1 5
<210> 105
   <211> 7
   <212> PRT
   <213> Artificial Sequence
<220>
   <223> fusion protein
<400> 105
   Met Gly Ser Gln Phe Ala Lys
   1 5
<210> 106
   <211> 7
   <212> PRT
   <213> Artificial Sequence
<220>
   <223> fusion protein
<400> 106
   Met Gly Ser Gln Ser Ser Lys
   1 5
<210> 107
   <211> 7
   <212> PRT
   <213> Artificial Sequence
<220>
   <223> fusion protein
<400> 107
   Met Gly Ser Gln Ser Ala Lys
   1 5
<210> 108
   <211> 7
   <212> PRT
   <213> Artificial Sequence
<220>
   <223> fusion protein
<400> 108
   Met Gly Ser Gln Gln Ser Lys
   1 5
<210> 109
   <211> 7
   <212> PRT
   <213> Artificial Sequence
<220>
   <223> fusion protein
<400> 109
   Met Gly Ser Gln Gln Ala Lys
   1 5
<210> 110
   <211> 9
   <212> PRT
   <213> Artificial Sequence
<220>
   <223> scaffold protein
<400> 110
   Met Gly Ala Lys Leu Ser Lys Lys Lys
   1               5
<210> 111
   <211> 167
   <212> PRT
   <213> Artificial Sequence
<220>
   <223> scaffold protein
<400> 111
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Ser Gly
               20                  25                  30
   Gly Ser Gly Gly Ser Asp Tyr Lys Asp Asp Asp Asp Lys Gly Gly Gly
           35                  40                  45
   Ser Gly Met Ala Ser Asn Phe Thr Gln Phe Val Leu Val Asp Asn Gly
       50                  55                  60
   Gly Thr Gly Asp Val Thr Val Ala Pro Ser Asn Phe Ala Asn Gly Ile
   65                  70                  75                  80
   Ala Glu Trp Ile Ser Ser Asn Ser Arg Ser Gln Ala Tyr Lys Val Thr
                   85                  90                  95
   Cys Ser Val Arg Gln Ser Ser Ala Gln Asn Arg Lys Tyr Thr Ile Lys
               100                 105                 110
   Val Glu Val Pro Lys Gly Ala Trp Arg Ser Tyr Leu Asn Met Glu Leu
           115                 120                 125
   Thr Ile Pro Ile Phe Ala Thr Asn Ser Asp Cys Glu Leu Ile Val Lys
       130                 135                 140
   Ala Met Gln Gly Leu Leu Lys Asp Gly Asn Pro Ile Pro Ser Ala Ile
   145                 150                 155                 160
   Ala Ala Asn Ser Gly Ile Tyr
                   165
<210> 112
   <211> 167
   <212> PRT
   <213> Artificial Sequence
<220>
   <223> scaffold protein
<400> 112
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Ser Gly
               20                  25                  30
   Gly Ser Gly Gly Ser Asp Tyr Lys Asp Asp Asp Asp Lys Gly Gly Gly
           35                  40                  45
   Ser Gly Met Ala Ser Asn Phe Thr Gln Phe Val Leu Val Asp Asn Gly
       50                  55                  60
   Gly Thr Gly Asp Val Thr Val Ala Pro Ser Asn Phe Ala Asn Gly Ile
   65                  70                  75                  80
   Ala Glu Trp Ile Ser Ser Asn Ser Arg Ser Gln Ala Tyr Lys Val Thr
                   85                  90                  95
   Cys Ser Val Arg Gln Ser Ser Ala Gln Lys Arg Lys Tyr Thr Ile Lys
               100                 105                 110
   Val Glu Val Pro Lys Gly Ala Trp Arg Ser Tyr Leu Asn Met Glu Leu
           115                 120                 125
   Thr Ile Pro Ile Phe Ala Thr Asn Ser Asp Cys Glu Leu Ile Val Lys
       130                 135                 140
   Ala Met Gln Gly Leu Leu Lys Asp Gly Asn Pro Ile Pro Ser Ala Ile
   145                 150                 155                 160
   Ala Ala Asn Ser Gly Ile Tyr
                   165
<210> 113
   <211> 296
   <212> PRT
   <213> Artificial Sequence
<220>
   <223> scaffold protein
<400> 113
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Ser Gly
               20                  25                  30
   Gly Ser Gly Gly Ser Asp Tyr Lys Asp Asp Asp Asp Lys Gly Gly Gly
           35                  40                  45
   Ser Gly Met Ala Ser Asn Phe Thr Gln Phe Val Leu Val Asp Asn Gly
       50                  55                  60
   Gly Thr Gly Asp Val Thr Val Ala Pro Ser Asn Phe Ala Asn Gly Ile
   65                  70                  75                  80
   Ala Glu Trp Ile Ser Ser Asn Ser Arg Ser Gln Ala Tyr Lys Val Thr
                   85                  90                  95
   Cys Ser Val Arg Gln Ser Ser Ala Gln Asn Arg Lys Tyr Thr Ile Lys
               100                 105                 110
   Val Glu Val Pro Lys Gly Ala Trp Arg Ser Tyr Leu Asn Met Glu Leu
           115                 120                 125
   Thr Ile Pro Ile Phe Ala Thr Asn Ser Asp Cys Glu Leu Ile Val Lys
       130                 135                 140
   Ala Met Gln Gly Leu Leu Lys Asp Gly Asn Pro Ile Pro Ser Ala Ile
   145                 150                 155                 160
   Ala Ala Asn Ser Gly Ile Tyr Gly Ser Gly Gly Ser Gly Gly Ser Gly
                   165                 170                 175
   Gly Ser Gly Met Ala Ser Asn Phe Thr Gln Phe Val Leu Val Asp Asn
               180                 185                 190
   Gly Gly Thr Gly Asp Val Thr Val Ala Pro Ser Asn Phe Ala Asn Gly
           195                 200                 205
   Ile Ala Glu Trp Ile Ser Ser Asn Ser Arg Ser Gln Ala Tyr Lys Val
       210                 215                 220
   Thr Cys Ser Val Arg Gln Ser Ser Ala Gln Asn Arg Lys Tyr Thr Ile
   225                 230                 235                 240
   Lys Val Glu Val Pro Lys Gly Ala Trp Arg Ser Tyr Leu Asn Met Glu
                   245                 250                 255
   Leu Thr Ile Pro Ile Phe Ala Thr Asn Ser Asp Cys Glu Leu Ile Val
               260                 265                 270
   Lys Ala Met Gln Gly Leu Leu Lys Asp Gly Asn Pro Ile Pro Ser Ala
           275                 280                 285
   Ile Ala Ala Asn Ser Gly Ile Tyr
   290 295
<210> 114
   <211> 296
   <212> PRT
   <213> Artificial Sequence
<220>
   <223> scaffold protein
<400> 114
   Met Gly Gly Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Ser Gly
               20                  25                  30
   Gly Ser Gly Gly Ser Asp Tyr Lys Asp Asp Asp Asp Lys Gly Gly Gly
           35                  40                  45
   Ser Gly Met Ala Ser Asn Phe Thr Gln Phe Val Leu Val Asp Asn Gly
       50                  55                  60
   Gly Thr Gly Asp Val Thr Val Ala Pro Ser Asn Phe Ala Asn Gly Ile
   65                  70                  75                  80
   Ala Glu Trp Ile Ser Ser Asn Ser Arg Ser Gln Ala Tyr Lys Val Thr
                   85                  90                  95
   Cys Ser Val Arg Gln Ser Ser Ala Gln Lys Arg Lys Tyr Thr Ile Lys
               100                 105                 110
   Val Glu Val Pro Lys Gly Ala Trp Arg Ser Tyr Leu Asn Met Glu Leu
           115                 120                 125
   Thr Ile Pro Ile Phe Ala Thr Asn Ser Asp Cys Glu Leu Ile Val Lys
       130                 135                 140
   Ala Met Gln Gly Leu Leu Lys Asp Gly Asn Pro Ile Pro Ser Ala Ile
   145                 150                 155                 160
   Ala Ala Asn Ser Gly Ile Tyr Gly Ser Gly Gly Ser Gly Gly Ser Gly
                   165                 170                 175
   Gly Ser Gly Met Ala Ser Asn Phe Thr Gln Phe Val Leu Val Asp Asn
               180                 185                 190
   Gly Gly Thr Gly Asp Val Thr Val Ala Pro Ser Asn Phe Ala Asn Gly
           195                 200                 205
   Ile Ala Glu Trp Ile Ser Ser Asn Ser Arg Ser Gln Ala Tyr Lys Val
   210 215 220
   Thr Cys Ser Val Arg Gln Ser Ser Ala Gln Lys Arg Lys Tyr Thr Ile
   225 230 235 240
   Lys Val Glu Val Pro Lys Gly Ala Trp Arg Ser Tyr Leu Asn Met Glu
   245 250 255
   Leu Thr Ile Pro Ile Phe Ala Thr Asn Ser Asp Cys Glu Leu Ile Val
   260 265 270
   Lys Ala Met Gln Gly Leu Leu Lys Asp Gly Asn Pro Ile Pro Ser Ala
   275 280 285
   Ile Ala Ala Asn Ser Gly Ile Tyr
   290 295
<210> 115
   <211> 680
   <212> RNA
   <213> Artificial Sequence
<220>
   <223> scaffold protein
<400> 115
   augaagccca ccgagaacaa cgaagacuuc aacaucgugg ccguggccag caacuucgcg 60
   accacggauc ucgaugcuga ccgcgggaag uugcccggca agaagcugcc gcuggaggug 120
   cucaaagagu uggaagccaa ugcccggaaa gcuggcugca ccaggggcug ucugaucugc 180
   cugucccaca ucaagugcac gcccaagaug aagaaguuca ucccaggacg cugccacacc 240
   uacgaaggcg acaaagaguc cgcacagggc ggcauaggcg aggcgaucgu cgacauuccu 300
   gagauuccug gguucaagga cuuggagccc uuggagcagu ucaucgcaca ggucgaucug 360
   uguguggacu gcacaacugg cugccucaaa gggcuugcca acgugcagug uucugaccug 420
   cucaagaagu ggcugccgca acgcugugcg accuuugcca gcaagaucca gggccaggug 480
   gacaagauca agggggccgg uggugacuaa ggauccaucg auaagcuuca ucgaaacaug 540
   aggaucaccc auaucugcag ucgacaucga aacaugagga ucacccaugu cugcagucga 600
   caucgaaaca ugaggaucac ccaugucugc agucgacauc gaaacaugag gaucacccau 660
   gucugcaguc gacaucgaaa 680
<210> 116
   <211> 9
   <212> PRT
   <213> Artificial Sequence
<220>
   <223> scaffold peptide
<220>
   <221> misc_feature
   <222> (3)..(3)
   <223> Xaa can be any naturally occurring amino acid
<400> 116
   Met Gly Xaa Lys Leu Ser Lys Lys Lys
   1 5
<210> 117
   <211> 9
   <212> PRT
   <213> Artificial Sequence
<220>
   <223> scaffold peptide
<220>
   <221> misc_feature
   <222> (3)..(3)
   <223> Xaa can be any naturally occurring amino acid
<400> 117
   Met Gly Xaa Lys Leu Ser Lys Lys Lys
   1 5
<210> 118
   <211> 8
   <212> PRT
   <213> Artificial Sequence
<220>
   <223> scaffold protein
<220>
   <221> MISC_FEATURE
   <222> (3)..(3)
   <223> Xaa = G, A, S
<220>
   <221> MISC_FEATURE
   <222> (4)..(3)
   <223> Xaa = K, Q
<220>
   <221> misc_feature
   <222> (4)..(4)
   <223> Xaa can be any naturally occurring amino acid
<220>
   <221> MISC_FEATURE
   <222> (5)..(5)
   <223> Xaa = L, F, S, Q
<220>
   <221> MISC_FEATURE
   <222> (6)..(6)
   <223> Xaa = S, A
<400> 118
   Met Gly Xaa Xaa Xaa Xaa Lys Lys
   1 5
<210> 119
   <211> 20
   <212> DNA
   <213> Artificial Sequence
<220>
   <223> scaffold protein
<400> 119
   tggaggtgct caaagagttg 20
<210> 120
   <211> 17
   <212> DNA
   <213> Artificial Sequence
<220>
   <223> scaffold protein
<400> 120
   ttgggcgtgc acttgat 17
<210> 121
   <211> 13
   <212> DNA
   <213> Artificial Sequence
<220>
   <223> scaffold protein
<400> 121
   gggcattggc ttc 13
<210> 122
   <211> 60
   <212> PRT
   <213> Artificial Sequence
<220>
   <223> scaffold protein
<400> 122
   Met Gly Gly Lys Leu Ser Lys Lys Lys Lys Gly Tyr Asn Val Asn Asp
   1 5 10 15
   Glu Lys Ala Lys Glu Lys Asp Lys Lys Ala Glu Gly Ala Ala Ser Ala
   20 25 30
   Gly Gly Gly Gly Ser Asp Tyr Lys Asp Asp Asp Asp Lys Gly Gly Gly
   35 40 45
   Gly Ser Val Ser Lys Gly Glu Glu Leu Phe Thr Gly
   50 55 60
<210> 123
   <211> 60
   <212> PRT
   <213> Artificial Sequence
<220>
   <223> scaffold protein
<400> 123
   Met Ala Gly Lys Leu Ser Lys Lys Lys Lys Gly Tyr Asn Val Asn Asp
   1 5 10 15
   Glu Lys Ala Lys Glu Lys Asp Lys Lys Ala Glu Gly Ala Ala Ser Ala
   20 25 30
   Gly Gly Gly Gly Ser Asp Tyr Lys Asp Asp Asp Asp Lys Gly Gly Gly
   35 40 45
   Gly Ser Val Ser Lys Gly Glu Glu Leu Phe Thr Gly
   50 55 60
<210> 124
   <211> 60
   <212> PRT
   <213> Artificial Sequence
<220>
   <223> scaffold protein
<400> 124
   Met Gly Ala Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Ser Ala
               20                  25                  30
   Gly Gly Gly Gly Ser Asp Tyr Lys Asp Asp Asp Asp Lys Gly Gly Gly
           35                  40                  45
   Gly Ser Val Ser Lys Gly Glu Glu Leu Phe Thr Gly
       50                  55                  60
<210> 125
   <211> 60
   <212> PRT
   <213> Artificial Sequence
<220>
   <223> scaffold protein
<400> 125
   Met Ala Ala Lys Leu Ser Lys Lys Lys Lys Gly Tyr Asn Val Asn Asp
   1               5                   10                  15
   Glu Lys Ala Lys Glu Lys Asp Lys Lys Ala Glu Gly Ala Ala Ser Ala
               20                  25                  30
   Gly Gly Gly Gly Ser Asp Tyr Lys Asp Asp Asp Asp Lys Gly Gly Gly
           35                  40                  45
   Gly Ser Val Ser Lys Gly Glu Glu Leu Phe Thr Gly
       50                  55                  60
<210> 126
   <211> 57
   <212> PRT
   <213> Artificial Sequence
<220>
   <223> scaffold protein
<400> 126
   Met Gly Gly Lys Leu Ser Lys Lys Lys Lys Gly Tyr Asn Val Asn Asp
   1 5 10 15
   Glu Lys Ala Lys Glu Lys Asp Lys Lys Ala Glu Ser Ala Gly Gly Gly
   20 25 30
   Gly Ser Asp Tyr Lys Asp Asp Asp Asp Lys Gly Gly Gly Gly Ser Val
   35 40 45
   Ser Lys Gly Glu Glu Leu Phe Thr Gly
   50 55
<210> 127
   <211> 54
   <212> PRT
   <213> Artificial Sequence
<220>
   <223> scaffold protein
<400> 127
   Met Gly Gly Lys Leu Ser Lys Lys Lys Lys Gly Tyr Asn Val Asn Asp
   1 5 10 15
   Glu Lys Ala Lys Glu Lys Asp Lys Ser Ala Gly Gly Gly Gly Ser Asp
   20 25 30
   Tyr Lys Asp Asp Asp Asp Lys Gly Gly Gly Gly Ser Val Ser Lys Gly
   35 40 45
   Glu Glu Leu Phe Thr Gly
   50
<210> 128
   <211> 51
   <212> PRT
   <213> Artificial Sequence
<220>
   <223> scaffold protein
<400> 128
   Met Gly Gly Lys Leu Ser Lys Lys Lys Lys Gly Tyr Asn Val Asn Asp
   1 5 10 15
   Glu Lys Ala Lys Glu Ser Ala Gly Gly Gly Gly Ser Asp Tyr Lys Asp
   20 25 30
   Asp Asp Asp Lys Gly Gly Gly Gly Ser Val Ser Lys Gly Glu Glu Leu
   35 40 45
   Phe Thr Gly
   50
<210> 129
   <211> 48
   <212> PRT
   <213> Artificial Sequence
<220>
   <223> scaffold protein
<400> 129
   Met Gly Gly Lys Leu Ser Lys Lys Lys Lys Gly Tyr Asn Val Asn Asp
   1 5 10 15
   Glu Lys Ser Ala Gly Gly Gly Gly Ser Asp Tyr Lys Asp Asp Asp Asp
   20 25 30
   Lys Gly Gly Gly Gly Ser Val Ser Lys Gly Glu Glu Leu Phe Thr Gly
   35 40 45
<210> 130
   <211> 45
   <212> PRT
   <213> Artificial Sequence
<220>
   <223> scaffold protein
<400> 130
   Met Gly Gly Lys Leu Ser Lys Lys Lys Lys Gly Tyr Asn Val Asn Ser
   1 5 10 15
   Ala Gly Gly Gly Gly Ser Asp Tyr Lys Asp Asp Asp Asp Lys Gly Gly
   20 25 30
   Gly Gly Ser Val Ser Lys Gly Glu Glu Leu Phe Thr Gly
   35 40 45
<210> 131
   <211> 42
   <212> PRT
   <213> Artificial Sequence
<220>
   <223> scaffold protein
<400> 131
   Met Gly Gly Lys Leu Ser Lys Lys Lys Lys Gly Tyr Ser Ala Gly Gly
   1               5                   10                  15
   Gly Gly Ser Asp Tyr Lys Asp Asp Asp Asp Lys Gly Gly Gly Gly Ser
               20                  25                  30
   Val Ser Lys Gly Glu Glu Leu Phe Thr Gly
           35                  40
<210> 132
   <211> 39
   <212> PRT
   <213> Artificial Sequence
<220>
   <223> scaffold protein
<400> 132
   Met Gly Gly Lys Leu Ser Lys Lys Lys Ser Ala Gly Gly Gly Gly Ser
   1               5                   10                  15
   Asp Tyr Lys Asp Asp Asp Asp Lys Gly Gly Gly Gly Ser Val Ser Lys
               20                  25                  30
   Gly Glu Glu Leu Phe Thr Gly
           35
<210> 133
   <211> 36
   <212> PRT
   <213> Artificial Sequence
<220>
   <223> scaffold protein
<400> 133
   Met Gly Gly Lys Leu Ser Ser Ala Gly Gly Gly Gly Ser Asp Tyr Lys
   1 5 10 15
   Asp Asp Asp Asp Lys Gly Gly Gly Gly Ser Val Ser Lys Gly Glu Glu
   20 25 30
   Leu Phe Thr Gly
   35
<210> 134
   <211> 33
   <212> PRT
   <213> Artificial Sequence
<220>
   <223> scaffold protein
<400> 134
   Met Gly Gly Ser Ala Gly Gly Gly Gly Ser Asp Tyr Lys Asp Asp Asp
   1 5 10 15
   Asp Lys Gly Gly Gly Gly Ser Val Ser Lys Gly Glu Glu Leu Phe Thr
   20 25 30
   Gly
<210> 135
   <211> 54
   <212> PRT
   <213> Artificial Sequence
<220>
   <223> scaffold protein
<400> 135
   Met Gly Gly Lys Leu Ser Lys Lys Lys Lys Gly Tyr Asn Val Asn Asp
   1 5 10 15
   Glu Lys Ala Lys Glu Lys Asp Lys Lys Ala Glu Gly Ala Ala Ser Ala
   20 25 30
   Gly Gly Gly Gly Ser Asp Tyr Lys Asp Asp Asp Asp Lys Gly Gly Gly
   35 40 45
   Gly Ser Val Ser Lys Gly
   50
<210> 136
   <211> 36
   <212> PRT
   <213> Artificial Sequence
<220>
   <223> scaffold protein
<400> 136
   Met Gly Gly Lys Leu Ser Lys Lys Lys Lys Gly Tyr Ser Ala Gly Gly
   1 5 10 15
   Gly Gly Ser Asp Tyr Lys Asp Asp Asp Asp Lys Gly Gly Gly Gly Ser
   20 25 30
   Val Ser Lys Gly
   35
<210> 137
   <211> 35
   <212> PRT
   <213> Artificial Sequence
<220>
   <223> scaffold protein
<400> 137
   Met Gly Gly Lys Leu Ser Lys Lys Lys Lys Gly Ser Ala Gly Gly Gly
   1 5 10 15
   Gly Ser Asp Tyr Lys Asp Asp Asp Asp Lys Gly Gly Gly Gly Ser Val
   20 25 30
   Ser Lys Gly
   35
<210> 138
   <211> 34
   <212> PRT
   <213> Artificial Sequence
<220>
   <223> scaffold protein
<400> 138
   Met Gly Gly Lys Leu Ser Lys Lys Lys Lys Ser Ala Gly Gly Gly Gly
   1 5 10 15
   Ser Asp Tyr Lys Asp Asp Asp Asp Lys Gly Gly Gly Gly Ser Val Ser
   20 25 30
   Lys Gly
<210> 139
   <211> 33
   <212> PRT
   <213> Artificial Sequence
<220>
   <223> scaffold protein
<400> 139
   Met Gly Gly Lys Leu Ser Lys Lys Lys Ser Ala Gly Gly Gly Gly Ser
   1               5                   10                  15
   Asp Tyr Lys Asp Asp Asp Asp Lys Gly Gly Gly Gly Ser Val Ser Lys
               20                  25                  30
   Gly
<210> 140
   <211> 32
   <212> PRT
   <213> Artificial Sequence
<220>
   <223> scaffold protein
<400> 140
   Met Gly Gly Lys Leu Ser Lys Lys Ser Ala Gly Gly Gly Gly Ser Asp
   1               5                   10                  15
   Tyr Lys Asp Asp Asp Asp Lys Gly Gly Gly Gly Ser Val Ser Lys Gly
               20                  25                  30
<210> 141
   <211> 31
   <212> PRT
   <213> Artificial Sequence
<220>
   <223> scaffold protein
<400> 141
   Met Gly Gly Lys Leu Ser Lys Ser Ala Gly Gly Gly Gly Ser Asp Tyr
   1               5                   10                  15
   Lys Asp Asp Asp Asp Lys Gly Gly Gly Gly Ser Val Ser Lys Gly
               20                  25                  30
<210> 142
   <211> 30
   <212> PRT
   <213> Artificial Sequence
<220>
   <223> scaffold protein
<400> 142
   Met Gly Gly Lys Leu Ser Ser Ala Gly Gly Gly Gly Ser Asp Tyr Lys
   1               5                   10                  15
   Asp Asp Asp Asp Lys Gly Gly Gly Gly Ser Val Ser Lys Gly
               20                  25                  30
<210> 143
   <211> 30
   <212> PRT
   <213> Artificial Sequence
<220>
   <223> scaffold protein
<400> 143
   Met Gly Gly Lys Leu Asp Lys Lys Lys Lys Gly Tyr Asn Val Asn Asp
   1               5                   10                  15
   Glu Lys Ala Lys Glu Lys Asp Lys Lys Ala Glu Gly Ala Ala
               20                  25                  30
<210> 144
   <211> 30
   <212> PRT
   <213> Artificial Sequence
<220>
   <223> scaffold protein
<400> 144
   Met Gly Gly Lys Leu Ala Lys Lys Lys Lys Gly Tyr Asn Val Asn Asp
   1               5                   10                  15
   Glu Lys Ala Lys Glu Lys Asp Lys Lys Ala Glu Gly Ala Ala
               20                  25                  30
<210> 145
   <211> 30
   <212> PRT
   <213> Artificial Sequence
<220>
   <223> scaffold protein
<400> 145
   Met Gly Gly Lys Gln Ser Lys Lys Lys Lys Gly Tyr Asn Val Asn Asp
   1 5 10 15
   Glu Lys Ala Lys Glu Lys Asp Lys Lys Ala Glu Gly Ala Ala
   20 25 30
<210> 146
   <211> 30
   <212> PRT
   <213> Artificial Sequence
<220>
   <223> scaffold protein
<400> 146
   Met Gly Ala Lys Lys Lys Lys Lys Arg Phe Ser Phe Lys Lys Ser Phe
   1 5 10 15
   Lys Leu Ser Gly Phe Ser Phe Lys Lys Asn Lys Lys Glu Ala
   20 25 30
<210> 147
   <211> 30
   <212> PRT
   <213> Artificial Sequence
<220>
   <223> scaffold protein
<400> 147
   Met Ala Ala Lys Lys Lys Lys Lys Arg Phe Ser Phe Lys Lys Ser Phe
   1 5 10 15
   Lys Leu Ser Gly Phe Ser Phe Lys Lys Asn Lys Lys Glu Ala
   20 25 30
<210> 148
   <211> 30
   <212> PRT
   <213> Artificial Sequence
<220>
   <223> scaffold protein
<400> 148
   Met Gly Ala Lys Lys Ser Lys Lys Arg Phe Ser Phe Lys Lys Ser Phe
   1 5 10 15
   Lys Leu Ser Gly Phe Ser Phe Lys Lys Asn Lys Lys Glu Ala
   20 25 30
<210> 149
   <211> 30
   <212> PRT
   <213> Artificial Sequence
<220>
   <223> scaffold protein
<400> 149
   Met Gly Ala Lys Lys Ala Lys Lys Arg Phe Ser Phe Lys Lys Pro Phe
   1               5                   10                  15
   Lys Leu Ser Gly Phe Ser Phe Lys Lys Asn Lys Lys Glu Ala
               20                  25                  30
<210> 150
   <211> 153
   <212> PRT
   <213> Artificial Sequence
<220>
   <223> scaffold protein
<400> 150
   Met Gly Gly Lys Leu Ser Lys Lys Lys Lys Ser Ala Gly Gly Ser Gly
   1               5                   10                  15
   Gly Ser Thr Ser Gly Ser Gly Asp Tyr Lys Asp Asp Asp Asp Lys Gly
               20                  25                  30
   Ser Gly Phe Glu Met Asp Gln Val Gln Leu Val Glu Ser Gly Gly Ala
           35                  40                  45
   Leu Val Gln Pro Gly Gly Ser Leu Arg Leu Ser Cys Ala Ala Ser Gly
       50                  55                  60
   Phe Pro Val Asn Arg Tyr Ser Met Arg Trp Tyr Arg Gln Ala Pro Gly
   65                  70                  75                  80
   Lys Glu Arg Glu Trp Val Ala Gly Met Ser Ser Ala Gly Asp Arg Ser
                   85                  90                  95
   Ser Tyr Glu Asp Ser Val Lys Gly Arg Phe Thr Ile Ser Arg Asp Asp
   100 105 110
   Ala Arg Asn Thr Val Tyr Leu Gln Met Asn Ser Leu Lys Pro Glu Asp
   115 120 125
   Thr Ala Val Tyr Tyr Cys Asn Val Asn Val Gly Phe Glu Tyr Trp Gly
   130 135 140
   Gln Gly Thr Gln Val Thr Val Ser Ser
   145 150
<210> 151
   <211> 4
   <212> PRT
   <213> Artificial Sequence
<220>
   <223> effector domain
<400> 151
   Lys Lys Lys Lys
   1
<210> 152
   <211> 5
   <212> PRT
   <213> Artificial Sequence
<220>
   <223> effector domain
<400> 152
   Lys Lys Lys Lys Lys
   1 5
<210> 153
   <211> 4
   <212> PRT
   <213> Artificial Sequence
<220>
   <223> effector domain
<400> 153
   Arg Arg Arg Arg
   1
<210> 154
   <211> 5
   <212> PRT
   <213> Artificial Sequence
<220>
   <223> effector domain
<400> 154
   Arg Arg Arg Arg Arg
   1 5
<210> 155
   <211> 4
   <212> PRT
   <213> Artificial Sequence
<220>
   <223> effector domain
<220>
   <221> MISC_FEATURE
   <222> (1)..(4)
   <223> Xaa = K, R
<400> 155
   Xaa Xaa Xaa Xaa
   1
<210> 156
   <211> 5
   <212> PRT
   <213> Artificial Sequence
<220>
   <223> effector domain
<220>
   <221> MISC_FEATURE
   <222> (1)..(5)
   <223> Xaa= K, R
<400> 156
   Xaa Xaa Xaa Xaa Xaa
   1 5
<210> 157
   <211> 7
   <212> PRT
   <213> Artificial Sequence
<220>
   <223> scaffold protein
<400> 157
   Gly Gly Lys Leu Ser Lys Lys
   1 5
<210> 158
   <211> 7
   <212> PRT
   <213> Artificial Sequence
<220>
   <223> scaffold protein
<400> 158
   Gly Ala Lys Leu Ser Lys Lys
   1               5
<210> 159
   <211> 7
   <212> PRT
   <213> Artificial Sequence
<220>
   <223> scaffold protein
<400> 159
   Gly Gly Lys Gln Ser Lys Lys
   1               5
<210> 160
   <211> 7
   <212> PRT
   <213> Artificial Sequence
<220>
   <223> scaffold protein
<400> 160
   Gly Gly Lys Leu Ala Lys Lys
   1               5
<210> 161
   <211> 8
   <212> PRT
   <213> Artificial Sequence
<220>
   <223> scaffold protein
<400> 161
   Gly Gly Lys Leu Ser Lys Lys Lys
   1               5
<210> 162
   <211> 8
   <212> PRT
   <213> Artificial Sequence
<220>
   <223> scaffold protein
<400> 162
   Gly Gly Lys Leu Ser Lys Lys Ser
   1 5
<210> 163
   <211> 8
   <212> PRT
   <213> Artificial Sequence
<220>
   <223> scaffold protein
<400> 163
   Gly Ala Lys Leu Ser Lys Lys Lys
   1 5
<210> 164
   <211> 8
   <212> PRT
   <213> Artificial Sequence
<220>
   <223> scaffold protein
<400> 164
   Gly Ala Lys Leu Ser Lys Lys Ser
   1 5
<210> 165
   <211> 8
   <212> PRT
   <213> Artificial Sequence
<220>
   <223> scaffold protein
<400> 165
   Gly Gly Lys Gln Ser Lys Lys Lys
   1 5
<210> 166
   <211> 8
   <212> PRT
   <213> Artificial Sequence
<220>
   <223> scaffold protein
<400> 166
   Gly Gly Lys Gln Ser Lys Lys Ser
   1 5
<210> 167
   <211> 8
   <212> PRT
   <213> Artificial Sequence
<220>
   <223> scaffold protein
<400> 167
   Gly Gly Lys Leu Ala Lys Lys Lys
   1               5
<210> 168
   <211> 8
   <212> PRT
   <213> Artificial Sequence
<220>
   <223> scaffold protein
<400> 168
   Gly Gly Lys Leu Ala Lys Lys Ser
   1               5
<210> 169
   <211> 14
   <212> PRT
   <213> Artificial Sequence
<220>
   <223> scaffold protein
<400> 169
   Gly Gly Lys Leu Ser Lys Lys Lys Lys Gly Tyr Asn Val Asn
   1               5                   10
<210> 170
   <211> 14
   <212> PRT
   <213> Artificial Sequence
<220>
   <223> scaffold protein
<400> 170
   Gly Ala Lys Leu Ser Lys Lys Lys Lys Gly Tyr Asn Val Asn
   1               5                   10
<210> 171
   <211> 14
   <212> PRT
   <213> Artificial Sequence
<220>
   <223> scaffold protein
<400> 171
   Gly Gly Lys Gln Ser Lys Lys Lys Lys Gly Tyr Asn Val Asn
   1               5                   10
<210> 172
   <211> 14
   <212> PRT
   <213> Artificial Sequence
<220>
   <223> scaffold protein
<400> 172
   Gly Gly Lys Leu Ala Lys Lys Lys Lys Gly Tyr Asn Val Asn
   1               5                   10
<210> 173
   <211> 14
   <212> PRT
   <213> Artificial Sequence
<220>
   <223> scaffold protein
<400> 173
   Gly Gly Lys Leu Ser Lys Lys Lys Lys Gly Tyr Ser Gly Gly
   1               5                   10
<210> 174
   <211> 14
   <212> PRT
   <213> Artificial Sequence
<220>
   <223> scaffold protein
<400> 174
   Gly Gly Lys Leu Ser Lys Lys Lys Lys Gly Ser Gly Gly Ser
   1               5                   10
<210> 175
   <211> 14
   <212> PRT
   <213> Artificial Sequence
<220>
   <223> scaffold protein
<400> 175
   Gly Gly Lys Leu Ser Lys Lys Lys Lys Ser Gly Gly Ser Gly
   1               5                   10
<210> 176
   <211> 14
   <212> PRT
   <213> Artificial Sequence
<220>
   <223> scaffold protein
<400> 176
   Gly Gly Lys Leu Ser Lys Lys Lys Ser Gly Gly Ser Gly Gly
   1               5                   10
<210> 177
   <211> 14
   <212> PRT
   <213> Artificial Sequence
<220>
   <223> scaffold protein
<400> 177
   Gly Gly Lys Leu Ser Lys Lys Ser Gly Gly Ser Gly Gly Ser
   1               5                   10
<210> 178
   <211> 14
   <212> PRT
   <213> Artificial Sequence
<220>
   <223> scaffold protein
<400> 178
   Gly Gly Lys Leu Ser Lys Ser Gly Gly Ser Gly Gly Ser Val
   1               5                   10
<210> 179
   <211> 14
   <212> PRT
   <213> Artificial Sequence
<220>
   <223> scaffold protein
<400> 179
   Gly Ala Lys Lys Ser Lys Lys Arg Phe Ser Phe Lys Lys Ser
   1               5                   10
<210> 180
   <211> 8
   <212> PRT
   <213> Artificial Sequence
<220>
   <223> scaffold protein
<220>
   <221> MISC_FEATURE
   <222> (4)..(4)
   <223> Xaa= K, Q
<220>
   <221> MISC_FEATURE
   <222> (5)..(5)
   <223> Xaa= L, F, S, Q
<220>
   <221> MISC_FEATURE
   <222> (6)..(6)
   <223> Xaa= S, A
<400> 180
   Met Gly Gly Xaa Xaa Xaa Lys Lys
   1               5
<210> 181
   <211> 8
   <212> PRT
   <213> Artificial Sequence
<220>
   <223> scaffold protein
<220>
   <221> MISC_FEATURE
   <222> (4)..(4)
   <223> Xaa= K, Q
<220>
   <221> MISC_FEATURE
   <222> (5)..(5)
   <223> Xaa= L, F, S, Q
<220>
   <221> MISC_FEATURE
   <222> (6)..(6)
   <223> Xaa= S, A
<400> 181
   Met Gly Ala Xaa Xaa Xaa Lys Lys
   1               5
<210> 182
   <211> 8
   <212> PRT
   <213> Artificial Sequence
<220>
   <223> scaffold protein
<220>
   <221> MISC_FEATURE
   <222> (4)..(4)
   <223> Xaa = K, Q
<220>
   <221> MISC_FEATURE
   <222> (5)..(5)
   <223> Xaa = L, F, S, Q
<220>
   <221> MISC_FEATURE
   <222> (6)..(6)
   <223> Xaa = S, A
<400> 182
   Met Gly Ser Xaa Xaa Xaa Lys Lys
   1               5
<210> 183
   <211> 8
   <212> PRT
   <213> Artificial Sequence
<220>
   <223> scaffold protein
<220>
   <221> MISC_FEATURE
   <222> (3)..(3)
   <223> Xaa = G, A, S
<220>
   <221> MISC_FEATURE
   <222> (5)..(5)
   <223> Xaa = L, F, S, Q
<220>
   <221> MISC_FEATURE
   <222> (6)..(6)
   <223> Xaa = S, A
<400> 183
   Met Gly Xaa Lys Xaa Xaa Lys Lys
   1               5
<210> 184
   <211> 8
   <212> PRT
   <213> Artificial Sequence
<220>
   <223> scaffold protein
<220>
   <221> MISC_FEATURE
   <222> (3)..(3)
   <223> Xaa= G, A, S
<220>
   <221> MISC_FEATURE
   <222> (5)..(5)
   <223> Xaa= L, F, S, Q
<220>
   <221> MISC_FEATURE
   <222> (6)..(6)
   <223> Xaa= S, A
<400> 184
   Met Gly Xaa Gln Xaa Xaa Lys Lys
   1               5
<210> 185
   <211> 8
   <212> PRT
   <213> Artificial Sequence
<220>
   <223> scaffold protein
<220>
   <221> MISC_FEATURE
   <222> (3)..(3)
   <223> Xaa = G, A, S
<220>
   <221> MISC_FEATURE
   <222> (4)..(4)
   <223> Xaa = K, Q
<220>
   <221> MISC_FEATURE
   <222> (6)..(6)
   <223> Xaa = S, A
<400> 185
   Met Gly Xaa Xaa Leu Xaa Lys Lys
   1               5
<210> 186
   <211> 8
   <212> PRT
   <213> Artificial Sequence
<220>
   <223> scaffold protein
<220>
   <221> MISC_FEATURE
   <222> (3)..(3)
   <223> Xaa = G, A, S
<220>
   <221> MISC_FEATURE
   <222> (4)..(4)
   <223> Xaa = K, Q
<220>
   <221> MISC_FEATURE
   <222> (6)..(6)
   <223> Xaa = S, A
<400> 186
   Met Gly Xaa Xaa Phe Xaa Lys Lys
   1               5
<210> 187
   <211> 8
   <212> PRT
   <213> Artificial Sequence
<220>
   <223> scaffold protein
<220>
   <221> MISC_FEATURE
   <222> (3)..(3)
   <223> Xaa = G, A, S
<220>
   <221> MISC_FEATURE
   <222> (4)..(4)
   <223> Xaa = K, Q
<220>
   <221> MISC_FEATURE
   <222> (6)..(6)
   <223> Xaa = S, A
<400> 187
   Met Gly Xaa Xaa Ser Xaa Lys Lys
   1               5
<210> 188
   <211> 8
   <212> PRT
   <213> Artificial Sequence
<220>
   <223> scaffold protein
<220>
   <221> MISC_FEATURE
   <222> (3)..(3)
   <223> Xaa = G, A, S
<220>
   <221> MISC_FEATURE
   <222> (4)..(4)
   <223> Xaa = K, Q
<220>
   <221> MISC_FEATURE
   <222> (6)..(6)
   <223> Xaa = S, A
<400> 188
   Met Gly Xaa Xaa Gln Xaa Lys Lys
   1               5
<210> 189
   <211> 8
   <212> PRT
   <213> Artificial Sequence
<220>
   <223> scaffold protein
<220>
   <221> MISC_FEATURE
   <222> (3)..(3)
   <223> Xaa= G, A, S
<220>
   <221> MISC_FEATURE
   <222> (4)..(4)
   <223> Xaa= K, Q
<220>
   <221> MISC_FEATURE
   <222> (5)..(5)
   <223> Xaa= L, F, S, Q
<400> 189
   Met Gly Xaa Xaa Xaa Ser Lys Lys
   1               5
<210> 190
   <211> 8
   <212> PRT
   <213> Artificial Sequence
<220>
   <223> scaffold protein
<220>
   <221> MISC_FEATURE
   <222> (3)..(3)
   <223> Xaa = G, A, S
<220>
   <221> MISC_FEATURE
   <222> (4)..(4)
   <223> Xaa = K, Q
<220>
   <221> MISC_FEATURE
   <222> (5)..(5)
   <223> Xaa = L, F, S, Q
<400> 190
   Met Gly Xaa Xaa Xaa Ala Lys Lys
   1 5
<210> 191
   <211> 7
   <212> PRT
   <213> Artificial Sequence
<220>
   <223> scaffold protein
<220>
   <221> MISC_FEATURE
   <222> (3)..(3)
   <223> Xaa = K, Q
<220>
   <221> MISC_FEATURE
   <222> (4)..(4)
   <223> Xaa = L, F, S, Q
<220>
   <221> MISC_FEATURE
   <222> (5)..(5)
   <223> Xaa = S, A
<400> 191
   Gly Gly Xaa Xaa Xaa Lys Lys
   1 5
<210> 192
   <211> 7
   <212> PRT
   <213> Artificial Sequence
<220>
   <223> scaffold protein
<220>
   <221> MISC_FEATURE
   <222> (3)..(3)
   <223> Xaa = K, Q
<220>
   <221> MISC_FEATURE
   <222> (4)..(4)
   <223> Xaa = L, F, S, Q
<220>
   <221> MISC_FEATURE
   <222> (5)..(5)
   <223> Xaa = S, A
<400> 192
   Gly Ala Xaa Xaa Xaa Lys Lys
   1 5
<210> 193
   <211> 7
   <212> PRT
   <213> Artificial Sequence
<220>
   <223> scaffold protein
<220>
   <221> MISC_FEATURE
   <222> (3)..(3)
   <223> Xaa = K, Q
<220>
   <221> MISC_FEATURE
   <222> (4)..(4)
   <223> Xaa = L, F, S, Q
<220>
   <221> MISC_FEATURE
   <222> (5)..(5)
   <223> Xaa = S, A
<400> 193
   Gly Ser Xaa Xaa Xaa Lys Lys
   1 5
<210> 194
   <211> 7
   <212> PRT
   <213> Artificial Sequence
<220>
   <223> scaffold protein
<220>
   <221> MISC_FEATURE
   <222> (2).. (2)
   <223> Xaa = G, A, S
<220>
   <221> MISC_FEATURE
   <222> (4)..(4)
   <223> Xaa = L, F, S, Q
<220>
   <221> MISC_FEATURE
   <222> (5)..(5)
   <223> Xaa = S, A
<400> 194
   Gly Xaa Lys Xaa Xaa Lys Lys
   1 5
<210> 195
   <211> 7
   <212> PRT
   <213> Artificial Sequence
<220>
   <223> scaffold protein
<220>
   <221> MISC_FEATURE
   <222> (2).. (2)
   <223> Xaa = G, A, S
<220>
   <221> MISC_FEATURE
   <222> (4)..(4)
   <223> Xaa = L, F, S, Q
<220>
   <221> MISC_FEATURE
   <222> (5)..(5)
   <223> Xaa = S, A
<400> 195
   Gly Xaa Gln Xaa Xaa Lys Lys
   1 5
<210> 196
   <211> 7
   <212> PRT
   <213> Artificial Sequence
<220>
   <223> scaffold protein
<220>
   <221> MISC_FEATURE
   <222> (2).. (2)
   <223> Xaa= G, A, S
<220>
   <221> MISC_FEATURE
   <222> (3)..(3)
   <223> Xaa= K, Q
<220>
   <221> MISC_FEATURE
   <222> (5)..(5)
   <223> Xaa= S, A
<400> 196
   Gly Xaa Xaa Leu Xaa Lys Lys
   1 5
<210> 197
   <211> 7
   <212> PRT
   <213> Artificial Sequence
<220>
   <223> scaffold protein
<220>
   <221> MISC_FEATURE
   <222> (2)..(2)
   <223> Xaa = G, A, S
<220>
   <221> MISC_FEATURE
   <222> (3)..(3)
   <223> Xaa = K, Q
<220>
   <221> MISC_FEATURE
   <222> (5)..(5)
   <223> Xaa = S, A
<400> 197
   Gly Xaa Xaa Phe Xaa Lys Lys
   1               5
<210> 198
   <211> 7
   <212> PRT
   <213> Artificial Sequence
<220>
   <223> scaffold protein
<220>
   <221> MISC_FEATURE
   <222> (2).. (2)
   <223> Xaa = G, A, S
<220>
   <221> MISC_FEATURE
   <222> (3)..(3)
   <223> Xaa = K, Q
<220>
   <221> MISC_FEATURE
   <222> (5)..(5)
   <223> Xaa = S, A
<400> 198
   Gly Xaa Xaa Ser Xaa Lys Lys
   1               5
<210> 199
   <211> 7
   <212> PRT
   <213> Artificial Sequence
<220>
   <223> scaffold protein
<220>
   <221> MISC_FEATURE
   <222> (2)..(2)
   <223> Xaa = G, A, S
<220>
   <221> MISC_FEATURE
   <222> (3)..(3)
   <223> Xaa = K, Q
<220>
   <221> MISC_FEATURE
   <222> (5)..(5)
   <223> Xaa = S, A
<400> 199
   Gly Xaa Xaa Gln Xaa Lys Lys
   1               5
<210> 200
   <211> 7
   <212> PRT
   <213> Artificial Sequence
<220>
   <223> scaffold protein
<220>
   <221> MISC_FEATURE
   <222> (2)..(2)
   <223> Xaa = G, A, S
<220>
   <221> MISC_FEATURE
   <222> (3)..(3)
   <223> Xaa = K, Q
<220>
   <221> MISC_FEATURE
   <222> (4)..(4)
   <223> Xaa = L, F, S, Q
<400> 200
   Gly Xaa Xaa Xaa Ser Lys Lys
   1               5
<210> 201
   <211> 7
   <212> PRT
   <213> Artificial Sequence
<220>
   <223> scaffold protein
<220>
   <221> MISC_FEATURE
   <222> (2)..(2)
   <223> Xaa = G, A, S
<220>
   <221> MISC_FEATURE
   <222> (3)..(3)
   <223> Xaa = K, Q
<220>
   <221> MISC_FEATURE
   <222> (4)..(4)
   <223> Xaa = L, F, S, Q
<400> 201
   Gly Xaa Xaa Xaa Ala Lys Lys
   1               5
<210> 202
   <211> 7
   <212> PRT
   <213> Artificial Sequence
<220>
   <223> scaffold protein
<220>
   <221> MISC_FEATURE
   <222> (2).. (2)
   <223> Xaa = G, A, S
<220>
   <221> MISC_FEATURE
   <222> (3)..(3)
   <223> Xaa = K, Q
<220>
   <221> MISC_FEATURE
   <222> (4)..(4)
   <223> Xaa = L, F, S, Q
<220>
   <221> MISC_FEATURE
   <222> (5)..(5)
   <223> Xaa = S, A
<400> 202
   Gly Xaa Xaa Xaa Xaa Lys Lys
   1               5
<210> 203
   <211> 6
   <212> PRT
   <213> Artificial Sequence
<220>
   <223> N-terminus domain
<400> 203
   Gly Gly Lys Leu Ser Lys
   1 5
<210> 204
   <211> 6
   <212> PRT
   <213> Artificial Sequence
<220>
   <223> N-terminus domain
<400> 204
   Gly Ala Lys Leu Ser Lys
   1 5
<210> 205
   <211> 6
   <212> PRT
   <213> Artificial Sequence
<220>
   <223> N-terminus domain
<400> 205
   Gly Gly Lys Gln Ser Lys
   1 5
<210> 206
   <211> 6
   <212> PRT
   <213> Artificial Sequence
<220>
   <223> N-terminus domain
<400> 206
   Gly Gly Lys Leu Ala Lys
   1 5
<210> 207
   <211> 4
   <212> PRT
   <213> Artificial Sequence
<220>
   <223> N-terminus domain
<400> 207
   Lys Lys Lys Gly
   1
<210> 208
   <211> 5
   <212> PRT
   <213> Artificial Sequence
<220>
   <223> N-terminus domain
<400> 208
   Lys Lys Lys Gly Tyr
   1 5
<210> 209
   <211> 6
   <212> PRT
   <213> Artificial Sequence
<220>
   <223> N-terminus domain
<400> 209
   Lys Lys Lys Gly Tyr Asn
   1 5
<210> 210
   <211> 7
   <212> PRT
   <213> Artificial Sequence
<220>
   <223> N-terminus domain
<400> 210
   Lys Lys Lys Gly Tyr Asn Val
   1 5
<210> 211
   <211> 8
   <212> PRT
   <213> Artificial Sequence
<220>
   <223> N-terminus domain
<400> 211
   Lys Lys Lys Gly Tyr Asn Val Asn
   1 5
<210> 212
   <211> 6
   <212> PRT
   <213> Artificial Sequence
<220>
   <223> N-terminus domain
<400> 212
   Lys Lys Lys Gly Tyr Ser
   1               5
<210> 213
   <211> 6
   <212> PRT
   <213> Artificial Sequence
<220>
   <223> N-terminus domain
<400> 213
   Lys Lys Lys Gly Tyr Gly
   1               5
<210> 214
   <211> 7
   <212> PRT
   <213> Artificial Sequence
<220>
   <223> N-terminus domain
<400> 214
   Lys Lys Lys Gly Tyr Gly Gly
   1               5
<210> 215
   <211> 5
   <212> PRT
   <213> Artificial Sequence
<220>
   <223> N-terminus domain
<400> 215
   Lys Lys Lys Gly Ser
   1               5
<210> 216
   <211> 6
   <212> PRT
   <213> Artificial Sequence
<220>
   <223> N-terminus domain
<400> 216
   Lys Lys Lys Gly Ser Gly
   1               5
<210> 217
   <211> 6
   <212> PRT
   <213> Artificial Sequence
<220>
   <223> N-terminus domain
<400> 217
   Lys Lys Lys Gly Ser Gly
   1               5
<210> 218
   <211> 7
   <212> PRT
   <213> Artificial Sequence
<220>
   <223> N-terminus domain
<400> 218
   Lys Lys Lys Gly Ser Gly Ser
   1               5
<210> 219
   <211> 4
   <212> PRT
   <213> Artificial Sequence
<220>
   <223> N-terminus domain
<400> 219
   Lys Lys Lys Ser
   1
<210> 220
   <211> 5
   <212> PRT
   <213> Artificial Sequence
<220>
   <223> N-terminus domain
<400> 220
   Lys Lys Lys Ser Gly
   1               5
<210> 221
   <211> 6
   <212> PRT
   <213> Artificial Sequence
<220>
   <223> N-terminus domain
<400> 221
   Lys Lys Lys Ser Gly Gly
   1               5
<210> 222
   <211> 7
   <212> PRT
   <213> Artificial Sequence
<220>
   <223> N-terminus domain
<400> 222
   Lys Lys Lys Ser Gly Gly Ser
   1               5
<210> 223
   <211> 8
   <212> PRT
   <213> Artificial Sequence
<220>
   <223> N-terminus domain
<400> 223
   Lys Lys Lys Ser Gly Gly Ser Gly
   1               5
<210> 224
   <211> 8
   <212> PRT
   <213> Artificial Sequence
<220>
   <223> N-terminus domain
<400> 224
   Lys Lys Ser Gly Gly Ser Gly Gly
   1               5
<210> 225
   <211> 10
   <212> PRT
   <213> Artificial Sequence
<220>
   <223> N-terminus domain
<400> 225
   Lys Lys Lys Ser Gly Gly Ser Gly Gly Ser
   1               5                   10
<210> 226
   <211> 8
   <212> PRT
   <213> Artificial Sequence
<220>
   <223> N-terminus domain
<400> 226
   Lys Arg Phe Ser Phe Lys Lys Ser
   1               5
<210> 227
   <211> 29
   <212> PRT
   <213> Artificial Sequence
<220>
   <223> Scaffold Protein
<400> 227
   Gly Gly Lys Leu Ser Lys Lys Lys Lys Gly Tyr Asn Val Asn Asp Glu
   1               5                   10                  15
   Lys Ala Lys Glu Lys Asp Lys Lys Ala Glu Gly Ala Ala
               20                  25
<210> 228
   <211> 28
   <212> PRT
   <213> Artificial Sequence
<220>
   <223> Scaffold Protein
<400> 228
   Gly Gly Lys Leu Ser Lys Lys Lys Lys Gly Tyr Asn Val Asn Asp Glu
   1               5                   10                  15
   Lys Ala Lys Glu Lys Asp Lys Lys Ala Glu Gly Ala
               20                  25
<210> 229
   <211> 27
   <212> PRT
   <213> Artificial Sequence
<220>
   <223> Scaffold Protein
<400> 229
   Gly Gly Lys Leu Ser Lys Lys Lys Lys Gly Tyr Asn Val Asn Asp Glu
   1               5                   10                  15
   Lys Ala Lys Glu Lys Asp Lys Lys Ala Glu Gly
               20                  25
<210> 230
   <211> 26
   <212> PRT
   <213> Artificial Sequence
<220>
   <223> Scaffold Protein
<400> 230
   Gly Gly Lys Leu Ser Lys Lys Lys Lys Gly Tyr Asn Val Asn Asp Glu
   1               5                   10                  15
   Lys Ala Lys Glu Lys Asp Lys Lys Ala Glu
               20                  25
<210> 231
   <211> 25
   <212> PRT
   <213> Artificial Sequence
<220>
   <223> Scaffold Protein
<400> 231
   Gly Gly Lys Leu Ser Lys Lys Lys Lys Gly Tyr Asn Val Asn Asp Glu
   1               5                   10                  15
   Lys Ala Lys Glu Lys Asp Lys Lys Ala
               20                  25
<210> 232
   <211> 24
   <212> PRT
   <213> Artificial Sequence
<220>
   <223> Scaffold Protein
<400> 232
   Gly Gly Lys Leu Ser Lys Lys Lys Lys Gly Tyr Asn Val Asn Asp Glu
   1               5                   10                  15
   Lys Ala Lys Glu Lys Asp Lys Lys
               20
<210> 233
   <211> 23
   <212> PRT
   <213> Artificial Sequence
<220>
   <223> Scaffold Protein
<400> 233
   Gly Gly Lys Leu Ser Lys Lys Lys Lys Gly Tyr Asn Val Asn Asp Glu
   1               5                   10                  15
   Lys Ala Lys Glu Lys Asp Lys
               20
<210> 234
   <211> 22
   <212> PRT
   <213> Artificial Sequence
<220>
   <223> Scaffold Protein
<400> 234
   Gly Gly Lys Leu Ser Lys Lys Lys Lys Gly Tyr Asn Val Asn Asp Glu
   1               5                   10                  15
   Lys Ala Lys Glu Lys Asp
               20
<210> 235
   <211> 21
   <212> PRT
   <213> Artificial Sequence
<220>
   <223> Scaffold Protein
<400> 235
   Gly Gly Lys Leu Ser Lys Lys Lys Lys Gly Tyr Asn Val Asn Asp Glu
   1               5                   10                  15
   Lys Ala Lys Glu Lys
               20
<210> 236
   <211> 20
   <212> PRT
   <213> Artificial Sequence
<220>
   <223> Scaffold Protein
<400> 236
   Gly Gly Lys Leu Ser Lys Lys Lys Lys Gly Tyr Asn Val Asn Asp Glu
   1               5                   10                  15
   Lys Ala Lys Glu
               20
<210> 237
   <211> 19
   <212> PRT
   <213> Artificial Sequence
<220>
   <223> Scaffold Protein
<400> 237
   Gly Gly Lys Leu Ser Lys Lys Lys Lys Gly Tyr Asn Val Asn Asp Glu
   1               5                   10                  15
   Lys Ala Lys
<210> 238
   <211> 18
   <212> PRT
   <213> Artificial Sequence
<220>
   <223> Scaffold Protein
<400> 238
   Gly Gly Lys Leu Ser Lys Lys Lys Lys Gly Tyr Asn Val Asn Asp Glu
   1               5                   10                  15
   Lys Ala
<210> 239
   <211> 17
   <212> PRT
   <213> Artificial Sequence
<220>
   <223> Scaffold Protein
<400> 239
   Gly Gly Lys Leu Ser Lys Lys Lys Lys Gly Tyr Asn Val Asn Asp Glu
   1 5 10 15
   Lys
<210> 240
   <211> 16
   <212> PRT
   <213> Artificial Sequence
<220>
   <223> Scaffold Protein
<400> 240
   Gly Gly Lys Leu Ser Lys Lys Lys Lys Gly Tyr Asn Val Asn Asp Glu
   1 5 10 15
<210> 241
   <211> 15
   <212> PRT
   <213> Artificial Sequence
<220>
   <223> Scaffold Protein
<400> 241
   Gly Gly Lys Leu Ser Lys Lys Lys Lys Gly Tyr Asn Val Asn Asp
   1 5 10 15
<210> 242
   <211> 13
   <212> PRT
   <213> Artificial Sequence
<220>
   <223> Scaffold Protein
<400> 242
   Gly Gly Lys Leu Ser Lys Lys Lys Lys Gly Tyr Asn Val
   1 5 10
<210> 243
   <211> 12
   <212> PRT
   <213> Artificial Sequence
<220>
   <223> Scaffold Protein
<400> 243
   Gly Gly Lys Leu Ser Lys Lys Lys Lys Gly Tyr Asn
   1 5 10
<210> 244
   <211> 11
   <212> PRT
   <213> Artificial Sequence
<220>
   <223> Scaffold Protein
<400> 244
   Gly Gly Lys Leu Ser Lys Lys Lys Lys Gly Tyr
   1               5                   10
<210> 245
   <211> 10
   <212> PRT
   <213> Artificial Sequence
<220>
   <223> Scaffold Protein
<400> 245
   Gly Gly Lys Leu Ser Lys Lys Lys Lys Gly
   1               5                   10
<210> 246
   <211> 9
   <212> PRT
   <213> Artificial Sequence
<220>
   <223> Scaffold Protein
<400> 246
   Gly Gly Lys Leu Ser Lys Lys Lys Lys
   1               5
<210> 247
   <211> 29
   <212> PRT
   <213> Artificial Sequence
<220>
   <223> Scaffold Protein
<400> 247
   Gly Ala Lys Lys Ser Lys Lys Arg Phe Ser Phe Lys Lys Ser Phe Lys
   1               5                   10                  15
   Leu Ser Gly Phe Ser Phe Lys Lys Asn Lys Lys Glu Ala
               20                  25
<210> 248
   <211> 28
   <212> PRT
   <213> Artificial Sequence
<220>
   <223> Scaffold Protein
<400> 248
   Gly Ala Lys Lys Ser Lys Lys Arg Phe Ser Phe Lys Lys Ser Phe Lys
   1 5 10 15
   Leu Ser Gly Phe Ser Phe Lys Lys Asn Lys Lys Glu
   20 25
<210> 249
   <211> 27
   <212> PRT
   <213> Artificial Sequence
<220>
   <223> Scaffold Protein
<400> 249
   Gly Ala Lys Lys Ser Lys Lys Arg Phe Ser Phe Lys Lys Ser Phe Lys
   1 5 10 15
   Leu Ser Gly Phe Ser Phe Lys Lys Asn Lys Lys
   20 25
<210> 250
   <211> 26
   <212> PRT
   <213> Artificial Sequence
<220>
   <223> Scaffold Protein
<400> 250
   Gly Ala Lys Lys Ser Lys Lys Arg Phe Ser Phe Lys Lys Ser Phe Lys
   1 5 10 15
   Leu Ser Gly Phe Ser Phe Lys Lys Asn Lys
   20 25
<210> 251
   <211> 25
   <212> PRT
   <213> Artificial Sequence
<220>
   <223> Scaffold Protein
<400> 251
   Gly Ala Lys Lys Ser Lys Lys Arg Phe Ser Phe Lys Lys Ser Phe Lys
   1               5                   10                  15
   Leu Ser Gly Phe Ser Phe Lys Lys Asn
               20                  25
<210> 252
   <211> 24
   <212> PRT
   <213> Artificial Sequence
<220>
   <223> Scaffold Protein
<400> 252
   Gly Ala Lys Lys Ser Lys Lys Arg Phe Ser Phe Lys Lys Ser Phe Lys
   1               5                   10                  15
   Leu Ser Gly Phe Ser Phe Lys Lys
               20
<210> 253
   <211> 23
   <212> PRT
   <213> Artificial Sequence
<220>
   <223> Scaffold Protein
<400> 253
   Gly Ala Lys Lys Ser Lys Lys Arg Phe Ser Phe Lys Lys Ser Phe Lys
   1               5                   10                  15
   Leu Ser Gly Phe Ser Phe Lys
               20
<210> 254
   <211> 22
   <212> PRT
   <213> Artificial Sequence
<220>
   <223> Scaffold Protein
<400> 254
   Gly Ala Lys Lys Ser Lys Lys Arg Phe Ser Phe Lys Lys Ser Phe Lys
   1               5                   10                  15
   Leu Ser Gly Phe Ser Phe
               20
<210> 255
   <211> 21
   <212> PRT
   <213> Artificial Sequence
<220>
   <223> Scaffold Protein
<400> 255
   Gly Ala Lys Lys Ser Lys Lys Arg Phe Ser Phe Lys Lys Ser Phe Lys
   1               5                   10                  15
   Leu Ser Gly Phe Ser
               20
<210> 256
   <211> 20
   <212> PRT
   <213> Artificial Sequence
<220>
   <223> Scaffold Protein
<400> 256
   Gly Ala Lys Lys Ser Lys Lys Arg Phe Ser Phe Lys Lys Ser Phe Lys
   1               5                   10                  15
   Leu Ser Gly Phe
               20
<210> 257
   <211> 19
   <212> PRT
   <213> Artificial Sequence
<220>
   <223> Scaffold Protein
<400> 257
   Gly Ala Lys Lys Ser Lys Lys Arg Phe Ser Phe Lys Lys Ser Phe Lys
   1               5                   10                  15
   Leu Ser Gly
<210> 258
   <211> 18
   <212> PRT
   <213> Artificial Sequence
<220>
   <223> Scaffold Protein
<400> 258
   Gly Ala Lys Lys Ser Lys Lys Arg Phe Ser Phe Lys Lys Ser Phe Lys
   1 5 10 15
   Leu Ser
<210> 259
   <211> 17
   <212> PRT
   <213> Artificial Sequence
<220>
   <223> Scaffold Protein
<400> 259
   Gly Ala Lys Lys Ser Lys Lys Arg Phe Ser Phe Lys Lys Ser Phe Lys
   1 5 10 15
   Leu
<210> 260
   <211> 16
   <212> PRT
   <213> Artificial Sequence
<220>
   <223> Scaffold Protein
<400> 260
   Gly Ala Lys Lys Ser Lys Lys Arg Phe Ser Phe Lys Lys Ser Phe Lys
   1 5 10 15
<210> 261
   <211> 15
   <212> PRT
   <213> Artificial Sequence
<220>
   <223> Scaffold Protein
<400> 261
   Gly Ala Lys Lys Ser Lys Lys Arg Phe Ser Phe Lys Lys Ser Phe
   1 5 10 15
<210> 262
   <211> 13
   <212> PRT
   <213> Artificial Sequence
<220>
   <223> Scaffold Protein
<400> 262
   Gly Ala Lys Lys Ser Lys Lys Arg Phe Ser Phe Lys Lys
   1 5 10
<210> 263
   <211> 12
   <212> PRT
   <213> Artificial Sequence
<220>
   <223> Scaffold Protein
<400> 263
   Gly Ala Lys Lys Ser Lys Lys Arg Phe Ser Phe Lys
   1 5 10
<210> 264
   <211> 11
   <212> PRT
   <213> Artificial Sequence
<220>
   <223> Scaffold Protein
<400> 264
   Gly Ala Lys Lys Ser Lys Lys Arg Phe Ser Phe
   1 5 10
<210> 265
   <211> 10
   <212> PRT
   <213> Artificial Sequence
<220>
   <223> Scaffold Protein
<400> 265
   Gly Ala Lys Lys Ser Lys Lys Arg Phe Ser
   1 5 10
<210> 266
   <211> 9
   <212> PRT
   <213> Artificial Sequence
<220>
   <223> Scaffold Protein
<400> 266
   Gly Ala Lys Lys Ser Lys Lys Arg Phe
   1 5
<210> 267
   <211> 8
   <212> PRT
   <213> Artificial Sequence
<220>
   <223> Scaffold Protein
<400> 267
   Gly Ala Lys Lys Ser Lys Lys Arg
   1 5
<210> 268
   <211> 7
   <212> PRT
   <213> Artificial Sequence
<220>
   <223> Scaffold Protein
<400> 268
   Gly Ala Lys Lys Ser Lys Lys
   1 5
<210> 269
   <211> 29
   <212> PRT
   <213> Artificial Sequence
<220>
   <223> Scaffold Protein
<400> 269
   Gly Ala Lys Lys Ala Lys Lys Arg Phe Ser Phe Lys Lys Ser Phe Lys
   1 5 10 15
   Leu Ser Gly Phe Ser Phe Lys Lys Asn Lys Lys Glu Ala
   20 25
<210> 270
   <211> 28
   <212> PRT
   <213> Artificial Sequence
<220>
   <223> Scaffold Protein
<400> 270
   Gly Ala Lys Lys Ala Lys Lys Arg Phe Ser Phe Lys Lys Ser Phe Lys
   1 5 10 15
   Leu Ser Gly Phe Ser Phe Lys Lys Asn Lys Lys Glu
   20 25
<210> 271
   <211> 27
   <212> PRT
   <213> Artificial Sequence
<220>
   <223> Scaffold Protein
<400> 271
   Gly Ala Lys Lys Ala Lys Lys Arg Phe Ser Phe Lys Lys Ser Phe Lys
   1               5                   10                  15
   Leu Ser Gly Phe Ser Phe Lys Lys Asn Lys Lys
               20                  25
<210> 272
   <211> 26
   <212> PRT
   <213> Artificial Sequence
<220>
   <223> Scaffold Protein
<400> 272
   Gly Ala Lys Lys Ala Lys Lys Arg Phe Ser Phe Lys Lys Ser Phe Lys
   1               5                   10                  15
   Leu Ser Gly Phe Ser Phe Lys Lys Asn Lys
               20                  25
<210> 273
   <211> 25
   <212> PRT
   <213> Artificial Sequence
<220>
   <223> Scaffold Protein
<400> 273
   Gly Ala Lys Lys Ala Lys Lys Arg Phe Ser Phe Lys Lys Ser Phe Lys
   1               5                   10                  15
   Leu Ser Gly Phe Ser Phe Lys Lys Asn
               20                  25
<210> 274
   <211> 24
   <212> PRT
   <213> Artificial Sequence
<220>
   <223> Scaffold Protein
<400> 274
   Gly Ala Lys Lys Ala Lys Lys Arg Phe Ser Phe Lys Lys Ser Phe Lys
   1 5 10 15
   Leu Ser Gly Phe Ser Phe Lys Lys
   20
<210> 275
   <211> 23
   <212> PRT
   <213> Artificial Sequence
<220>
   <223> Scaffold Protein
<400> 275
   Gly Ala Lys Lys Ala Lys Lys Arg Phe Ser Phe Lys Lys Ser Phe Lys
   1 5 10 15
   Leu Ser Gly Phe Ser Phe Lys
   20
<210> 276
   <211> 22
   <212> PRT
   <213> Artificial Sequence
<220>
   <223> Scaffold Protein
<400> 276
   Gly Ala Lys Lys Ala Lys Lys Arg Phe Ser Phe Lys Lys Ser Phe Lys
   1 5 10 15
   Leu Ser Gly Phe Ser Phe
   20
<210> 277
   <211> 21
   <212> PRT
   <213> Artificial Sequence
<220>
   <223> Scaffold Protein
<400> 277
   Gly Ala Lys Lys Ala Lys Lys Arg Phe Ser Phe Lys Lys Ser Phe Lys
   1 5 10 15
   Leu Ser Gly Phe Ser
   20
<210> 278
   <211> 20
   <212> PRT
   <213> Artificial Sequence
<220>
   <223> Scaffold Protein
<400> 278
   Gly Ala Lys Lys Ala Lys Lys Arg Phe Ser Phe Lys Lys Ser Phe Lys
   1               5                   10                  15
   Leu Ser Gly Phe
               20
<210> 279
   <211> 19
   <212> PRT
   <213> Artificial Sequence
<220>
   <223> Scaffold Protein
<400> 279
   Gly Ala Lys Lys Ala Lys Lys Arg Phe Ser Phe Lys Lys Ser Phe Lys
   1               5                   10                  15
   Leu Ser Gly
<210> 280
   <211> 18
   <212> PRT
   <213> Artificial Sequence
<220>
   <223> Scaffold Protein
<400> 280
   Gly Ala Lys Lys Ala Lys Lys Arg Phe Ser Phe Lys Lys Ser Phe Lys
   1               5                   10                  15
   Leu Ser
<210> 281
   <211> 17
   <212> PRT
   <213> Artificial Sequence
<220>
   <223> Scaffold Protein
<400> 281
   Gly Ala Lys Lys Ala Lys Lys Arg Phe Ser Phe Lys Lys Ser Phe Lys
   1               5                   10                  15
   Leu
<210> 282
   <211> 16
   <212> PRT
   <213> Artificial Sequence
<220>
   <223> Scaffold Protein
<400> 282
   Gly Ala Lys Lys Ala Lys Lys Arg Phe Ser Phe Lys Lys Ser Phe Lys
   1               5                   10                  15
<210> 283
   <211> 15
   <212> PRT
   <213> Artificial Sequence
<220>
   <223> Scaffold Protein
<400> 283
   Gly Ala Lys Lys Ala Lys Lys Arg Phe Ser Phe Lys Lys Ser Phe
   1               5                   10                  15
<210> 284
   <211> 14
   <212> PRT
   <213> Artificial Sequence
<220>
   <223> Scaffold Protein
<400> 284
   Gly Ala Lys Lys Ala Lys Lys Arg Phe Ser Phe Lys Lys Ser
   1               5                   10
<210> 285
   <211> 13
   <212> PRT
   <213> Artificial Sequence
<220>
   <223> Scaffold Protein
<400> 285
   Gly Ala Lys Lys Ala Lys Lys Arg Phe Ser Phe Lys Lys
   1               5                   10
<210> 286
   <211> 12
   <212> PRT
   <213> Artificial Sequence
<220>
   <223> Scaffold Protein
<400> 286
   Gly Ala Lys Lys Ala Lys Lys Arg Phe Ser Phe Lys
   1 5 10
<210> 287
   <211> 11
   <212> PRT
   <213> Artificial Sequence
<220>
   <223> Scaffold Protein
<400> 287
   Gly Ala Lys Lys Ala Lys Lys Arg Phe Ser Phe
   1 5 10
<210> 288
   <211> 10
   <212> PRT
   <213> Artificial Sequence
<220>
   <223> Scaffold Protein
<400> 288
   Gly Ala Lys Lys Ala Lys Lys Arg Phe Ser
   1 5 10
<210> 289
   <211> 9
   <212> PRT
   <213> Artificial Sequence
<220>
   <223> Scaffold Protein
<400> 289
   Gly Ala Lys Lys Ala Lys Lys Arg Phe
   1 5
<210> 290
   <211> 8
   <212> PRT
   <213> Artificial Sequence
<220>
   <223> Scaffold Protein
<400> 290
   Gly Ala Lys Lys Ala Lys Lys Arg
   1 5
<210> 291
   <211> 7
   <212> PRT
   <213> Artificial Sequence
<220>
   <223> Scaffold Protein
<400> 291
   Gly Ala Lys Lys Ala Lys Lys
   1 5
<210> 292
   <211> 28
   <212> PRT
   <213> Artificial Sequence
<220>
   <223> Scaffold Protein
<400> 292
   Gly Ala Gln Glu Ser Lys Lys Lys Lys Lys Lys Arg Phe Ser Phe Lys
   1 5 10 15
   Lys Ser Phe Lys Leu Ser Gly Phe Ser Phe Lys Lys
   20 25
<210> 293
   <211> 27
   <212> PRT
   <213> Artificial Sequence
<220>
   <223> Scaffold Protein
<400> 293
   Gly Ala Gln Glu Ser Lys Lys Lys Lys Lys Lys Arg Phe Ser Phe Lys
   1 5 10 15
   Lys Ser Phe Lys Leu Ser Gly Phe Ser Phe Lys
   20 25
<210> 294
   <211> 26
   <212> PRT
   <213> Artificial Sequence
<220>
   <223> Scaffold Protein
<400> 294
   Gly Ala Gln Glu Ser Lys Lys Lys Lys Lys Lys Arg Phe Ser Phe Lys
   1               5                   10                  15
   Lys Ser Phe Lys Leu Ser Gly Phe Ser Phe
               20                  25
<210> 295
   <211> 25
   <212> PRT
   <213> Artificial Sequence
<220>
   <223> Scaffold Protein
<400> 295
   Gly Ala Gln Glu Ser Lys Lys Lys Lys Lys Lys Arg Phe Ser Phe Lys
   1               5                   10                  15
   Lys Ser Phe Lys Leu Ser Gly Phe Ser
               20                  25
<210> 296
   <211> 24
   <212> PRT
   <213> Artificial Sequence
<220>
   <223> Scaffold Protein
<400> 296
   Gly Ala Gln Glu Ser Lys Lys Lys Lys Lys Lys Arg Phe Ser Phe Lys
   1               5                   10                  15
   Lys Ser Phe Lys Leu Ser Gly Phe
               20
<210> 297
   <211> 23
   <212> PRT
   <213> Artificial Sequence
<220>
   <223> Scaffold Protein
<400> 297
   Gly Ala Gln Glu Ser Lys Lys Lys Lys Lys Lys Arg Phe Ser Phe Lys
   1               5                   10                  15
   Lys Ser Phe Lys Leu Ser Gly
               20
<210> 298
   <211> 22
   <212> PRT
   <213> Artificial Sequence
<220>
   <223> Scaffold Protein
<400> 298
   Gly Ala Gln Glu Ser Lys Lys Lys Lys Lys Lys Arg Phe Ser Phe Lys
   1               5                   10                  15
   Lys Ser Phe Lys Leu Ser
               20
<210> 299
   <211> 21
   <212> PRT
   <213> Artificial Sequence
<220>
   <223> Scaffold Protein
<400> 299
   Gly Ala Gln Glu Ser Lys Lys Lys Lys Lys Lys Arg Phe Ser Phe Lys
   1               5                   10                  15
   Lys Ser Phe Lys Leu
               20
<210> 300
   <211> 20
   <212> PRT
   <213> Artificial Sequence
<220>
   <223> Scaffold Protein
<400> 300
   Gly Ala Gln Glu Ser Lys Lys Lys Lys Lys Lys Arg Phe Ser Phe Lys
   1 5 10 15
   Lys Ser Phe Lys
   20
<210> 301
   <211> 19
   <212> PRT
   <213> Artificial Sequence
<220>
   <223> Scaffold Protein
<400> 301
   Gly Ala Gln Glu Ser Lys Lys Lys Lys Lys Lys Arg Phe Ser Phe Lys
   1 5 10 15
   Lys Ser Phe
<210> 302
   <211> 18
   <212> PRT
   <213> Artificial Sequence
<220>
   <223> Scaffold Protein
<400> 302
   Gly Ala Gln Glu Ser Lys Lys Lys Lys Lys Lys Arg Phe Ser Phe Lys
   1 5 10 15
   Lys Ser
<210> 303
   <211> 17
   <212> PRT
   <213> Artificial Sequence
<220>
   <223> Scaffold Protein
<400> 303
   Gly Ala Gln Glu Ser Lys Lys Lys Lys Lys Lys Arg Phe Ser Phe Lys
   1 5 10 15
   Lys
<210> 304
   <211> 16
   <212> PRT
   <213> Artificial Sequence
<220>
   <223> Scaffold Protein
<400> 304
   Gly Ala Gln Glu Ser Lys Lys Lys Lys Lys Lys Arg Phe Ser Phe Lys
   1 5 10 15
<210> 305
   <211> 15
   <212> PRT
   <213> Artificial Sequence
<220>
   <223> Scaffold Protein
<400> 305
   Gly Ala Gln Glu Ser Lys Lys Lys Lys Lys Lys Arg Phe Ser Phe
   1 5 10 15
<210> 306
   <211> 14
   <212> PRT
   <213> Artificial Sequence
<220>
   <223> Scaffold Protein
<400> 306
   Gly Ala Gln Glu Ser Lys Lys Lys Lys Lys Lys Arg Phe Ser
   1 5 10
<210> 307
   <211> 13
   <212> PRT
   <213> Artificial Sequence
<220>
   <223> Scaffold Protein
<400> 307
   Gly Ala Gln Glu Ser Lys Lys Lys Lys Lys Lys Arg Phe
   1 5 10
<210> 308
   <211> 12
   <212> PRT
   <213> Artificial Sequence
<220>
   <223> Scaffold Protein
<400> 308
   Gly Ala Gln Glu Ser Lys Lys Lys Lys Lys Lys Arg
   1 5 10
<210> 309
   <211> 11
   <212> PRT
   <213> Artificial Sequence
<220>
   <223> Scaffold Protein
<400> 309
   Gly Ala Gln Glu Ser Lys Lys Lys Lys Lys Lys
   1 5 10
<210> 310
   <211> 10
   <212> PRT
   <213> Artificial Sequence
<220>
   <223> Scaffold Protein
<400> 310
   Gly Ala Gln Glu Ser Lys Lys Lys Lys Lys
   1 5 10
<210> 311
   <211> 9
   <212> PRT
   <213> Artificial Sequence
<220>
   <223> Scaffold Protein
<400> 311
   Gly Ala Gln Glu Ser Lys Lys Lys Lys
   1 5
<210> 312
   <211> 8
   <212> PRT
   <213> Artificial Sequence
<220>
   <223> Scaffold Protein
<400> 312
   Gly Ala Gln Glu Ser Lys Lys Lys
   1 5
<210> 313
   <211> 7
   <212> PRT
   <213> Artificial Sequence
<220>
   <223> Scaffold Protein
<400> 313
   Gly Ala Gln Glu Ser Lys Lys
   1               5
<210> 314
   <211> 30
   <212> PRT
   <213> Artificial Sequence
<220>
   <223> Scaffold Protein
<400> 314
   Gly Ser Gln Ser Ser Lys Lys Lys Lys Lys Lys Phe Ser Phe Lys Lys
   1               5                   10                  15
   Pro Phe Lys Leu Ser Gly Leu Ser Phe Lys Arg Asn Arg Lys
               20                  25                  30
<210> 315
   <211> 29
   <212> PRT
   <213> Artificial Sequence
<220>
   <223> Scaffold Protein
<400> 315
   Gly Ser Gln Ser Ser Lys Lys Lys Lys Lys Lys Phe Ser Phe Lys Lys
   1               5                   10                  15
   Pro Phe Lys Leu Ser Gly Leu Ser Phe Lys Arg Asn Arg
               20                  25
<210> 316
   <211> 28
   <212> PRT
   <213> Artificial Sequence
<220>
   <223> Scaffold Protein
<400> 316
   Gly Ser Gln Ser Ser Lys Lys Lys Lys Lys Lys Phe Ser Phe Lys Lys
   1 5 10 15
   Pro Phe Lys Leu Ser Gly Leu Ser Phe Lys Arg Asn
   20 25
<210> 317
   <211> 27
   <212> PRT
   <213> Artificial Sequence
<220>
   <223> Scaffold Protein
<400> 317
   Gly Ser Gln Ser Ser Lys Lys Lys Lys Lys Lys Phe Ser Phe Lys Lys
   1 5 10 15
   Pro Phe Lys Leu Ser Gly Leu Ser Phe Lys Arg
   20 25
<210> 318
   <211> 26
   <212> PRT
   <213> Artificial Sequence
<220>
   <223> Scaffold Protein
<400> 318
   Gly Ser Gln Ser Ser Lys Lys Lys Lys Lys Lys Phe Ser Phe Lys Lys
   1 5 10 15
   Pro Phe Lys Leu Ser Gly Leu Ser Phe Lys
   20 25
<210> 319
   <211> 25
   <212> PRT
   <213> Artificial Sequence
<220>
   <223> Scaffold Protein
<400> 319
   Gly Ser Gln Ser Ser Lys Lys Lys Lys Lys Lys Phe Ser Phe Lys Lys
   1 5 10 15
   Pro Phe Lys Leu Ser Gly Leu Ser Phe
   20 25
<210> 320
   <211> 24
   <212> PRT
   <213> Artificial Sequence
<220>
   <223> Scaffold Protein
<400> 320
   Gly Ser Gln Ser Ser Lys Lys Lys Lys Lys Lys Phe Ser Phe Lys Lys
   1               5                   10                  15
   Pro Phe Lys Leu Ser Gly Leu Ser
               20
<210> 321
   <211> 23
   <212> PRT
   <213> Artificial Sequence
<220>
   <223> Scaffold Protein
<400> 321
   Gly Ser Gln Ser Ser Lys Lys Lys Lys Lys Lys Phe Ser Phe Lys Lys
   1               5                   10                  15
   Pro Phe Lys Leu Ser Gly Leu
               20
<210> 322
   <211> 22
   <212> PRT
   <213> Artificial Sequence
<220>
   <223> Scaffold Protein
<400> 322
   Gly Ser Gln Ser Ser Lys Lys Lys Lys Lys Lys Phe Ser Phe Lys Lys
   1               5                   10                  15
   Pro Phe Lys Leu Ser Gly
               20
<210> 323
   <211> 21
   <212> PRT
   <213> Artificial Sequence
<220>
   <223> Scaffold Protein
<400> 323
   Gly Ser Gln Ser Ser Lys Lys Lys Lys Lys Lys Phe Ser Phe Lys Lys
   1               5                   10                  15
   Pro Phe Lys Leu Ser
               20
<210> 324
   <211> 20
   <212> PRT
   <213> Artificial Sequence
<220>
   <223> Scaffold Protein
<400> 324
   Gly Ser Gln Ser Ser Lys Lys Lys Lys Lys Lys Phe Ser Phe Lys Lys
   1               5                   10                  15
   Pro Phe Lys Leu
               20
<210> 325
   <211> 19
   <212> PRT
   <213> Artificial Sequence
<220>
   <223> Scaffold Protein
<400> 325
   Gly Ser Gln Ser Ser Lys Lys Lys Lys Lys Lys Phe Ser Phe Lys Lys
   1               5                   10                  15
   Pro Phe Lys
<210> 326
   <211> 18
   <212> PRT
   <213> Artificial Sequence
<220>
   <223> Scaffold Protein
<400> 326
   Gly Ser Gln Ser Ser Lys Lys Lys Lys Lys Lys Phe Ser Phe Lys Lys
   1               5                   10                  15
   Pro Phe
<210> 327
   <211> 17
   <212> PRT
   <213> Artificial Sequence
<220>
   <223> Scaffold Protein
<400> 327
   Gly Ser Gln Ser Ser Lys Lys Lys Lys Lys Lys Phe Ser Phe Lys Lys
   1               5                   10                  15
   Pro
<210> 328
   <211> 16
   <212> PRT
   <213> Artificial Sequence
<220>
   <223> Scaffold Protein
<400> 328
   Gly Ser Gln Ser Ser Lys Lys Lys Lys Lys Lys Phe Ser Phe Lys Lys
   1               5                   10                  15
<210> 329
   <211> 15
   <212> PRT
   <213> Artificial Sequence
<220>
   <223> Scaffold Protein
<400> 329
   Gly Ser Gln Ser Ser Lys Lys Lys Lys Lys Lys Phe Ser Phe Lys
   1               5                   10                  15
<210> 330
   <211> 14
   <212> PRT
   <213> Artificial Sequence
<220>
   <223> Scaffold Protein
<400> 330
   Gly Ser Gln Ser Ser Lys Lys Lys Lys Lys Lys Phe Ser Phe
   1               5                   10
<210> 331
   <211> 13
   <212> PRT
   <213> Artificial Sequence
<220>
   <223> Scaffold Protein
<400> 331
   Gly Ser Gln Ser Ser Lys Lys Lys Lys Lys Lys Phe Ser
   1 5 10
<210> 332
   <211> 12
   <212> PRT
   <213> Artificial Sequence
<220>
   <223> Scaffold Protein
<400> 332
   Gly Ser Gln Ser Ser Lys Lys Lys Lys Lys Lys Phe
   1 5 10
<210> 333
   <211> 11
   <212> PRT
   <213> Artificial Sequence
<220>
   <223> Scaffold Protein
<400> 333
   Gly Ser Gln Ser Ser Lys Lys Lys Lys Lys Lys
   1 5 10
<210> 334
   <211> 10
   <212> PRT
   <213> Artificial Sequence
<220>
   <223> Scaffold Protein
<400> 334
   Gly Ser Gln Ser Ser Lys Lys Lys Lys Lys
   1 5 10
<210> 335
   <211> 9
   <212> PRT
   <213> Artificial Sequence
<220>
   <223> Scaffold Protein
<400> 335
   Gly Ser Gln Ser Ser Lys Lys Lys Lys
   1 5
<210> 336
   <211> 8
   <212> PRT
   <213> Artificial Sequence
<220>
   <223> Scaffold Protein
<400> 336
   Gly Ser Gln Ser Ser Lys Lys Lys
   1 5
<210> 337
   <211> 7
   <212> PRT
   <213> Artificial Sequence
<220>
   <223> Scaffold Protein
<400> 337
   Gly Ser Gln Ser Ser Lys Lys
   1 5
<210> 338
   <211> 7
   <212> PRT
   <213> Artificial Sequence
<220>
   <223> fusion protein
<400> 338
   Gly Gly Lys Phe Ser Lys Lys
   1 5
<210> 339
   <211> 7
   <212> PRT
   <213> Artificial Sequence
<220>
   <223> fusion protein
<400> 339
   Gly Gly Lys Phe Ala Lys Lys
   1 5
<210> 340
   <211> 7
   <212> PRT
   <213> Artificial Sequence
<220>
   <223> fusion protein
<400> 340
   Gly Gly Lys Ser Ser Lys Lys
   1 5
<210> 341
   <211> 7
   <212> PRT
   <213> Artificial Sequence
<220>
   <223> fusion protein
<400> 341
   Gly Gly Lys Ser Ala Lys Lys
   1 5
<210> 342
   <211> 7
   <212> PRT
   <213> Artificial Sequence
<220>
   <223> fusion protein
<400> 342
   Gly Gly Lys Gln Ala Lys Lys
   1 5
<210> 343
   <211> 7
   <212> PRT
   <213> Artificial Sequence
<220>
   <223> fusion protein
<400> 343
   Gly Gly Gln Leu Ser Lys Lys
   1 5
<210> 344
   <211> 7
   <212> PRT
   <213> Artificial Sequence
<220>
   <223> fusion protein
<400> 344
   Gly Gly Gln Leu Ala Lys Lys
   1 5
<210> 345
   <211> 7
   <212> PRT
   <213> Artificial Sequence
<220>
   <223> fusion protein
<400> 345
   Gly Gly Gln Phe Ser Lys Lys
   1 5
<210> 346
   <211> 7
   <212> PRT
   <213> Artificial Sequence
<220>
   <223> fusion protein
<400> 346
   Gly Gly Gln Phe Ala Lys Lys
   1 5
<210> 347
   <211> 7
   <212> PRT
   <213> Artificial Sequence
<220>
   <223> fusion protein
<400> 347
   Gly Gly Gln Ser Ser Lys Lys
   1 5
<210> 348
   <211> 7
   <212> PRT
   <213> Artificial Sequence
<220>
   <223> fusion protein
<400> 348
   Gly Gly Gln Ser Ala Lys Lys
   1 5
<210> 349
   <211> 7
   <212> PRT
   <213> Artificial Sequence
<220>
   <223> fusion protein
<400> 349
   Gly Gly Gln Gln Ser Lys Lys
   1               5
<210> 350
   <211> 7
   <212> PRT
   <213> Artificial Sequence
<220>
   <223> fusion protein
<400> 350
   Gly Gly Gln Gln Ala Lys Lys
   1               5
<210> 351
   <211> 7
   <212> PRT
   <213> Artificial Sequence
<220>
   <223> fusion protein
<400> 351
   Gly Ala Lys Leu Ala Lys Lys
   1               5
<210> 352
   <211> 7
   <212> PRT
   <213> Artificial Sequence
<220>
   <223> fusion protein
<400> 352
   Gly Ala Lys Phe Ser Lys Lys
   1               5
<210> 353
   <211> 7
   <212> PRT
   <213> Artificial Sequence
<220>
   <223> fusion protein
<400> 353
   Gly Ala Lys Phe Ala Lys Lys
   1 5
<210> 354
   <211> 7
   <212> PRT
   <213> Artificial Sequence
<220>
   <223> fusion protein
<400> 354
   Gly Ala Lys Ser Ser Lys Lys
   1 5
<210> 355
   <211> 7
   <212> PRT
   <213> Artificial Sequence
<220>
   <223> fusion protein
<400> 355
   Gly Ala Lys Ser Ala Lys Lys
   1 5
<210> 356
   <211> 7
   <212> PRT
   <213> Artificial Sequence
<220>
   <223> fusion protein
<400> 356
   Gly Ala Lys Gln Ser Lys Lys
   1 5
<210> 357
   <211> 7
   <212> PRT
   <213> Artificial Sequence
<220>
   <223> fusion protein
<400> 357
   Gly Ala Lys Gln Ala Lys Lys
   1 5
<210> 358
   <211> 7
   <212> PRT
   <213> Artificial Sequence
<220>
   <223> fusion protein
<400> 358
   Gly Ala Gln Leu Ser Lys Lys
   1 5
<210> 359
   <211> 7
   <212> PRT
   <213> Artificial Sequence
<220>
   <223> fusion protein
<400> 359
   Gly Ala Gln Leu Ala Lys Lys
   1 5
<210> 360
   <211> 7
   <212> PRT
   <213> Artificial Sequence
<220>
   <223> fusion protein
<400> 360
   Gly Ala Gln Phe Ser Lys Lys
   1 5
<210> 361
   <211> 7
   <212> PRT
   <213> Artificial Sequence
<220>
   <223> fusion protein
<400> 361
   Gly Ala Gln Phe Ala Lys Lys
   1 5
<210> 362
   <211> 7
   <212> PRT
   <213> Artificial Sequence
<220>
   <223> fusion protein
<400> 362
   Gly Ala Gln Ser Ser Lys Lys
   1 5
<210> 363
   <211> 7
   <212> PRT
   <213> Artificial Sequence
<220>
   <223> fusion protein
<400> 363
   Gly Ala Gln Ser Ala Lys Lys
   1 5
<210> 364
   <211> 7
   <212> PRT
   <213> Artificial Sequence
<220>
   <223> fusion protein
<400> 364
   Gly Ala Gln Gln Ser Lys Lys
   1 5
<210> 365
   <211> 7
   <212> PRT
   <213> Artificial Sequence
<220>
   <223> fusion protein
<400> 365
   Gly Ala Gln Gln Ala Lys Lys
   1 5
<210> 366
   <211> 7
   <212> PRT
   <213> Artificial Sequence
<220>
   <223> fusion protein
<400> 366
   Gly Ser Lys Leu Ser Lys Lys
   1 5
<210> 367
   <211> 7
   <212> PRT
   <213> Artificial Sequence
<220>
   <223> fusion protein
<400> 367
   Gly Ser Lys Leu Ala Lys Lys
   1 5
<210> 368
   <211> 7
   <212> PRT
   <213> Artificial Sequence
<220>
   <223> fusion protein
<400> 368
   Gly Ser Lys Phe Ser Lys Lys
   1 5
<210> 369
   <211> 7
   <212> PRT
   <213> Artificial Sequence
<220>
   <223> fusion protein
<400> 369
   Gly Ser Lys Phe Ala Lys Lys
   1 5
<210> 370
   <211> 7
   <212> PRT
   <213> Artificial Sequence
<220>
   <223> fusion protein
<400> 370
   Gly Ser Lys Ser Ser Lys Lys
   1 5
<210> 371
   <211> 7
   <212> PRT
   <213> Artificial Sequence
<220>
   <223> fusion protein
<400> 371
   Gly Ser Lys Ser Ala Lys Lys
   1 5
<210> 372
   <211> 7
   <212> PRT
   <213> Artificial Sequence
<220>
   <223> fusion protein
<400> 372
   Gly Ser Lys Gln Ser Lys Lys
   1 5
<210> 373
   <211> 7
   <212> PRT
   <213> Artificial Sequence
<220>
   <223> fusion protein
<400> 373
   Gly Ser Lys Gln Ala Lys Lys
   1 5
<210> 374
   <211> 7
   <212> PRT
   <213> Artificial Sequence
<220>
   <223> fusion protein
<400> 374
   Gly Ser Gln Leu Ser Lys Lys
   1 5
<210> 375
   <211> 7
   <212> PRT
   <213> Artificial Sequence
<220>
   <223> fusion protein
<400> 375
   Gly Ser Gln Leu Ala Lys Lys
   1 5
<210> 376
   <211> 7
   <212> PRT
   <213> Artificial Sequence
<220>
   <223> fusion protein
<400> 376
   Gly Ser Gln Phe Ser Lys Lys
   1 5
<210> 377
   <211> 7
   <212> PRT
   <213> Artificial Sequence
<220>
   <223> fusion protein
<400> 377
   Gly Ser Gln Phe Ala Lys Lys
   1 5
<210> 378
   <211> 7
   <212> PRT
   <213> Artificial Sequence
<220>
   <223> fusion protein
<400> 378
   Gly Ser Gln Ser Ala Lys Lys
   1 5
<210> 379
   <211> 7
   <212> PRT
   <213> Artificial Sequence
<220>
   <223> fusion protein
<400> 379
   Gly Ser Gln Gln Ser Lys Lys
   1 5
<210> 380
   <211> 7
   <212> PRT
   <213> Artificial Sequence
<220>
   <223> fusion protein
<400> 380
   Gly Ser Gln Gln Ala Lys Lys
   1 5
<210> 381
   <211> 6
   <212> PRT
   <213> Artificial Sequence
<220>
   <223> fusion protein
<400> 381
   Gly Gly Lys Phe Ser Lys
   1 5
<210> 382
   <211> 6
   <212> PRT
   <213> Artificial Sequence
<220>
   <223> fusion protein
<400> 382
   Gly Gly Lys Phe Ala Lys
   1 5
<210> 383
   <211> 6
   <212> PRT
   <213> Artificial Sequence
<220>
   <223> fusion protein
<400> 383
   Gly Gly Lys Ser Ser Lys
   1 5
<210> 384
   <211> 6
   <212> PRT
   <213> Artificial Sequence
<220>
   <223> fusion protein
<400> 384
   Gly Gly Lys Ser Ala Lys
   1 5
<210> 385
   <211> 6
   <212> PRT
   <213> Artificial Sequence
<220>
   <223> fusion protein
<400> 385
   Gly Gly Lys Gln Ala Lys
   1 5
<210> 386
   <211> 6
   <212> PRT
   <213> Artificial Sequence
<220>
   <223> fusion protein
<400> 386
   Gly Gly Gln Leu Ser Lys
   1 5
<210> 387
   <211> 6
   <212> PRT
   <213> Artificial Sequence
<220>
   <223> fusion protein
<400> 387
   Gly Gly Gln Leu Ala Lys
   1 5
<210> 388
   <211> 6
   <212> PRT
   <213> Artificial Sequence
<220>
   <223> fusion protein
<400> 388
   Gly Gly Gln Phe Ser Lys
   1 5
<210> 389
   <211> 6
   <212> PRT
   <213> Artificial Sequence
<220>
   <223> fusion protein
<400> 389
   Gly Gly Gln Phe Ala Lys
   1 5
<210> 390
   <211> 6
   <212> PRT
   <213> Artificial Sequence
<220>
   <223> fusion protein
<400> 390
   Gly Gly Gln Ser Ser Lys
   1 5
<210> 391
   <211> 6
   <212> PRT
   <213> Artificial Sequence
<220>
   <223> fusion protein
<400> 391
   Gly Gly Gln Ser Ala Lys
   1 5
<210> 392
   <211> 6
   <212> PRT
   <213> Artificial Sequence
<220>
   <223> fusion protein
<400> 392
   Gly Gly Gln Gln Ser Lys
   1 5
<210> 393
   <211> 6
   <212> PRT
   <213> Artificial Sequence
<220>
   <223> fusion protein
<400> 393
   Gly Gly Gln Gln Ala Lys
   1 5
<210> 394
   <211> 6
   <212> PRT
   <213> Artificial Sequence
<220>
   <223> fusion protein
<400> 394
   Gly Ala Lys Leu Ala Lys
   1 5
<210> 395
   <211> 6
   <212> PRT
   <213> Artificial Sequence
<220>
   <223> fusion protein
<400> 395
   Gly Ala Lys Phe Ser Lys
   1 5
<210> 396
   <211> 6
   <212> PRT
   <213> Artificial Sequence
<220>
   <223> fusion protein
<400> 396
   Gly Ala Lys Phe Ala Lys
   1 5
<210> 397
   <211> 6
   <212> PRT
   <213> Artificial Sequence
<220>
   <223> fusion protein
<400> 397
   Gly Ala Lys Ser Ser Lys
   1 5
<210> 398
   <211> 6
   <212> PRT
   <213> Artificial Sequence
<220>
   <223> fusion protein
<400> 398
   Gly Ala Lys Ser Ala Lys
   1 5
<210> 399
   <211> 6
   <212> PRT
   <213> Artificial Sequence
<220>
   <223> fusion protein
<400> 399
   Gly Ala Lys Gln Ser Lys
   1 5
<210> 400
   <211> 6
   <212> PRT
   <213> Artificial Sequence
<220>
   <223> fusion protein
<400> 400
   Gly Ala Lys Gln Ala Lys
   1 5
<210> 401
   <211> 6
   <212> PRT
   <213> Artificial Sequence
<220>
   <223> fusion protein
<400> 401
   Gly Ala Gln Leu Ser Lys
   1 5
<210> 402
   <211> 6
   <212> PRT
   <213> Artificial Sequence
<220>
   <223> fusion protein
<400> 402
   Gly Ala Gln Leu Ala Lys
   1 5
<210> 403
   <211> 6
   <212> PRT
   <213> Artificial Sequence
<220>
   <223> fusion protein
<400> 403
   Gly Ala Gln Phe Ser Lys
   1 5
<210> 404
   <211> 6
   <212> PRT
   <213> Artificial Sequence
<220>
   <223> fusion protein
<400> 404
   Gly Ala Gln Phe Ala Lys
   1 5
<210> 405
   <211> 6
   <212> PRT
   <213> Artificial Sequence
<220>
   <223> fusion protein
<400> 405
   Gly Ala Gln Ser Ser Lys
   1 5
<210> 406
   <211> 6
   <212> PRT
   <213> Artificial Sequence
<220>
   <223> fusion protein
<400> 406
   Gly Ala Gln Ser Ala Lys
   1 5
<210> 407
   <211> 6
   <212> PRT
   <213> Artificial Sequence
<220>
   <223> fusion protein
<400> 407
   Gly Ala Gln Gln Ser Lys
   1 5
<210> 408
   <211> 6
   <212> PRT
   <213> Artificial Sequence
<220>
   <223> fusion protein
<400> 408
   Gly Ala Gln Gln Ala Lys
   1 5
<210> 409
   <211> 6
   <212> PRT
   <213> Artificial Sequence
<220>
   <223> fusion protein
<400> 409
   Gly Ser Lys Leu Ser Lys
   1 5
<210> 410
   <211> 6
   <212> PRT
   <213> Artificial Sequence
<220>
   <223> fusion protein
<400> 410
   Gly Ser Lys Leu Ala Lys
   1 5
<210> 411
   <211> 6
   <212> PRT
   <213> Artificial Sequence
<220>
   <223> fusion protein
<400> 411
   Gly Ser Lys Phe Ser Lys
   1 5
<210> 412
   <211> 6
   <212> PRT
   <213> Artificial Sequence
<220>
   <223> fusion protein
<400> 412
   Gly Ser Lys Phe Ala Lys
   1 5
<210> 413
   <211> 6
   <212> PRT
   <213> Artificial Sequence
<220>
   <223> fusion protein
<400> 413
   Gly Ser Lys Ser Ser Lys
   1 5
<210> 414
   <211> 6
   <212> PRT
   <213> Artificial Sequence
<220>
   <223> fusion protein
<400> 414
   Gly Ser Lys Ser Ala Lys
   1 5
<210> 415
   <211> 6
   <212> PRT
   <213> Artificial Sequence
<220>
   <223> fusion protein
<400> 415
   Gly Ser Lys Gln Ser Lys
   1 5
<210> 416
   <211> 6
   <212> **PRT**
   <213> **Artificial** Sequence
<220>
   <223> fusion protein
<400> 416
   Gly Ser Lys Gln Ala Lys
   1 5
<210> 417
   <211> 6
   <212> **PRT**
   <213> **Artificial** Sequence
<220>
   <223> fusion protein
<400> 417
   Gly Ser Gln Leu Ser Lys
   1 5
<210> 418
   <211> 6
   <212> PRT
   <213> Artificial Sequence
<220>
   <223> fusion protein
<400> 418
   Gly Ser Gln Leu Ala Lys
   1 5
<210> 419
   <211> 6
   <212> PRT
   <213> Artificial Sequence
<220>
   <223> fusion protein
<400> 419
   Gly Ser Gln Phe Ser Lys
   1 5
<210> 420
   <211> 6
   <212> PRT
   <213> Artificial Sequence
<220>
   <223> fusion protein
<400> 420
   Gly Ser Gln Phe Ala Lys
   1 5
<210> 421
   <211> 6
   <212> PRT
   <213> Artificial Sequence
<220>
   <223> fusion protein
<400> 421
   Gly Ser Gln Ser Ser Lys
   1 5
<210> 422
   <211> 6
   <212> PRT
   <213> Artificial Sequence
<220>
   <223> fusion protein
<400> 422
   Gly Ser Gln Ser Ala Lys
   1 5
<210> 423
   <211> 6
   <212> PRT
   <213> Artificial Sequence
<220>
   <223> fusion protein
<400> 423
   Gly Ser Gln Gln Ser Lys
   1 5
<210> 424
   <211> 6
   <212> PRT
   <213> Artificial Sequence
<220>
   <223> fusion protein
<400> 424
   Gly Ser Gln Gln Ala Lys
   1 5
<210> 425
   <211> 8
   <212> PRT
   <213> Artificial Sequence
<220>
   <223> Fusion Protein
<220>
   <221> misc_feature
   <222> (2)..(2)
   <223> Xaa can be any naturally occurring amino acid
<400> 425
   Gly Xaa Lys Leu Ser Lys Lys Lys
   1               5